(19) Europäisches Patentamt
European Patent Office
Office européen des brevets

(11) Publication number: **0 365 324 B1**

(12) **EUROPEAN PATENT SPECIFICATION**

(45) Date of publication of patent specification: **04.05.94**

(51) Int. Cl.5: **C07D 471/10**, C07D 498/10, C07D 513/10, A61K 31/47, A61K 31/54, A61K 31/55

(21) Application number: **89310762.3**

(22) Date of filing: **19.10.89**

# (54) Spiro-isoquinoline-pyrrolidine tetrones and analogs thereof useful as aldose reductase inhibitors.

(30) Priority: **20.10.88 US 260434**
**26.05.89 US 357563**
**31.08.89 GB 8919736**

(43) Date of publication of application:
**25.04.90 Bulletin 90/17**

(45) Publication of the grant of the patent:
**04.05.94 Bulletin 94/18**

(84) Designated Contracting States:
**AT BE CH DE ES FR GR IT LI LU NL SE**

(56) References cited:
**EP-A- 0 079 675**
**EP-A- 0 129 747**
**FR-A- 2 544 317**
**US-A- 4 130 714**
**US-A- 4 210 756**

**CHEMICAL ABSTRACTS, vol. 71, no. 25, December 22, 1969, Columbus, Ohio, USA SAUL B.KADIN "Unusually facile anilide ethanolysis" page 430, column 2, Abstract-no. 124 183x**

**CHEMICAL ABSTRACTS, vol. 75, abstract-nr. 20423s**

**CHEMICAL ABSTRACTS, vol. 75, abstract-nr. 5914j**

**PHARMAZEUTISCHE CHEMIE, 1982, p. 44 ; E. SCHROEDER**

(73) Proprietor: **AMERICAN HOME PRODUCTS CORPORATION**
**Five Giralda Farms**
**Madison, New Jersey 07940-0874(US)**

(72) Inventor: **Malamas, Michael Sotirios**
**2443 Oleander Circle**
**Jamison Pennsylvania 18929(US)**

(74) Representative: **Wileman, David Francis Dr. et al**
**c/o Wyeth Laboratories**
**Huntercombe Lane South**
**Taplow Maidenhead Berkshire SL6 OPH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 365 324 B1

## Description

This invention relates to spiro-isoquinoline-pyrrolidine tetrones and their pharmaceutically acceptable salts thereof, to processes for their preparation, to methods for using the compounds, and to pharmaceutical preparations thereof. The compounds have pharmaceutical properties which render them beneficial for the prevention or treatment of complications associated with diabetes mellitus.

The use of insulin and/or oral hypoglycemic agents in the treatment of diabetes mellitus has prolonged the life of many of these patients. However, their use has not had a demonstrable impact on the development of diabetic complications such as neuropathy, nephropathy, retinopathy, cataracts and vascular disease which accompany the underlying metabolic disorder. There is little question that chronic hyperglycemia plays a major role in the genesis of these complications, and that complete normalization of blood glucose would likely prevent most if not all complications. For a number of reasons, though, chronic normalization of blood glucose has not been achieved with the currently available therapies. The development of diabetic complications has recently been linked to the increased accumulation of tissue sorbitol resulting from chronic hyperglycemia. Therapeutic reduction of sorbitol accumulation could potentially prevent the development of diabetic complications.

In mammals, including humans, the key enzyme involved in the conversion of glucose to sorbitol (e.g. the sorbitol pathway) is aldose reductase. J.H. Kinoshita and collaborators [see J.H. Kinoshita et al, Biochem. Biophys. Acta, 158, 472 (1968) and references cited therein] have demonstrated that this enzyme plays a central role in the etiology of galactosemic cataracts by effecting the conversion of galactose to dulcitol (galactitol), and that an agent capable of inhibiting aldose reductase can prevent the detrimental accumulation of dulcitol in the lens. Furthermore, a relationship between elevated levels of glucose and an undesirable accumulation of sorbitol has been demonstrated in the lens, peripheral nerves and kidney of diabetic animals [see A. Pirie and R. van Heyningen, Exp. Eye Res., 3, 124 (1964); L.T. Chylack and J.H. Kinoshita, Invest. Ophthal., 8, 401 (1969) and J.D. Ward and R.W.R. Baker, Diabetol., 6, 531(1970)].

European Patent 168,181-A discloses 1-(4-bromo-2-fluorophenylmethyl-7-chlorospiro-(indole-3,3'-pyrrolidine)-2,2',5'-trione of formula

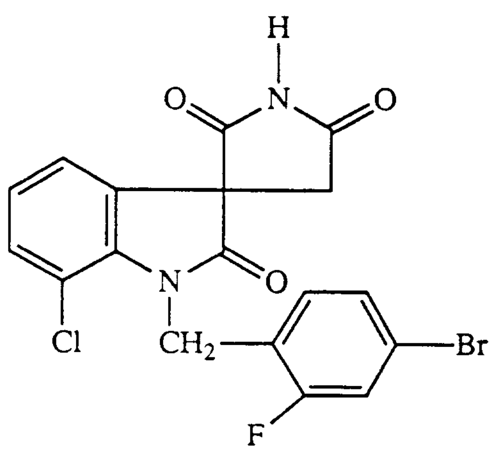

useful as an aldose reductase inhibitor for treating complications of diabetes and galactosemia.

French Patent 2,544,317 discloses compounds of formula

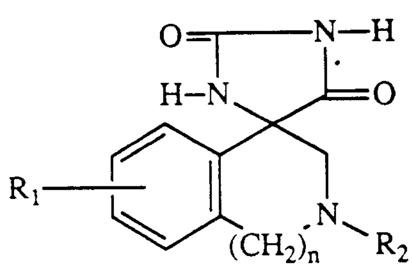

wherein $R_1$ represents hydrogen, halogen, hydroxy or methoxy; $R_2$ represents hydrogen, lower alkyl, phenyl lower alkyl, alkanoyl or p-toluenesulfonyl, useful as aldose reductase inhibitors.

The present invention provides spiro-isoquinoline-pyrrolidine tetrones and their analogs represented by formula (I):

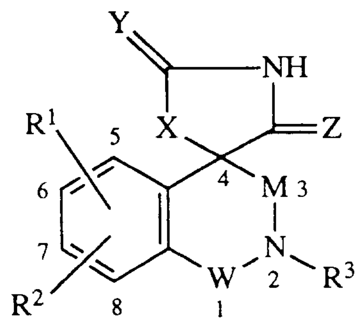

(I)

wherein $R^1$ and $R^2$ are independently hydrogen, alkyl containing 1 to 6 carbon atoms, halogen, lower alkoxy containing 1 to 6 carbon atoms, trifluoromethyl, lower alkylthio wherein lower alkyl contains 1 to 6 carbon atoms, dialkylamino wherein alkyl contains 1 to 6 carbon atoms, nitro, aryl or aryl (lower alkyl) oxy wherein aryl contains 6 to 10 carbon atoms and lower alkyl contains 1 to 6 carbon atoms; $R^3$ is hydrogen, lower alkyl containing 1 to 6 carbon atoms, aryl, aryl (lower alkyl) or halogen substituted aryl (lower alkyl) wherein aryl contains 6 to 10 carbon atoms and lower alkyl contains 1 to 6 carbon atoms or heterocyclic (lower alkyl) of structural formula

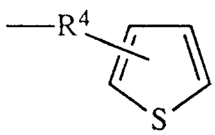

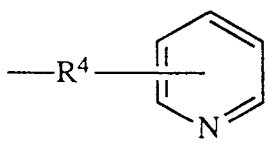

wherein $R^4$ is lower alkylene containing 1 to 3 carbon atoms; X is lower alkylene containing 1 to 3 carbon atoms, oxygen, sulfur or -NH- Y and Z are independently oxygen or sulfur; M and W are independently carbonyl, thiocarbonyl, sulfonyl, sulfoxo or lower alkylene containing 1 to 2 carbon atoms, with the provisos that (i) M and W are not both lower alkylene and when M is $CH_2$,W is other than sulfonyl and the pharmaceutically acceptable salts thereof. The fused benzene ring can be replaced by thiophene, pyridine or furan.

A preferred group of compounds of the present invention is represented by formula (II)

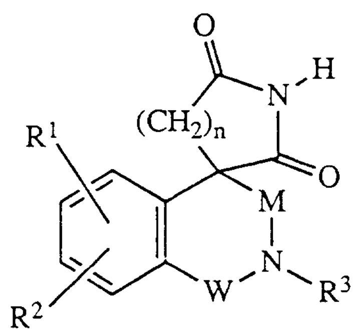

(II)

wherein $R^1$ and $R^2$ are independently hydrogen, alkyl containing 1 to 6 carbon atoms, halogen, lower alkoxy containing 1 to 6 carbon atoms, trifluoromethyl, lower alkylthio wherein lower alkyl contains 1 to 6 carbon atoms; $R^3$ is hydrogen, lower alkyl containing 1 to 6 carbon atoms; M and W are carbonyl or thiocarbonyl; n is 1 to 3, and the pharmaceutically acceptable salts thereof.

A second preferred group of compounds of the present invention is represented by formula (II)

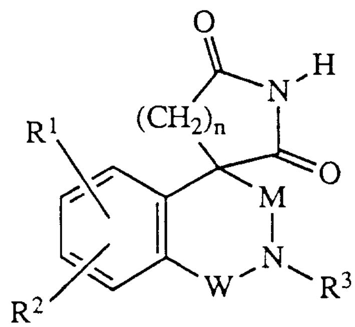

(II)

wherein $R^1$ and $R^2$ are independently hydrogen, alkyl containing 1 to 6 carbon atoms, halogen, lower alkoxy containing 1 to 6 carbon atoms, trifluoromethyl, lower alkylthio wherein lower alkyl contains 1 to 6 carbon atoms; $R^3$ is lower alkyl, aryl-(lower alkyl) or dihalogen substituted aryl-(lower alkyl) wherein aryl contains 6 to 10 carbon atoms and lower alkyl contains 1 to 6 carbon atoms; M and W are carbonyl or thiocarbonyl; n is 1 to 3, and the pharmaceutically acceptable salts thereof.

A still further preferred group of compounds of the present invention is represented by formula (III)

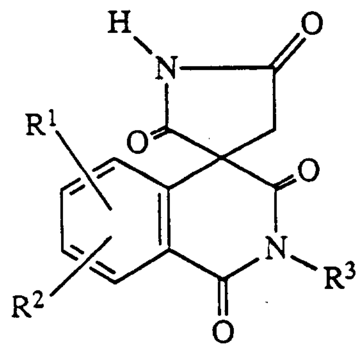

(III)

wherein $R^1$ and $R^2$ are hydrogen or halogen; $R^3$ is lower alkyl containing 1 to 6 carbon atoms, benzyl or dihalogen substituted benzyl, and the pharmaceutically acceptable salts thereof.

In formula I, Examples of Y and Z are oxygen. Examples of M and W are C = O. Examples of X are $-CH_2-$, $CH_2CH_2$ and $-CH_2CH_2CH_2-$.

$R^3$ may be for example hydrogen, methyl, ethyl, propyl, butyl, or dihalophenylmethyl, eg. 4-bromo-2-fluorophenylmethyl or 3,4-chichlorophenylmethyl.

The most preferred compounds of the present invention are set forth below:

2-[(4-bromo-2-fluorophenyl)methyl]spiro[isoquinoline-4(1H),3'-pyrrolidine]-1,2',3,5'(2H)-tetrone;
[2-[(4-bromo-2-fluorophenyl)methyl]-7-chloro]spiro[isoquinoline-4(1H),3'-pyrrolidine]-1,2',3,5'(2H)-tetrone;
[2-[(4-bromo-2-fluorophenyl)methyl]-6-chloro]spiro[isoquinoline-4(1H),3'-pyrrolidine)-1,2',3,5'(2H)-tetrone;
[2-[4-bromo-2-fluorophenyl)methyl]-5-fluoro]spiro[isoquinoline-4(1H),3'-pyrrolidine]-1,2',3,5'(2H)-tetrone;
[2-[(4-bromo-2-fluorophenyl)methyl]-6-fluoro]spiro[isoquinoline-4(1H),3'-pyrrolidine]-1,2',3,5'(2H)-tetrone;
[2[(4-bromo-2-fluorophenyl)methyl]-7-fluoro] spiro[isoquinoline-4-(1H),3'-pyrrolidine]-1,2',3,5'(2H)-tetrone;
[2-[(4-bromo-2-fluorophenyl)methyl]-8-fluoro]spiro[isoquinoline-4(1H),3'-pyrrolidine]-1,2',3,5'(2H)-tetrone;
[6-bromo-2-[(4-bromo-2-fluorophenyl)methyl]-7-methoxy]spiro[isoquinoline-4(1H),3'-pyrrolidine]-1,2',3,5'(2H)-tetrone;
[2-[(4-bromo-2-fluorophenyl)methyl]-7-methoxy]spiro[isoquinoline-4(1H),3'-pyrrolidine]-1,2',3,5'(2H)-tetrone;
(R)-(+)-2-[(4-bromo-2-fluorophenyl)methyl]spiro[isoquinoline-4(1H),3'-pyrrolidine]-1,2',3,5'(2H)-tetrone;
(S)-(-)-2-[(4-bromo-2-fluorophenyl)methyl]spiro[isoquinoline-4(1H),3'-pyrrolidine]-1,2',3,5'(2H)-tetrone;
(R)-(+)-[2-[(4-bromo-2-fluorophenyl)methyl]-6-fluoro]spiro[isoquinoline-4(1H),3'-pyrrolidine]-1,2',3,5'(2H)-tetrone;
(S)-(-)-[2-[(4-bromo-2-fluorophenyl)methyl]-6-fluoro]spiro[isoquinoline-4(1H),3'-pyrrolidine]-1,2',3,5'(2H)-tetrone;
2-[(3,4-dichlorophenyl)methyl]spiro[isoquinoline-4(1H),3'-pyrrolidine]-1,2',3,5'(2H)-tetrone;

2-methylspiro[isoquinoline-4(1H),3'-pyrrolodine-1,2',3,5'(2H)-tetrone;
6-chloro-2-methylspiro[isoquinoline-4(1H),3'-pyrrolidine]-1,2',3,5'(2H)-tetrone:
6-bromo-2-methylspiro[isoquinoline-4(1H),3'-pyrrolidine]-1,2',3,5'(2H)-tetrone;
7-chloro-2-methylspiro[isoquinoline-4(1H),3'-pyrrolidine]-1,2',3,5'(2H)-tetrone;
2-ethylspiro[isoquinoline-4(1H),3'-pyrrolidine]-1,2',3,5',(2H)-tetrone;
2-propylspiro[isoquinoline-4(1H),3',-pyrrolidine]-1,2',3,5',(2H)-tetrone;
2-butylspiro[isoquinoline-4(1H),3'-pyrrolidine]-1,2',3,5'(2H)-tetrone;
and the pharmaceutically acceptable salts thereof.

The compounds of formula (I) all possess at least one asymmetric carbon atom, namely the spiro carbon atom at position 3' of the pyrrolidine ring. The compounds of formula (I) therefore exist, and may be isolated, in two or more stereoisomeric forms. This invention encompasses the compounds of formula (I) in any optically active form or mixtures thereof, eg.racemate.

The spiro-isoquinoline-pyrrolidine tetrones and their enantiomers were prepared by the processes described hereinafter.

A method is provided for preventing or relieving diabetes mellitus associated complications in a diabetic mammal by administering to said mammal a prophylactic or alleviating amount of the compounds of formula (I). Such complications include neuropathy, nephropathy, retinopathy, keratopathy, diabetic uveitis, and cataracts.

A compound of formula (I), or a pharmaceutically acceptable salt thereof, when admixed with a pharmaceutically acceptable carrier, forms a pharmaceutical composition which can be used according to the preceding method.

The compounds of formula (I) form salts with suitable therapeutically acceptable inorganic and organic bases. These derived salts possess the same activity as their parent compound and are included within the scope of this invention. The compounds of formula (I) are transformed in excellent yield into the corresponding therapeutically acceptable salts by neutralization with the appropriate inorganic or organic base. The salts are administered usually in the same manner as the parent compounds. Suitable inorganic bases to form these salts include, for example, the hydroxides, carbonates or bicarbonates of the therapeutically acceptable alkali metals or alkaline earth metals, for example, sodium, potassium, magnesium, calcium and the like. Suitable organic bases include the following amines: benzylamine; lower mono-, di-, and trialkylamines, the alkyl radicals of which contain up to three carbon atoms, such as methylamine, dimethylamine, trimethylamine, ethylamine, di- and triethylamine, methylethylamine, and the like; mono-, di-, and trialkanolamines, the alkanol radicals of which contain up to three carbon atoms, for example, mono-, di-, and triethanolamine; alkylene-diamines which contain up to six carbon atoms, such as hexamethylenediamine; cyclic saturated or unsaturated bases containing up to six carbon atoms, such as pyrrolidine, piperidine, morpholine, piperazine and their N-alkyl and N-hydroxyalkyl derivatives, such as N-methyl-morpholine and N-(2-hydroxyethyl)-piperidine, as well as pyridine. Furthermore, there may be mentioned the corresponding quaternary salts, such as the tetraalkyl (for example tetramethyl), alkyl-alkanol (for example methyl-triethanol and trimethyl-monoethanol) and cyclic ammonium salts, for example the N-methyl-pyridinium, N-methyl-N-(2-hydroxyethyl)-morpholinium, N,N-dimethyl-morpholinium, N-methyl-N-(2-hydroxyethyl)-morpholinium, N,N-dimethylpiperidinium salts, which are characterized by having good water-solubility. In principle, however, there can be used all the ammonium salts which are physiologically compatible.

The transformations to the salts can be carried out by a variety of methods known in the art. For example, in the case of the inorganic salts, it is preferred to dissolve the compound of formula (I) in water containing at least one equivalent amount of a hydroxide, carbonate, or bicarbonate corresponding to the inorganic salt desired. Advantageously, the reaction is performed in a water-miscible, inert organic solvent, for example, methanol, ethanol, dioxane, and the like in the presence of water. For example, such use of sodium hydroxide, sodium carbonate or sodium bicarbonate gives a solution of the sodium salt. Evaporation of the solution or addition of a water-miscible solvent of a more moderate polarity, for example, a lower alkanol, for instance, butanol, or a lower alkanone, for instance, ethyl methyl ketone, gives the solid inorganic salt if that form is desired.

To produce an amine salt, the acidic compound of formula (I) is dissolved in a suitable solvent of either moderate or low polarity, for example, ethanol, methanol, ethyl acetate, diethyl ether and benzene. At least an equivalent amount of the amine corresponding to the desired cation is then added to that solution. If the resulting salt does not precipitate, it can usually be obtained in solid form by addition of a miscible diluent of lower polarity, for example, benzene or petroleum ether, or by evaporation. If the amine is relatively volatile, any excess can easily be removed by evaporation. It is preferred to use substantially equivalent amounts of the less volatile amines.

Salts wherein the cation is quaternary ammonium are produced by mixing the compound of formula (I) with an equivalent amount of the corresponding quaternary ammonium hydroxide in water solution, followed by evaporation of the water.

The compounds of formula I or pharmaceutically salts thereof may be administered to mammals, for example, man, cattle, or rabbits, either alone or in dosage forms, i.e., capsules or tablets, combined with pharmacologically acceptable excipients.

The compounds of this invention may be given orally. However, the method of administering the present active ingredients of this invention is not to be construed as limited to a particular mode of administration. For example, the compounds may be administered topically directly to the eye in the form of drops of sterile, buffered ophthalmic solutions, preferably of pH 7.2-7.6. Also, they may be administered orally in solid form containing such excipients as starch, milk sugar, certain types of clay and so forth. They may also be administered orally in the form of solutions or they may be injected parenterally. For parenteral administration they may be used in the form of a sterile solution, preferably of pH 7.2-7.6, containing a pharmaceutically acceptable buffer.

The dosage of the compound of formula I will vary with the form of administration and the particular compound chosen. Furthermore, it will vary with the particular host under treatment. Generally, treatment is initiated with small dosages substantially less than the optimal dose of the compound. Thereafter, the dosage is increased by small increments until efficacy is obtained. In general, the compounds of this invention are most desirably administered at a concentration level that will generally afford effective results without causing any harmful or deleterious side effects. For topical administration, a 0.05-1.0% solution may be administered dropwise in the eye. The frequency of instillation varies with the subject under treatment from a drop every two or three days to once daily. For oral or parenteral administration a preferred level of dosage ranges from about 0.1 mg to about 1.0 mg per kilo of body weight per day, although aforementioned variations will occur. However, a dosage level that is in the range of from about 0.1 mg to about 1.0 mg per kilo of body weight per day is most satisfactory.

Unit dosage forms such as capsules, tablets, pills and the like may contain from about 5.0 mg to about 25.0 mg of the active ingredients of this invention with a pharmaceutical carrier. Thus, for oral administration, capsules can contain from between about 5.0 mg to about 25.0 mg of the active ingredients of this invention with or without a pharmaceutical diluent. Tablets, either effervescent or noneffervescent, can contain between about 5.0 to 25.0 mg of the active ingredients of this invention together with conventional pharmaceutical carriers. Thus tablets, which may be coated and either effervescent or noneffervescent, may be prepared according to the known art. Inert diluents or carriers, for example, magnesium carbonate or lactose, can be used together with conventional disintegrating agents for example, magnesium stearate.

The compounds of formula I can also be used in combination with insulin or oral hypoglycemic agents to produce a beneficial effect in the treatment of diabetes mellitus. In this instance, commercially available insulin preparations or oral hypoglycemic agents, exemplified by acetohexamide, chlorpropamide, tolazamide, tolbutamide and phenformin, are suitable. The compounds hereof can be administered sequentially or simultaneously with insulin or the oral hypoglycemic agent. Suitable methods of administration, compositions and doses of the insulin preparation or oral hypoglycemic agent are described in medical textbooks; for instance, Physicians' Desk Reference, 42 ed., Medical Economics Co., Oradell, N.J., U.S.A., 1988.

The aldose reductase inhibiting property of the compounds of this invention and the utilization of the compounds in preventing, diminishing and alleviating diabetic complications are demonstrable in experiments using galactosemic rats, see Dvornik et al., Science, 182, 1146 (1973). Such experiments are exemplified hereinbelow after the listing of the following general comments pertaining to these experiments:

(a) Four or more groups of six male rats, 50-70 g, Sprague-Dawley strain, were used. The first group, the control group, was fed a mixture of laboratory chow (rodent Laboratory Chow, Purina) and glucose at 20% (w/w %) concentration. An untreated galactosemic group was fed a similar diet in which galactose was substituted for glucose. The third group was fed a diet prepared by mixing a given amount of the test compound with the galactose containing diet. The concentration of galactose in the diet of the treated groups was the same as that for the untreated galactosemic group.

(b) After four days, the animals were killed by euthanization. Both the lens and sciatic nerve were removed, weighed and stored frozen for polyol determination.

(c) The polyol determination was performed by a modification of the procedure of M. Kraml and L. Cosyns, Clin. Biochem., 2,373 (1969). Only two minor reagent changes were made: (a) The rinsing mixture was an aqueous 5% (w/v) trichloroacetic acid solution and (b) the stock solution was prepared by dissolving 25 mg of dulcitol in 100 ml of an aqueous trichloroacetic acid solution. [N.B.: For each experiment the average value found in the tissue from rats fed the glucose diet was subtracted from the

individual values found in the corresponding tissue in galactose-fed rats to obtain the amount of polyol accumulated.] The aldose reductase inhibiting effects of the compounds of formula (I) were also tested by employing an in vitro testing procedure similar to that described by S. Hayman and J.H. Kinoshita, J. Biol. Chem., 240,877 (1965). In the present case the procedure of Hayman and Kinoshita was modified in that the final chromatography step was omitted in the preparation of the enzyme from bovine lens.

The following tabulated results show that the spiro-isoquinoline-pyrrolidine tetrones of this invention show the property that they are active both in vitro and in vivo and diminish the accumulation of dulcitol in the lenses and sciatic nerves of rats fed galactose. The figures under L and N represent the percentage decrease of dulcitol accumulation in the tissues of the lens and sciatic nerve, respectively, for treated rats as compared to untreated rats.

**Table 1**

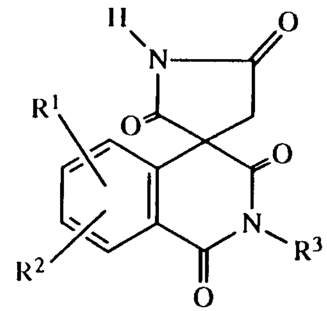

(III)

| $R^1$ | $R^2$ | $R^3$ | % Inhibition IN VITRO $10^{-5}$M | $10^{-6}$M | $10^{-7}$M | $4x10^{-8}$M | % Lowering Dulcitol Accumulation IN VIVO mg/kg | (%)L | (%)N |
|---|---|---|---|---|---|---|---|---|---|
| -H | -H | 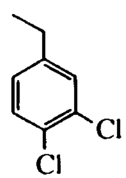 | 100 | 97 | 87 | 42 | 1.0<br>3.0<br>10.0<br>48.0 | 10.0<br>18.0<br>35.0<br>41.0 | 64.0<br>76.0<br>92.0<br>85.0 |
| -H | -H | 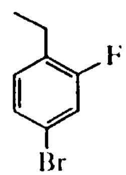 | 96 | 94 | 94 | 51 | 0.1<br>0.3<br>0.5<br>1.0 | NS<br>NS<br>12.0<br>16.0 | 26.0<br>60.0<br>82.0<br>95.0 |

| R1 | R2 | R3 | % Inhibition IN VITRO 10$^{-5}$M | 10$^{-6}$M | 10$^{-7}$M | 4x10$^{-8}$M | % Lowering Dulcitol Accumulation IN VIVO mg/kg | (%)L | (%)N |
|---|---|---|---|---|---|---|---|---|---|
| 6-Cl | -H | 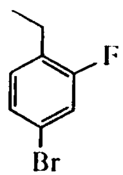 | 97 | 92 | 55 | 8 | 0.6 | 9 | 29 |
| -H | 7-Cl | 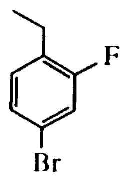 | 96 | 92 | 42 | 25 | 0.6 | NS | 63 |
| 5-F | -H | 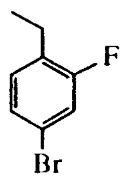 | 98 | 98 | 92 | 44 | 0.5<br>1.0 | 14.2<br>23.9 | 52.5<br>77.0 |
| 6-F | -H | 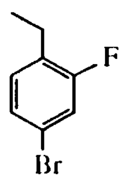 | 97 | 96 | 86 | 38 | 0.3<br>0.5<br>0.5<br>0.9 | NS<br>NS<br>NS<br>NS | 39.7<br>82.9<br>92.1<br>93.5 |

| R1 | R2 | R3 | % Inhibition IN VITRO 10⁻⁵M | | | | % Lowering Dulcitol Accumulation IN VIVO | | |
|---|---|---|---|---|---|---|---|---|---|
| | | | $10^{-5}$M | $10^{-6}$M | $10^{-7}$M | $4x10^{-8}$M | mg/kg | (%)L | (%)N |
| -H | 7-F | 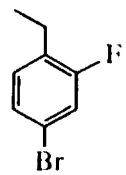 | 95 | 93 | 92 | 42 | 0.5<br>1.0 | NS<br>NS | NS<br>50.2 |
| -H | 8-F | 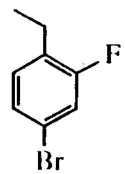 | 96 | 94 | 92 | 43 | 0.5<br>1.0 | NS<br>16.3 | 50.6<br>57.6 |
| -H | 7-$OCH_3$ | 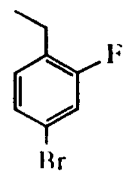 | 97 | 96 | 90 | 72 | 25.7 | 45.3 | 88.1 |
| 6-Br | 7-$OCH_3$ | 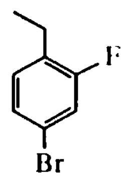 | 98 | 97 | 88 | 70 | 0.5<br>26.4 | NS<br>48.8 | 55.2<br>89.5 |

| | | | | % Inhibition IN VITRO | | | | % Lowering Dulcitol Accumulation IN VIVO | | |
|---|---|---|---|---|---|---|---|---|---|---|
| | $R^1$ | $R^2$ | $R^3$ | $10^{-5}M$ | $10^{-6}M$ | $10^{-7}M$ | $4x10^{-8}M$ | mg/kg | (%)L | (%)N |
| (S)-(-) | -H | -H | 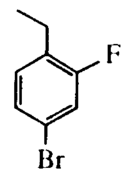 | 87 | 51 | -- | — | 0.5<br>4.8 | NS<br>NS | NS<br>83 |
| (R)-(+) | -H | -H | 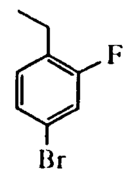 | 96 | 93 | 91 | 87 | 0.5<br>5.1 | NS<br>NS | 83<br>100 |
| (S)-(-) | -H | -F | 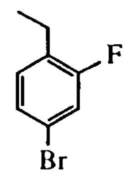 | 98 | 97 | 46 | 29 | 0.4<br>0.8 | NS<br>21 | 40<br>70 |
| (R)-(+) | -H | -F | 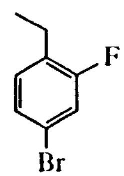 | 99 | 95 | 93 | 93 | 0.4<br>0.8 | 31<br>26 | 100<br>100 |

| | | | % Inhibition IN VITRO | | | | % Lowering Dulcitol Accumulation IN VIVO | | |
|---|---|---|---|---|---|---|---|---|---|
| $R^1$ | $R^2$ | $R^3$ | $10^{-5}M$ | $10^{-6}M$ | $10^{-7}M$ | $4x10^{-8}M$ | mg/kg | (%)L | (%)N |
| -H | -H | $-CH_3$ | 95 | 91 | 63 | 38 | 1.0<br>3.0<br>10.0<br>43.0 | 15.0<br>43.0<br>62.0<br>86.0 | 37.0<br>66.0<br>79.0<br>89.0 |
| 6-Cl | -H | $-CH_3$ | 93 | 94 | 73 | 43 | 1<br>3 | 33<br>59 | 75<br>75 |
| 6-Br | -H | $-CH_3$ | 100 | 100 | 85 | 43 | 1 | NS | 54 |
| -H | 7-Cl | $-CH_3$ | 95 | 92 | 61 | 34 | 3 | 14 | 21 |
| -H | -H | $-CH_2CH_3$ | 96 | 76 | 27 | 17 | 11 | NS | NS |
| -H | -H | $-(CH_2)_2CH_3$ | 94 | 62 | 19 | 8 | 11 | NS | NS |
| -H | -H | $-(CH_2)_3CH_3$ | 99 | 86 | 17 | 19 | 10 | NS | NS |

NS = statistically non-significant change

This invention provides processes for preparing compounds of formula I. These processes include:

a)cyclising an amide of formula (A)

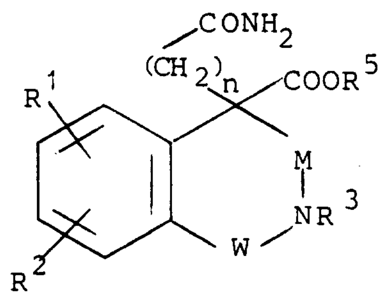

(A)

wherein $R^1$, $R^2$, $R^3$, n, M and W are as defined above and $COOR^5$ is an ester group, using a suitable base such as sodium hydride or lithium bis-(trimethylsilyl)amide depending on the value for $R^3$,M,W to give a compound of formula I wherein Y and Z are both oxygen and X is $-(CH_2)_n-$ wherein n is 1 to 3;
b) reacting a compound of formula (B)

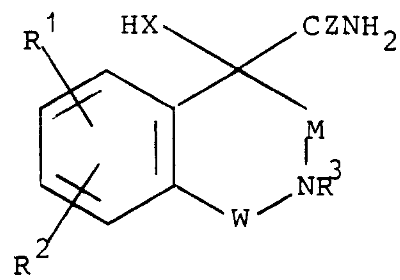

(B)

wherein $R^1$, $R^2$, $R^3$ ,M and W are as defined above and X is O, S or NH and Z is O or S, with $CYCl_2$ wherein Y is O
or S to give a compound of formula I wherein X is O, S or NH and Y and Z independently represent O or S;
or (c) reacting a compound of formula (C)

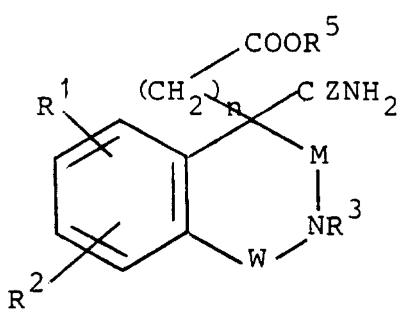

(C)

wherein $R^1$, $R^2$, $R^3$ ,M, W, n, Z and $COOR^5$ are as defined above using a suitable base such as sodium

hydride or lithium bis-(trimethylsilyl)amide depending on the value of $R^3$,M,W to give a compound of formula I wherein Y is O, Z is O or S and n is 1 to 3;
or (d) reacting a compound of formula (D)

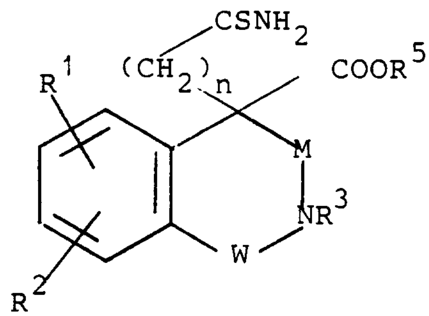

(D)

wherein $R^1$, $R^2$, $R^3$,M, W, n, and $COOR^5$ are as defined above with strong base, eg. an alkali metal hydride to give a compound of formula I wherein X is $-(CH_2)_n-$in which n is 1 to 3, and Y is S and Z is O;
or (e)
reacting a compound of formula

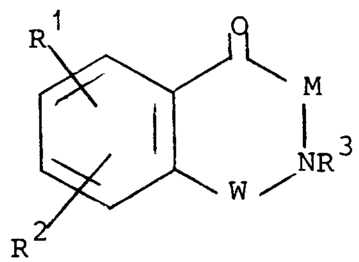

(F)

wherein $R^1$, $R^2$, $R^3$, M and W are as defined above with ammonium carbonate and an alkali metal cyanide to give a compound of formula I wherein X is -NH- and Y and Z are both oxygen;
or
(f) reacting a compound of formula (F) successfully with hydrogen cyanide, thionyl chloride and thiourea to give a compound of formula (I) wherein X is S and Y and Z are
both oxygen;
or
(g) reacting a compound of formula (F) with trimethylsilyl cyanide followed by HCl/alkanol and phosgene or thiophosgene to give a compound of formula (I) wherein X is O or S and Y and Z are both oxygen.

Starting materials of formulae (A),(B),(C) and (D) can be prepared via intermediates of formula (G) and (G-1) shown below:

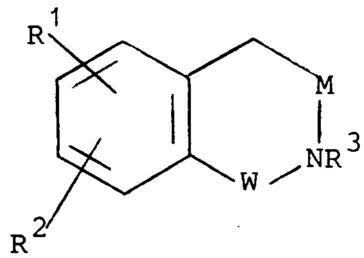

(G-1)

and

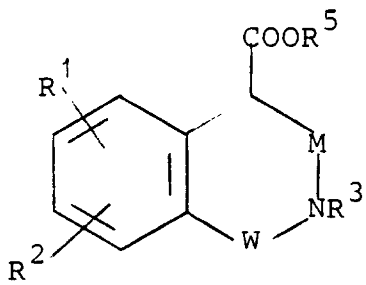

(G)

wherein $R^1$, $R^2$, $R^3$,M, W, and $COOR^5$ are as defined above. Examples of $R^5$ are alkyl group such as $C_1$-$C_4$ alkyl especially methyl.

For example compounds of formula (A) are prepared by reacting a compound of formula (G) with $Br(CH_2)_nCO_2$-${}^tBu$ in the presence of a suitable base eg. $Li$-$N(SiMe_3)_2$ to give a compound of formula

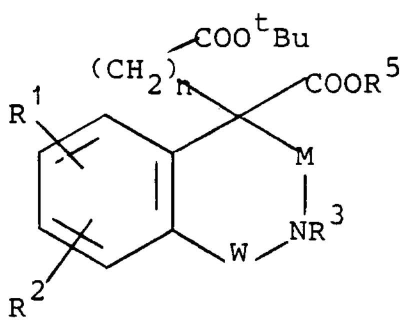

(H)

wherein $R^1$, $R^2$, $R^3$,M and W are as defined above and $COOR^5$ is an ester group (other than ${}^tBu$ ester); treating the compound of formula (G) with trifluoracetic acid to remove the ${}^tBu$ group, reacting the resulting acid with a coupling agent, eg. 1-(2-dimethyl-aminopropyl)-2-ethylcarbodiimide in the presence of 1-hydroxy benzotriazole, followed by ammonia to give the amide of formula (A).

Compounds of formula (B) are prepared by (1) reacting a compound of formula (G) with potassium trimethylsilanoate

followed in turn by thionylchloride, N-bromosuccinimide and methanol to give a compound of formula (H)

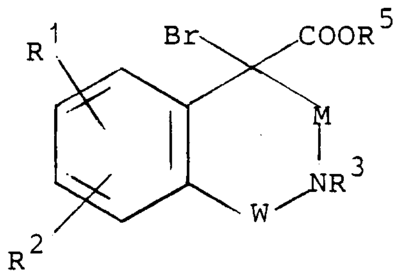

(J)

wherein $R^1$, $R^2$ $R^3$,M, W and $COOR^5$ are as defined above, and (2) reacting the compound of formula (J) with KSH, NaSH, potassium hydroxide or ammonia to give an ester of formula

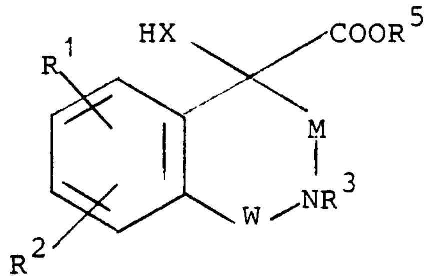

(K)

wherein $R^1$, $R^2$, $R^3$,M, W, X and $COOR^5$ are as defined above,
and (3) converting the ester of formula (K) to the amide of formula (B) by standard methods of amidation. A carboxy amide of formula (B) can be converted to a thioamide using $P_2S_5$ or Lawesson's reagent.

Compounds of formula (C) can be prepared by converting a compound of formula (G) to a nitrile of formula (L).

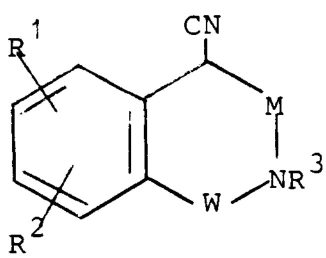

(L)

using standard techniques and then reacting the nitrile with $Br(CH_2)_nCOOR^5$ to give a compound of formula (M)

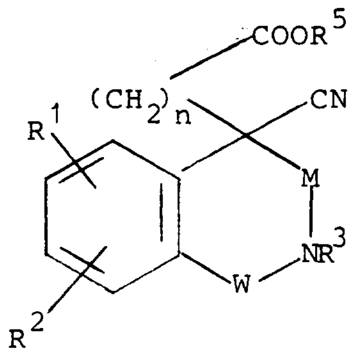

(M)

in which formula $R^1$, $R^2$ $R^3$, n, M, W and $COOR^5$ are as hereinbefore defined. The compound of formula (M) is then converted to the desired amide (C) using standard techniques eg. acid hydrolysis to give the carboxyamide or reaction with $HSP(=S)(OEt)_2$ /HCl to give the thioamide.

Compounds of formula (D) may be prepared by reacting a compound of formula (G) with $Br(CH_2)_nCN$ to give a nitrile of formula (N)

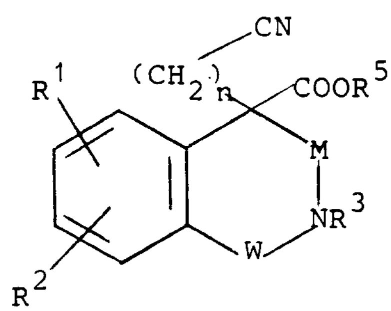

(N)

and then converting the nitrile to a thioamide using $HSP(=S)(OEt)_2$ /HCl

Compounds of formula (E) can be prepared by reacting a compound of formula (F)

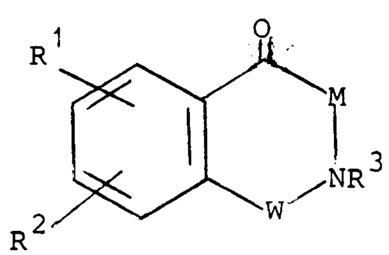

(F)

with $NCCH_2COOMe$ to give a compound of formula

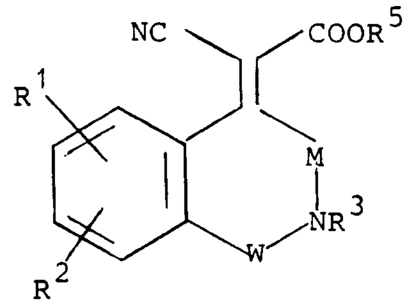

(P)

and then reacting compound (P) with KCN.

Compounds of formula F in many cases are known compounds or can be prepared by analogous methods see for example Process G shown hereinafter.

Compounds of formula (G) can in general be prepared from compounds of formula (G-1) by reaction with $CNCOOR^5$ and lithium bis-(trimethylsilyl)amide depending on the value for M and whether M activates the $\alpha$ carbon. Where it is desired to make a compound of formula (G) having a value of M such that the $\alpha$-carbon is not susceptible to introduction of $COOR^5$ then such a compound can in general be prepared from a compound (G-1) having an "activating" M group by introducing-COOR5 and then changing the M group to the desired value.

Compounds of formula (G-1) wherein M and W are both C=O can be prepared by reacting a compound of formula (Q)

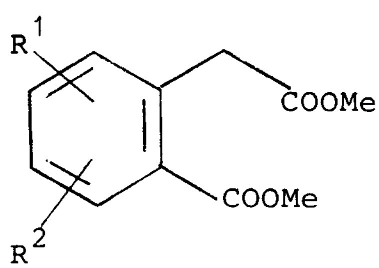

(Q)

with an amine of formula $H_2NR^3$.

Compounds of formula (G-1) wherein M is CO and W is $CH_2$ can be prepared by reacting a compound of formula (R)

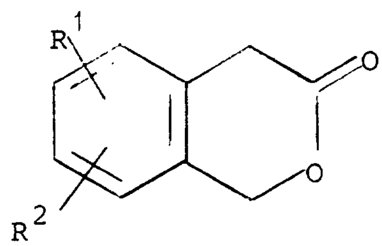

(R)

with an amine of formula $H_2NR^3$ in the presence of HBr in ethanol solvent. The compounds of formula (R) may be obtained by reacting a substituted or unsubstituted phenylacetic acid with formaldehyde in the

presence of acetic anhydride.

Compound of formula (G-1) wherein M is CO and W is $SO_2$ or SO may be prepared by reacting a compound of formula

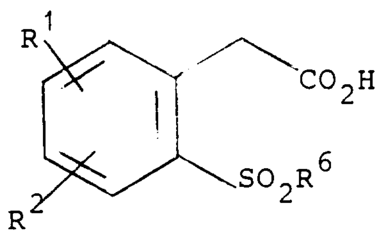

(S)

where $R^6$ = H or Cl, with an amine of formula $H_2NR^3$. The compound of formula (R) wherein $R^6$ = Cl may be obtained by reacting a compound of formula

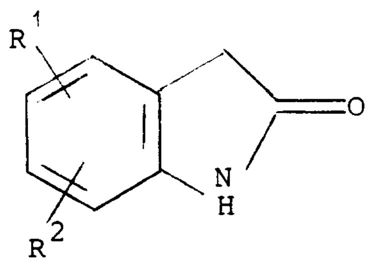

(T)

with sodium hydroxide, sodium nitrite, hydrochloric acid sulphur dioxide and CuCl.

Compounds of formula (G-1) wherein M is $SO_2$ or SO and W is CO may be prepared by reacting a compound of formula

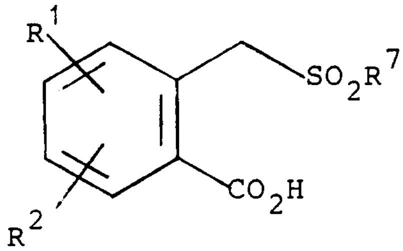

(U)

where $R^7$ is OH, Cl or H, with an amine of formula $H_2NR^3$.

Compounds of formula (G-1) wherein M and W both independently represent $SO_2$ or SO can be prepared by reacting a compound of formula (V)

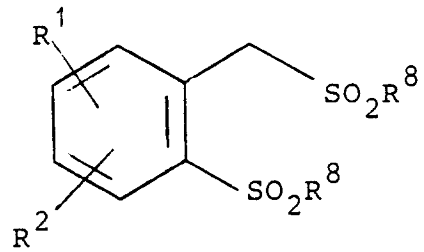

(V)

with an amine of formula $H_2NR^3$, wherein $R^8$ is Cl or H.

Compounds of formula (G) wherein M is $C_1$ or $C_2$ alkylene and W is CO, $SO_2$ or SO can be prepared by treating a corresponding compound of formula (G-1) with N-bromosuccinimide followed by KCN and then methanolic HCl.

Compounds of formula (G-1) wherein M is CO, $R^3$ is H and W is $-CH_2CH_2-$ can be prepared from a *β*-tetralone of formula (W):

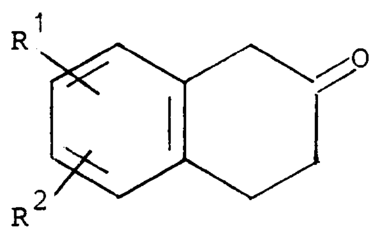

(W)

by ring expansion, eg. Beckmann rearrangement of the oxime under standard conditions.

Compounds of formula (G-1) wherein M is $SO_2$ or SO and W is $CH_2CH_2$ can be prepared by reacting a compound of formula

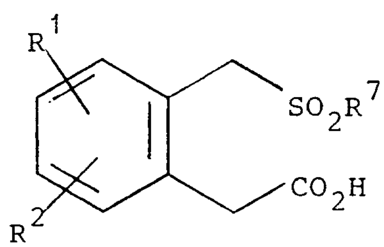

(X)

where $R^7$ is H, Cl or OH with an amine $H_2NR^3$ followed by thiation and reduction using Raney nickel.

Compounds of formulae (S),(U),(V) and (X) where $R^6$, $R^7$ or $R^8$ is hydrogen may be prepared by reducing corresponding compounds where $R^6$, $R^7$ or $R^8$ is Cl eg. using zinc or sodium metabisulphite.

Compounds of formulae (G) and (G-1) wherein M is CO and/or W is CO can be converted to other compounds of formulae (G) and (G-1) wherein one or both of M and W are CS by thiation, eg. using $P_2S_5$ or Lawesson's reagent.

Compounds of formulae (G) and (G-1) wherein one or both of M and W represent CS can be reduced, eg. using Raney nickel to give compounds of formulae (G) and G-1) wherein one or both of M and W are $-CH_2-$.

Compounds of formula (G) and (G-1) wherein $R^3$ represents hydrogen can be converted to compounds of formula G and G-1 wherein $R^3$ is as defined in connection with formula I by alkylating, arylating or aralkylating or acylating in known manner with an agent containing the group $R^3$.

Other routes to the various intermediates will be apparent to the skilled chemist.

Compounds of formula I wherein the benzene ring is replaced by a fused thiophene, furan or pyridine ring may be prepared by similar procedures to those described above using the appropriate starting materials - see for example Processes I and J hereinafter described.

Starting materials for the processes herein described are known compounds or may be prepared by analogous methods.

This invention also provides intermediates of formula A, C, D, M, and N above, which compounds are represented by the single general formula XXIV shown below:

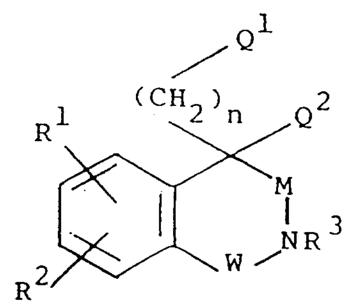

(XXIV)

wherein one of $Q^1$ and $Q^2$ is $CZNH_2$ or CN; the other one of $Q^1$ and $Q^2$ is $-COOR^5$ wherein $COOR^5$ is an ester group; Z, $R^1$, $R^2$, $R^3$, M and W are as defined in connection with formula I and n is 1 to 3.

In formula I, $Q^1$ may be for example $CONH_2$, $R^1$ and $R^2$ may be hydrogen or halogen, n may be l, M and W may be both carbonyl and $R^3$ may be loweralkyl of 1-6 carbon atoms, benzyl, or dihalogen substituted benzyl. This invention also provides a process for preparing a compound of formula XXIV which comprises one of the following.

a) reacting an acid of formula

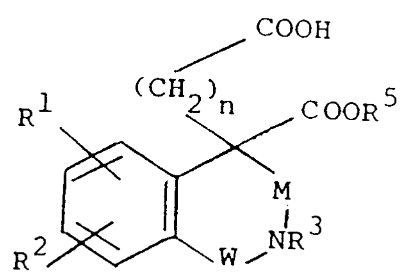

XXV

wherein $R^1$, $R^2$, $R^3$, M, W and $COOR^5$ are as defined above with a coupling agent and ammonia to give a corresponding compound of formula XXIV wherein $Q^1$ is $CONH_2$

or

b) reacting a compound of formula L

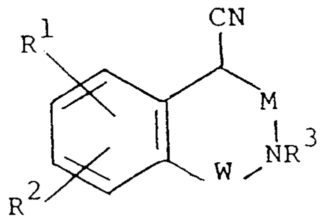

(L)

wherein $R^1$, $R^2$, M, W and $R^3$ are as defined above with a compound of formula

$Br(CH_2)_nCOOR^5$

wherein n is 1 to 3 and $COOR^5$ is an ester group to give a compound of formula XXIV wherein $Q^1$ is $COOR^5$ and $Q^2$ is CN;
c) reacting a compound of formula G

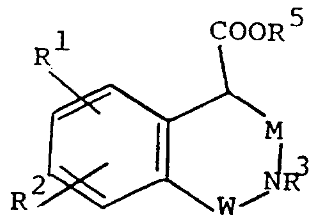

(G)

wherein $R^1$, $R^2$, $R^3$, M, W and $COOR^5$ are as defined above with a compound of formula

$Br(CH_2)_nCN$

wherein n is as defined above to give a compound of formula XXIV wherein $Q^1$ is CN and $Q^2$ is $COOR^5$; or
d) converting a nitrile of formula XXIV wherein $Q^2$ is CN to an amide or thioamide of formula XXIV wherein $Q^2$ is $CONH_2$ or $CSNH_2$ in known manner;
or
e) converting a nitrile of formula XXIV wherein $Q^1$ is CN to thioamide of formula XXIV wherein $Q^1$ is $CSNH_2$ using $HSP(=S)(OEt)_2/HCl$.
The following processes exemplify preferred embodiments

## Process A

The spiro-isoquinoline-pyrrolidine tetrones of the present invention can be prepared by the following reaction scheme,

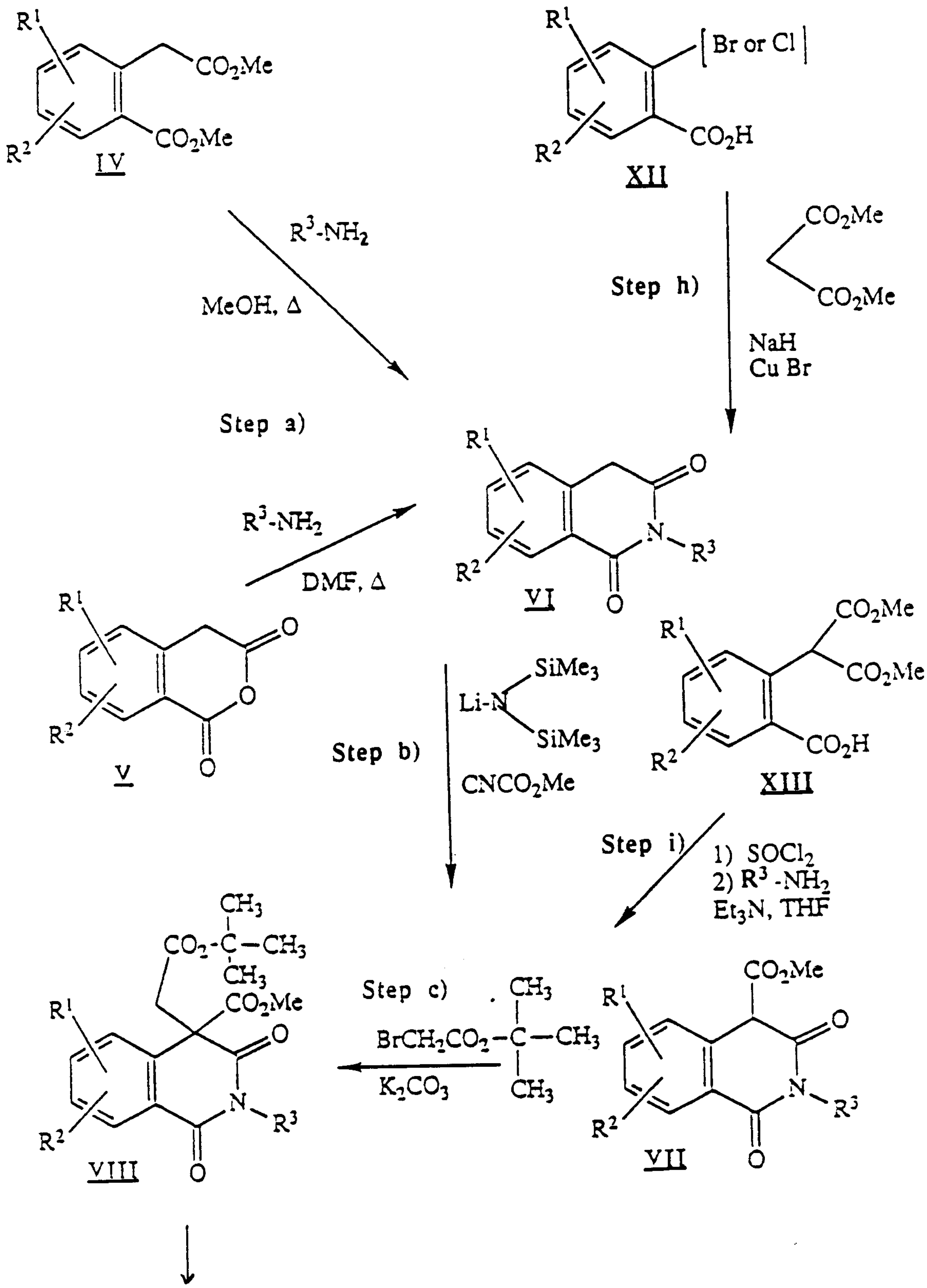
IV
XII
$R^3-NH_2$
MeOH, Δ
Step a)
Step h)
NaH
Cu Br
VI
$R^3-NH_2$
DMF, Δ
V
Step b)
$CNCO_2Me$
XIII
Step i)
1) $SOCl_2$
2) $R^3-NH_2$
$Et_3N$, THF
Step c)
$K_2CO_3$
VIII
VII

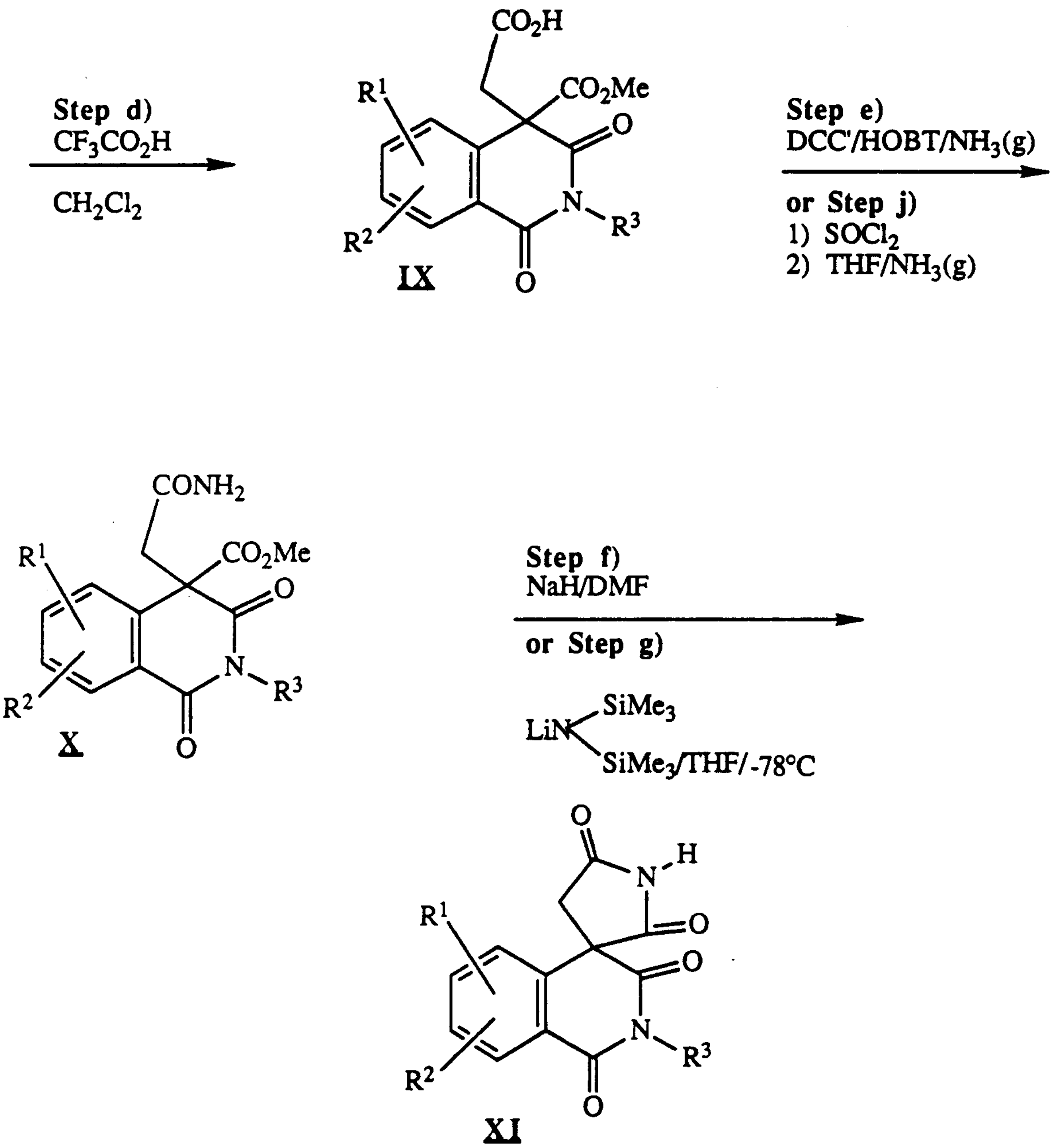


IX

X

XI

wherein $R^1$ and $R^2$ are hydrogen or halogen; $R^3$ is alkyl containing 1 to 6 carbon atoms, aryl (lower alkyl) or dihalogen substituted aryl (lower alkyl) wherein aryl contains 6 to 10 carbon atoms and lower alkyl contains 1 to 6 carbon atoms. The process of the production of the compounds of formula (I) wherein $R^1$ and $R^2$ are chlorine or hydrogen; X is methylene; Y,Z are oxygen; M,W are carbonyl; $R^3$ is alkyl (methyl, ethyl, propyl, butyl) or aralkyl (3,4-dichlorobenzyl, or 4-bromo-2-fluorobenzyl) comprises:

Step a) Reacting either homophthalic acid dimethyl ester of formula (IV) or homophthalic anhydride of formula (V) wherein $R^1$ and $R^2$ are as defined above with an amine $R^3$-$NH_2$ wherein $R^3$ is as defined above to produce the homophthalimide of formula (VI) wherein $R^1$, $R^2$, $R^3$ are as defined above.

The homophthalic acid dimethyl esters of formula (IV) and the homophthalic anhydrides of formula (V) required for the present invention are commercially available compounds or can be prepared by known methods.

The homophthalic acid dimethyl ester compound (IV) can be reacted with a saturated methanolic ammonium solution in a pressure vessel and temperature in the range of 60-80 ° C. The homophthalic anhydride compound (V) can be reacted with an amine at high temperature 160-180 ° C in a conventional solvent which does not adversely influence the reaction such as N,N-dimethyformamide. However, reaction of a volatile amine with the homophthalic anhydride compound (V) can be produced by reaction

of a saturated tetrahydrofuran solution of the appropriate amine with homophthalic anhydride at room temperature and subsequent removal of the volatile solvent, and introduction of N,N-dimethylformamide as the solvent.

Further reaction at high temperature (160-180 °C) is required to produce the compound of formula (VI).

Step b) The compound of formula (VI) wherein $R^1$, $R^2$, $R^3$ are as defined above is reacted with a base, for example, lithium bis(trimethylsilyl)amide in a conventional solvent which does not adversely influence the reaction, for example, tetrahydrofuran, and subsequent addition of a reactive carbomethoxylating agent such as methyl cyanoformate to produce the compound of formula (VII) wherein $R^1$, $R^2$, $R^3$ are as defined above.

Step c) The compound of formula (VII), wherein $R^1$, $R^2$, $R^3$ are as defined above, is reacted with an inorganic base such as potassium carbonate in a conventional solvent which does not adversely influence the reaction, for example, N,N-dimethyformamide, and subsequent addition of the tert-butyl bromoacetate produces the compound of the formula (VIII), wherein $R^1$, $R^2$, $R^3$ are as defined above.

Step d) The compound of formula (VIII), wherein $R^1$, $R^2$, $R^3$ are as defined above, is hydrolyzed with an organic acid such as trifluoroacetic acid in a conventional solvent which does not adversely influence the reaction, for example, methylene chloride, to produce the compound of formula (IX), wherein $R^1$, $R^2$, $R^3$ are as defined above.

Step e) The compound of formula (IX), wherein $R^1$, $R^2$, $R^3$ are as defined above, is reacted with a coupling agent such as 1-(2-dimethylaminopropyl)-2-ethylcarbodiimide DCC'/l-hydroxybenzotriazole, (HOBT) in a conventional solvent which does not adversely influence the reaction, for example, N,N-dimethylformamide, and subsequent addition of tetrahydrofuran ammonium solution produces the compound of the formula (X), wherein $R^1$, $R^2$, $R^3$ are as defined above.

Step f) The compound of formula (X), wherein $R^1$ and $R^2$ are as defined above, and $R^3$ is an appropriately substituted aralkyl as defined above is reacted with a base such as sodium hydride in a conventional solvent which does not adversely influence the reaction, for example, N,N-dimethylformamide, to produce the compound of the formula (XI), wherein $R^1$, $R^2$ are as defined above, $R^3$ is an appropriately substituted aralkyl as defined above.

When $R^3$ is an alkyl as defined above, the process of Step f) produces very low yields.

Step g) The compound of formula (X), wherein $R^1$, $R^2$ are as defined above, $R^3$ is an alkyl as defined above, is reacted with a base such as lithium bis(trimethylsilyl)amide, in a conventional solvent which does not adversely influence the reaction, for example, tetrahydrofuran, at low termperature (-78 °C) to produce the compound of the formula (XI), wherein $R^1$, $R^2$ are as defined above, $R^3$ is an alkyl, as defined above.

Step h) Reacting either 2-bromobenzoic acid or 2-chlorobenzoic acid of formula (XII) wherein $R^1$ and $R^2$ are as defined above with dimethyl malonate and NaH in the presence of a catalytic amount of CuBr to produce the propanedioic acid dimethyl ester of formula (XIII) wherein $R^1$, $R^2$ and $R^3$ are as defined above.

The 2-bromobenzoic acids or 2-chlorobenzoic acids of formula (XII) required for the present invention are commercially available compounds or can be prepared by known methods.

Step i) The propanedioic acid dimethyl ester of formula (XIII) can be reacted with thionyl chloride under refluxing conditions to produce the corresponding acid chloride which upon treatment with an amine $R^3$-$NH_2$ in the presence of triethylamine in a conventional solvent which does not adversely influence the reaction, for example, tetrahydrofuran, can produce the compound of formula (VII) wherein $R^1$, $R^2$ and $R^3$ are as defined above.

Step j) The compound of formula (IX), wherein $R^1$, $R^2$ and $R^3$ are as defined above, can be reacted with thionyl chloride under refluxing conditions to produce the corresponding acid chloride, which upon treatment with a saturated tetrahydrofuran ammonium solution can produce the compound of formula (X), wherein $R^1$, $R^2$ and $R^3$ are as defined above.

## Process B

The enantiomers of the spiro-isoquinoline-pyrrolidine tetrones of the present invention were prepared by the following reaction scheme,

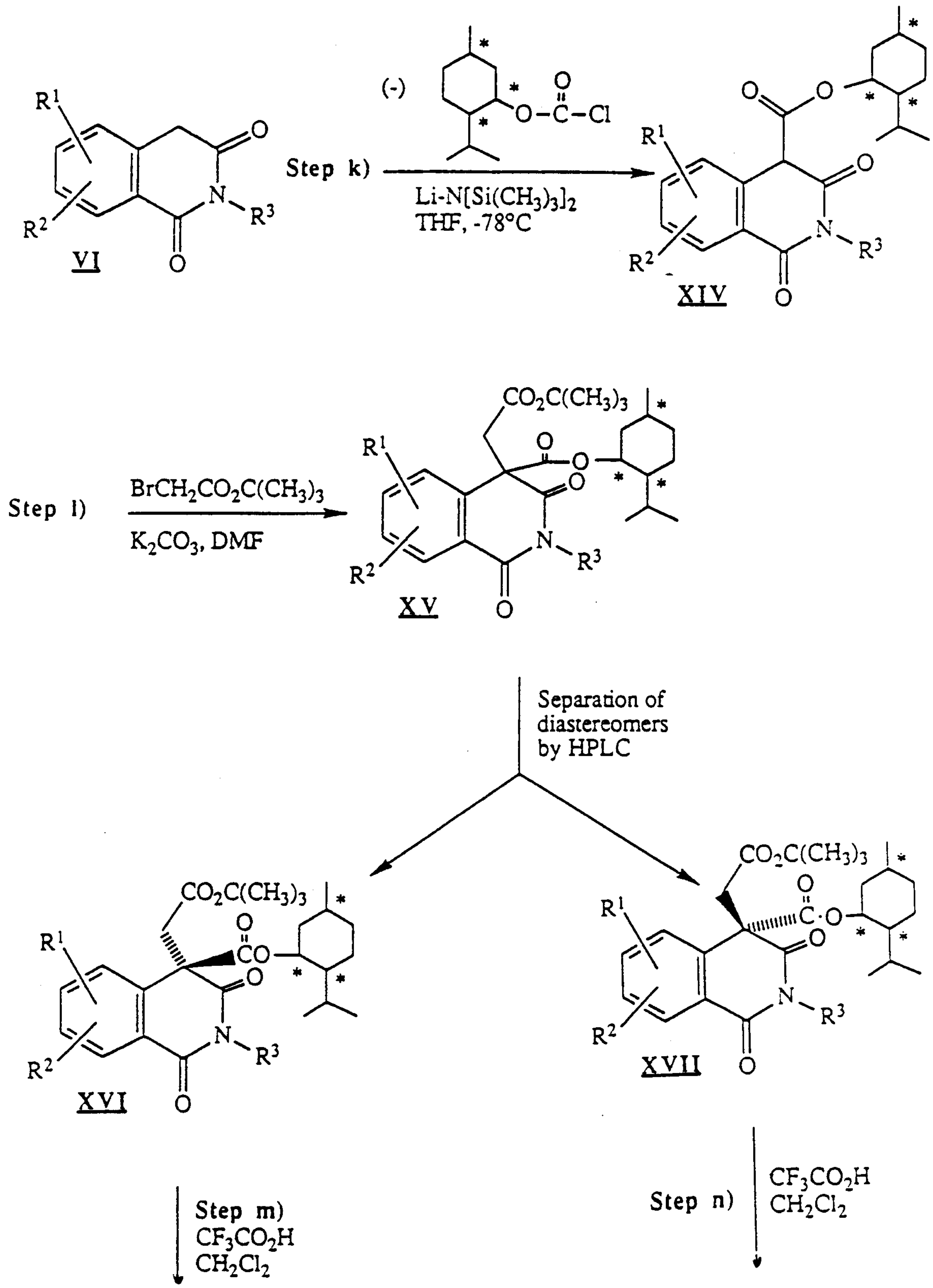


VI

XIV

XV

XVI

XVII

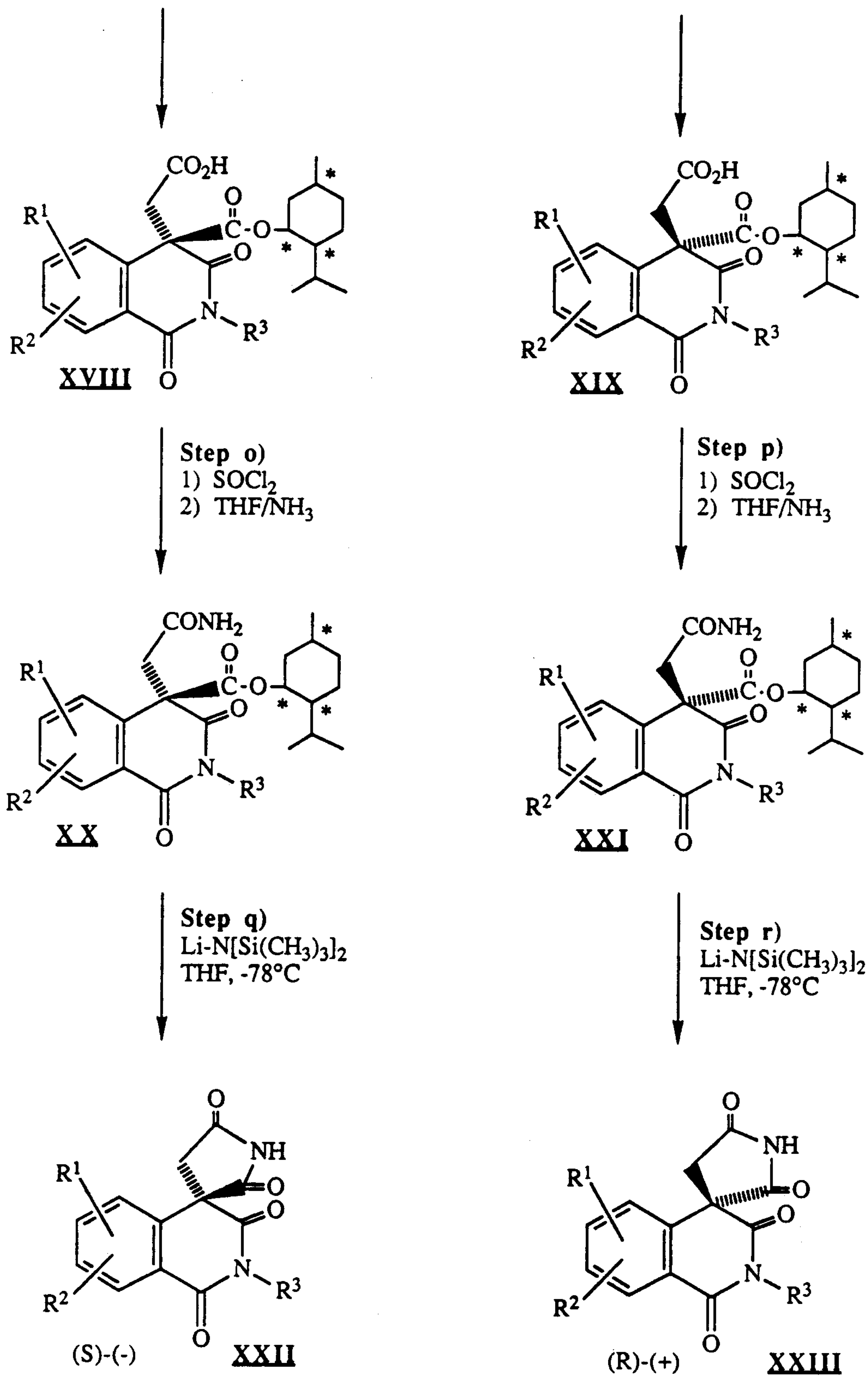


XVIII XIX XX XXI

(S)-(-) XXII (R)-(+) XXIII

wherein $R^1$ and $R^2$ are hydrogen or halogen; $R^3$ is alkyl containing 1 to 6 carbon atoms, aryl (lower alkyl) or dihalogen-substituted aryl (lower alkyl) wherein aryl contains 6 to 10 carbon atoms and lower alkyl contains 1 to 6 carbon atoms. The process for the production of the compounds of formula (I) wherein $R^1$ and $R^2$ are chlorine or hydrogen; X is methylene; Y and Z are oxygen; M and W are carbonyl; $R^3$ is alkyl (methyl, ethyl, propyl, butyl) or aralkyl (3,4-dichlorobenzyl, or 4-bromo-2-fluorobenzyl) comprises:

Step k) The compound of formula (VI), wherein $R^1$, $R^2$ and $R^3$ are as defined above is reacted with a base, for example, lithium bis(trimethylsilyl)amide in a conventional solvent which does not adversely influence the reaction, for example, tetrahydrofuran, and subsequent addition of (-)-menthyl chloroformate to produce the compound of formula (XIV), wherein $R^1$, $R^2$ and $R^3$ are as defined above.

Step l) The compound of formula (XIV), wherein $R^1$, $R^2$ and $R^3$ are as defined above, is reacted with an inorganic base such as potassium carbonate in a conventional solvent which does not adversely influence the reaction, for example, N,N-dimethylformamide, and subsequent addition of the tert-butyl bromoacetate produces the compound of formula (XV), wherein $R^1$, $R^2$ and $R^3$ are as defined above. The compound of formula (XV), wherein $R^1$, $R^2$ and $R^3$ are as defined above, represents a mixture of two diastereomers which are separable by HPLC to produce diastereomer of formula (XVI) and diastereomer of formula (XVII), wherein $R^1$, $R^2$ and $R^3$ are as defined above.

Step m) The compound of formula (XVI), wherein $R^1$, $R^2$ and $R^3$ are as defined above, is hydrolyzed with an organic acid such as trifluoroacetic acid in a conventional solvent which does not adversely influence the reaction, for example, methylene chloride, to produce the compound of formula (XVIII), wherein $R^1$, $R^2$ and $R^3$ are as defined above.

Step n) The compound of formula (XVII), wherein $R^1$, $R^2$ and $R^3$ are as defined above, is hydrolyzed with an organic acid such as trifluoroacetic acid in a conventional solvent which does not adversely influence the reaction, for example methylene chloride, to produce the compound of formula (XIX), wherein $R^1$, $R^2$ and $R^3$ are as defined above.

Step o) The compound of formula (XVIII), wherein $R^1$, $R^2$ and $R^3$ are as defined above, is reacted with thionyl chloride to produce the corresponding acid chloride and subsequent addition to a tetrahydrofuran ammonium solution produces the compound of the formula (XX), wherein $R^1$, $R^2$ and $R^3$ are as defined above.

Step p) The compound of formula (XIX), wherein $R^1$, $R^2$ and $R^3$ are as defined above, is reacted with thionyl chloride to produce the corresponding acid chloride and subsequent addition to a tetrahydrofuran ammonium solution produces the compound of formula (XXI), wherein $R^1$, $R^2$ and $R^3$ are as defined above.

Step q) The compound of formula (XX), wherein $R^1$, $R^2$ and $R^3$ are as defined above, is reacted with a base such as lithium bis(trimethylsilyl)amide, in a conventional solvent which does not adversely influence the reaction, for example, tetrahydrofuran, at low temperature (-78°C) to produce the compound of formula (XXII), wherein $R^1$, $R^2$ and $R^3$ are as defined above.

Step r) The compound of formula (XXI), wherein $R^1$, $R^2$ and $R^3$ are as defined above, is reacted with a base such as lithium bis(trimethylsilyl)amide, in a conventional solvent which does not adversely influence the reaction, for example tetrahydrofuran, at low temperature (-78° C) to produce the compound of formula (XXIII), wherein $R^1$, $R^2$ and $R^3$ are as defined above.

The absolute configuration of the compound of formula (XXII), wherein $R^1$ and $R^2$ are hydrogen and $R^3$ is (4-bromo-2-fluorophenyl)methyl was determined by a single crystal x-ray analysis. It was established that the compound of formula (XXII) possesses the (S) absolute configuration.

Some of the compounds of formula (I) wherein M is carbonyl and W is lower alkylene can be made by the Process C

**Process C**

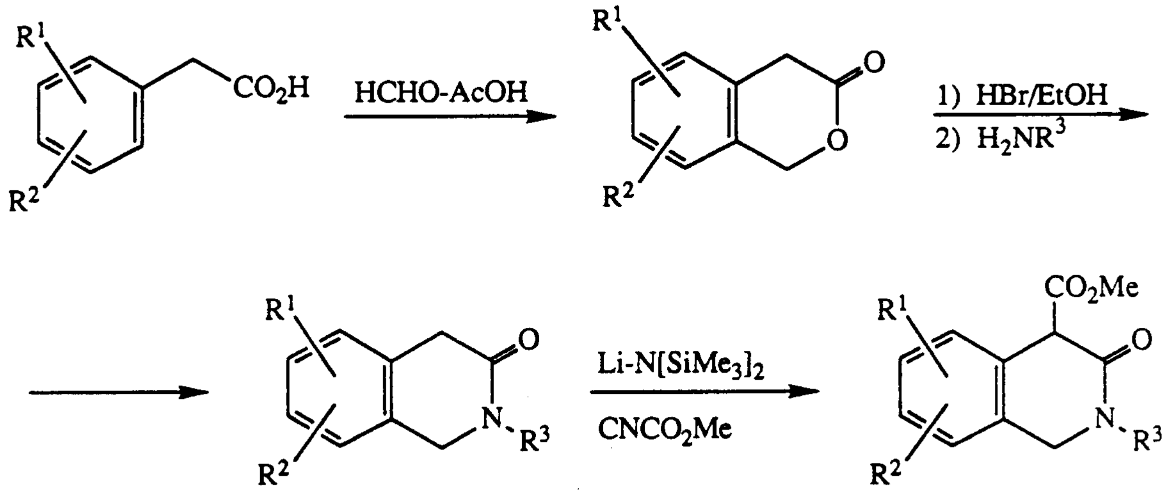


**J. Het. Chem., Vol. 4, No. 3, 315 (1967).**

Other compounds of the present invention of formula (I) wherein M is a carbonyl and W is a sulfonyl can be made by the Process D

**Process D**

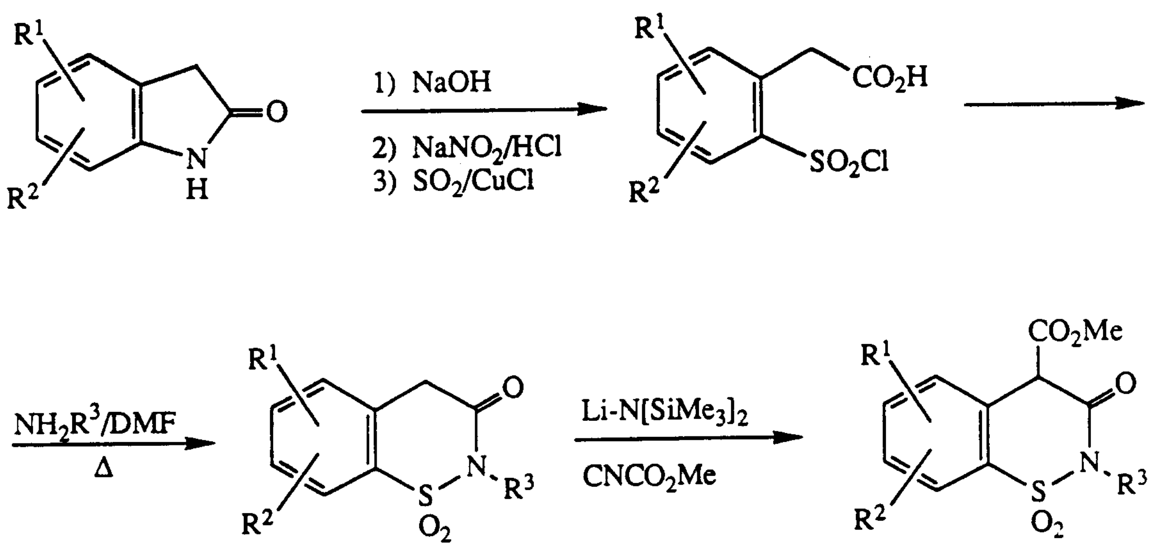


Other compounds of the present invention of formula (I) wherein M is sulfonyl and W is carbonyl can be made by the Process E

Process E

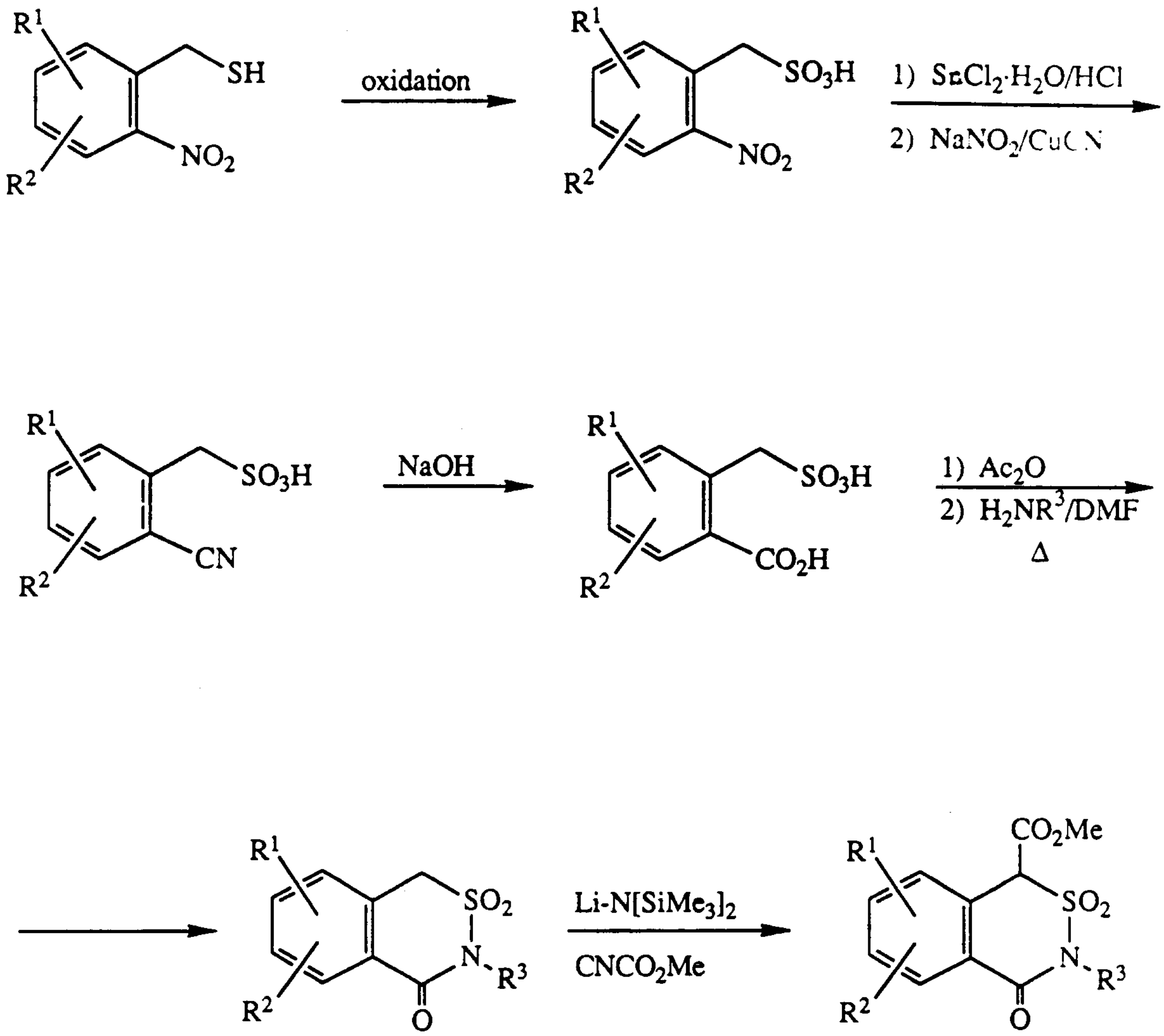


Other compounds of the present invention of formula (I) wherein M is carbonyl and W is thiocarbonyl can be made by the Process F

**Process F**

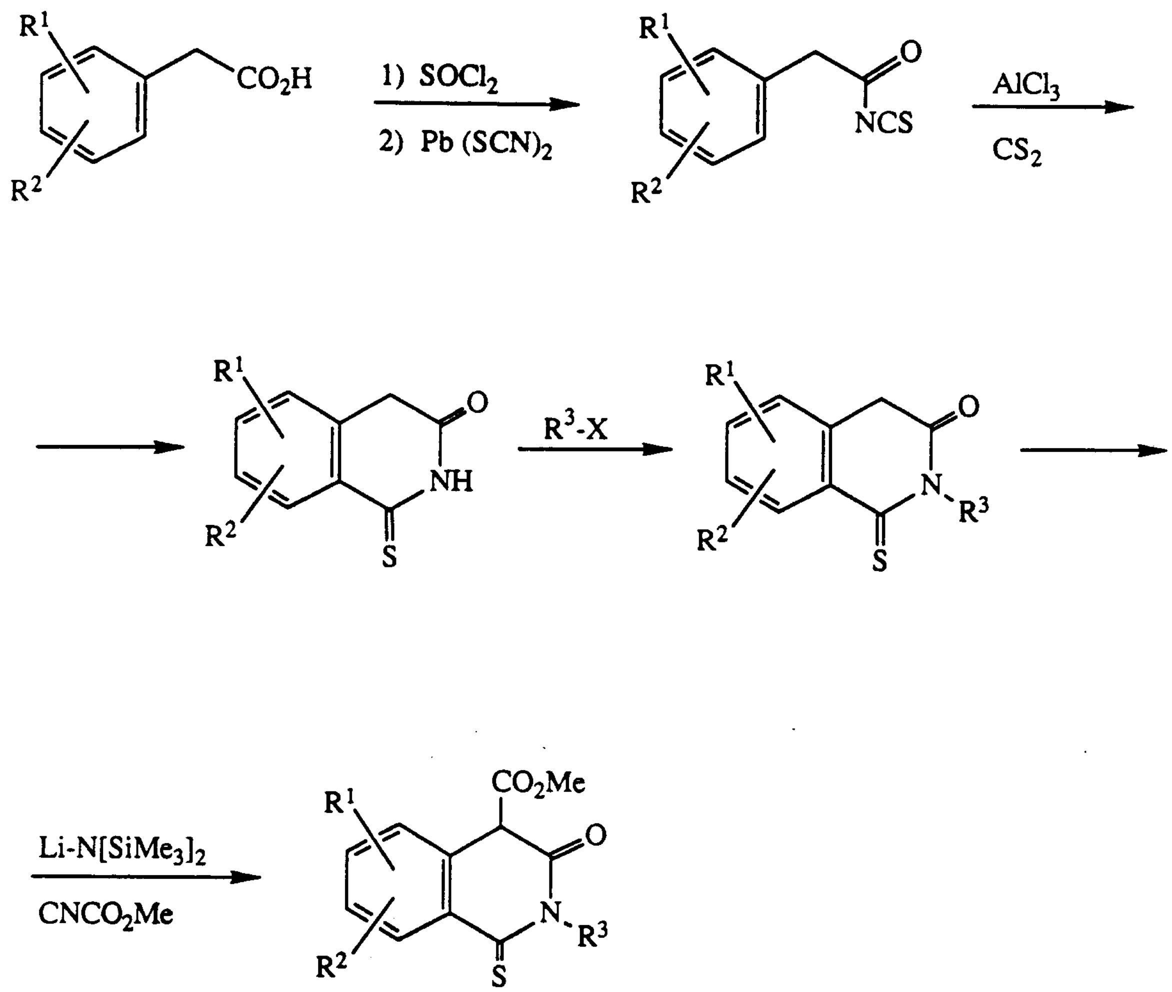


Other compounds of the present invention of formula (I) wherein M and W are carbonyl, Y and Z are oxygen and X is oxygen, sulfur or nitrogen can be made by the Process G

Process G

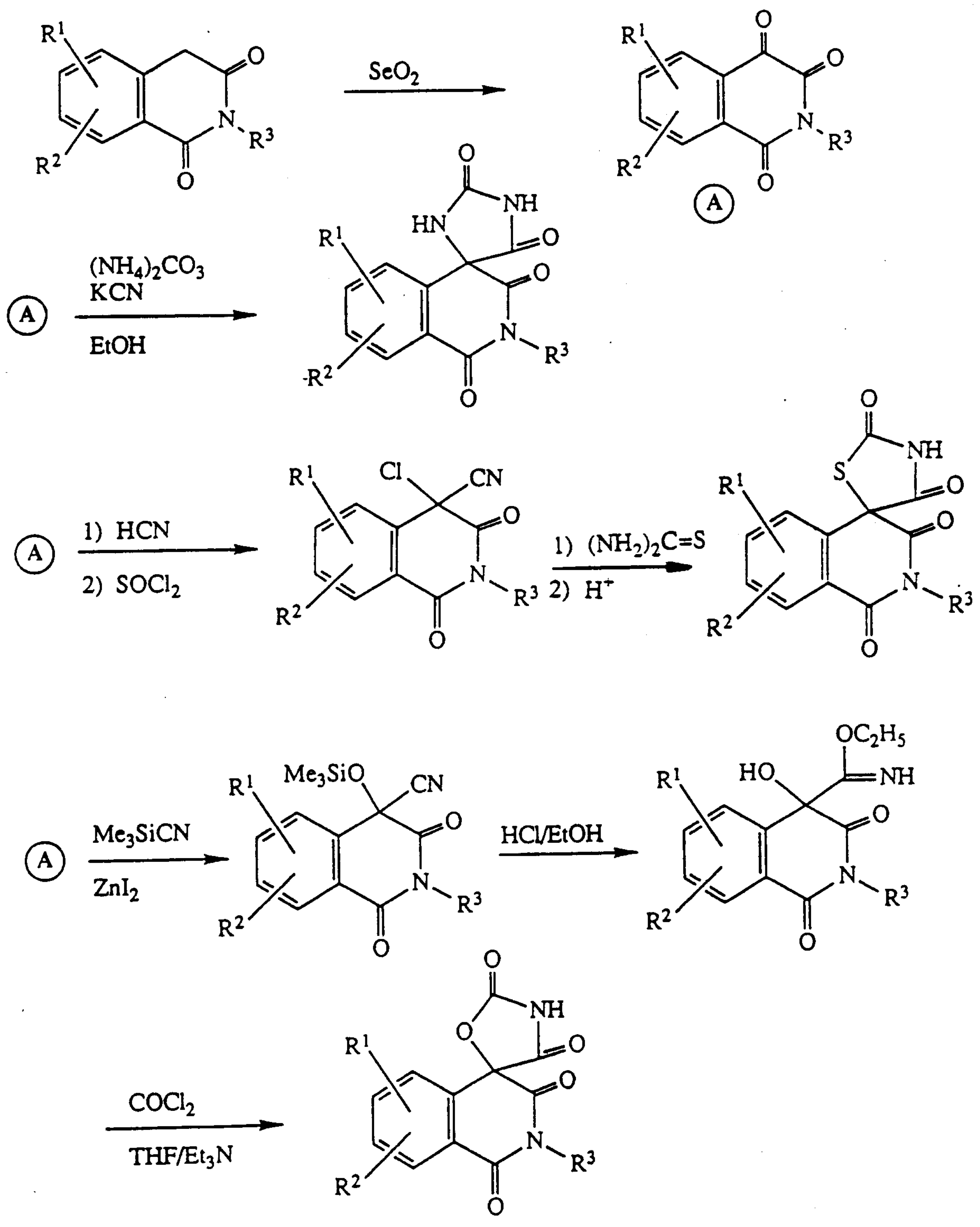


Can. Journal of Chem. 47 858 (1969);
J. Het. Chem. 18, 143 (1981);
Tetrahedron. 36, 943 (1980);
J. Org. Chem. 25, 1920 (1960);
Chem. Pharm. Bull. 30 (10), 3601 (1982);
J. Med. Chem. 29, 770 (1986).

Other compounds of the present invention of formula (I) wherein M and W are carbonyl and wherein the fused benzene ring is replaced by a thiophene can be made by the Process I

**Process I**

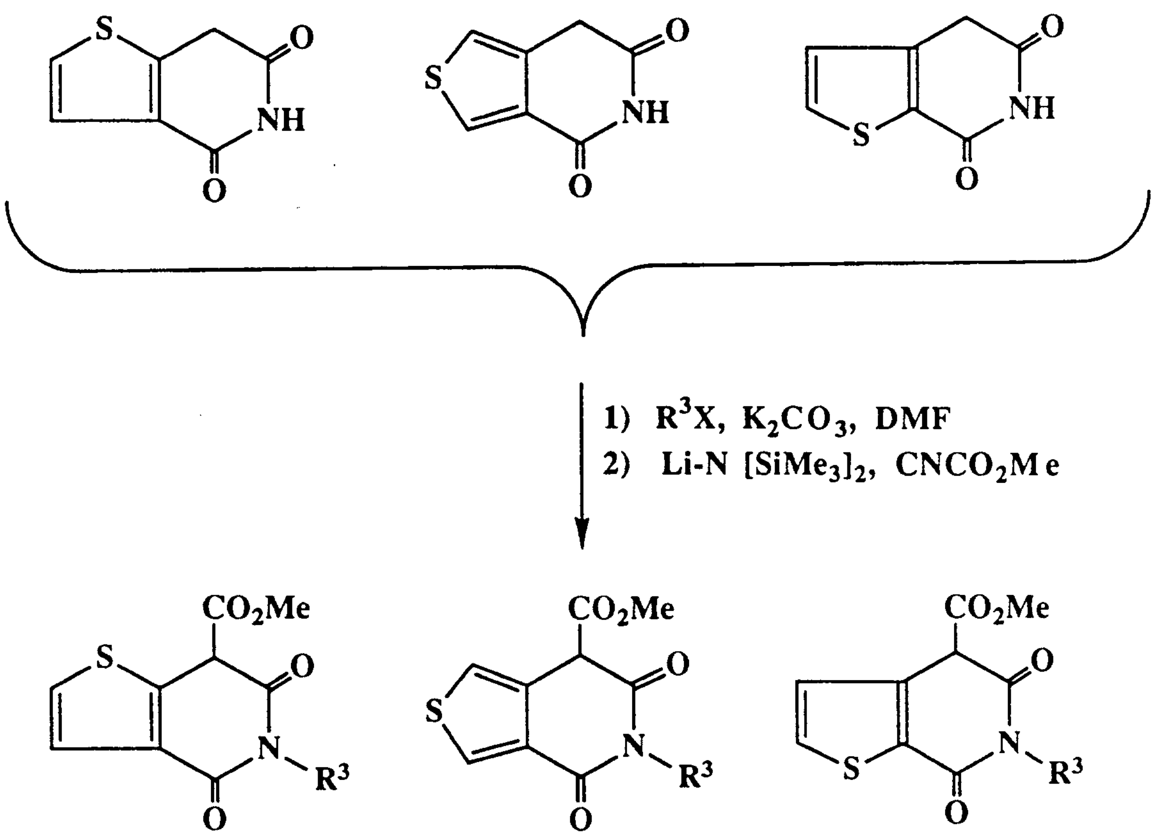


J. Chem. Soc. Perkin I, 1390 (1975);
OPPI BRIEFS 8 (4), 286 (1986).

Other compounds of the present invention of formula (I) wherein M and W are carbonyl and wherein the fused benzene ring is replaced by pyridine can be made by the Process J

**Process J**

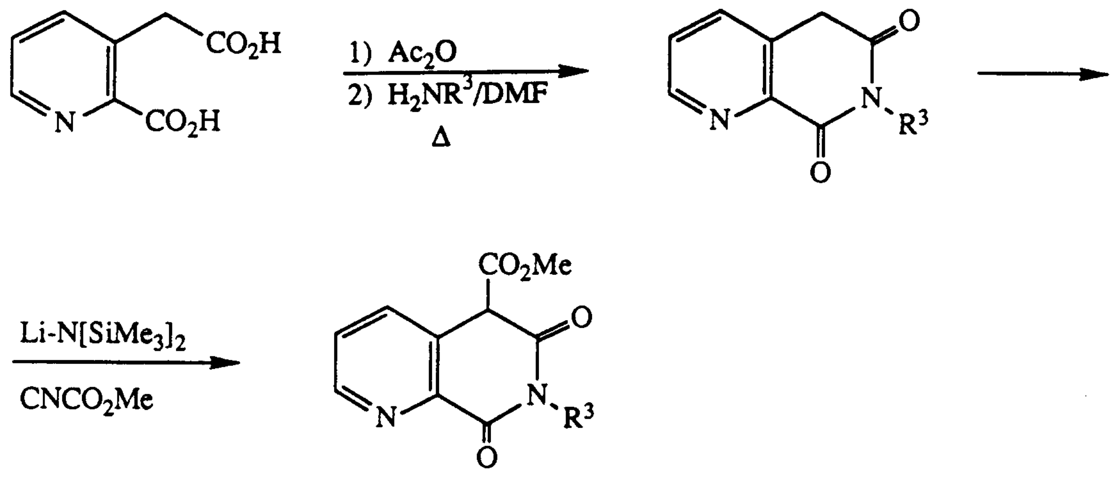


Chem. Abst. 41: 780g;
US. Patent 2,408,020.

Other compounds of the present invention of formula (I) wherein M and W are sulfonyl can be made by the Process K

**Process K**

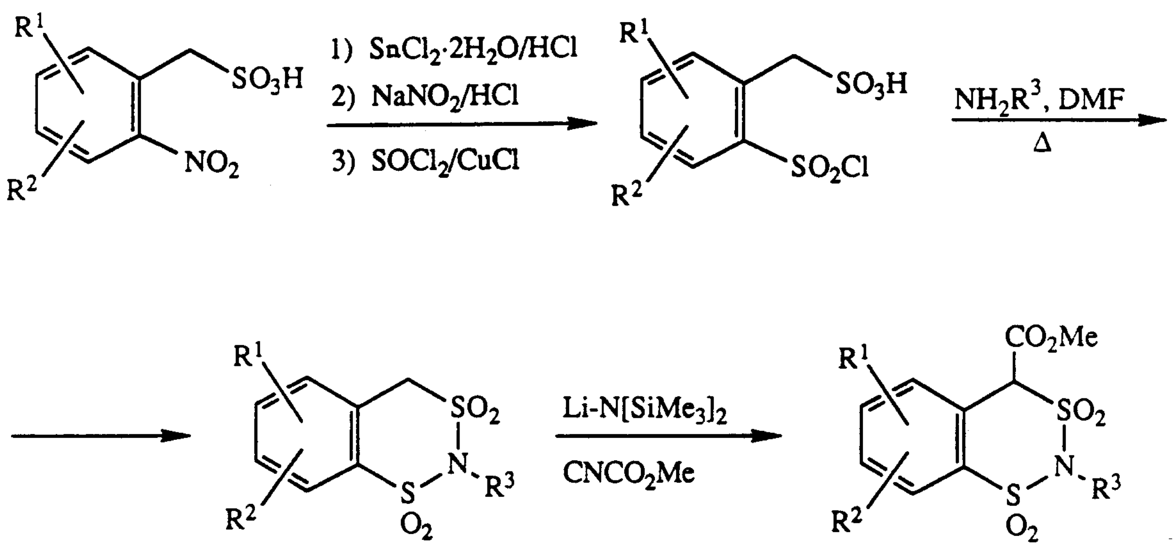


The carbomethoxy intermediate produced by Process C, D, E, F, I, J and K may be converted to compounds of formula (I) by Process A.

The following examples further illustrate this invention:

**EXAMPLE 1**

2-[(3,4-Dichlorophenyl)methyl]spiro[isoquinoline-4(1H),3'-pyrrolidine]-1,2',3,5'(2H)-tetrone

**Step a)** Preparation of 2-[(3,4-Dichlorophenyl)methyl]-1,3(2H, 4H)-isoquinolinedione

To a mixture of homophthalic anhydride (20.0 g, 123.45 mmol) in DMF (300 mL) was added 3,4-dichlorobenzylamine (25.0 g, 142.04 mmol) and the mixture was stirred at 180 °C for 5 hours. After cooling

to room temperature the mixture was poured into $H_2O$, extracted with EtOAc and dried over $MgS0_4$. The crude product was recrystallized from EtOAc/$Et_2O$/hexane (at 0° C) to give a brownish solid m.p. 129-130°C (25.21 g, 57.5 %).
NMR (DMSO-$d_6$, 400 MHz): δ 4.22 (s, 2H, -$\underline{CH_2}$CO-), 5.0 (s, 2H, -N$\underline{CH_2}$-), 7.3 (dd, J=8.49 Hz, 1.98 Hz, 1N, Ar-$\underline{H}$), 7.4 (d, J=8.54 Hz, 1H, Ar-$\underline{H}$), 7.47 (t, J=7.4 Hz, 1H, Ar-$\underline{H}$), 7.54 (d, J=8.3 Hz, 1H, Ar-$\underline{H}$), 7.57 (d, J=1.78 Hz, 1H, Ar-$\underline{H}$), 7.67 (dt, J=8.37 Hz, 0.97 Hz, 1H, Ar-$\underline{H}$), 8.03 (d, J=7.83 Hz, 1H, Ar-$\underline{H}$)
IR (KBr, $cm^{-1}$): 1650(s), 1330(s), 970(m), 740(m)
MS (m/e): 319($M^+$), 256($M^+$-Cl, CO), 118($M^+$-CONH, -$CH_2C_6H_3Cl_2$)

| | | | |
|---|---|---|---|
| Anal. Calcd.: | C, 60.02; | H, 3.46; | N, 4.37 |
| Found: | C, 59.81; | H, 3.60; | N, 4.36. |

The following compounds were obtained in substantially the same manner as that of Example 1, Step a).

2-[(4-Bromo-2-fluorophenyl)methyl]-1,3(2H, 4H)-isoquinolinedione

NMR (DMSO-$d_6$, 200 MHz): δ 4.24 (s, 2H, -$\underline{CH_2}$CON-), 5.04 (s, 2H, -N$\underline{CH_2}$-), 7.23 (t, J=7.8 Hz, 1H, Ar-$\underline{H}$), 7.3 (d, J=7.65 Hz, 1H, Ar-$\underline{H}$), 7.4-7.56 (m, 3H, Ar-$\underline{H}$), 7.65 (t, J=7.6 Hz, Ar-$\underline{H}$), 8.06 (d, J=7.8 Hz, 1H, Ar-$\underline{H}$)
IR (KBr, $cm^{-1}$): 1675(s), 1350(m), 975(m)
MS (m/e): 347($M^+$)

| | | | |
|---|---|---|---|
| Anal. Calcd.: | C, 55.19; | H, 3.18; | N, 4.02 |
| Found: | C, 54.83; | H, 3.14; | N, 4.07 |

M.P. 128-129°C.

2[(4-Bromo-2-fluorophenyl)methyl]-6-chloro-1,3(2H, 4H)-isoquinolinedione

NMR (DMSO-$d_6$, 400MHz): δ 4.23 (s, 2H, -$\underline{CH_2}$CON-), 5.0 (s, 2H, -N$\underline{CH_2}C_6H_3$BrF), 7.19 (t, J=8.20 Hz, 1H, Ar-$\underline{H}$), 7.29 (dd, J=8.32 Hz, 1.71 Hz, 1H, Ar-$\underline{H}$), 7.5-7.56 (m, 3H, Ar-$\underline{H}$), 8.01 (d, J=8.31 Hz, 1H, Ar-$\underline{H}$)
IR (KBr, $cm^{-1}$): 3400 (m), 1710 (m), 1660 (s), 1480 (m), 740 (m)
MS (m/e): 381 ($M^+$)

| | | | |
|---|---|---|---|
| Anal. Calcd.: | C, 50.23; | H, 2.63; | N, 3.66 |
| Found: | C, 50.11; | H, 2.60; | N, 3.63 |

M.P. 157-159°C.

2-[(4-Bromo-2-fluorophenyl)methyl]-7-chloro-1,3(2H, 4H)-isoquinolinedione

NMR (DMSO-$d_6$, 400MHz): δ 4.21 (s, 2H, -$\underline{CH_2}$CON-), 5.01 (s, 2H, -N$\underline{CH_2}C_6H_3$BrF), 7.2 (t, J=8.2 Hz, 1H, Ar-$\underline{H}$), 7.29 (dd, J=8.35 Hz, 1.85 Hz, 1H, Ar-$\underline{H}$) 7.46 (d, J=8.27 Hz, 1H, Ar-$\underline{H}$), 7.51 (dd, J=9.9 Hz, 1.8 Hz, 1H, Ar-$\underline{H}$), 7.75 (dd, J=8.3 Hz, 2.3 Hz, 1H, Ar-$\underline{H}$), 7.97 (d, J=2.3 Hz, 1H, Ar-$\underline{H}$)
IR (KBr, $cm^{-1}$): 3420(m), 1715 (s), 1680 (s), 1645(s), 1480 (m)
MS (m/e): 381($M^+$)

| | | | |
|---|---|---|---|
| Anal. Calcd.: | C, 50.23; | H, 2.63; | N, 3.66 |
| Found: | C, 50.11; | H, 2.58; | N, 3.62 |

M.P. 152-154°C.

2-[(4-Bromo-2-fluorophenyl)methyl]-6-fluoro-1,3(2H, 3H)-isoquinolinedione

NMR (DMSO-$d_6$, 400 MHz): δ 4.24 (s, 2H, Ar$CH_2$CO-), 5.01(s, 2H, -$NCH_2$-), 7.19 (t, J=8.24 Hz, 1H, Ar-H), 7.3-7.35 (m, 3H, Ar-H), 7.51 (dd, J=9.87 Hz, 1.87 Hz, 1H, Ar-H), 8.09 (dd, J=8.4 Hz, 5.94 Hz, 1H, Ar-H)
IR (KBr, $cm^{-1}$): 1660 (s), 1340 (s), 760 (m)
MS (m/e): 365 ($M^+$), 337 ($M^+$-CO)

| | | | |
|---|---|---|---|
| Anal. Calcd.: | C, 52.48; | H, 2.75; | N, 3.83 |
| Found: | C, 52.55; | H, 2.91; | N, 3.73 |

M.P. 146-147 °C.

**Step b)** Preperation of 2-[(3,4-Dichlorophenyl)methyl]-1,2,3,4-tetrahydro-1,3-dioxo-4-isoquinolinecarboxylic Acid Methyl Ester

To a cold (-78 °C) solution of 2-[(3,4-dichlorophenyl)methyl]-1,3(2H, 4H)-isoquinolinedione (11.2 g, 35.0 mmol) in anhydrous THF (100 mL) was added lithium bis(trimethylsilyl)amide (42.0 mL, 42 mmol, 1.0 M in THF) dropwise over a 10 minute period. After stirring for 3 hours, methyl cyanoformate (3.33 mL, 42.0 mmol) was added and the reaction mixture was allowed to warm up to room temperature. The mixture during that period turned a dark color. It was stirred an additional 30 minutes and quenched with $H_2O$. The dark solution was poured into $H_2O$, acidified with HCl (2N), extracted with EtOAc and dried over $MgSO_4$. The crude product was recrystallized from EtOAc/hexane (at -20 ° C) to yield a yellow solid m.p. 164-166 °C (7.5 g, 56.6%).
NMR (DMSO-$d_6$, 400 MHz): δ [3.67 (s), 3.99 (s), tautomeric, 3H, -$CO_2CH_3$], [5.07 (q), J=15.4 Hz, 5.28 (s), tautomeric, 2H, -$NCH_2$-], [7.27-8.43 (m), tautomeric, 7H, Ar-H]
IR (KBr, $cm^{-1}$): 1680 (s), 1610 (s), 1490 (s), 1355 (s), 1015 (s), 790 (m)
MS (m/e): 377 ($M^+$)

| | | | |
|---|---|---|---|
| Anal. Calcd.: | C, 57.16; | H, 3.46; | N, 3.70 |
| Found: | C, 57.03; | H, 3.39; | N, 3.68. |

The following compounds were obtained in substantially the same manner as that of Example 1, Step b).

2-[(4-Bromo-2-fluorophenyl)methyl]-1,2,3,4-tetrahydro-1,3-dioxo-4-isoquinoline-carboxylic Acid Methyl Ester

NMR (DMSO-$d_6$, 400 MHz): δ [3.67 (s), 3.99 (s), tautomeric, -$CO_2$Me,3H], [5.06 (q), J=15.4 Hz, 5.29 (s), tautomeric, $NCH_2$, 2H], 7.07-8.44 (m, tautomeric, Ar-H, 7H)
IR (KBr, $cm^{-1}$) 1675 (s), 1610 (s), 1490 (s), 1350 (m), 875 (m).
MS (m/e): 405 ($M^+$), 187[$M^+$-(4-bromo-2-fluorophenyl)methyl]

| | | | |
|---|---|---|---|
| Anal. Calcd.: | C, 53.22; | H, 3.23; | N, 3.45 |
| Found: | C, 53.06; | H, 3.11; | N, 3.34 |

M.P. 149-150 °C.

2-[(4-Bromo-2-fluorophenyl)methyl]-7-chloro-1,2,3,4-tetrahydro-1,3-dioxo-4-isoquinolinecarboxylic Acid Methyl Ester

NMR (DMSO-$d_6$, 400 MHz): δ [3.66 (s), 3.98 (s), 3H, tautomeric, -$CO_2CH_3$], [5.06 (q), 5.28 (s), 2H, tautomeric, -$NCH_2C_6H_3$BrF], 7.1-8.4 (6H, tautomeric, Ar-H)
IR(KBr, $cm^{-1}$): 3420 (m), 1670 (s), 1600 (s), 1480 (s), 790 (m).
MS (m/e): 439 ($M^+$)

| | | | |
|---|---|---|---|
| Anal. Calcd.: | C, 49.06; | H, 2.74; | N, 3.18 |
| Found: | C, 48.96; | H, 2.80; | N, 3.18 |

M.P. 161-162 ° C.

2[(4-Bromo-2-fluorophenyl)methyl]-6-chloro-1,2,3,4-tetrahydro-1,3-dioxo-4-isoquinolinecarboxylic Acid Methyl Ester

NMR (DMSO-$d_6$, 400 MHz): δ [3.68 (s), 3.98 (s), tautomeric, 3H, -$CO_2CH_3$], [5.0 (q), 5.26 (s), tautomeric, 2H, -N$CH_2C_6H_3$BrF], 7.0-8.4 (m, tautomeric, 6H, Ar-H)
IR (KBr, $cm^{-1}$): 3430 (m), 1670 (s), 1600 (s), 1480 (m), 785 (m)
MS (m/e): 439 ($M^+$)

| | | | |
|---|---|---|---|
| Anal. Calcd.: | C, 49.06; | H, 2.74; | N, 3.18 |
| Found: | C, 49.07; | H, 2.79; | N, 3.08 |

M.P. 181-182 ° C.

6-Chloro-1,2,3,4-tetrahydro-2-methyl-1,3-dioxo-4-isoquinolinecarboxylic Acid Methyl Ester

NMR (DMSO-$d_6$, 200 MHz): δ [3.23 (s), 3.44 (s), tautomeric, 3H, -N$CH_3$], [3.71 (s), 4.03 (s), tautomeric, 3H, -$CO_2CH_3$], 7.3-8.4 (tautomeric, Ar-H, 3H)
IR (KBr, $cm^{-1}$): 3440 (m), 1680 (s), 1600 (s), 1440 (m), 1320 (s), 790 (m)
MS (m/e): 267($M^+$), 235 ($M^+$-OMe)

| | | | |
|---|---|---|---|
| Anal. Calcd.: | C, 53.85; | H, 3.77; | N, 5.23 |
| Found: | C, 53.66; | H, 3.63; | N, 5.14 |

M.P. 166-167 ° C.

7-Chloro-1,2,3,4-tetrahydro-2-methyl-1,3-dioxo-4-isoquinolinecarboxylic Acid Methyl Ester

NMR (DMSO-$d_6$, 200 MHz): δ [3.22 (s), 3.45 (s), tautomeric, 3H, N-$CH_3$], [3.7 (s), 4.02 (s), tautomeric, 3H, -$CO_2CH_3$], 7.5-8.4 (tautomeric, Ar-H, 3H)
IR (KBr, $cm^{-1}$): 3440 (m), 1770 (s), 1600 (s), 1330 (s), 830 (s), 795 (m)
MS (m/e): 267 ($M^+$), 235 ($M^+$-OMe-H)

| | | | |
|---|---|---|---|
| Anal. Calcd. for ($C_{12}H_{10}ClNO_4$x1/3 $H_2O$): | C, 52.65; | H, 3.90; | N, 5.12 |
| Found: | C, 52.64; | H, 3.68; | N, 5.18 |

M.P. 158-159 ° C.

1,2,3,4-Tetrahydro-2-methyl-1,3-dioxo-4-isoquinolinecarboxylic Acid Methyl Ester

NMR (DMSO-$d_6$, 200 MHz): δ [3.24 (s), 3.46 (s), tautomeric, 3H, -N$CH_3$], [3.7 (s), 4.03 (s), tautomeric, 3H, -$CO_2CH_3$], 7.4-8.45 (tautomeric, 4H, Ar-H)
IR (KBr, $cm^{-1}$): 3400 (brm), 1670 (s), 1600 (s), 1420 (m), 780 (m)
MS (m/e): 233 ($M^+$), 118 ($M^+$-$CO_2$Me, -CON$CH_3$)

| | | | |
|---|---|---|---|
| Anal. Calcd.: | C, 61.80; | H, 4.75; | N, 6.01 |
| Found: | C, 61.62; | H, 4.89; | N, 5.92 |

M.P. 130-131 ° C.

**Step c)** Preparation of 2-[(3,4-Dichlorophenyl)methyl]-1,2,3,4-tetrahydro-4-(methoxycarbonyl)-1,3-dioxo-4-isoquinolineacetic Acid 1,1-Dimethylethyl Ester

To a suspension of 2-[(3,4-dichlorophenyl)methyl]-1,2,3,4-tetrahydro-1,3-dioxo-4-isoquinolinecarboxylic acid methyl ester (6.3 g, 16.66 mmol), $K_2CO_3$ (3.45 g, 24.99 mmol) in DMF (100 mL) was added tert-butyl bromoacetate (4.03 mL, 24.99 mmol). After stirring at 70°C for 1 hour, the mixture was poured into $H_2O$, extracted with EtOAc and dried over $MgSO_4$. The crude product was purified by flash chromatography (hexane/EtOAc 4:1) to yield a yellowish oil (5.9 g, 71.9%).

NMR (DMSO-$d_6$, 400 MHz): δ 1.02 (s, 9H, $CO_2$-tertbutyl), 3.56 (s, 3H, -$CO_2CH_3$), 3.62 (q, J=17.2 Hz, 2H, -$CH_2CO_2$-tertbutyl), 5.14 (s, 2H, -N$CH_2$-), 7.32 (dd, J=8.42 Hz, 2.04 Hz, 1H, Ar-H), 7.52 (d, J=2.03 Hz, 1H, Ar-H), 7.59-7.62 (m, 3H, Ar-H), 7.78 (dt, J=7.64 Hz, 1.39 Hz, 1H, Ar-H), 8.19 (dd, J=8.51 Hz, 1.66 Hz, 1H, Ar-H)

IR ($CHCl_3$, $cm^{-1}$): 1750 (s), 1720 (s), 1675 (s), 1355 (m), 1140 (s), 740 (m)

MS (m/e): 491 ($M^+$), 304 ($M^+$-CONH, -$CH_2C_6H_3Cl_2$)

The following compounds were obtained in substantially the same manner as that of Example 1, Step c).

2-[(4-Bromo-2-fluorophenyl)methyl]-1,2,3,4-tetrahydro-4-(methoxycarbonyl)-1,3-dioxo-4-isoquinolineacetic Acid 1,1-Dimethylethyl Ester

NMR (DMSO-$D_6$, 400 MHz): δ 1.04 [s, 9H, -C($CH_3$)$_3$], 3.53 (s, 3H, -$CO_2CH_3$),3.60 [dd, J=17.7 Hz, 2H, -$CH_2CO_2C(CH_3)_3$], 5.14 (s, 2H, N$CH_2$-), 7.17 (t, J=8.25 Hz, 1H, Ar-H), 7.36 (dd, J=8.36 Hz, 1.75 Hz, 1H, Ar-H), 7.6 (m, 3H, Ar-H), 7.77 (dt, J=7.2 Hz, 1.27 Hz, 1H, Ar-H), 8.19 (dd, J=8.25 Hz, 1.54 Hz, 1H Ar-H)

IR ($CHCl_3$, $cm^{-1}$): 1720 (s), 1675 (s), 1360 (s), 765 (m)

MS (m/e): 520 $(M+H)^+$, 464 [$M^+$-$C(CH_3)_3$].

1,2,3,4-Tetrahydro-4-(methoxycarbonyl)-2-methyl-1,3-dioxo-4-isoquinoline-acetic Acid 1,1-Dimethylethyl Ester

NMR (DMSO-$d_6$, 200 MHz): δ 1.01 (s, 9H, -$CO_2$-tertbutyl), 3.31 (s, 3H, N-$CH_3$), 3.58 (m, 5H, -$CO_2CH_3$, -$CH_2CO_2$-tertbutyl), 7.61 (m, 2H), 7.74 (dt, J=7.6 Hz, 1.6 Hz, 1H, Ar-H), 8.18 (dd, J=8.4Hz, 2.0Hz, 1H, Ar-H)

IR (KBr, $cm^{-1}$): 3420 (m), 1750 (s), 1730 (s), 1670 (s), 1465 (m), 740 (m)

MS (m/e): 347 ($M^+$)

| | | | |
|---|---|---|---|
| Anal. Calcd.: | C, 62.24; | H, 6.09; | N, 4.03 |
| Found: | C, 62.20; | H, 6.08; | N, 4.02 |

M.P. 89-90° C.

6-Chloro-1,2,3,4-tetrahydro-4-(methoxycarbonyl)-2-methyl-1,3-dioxo-4-isoquinolineacetic Acid 1,1-Dimethylethyl Ester

NMR (DMSO-$d_6$, 200 MHz): δ 1.06 (s, 9H, -$CO_2$-tertbutyl), 3.3 (s, 3H, -N$CH_3$), 3.6 (s, 3H, -$CO_2CH_3$), 3.67 (q, J=17.5 Hz, 2H, -$CH_2CO_2$-tertbutyl), 7.68 (dd, J=9.0 Hz, 1.6 Hz, 1H, Ar-H), 7.77 (d, J=2.0 Hz, 1H, Ar-H), 8.21 (d, J=8.2 Hz, 1H, Ar-H)

IR (KBr, $cm^{-1}$): 3440 (m), 1740 (s), 1720 (s), 1680 (s), 1360 (s), 1060 (s), 770 (m)

MS (m/e): 381 ($M^+$)

| | | | |
|---|---|---|---|
| Anal. Calcd.: | C, 56.82; | H, 5.28; | N, 3.67 |
| Found: | C, 57.00; | H, 5.41; | N, 3.66 |

M.P. 135-136°C.

7-Chloro-1,2,3,4-tetrahydro-4-(methoxycarbonyl)-2-methyl-1,3-dioxo-4-isoquinolineacetic Acid 1,1-Dimethylethyl Ester

NMR (DMSO-$d_6$, 200 MHz): δ 1.06 (s, 9H, $CO_2$-tertbutyl), 3.31 (s, 3H, -$NCH_3$), 3.59 (s, 5H, -$CO_2CH_3$, -$CH_2CO_2$-tertbutyl), 7.65 (d, J = 8.4 Hz, 1H, Ar-H), 7.84 (d, J = 8.2 Hz, 2.8 Hz, 1H, Ar-H), 8.15 (d, J = 2.2 Hz, 1H, Ar-H)
IR (KBr, $cm^{-1}$): 3440 (m), 1760 (s), 1740 (m), 1725 (m), 1680 (s), 1440 (m), 770 (m)
MS (m/e): 381 ($M^+$), 308 ($M^+$-O-tertbutyl)

| | | | |
|---|---|---|---|
| Anal. Calcd.: | C, 56.62; | H, 5.28; | N, 3.67 |
| Found: | C, 56.44; | H, 5.23; | N, 3.72 |

M.P. 103-104 °C.

2-Ethyl-1,2,3,4-tetrahydro-4-(methoxycarbonyl)-1,3-dioxo-4-isoquinolineacetic Acid 1,1-Dimethylethyl Ester

NMR (DMSO-$d_6$, 200 MHz): δ 1.04 (s, 9H, $CO_2$-tertbutyl), 1.14 (t, J = 7.0 Hz, 3H, -$NCH_2CH_3$), 3.58 (s, 2H, -$CH_2CO$-), 4.0 (q, J = 7.0 Hz, 2H, -$NCH_2CH_3$), 7.59-7.61 (m, 2H, Ar-H), 7.75 (t, J = 7.8 Hz, 1H, Ar-H), 8.18 (d, J = 7.9 Hz, 1H, Ar-H)
IR (KBr, $cm^{-1}$): 1730 (s), 1670 (s), 1465 (m), 1160 (s), 710 (m)
MS (m/e): 361 ($M^+$), 305 ($M^+$-$C_4H_8$), 288 ($M^+$-O-tertbutyl), 246 ($M^+$-$CH_2CO_2$-tertbutyl).

6-Bromo-1,2,3,4-tetrahydro-4-(methoxycarbonyl)-2-methyl-1,3-dioxo-4-isoquinolineacetic Acid 1,1-Dimethylethyl Ester

NMR (DMSO-$d_6$, 200 MHz): δ 1.05 [s, 9H, -$C(CH_3)_3$], 3.28 (s, 3H, -$NCH_3$), 3.59 (s, 3H, -$CO_2CH_3$), 3.58 (d, J = 17.03 Hz, 1H, -$CH_2CO_2$-), 3.67 (d, J = 17.03 Hz, 1H, -$CH_2CO_2$-), 7.81 (dd, J = 8.4 Hz, 1.85 Hz, 1H, Ar-H), 7.88 (d, J = 1.81 Hz, 1H, Ar-H), 8.08 (d, J = 8.4 Hz, 1H, Ar-H)
IR (KBr, $cm^{-1}$): 1740 (s), 1710 (s), 1670 (s), 755 (m)
MS (m/e): 425 ($M^+$), 370 ($M^+$-$C_4H_7$), 352 ($M^+$-$C_4H_9O$)

| | | | |
|---|---|---|---|
| Anal. Calcd.: | C, 50.72; | H, 4.73; | N, 3.29 |
| Found: | C, 50.47; | H, 4.68; | N, 3.12 |

M.P. 152-153 °C.

**Step d)** Preparation of 2-[(3,4-Dichlorophenyl)methyl]-1,2,3,4-tetrahydro-4-(methoxycarbonyl)-1,3-dioxo-4-isoquinolineacetic Acid

A mixture of 2-[(3,4-dichlorophenyl)methyl]-1,2,3,4-tetrahydro-4-(methoxycarbonyl)-1,3-dioxo-4-isoquinolineacetic acid 1,1-dimethylethyl ester (5.8 g, 11.78 mmol), $CH_2Cl_2$ (50 mL) and $CF_3CO_2H$ (10 mL) was stirred at room temperature for 10 hours. The volatiles were removed in vacuo, and the residue was recrystallized from acetone/ether/hexane (at -20 ° C) to yield a white solid m.p. 153-154 °C (4.0 g, 89.5%).
NMR (DMSO-$d_6$, 400 MHz): δ 3.56 (s, 3H, -$CO_2CH_3$), 3.65 (q, J = 17.7 Hz, 2H, -$CH_2$ $CO_2H$), 5.12 (q, J = 15.4 Hz, 2H, -$NCH_2$-), 7.27 (dd, J = 8.35 Hz, 1.97 Hz, 1H, Ar-H), 7.52 (d, J = 1.92 Hz, 1H, Ar-H); 7.58-7.62 (m, 3H), 7.76 (dt, J = 7.35 Hz, 0.87 Hz, 1H, Ar-H), 8.16 (dd, J = 7.82 Hz, 1.16 Hz, 1H, Ar-H)
IR (KBr, $cm^{-1}$): 3430 (m), 3500-2500 (br), 1755 (s), 1710 (s), 1670 (s), 1350 (m), 1230 (s), 745 (m)
MS (m/e): 435 ($M^+$)

| | | | |
|---|---|---|---|
| Anal. Calcd.: | C, 55.07; | H, 3.47; | N, 3.21 |
| Found: | C, 55.15; | H, 3.84; | N, 2.94. |

The following compounds were obtained in substantially the same manner as that of Example 1, Step d).

2-[(4-Bromo-2-fluorophenyl)methyl]-1,2,3,4-tetrahydro-4-(methoxycarbonyl)-1,3-dioxo-4-isoquinolineacetic Acid

NMR (DMSO-$d_6$, 400 MHz): δ 3.54 (s, 3H, $-CO_2CH_3$), 3.64 (q, J = 17.67 Hz, 2H, $-CH_2$ $CO_2H$), 5.12 (q, J = 15.34 Hz, 2H, $-NCH_2-$), 7.14 (t, J = 8.22 Hz, 1H, Ar-H), 7.3 (d, J = 8.3 Hz, 1H, Ar-H), 7.5-7.6 (m, 3H, Ar-H), 7.76 (d, 7.4 Hz, 1H, Ar-H), 8.16 (d, J = 7.8 Hz, 1H, Ar-H), 12.35 (s, 1H, $-CO_2H$)
IR (KBr, $cm^{-1}$): 3280 (m), 3500-2500 (br), 1750 (s), 1720 (s), 1675 (s), 1350 (s), 875 (m)
MS (m/e): 463 ($M^+$), 445 ($M^+$-H,-OH)

| | | | |
|---|---|---|---|
| Anal. Calcd. for $C_{20}H_{15}BrFNO_6 \cdot 0.2H_20$: | C, 51.28; | H, 3.30; | N, 2.99 |
| Found: | C, 51.26; | H, 3.48; | N, 2.95 |

M.P. 139-140 °C.

2-[(4-Bromo-2-fluorophenyl)methyl]-7-chloro-1,2,3,4-tetrahydro-4-(methoxycarbonyl)-1,3-dioxo-4-isoquinolineacetic Acid

NMR (DMSO-$d_6$, 200 MHz): δ 3.67 (s, 3H, $-CO_2CH_3$), 3.71 (q, J = l7.5 Hz, 2H, $-CH_2CO_2H$), 5.14 (q, J = 17.2 Hz, 2H, $-NCH_2C_6H_3BrF$), 7.15 (t, J = 8.4 Hz, 1H, Ar-H), 7.36 (dd, J = 8.2 Hz, 2.0 Hz, 1H, Ar-H), 7.55 (dd, J = 9.8 Hz, 2.0 Hz, 1H, Ar-H), 7.70 (d, J = 8.4 Hz, 1H, Ar-H), 7.87 (dd, J = 8.8 Hz, 2.8 Hz, 1H, Ar-H), 8.14 (d, J = 2.2 Hz, 1H, Ar-H), 12.8 (brs, 1H, $-CO_2H$)
IR (KBr, $cm^{-1}$): 3500-2500 (brs), 1730 (s), 1710 (s), 1665 (s), 1420 (m), 750 (m)

| | | | |
|---|---|---|---|
| Anal. Calcd.: | C, 48.17; | H, 2.83; | N, 2.81 |
| Found: | C, 47.96; | H, 2.85; | N, 2.85 |

M.P. 86-88 °C.

2-[(4-Bromo-2-fluorophenyl)methyl]-6-chloro-1,2,3,4-tetrahydro-4-(methoxycarbonyl)-1,3-dioxo-4-isoquinolineacetic Acid

NMR (DMSO-$d_6$, 200 MHz): δ 3.58 (s, 3H, $-CO_2CH_3$), 3.68 (d, J = 17.5 Hz, 1H, $-CH_2CO_2H$), 3.85 (d, J = 17.5 Hz, 1H, $-CH_2CO_2H$), 5.15 (s, 2H, $-NCH_2-$), 7.15 (t, J = 8.3 Hz, 1H, Ar-H), 7.35 (d, J = 8.3 Hz, 1H, Ar-H), 7.55 (d, J = 9.6 Hz, 1H, Ar-H), 7.68 (d, J = 8.2 Hz, 1H, Ar-H), 7.85 (s, 1H, Ar-H), 8.2 (d, J = 8.1 Hz, 1H, Ar-H)
MS (m/e): 497 ($M^+$)

| | | | |
|---|---|---|---|
| Anal. Calcd.: | C, 48.17; | H, 2.83; | N, 2.81 |
| Found: | C, 47.85; | H, 2.85; | N, 2.86. |

2-[(4-Bromo-2-fluorophenyl)methyl]-6-fluoro-1,2,3,4-tetrahydro-4-(methoxycarbony])-1,3-dioxo-4-isoquinolineacetic Acid

NMR (DMSO-$d_6$, 400 MHz): δ 3.56 (s, 3H, $-CO_2CH_3$), 3.6 (d, J = 17.9 Hz, 1H, $-CH_2CO_2H$), 3.8 (d, J = 17.9 Hz, 1H, $-CH_2CO_2H$), 5.1 (dd, J = 15.5 Hz, 2H, $-NCH_2-$), 7.12 (t, J = 8.23 Hz, 1H, Ar-H), 7.31 (dd, J = 8.28 Hz, 1.68 Hz, 1H, Ar-H), 7.45 (dt, J = 8.56 Hz, 2.5 Hz, 1H, Ar-H), 7.54 (dd, J = 9.77 Hz, 1.89 Hz, 1H, Ar-H), 7.64 (dd, J = 9.61 Hz, 2.46 Hz, 1H, Ar-H), 8.23 (dd, J = 8.79 Hz, 5.81 Hz, 1H, Ar-H), 12.67 (brs, 1H, $-CO_2H$)
IR (KBr, $cm^{-1}$): 3400-2700 (br), 1745 (s), 1710 (s), 1670 (s), 770 (m)
MS (m/e): 481 (M+), 405 ($M^+$-$CO_2$-$CH_3OH$)

| | | | |
|---|---|---|---|
| Anal. Calcd.: | C, 49.81; | H, 2.93; | N, 2.90 |
| Found: | C, 49.94; | H, 3.03; | N, 2.84 |

M.P. 132-133.5 °C.

1,2,3,4-Tetrahydro-4-(methoxycarbonyl)-2-methyl-1,3-dioxo-4-isoquinolineacetic Acid

NMR (DMSO-$d_6$, 200 MHz): δ 3.28 (s, 3H, N-$\underline{CH_3}$), 3.57 (s, 3H, -$CO_2\underline{CH_3}$), 3.61 (s, 2H, -$\underline{CH_2}CO_2H$), 7.54 (m, 2H, Ar-$\underline{H}$), 7.75 (dt, J = 8.2 Hz, 1.6 Hz, 1H, Ar-$\underline{H}$), 8.18 (d, J = 7.8 Hz, 1H, Ar-$\underline{H}$), 12.3 (brs, 1H, -$CO_2\underline{H}$)
IR (KBr, $cm^{-1}$): 3420 (m), 3500-2500 (brs), 1755 (s), 1730 (s), 1710 (s), 1660 (s), 1455 (m), 745 (m)
MS (m/e): 291 ($M^+$)

| | | | |
|---|---|---|---|
| Anal. Calcd.: | C, 57.73; | H, 4.50; | N, 4.81 |
| Found: | C, 57.55; | H, 4.68; | N, 4.75 |

M.P. 188-189 ° C.

7-Chloro-1,2,3,4-tetrahydro-4-(methoxycarbonyl)-2-methyl-1,3-dioxo-4-isoquinolineacetic Acid

NMR (DMSO-$d_6$, 200 MHz): δ 3.29 (s, 3H, -N$\underline{CH_3}$), 3.59 (s, 3H, -$CO_2\underline{CH_3}$), 3.63 (s, 2H, -$\underline{CH_2}CO_2H$), 7.68 (d, J = 9.0 Hz, 1H, Ar-$\underline{H}$), 7.81 (dd, J = 8.8 Hz, 2.0 Hz, 1H, Ar-$\underline{H}$), 8.12 (d, J = 1.8 Hz, 1H, Ar-$\underline{H}$)
IR (KBr, $cm^{-1}$): 3430 (m), 3500-2500 (brm), 1755 (s), 1720 (s), 1675 (s), 1440 (m), 1240 (s), 770 (m)
MS (m/e): 325 ($M^+$)

| | | | |
|---|---|---|---|
| Anal. Calcd.: | C, 51.63; | H, 3.71; | N, 4.30 |
| Found: | C, 51.41; | H, 3.66; | N, 4.26 |

M.P. 161-162 ° C.

6-Chloro-1,2,3,4-tetrahydro-4-(methoxycarbonyl)-2-methyl-1,3-dioxo-4-isoquinolineacetic Acid

NMR (DMSO-$d_6$, 200 MHz): δ 3.27 (s, 3H, -N$\underline{CH_3}$), 3.59 (s, 3H, -$CO_2\underline{CH_3}$), 3.64 (q, J = 17.5 Hz, 2H, -$\underline{CH_2}CO_2H$), 7.65 (dd, J = 8.6 Hz, 2.0 Hz, 1H, Ar-$\underline{H}$), 7.78 (d, J = 2.0 Hz, 1H, Ar-$\underline{H}$), 8.18 (d, J = 8.0 Hz, 1H, Ar-$\underline{H}$)
IR (KBr, $cm^{-1}$): 3440 (m), 3500-2500 (brm), 1750 (s), 1710 (s), 1675 (s), 1430 (m), 1240 (s), 770 (m)
MS (m/e): 325 ($M^+$)

| | | | |
|---|---|---|---|
| Anal. Calcd.: | C, 51.63; | H,3.71; | N,4.30 |
| Found: | C, 51.73; | H, 3.70; | N, 4.28 |

M.P. 195-196 ° C.

2-Ethyl-1,2,3,4-tetrahydro-4-(methoxycarbonyl)-1,3-dioxo-4-isoquinolineacetic Acid

NMR (DMSO-$d_6$, 400 MHz): δ 1.09 (t, J = 7.04 Hz, 3H, -$NCH_2\underline{CH_3}$), 3.55 (s, 3H, -$CO_2\underline{CH_3}$), 3.94 (q, J = 7.0 Hz, 2H, -N$\underline{CH_2}CH_3$), 7.54-7.58 (m, 2H, Ar-$\underline{H}$), 7.72 (dt, J = 7.6 Hz, 1.48 Hz, 1H, Ar-$\underline{H}$), 8.14 (dd, J = 7.91 Hz, 1.14 Hz, 1H, Ar-$\underline{H}$), 12.6 (brs, 1H,-$CO_2\underline{H}$)
IR (KBr, $cm^{-1}$): 3450 (m), 3500-2500 (brm), 1750 (s), 1710 (s), 1665 (s), 1640 (s), 1465 (m), 745 (m)
MS (m/e): 305 ($M^+$), 246 ($M^+$-$CO_2Me$)

| | | | |
|---|---|---|---|
| Anal. Calcd.: | C, 59.01; | H, 4.95; | N, 4.59 |
| Found: | C, 58.87; | H, 5.05; | N, 4.60 |

M.P. 145-146 ° C.

1,2,3,4-Tetrahydro-4-(methoxycarbonyl)-2-propyl-1,3-dioxo-4-isoquinolineacetic Acid

NMR (DMSO-$d_6$, 200 MHz): δ 0.87 (t, J = 7.0 Hz, 3H, -$NCH_2CH_2\underline{CH_3}$), 1.54-1.62 (m, 2H, -$NCH_2\underline{CH_2}CH_3$), 3.56 (s, 3H, -$CO_2\underline{CH_3}$), 3.62 (s, 2H, -$\underline{CH_2}CO_2H$), 3.91 (t, J = 7.0 Hz, 2H, -N$\underline{CH_2}CH_2CH_3$), 7.5-7.62 (m, 2H, Ar-H), 7.81 (t, J = 7.8 Hz, 1H, Ar-H), 8.15 (d, J = 7.65 Hz, 1H, Ar-H)
IR (KBr, $cm^{-1}$): 3500-2500 (brs), 1750 (s), 1710 (s), 1665 (s), 1430 (m), 750 (m)
MS (m/e): 319 ($M^+$), 260 ($M^+$-$CH_2CO_2H$)

| | | | |
|---|---|---|---|
| Anal. Calcd.: | C, 60.18; | H, 5.37; | N, 4.39 |
| Found: | C, 59.96; | H, 5.28; | N, 4.34 |

M.P. 119-120 °C.

2-Butyl-1,2,3,4-tetrahydro-4-(methoxycarbonyl)-1,3-dioxo-4 isoquinolineacetic Acid

NMR (DMSO-$d_6$, 200 MHz): δ 0.89 (t, J = 7.6 Hz, 3H, -$NCH_2CH_2CH_2CH_3$), 1.21-1.38 (m, 2H, -N,$CH_2CH_2CH_2CH_3$), 1.4-1.6 (m, 1H, -$NCH_2CH_2CH_2CH_3$), 3.56 (s, 3H, -$CO_2CH_3$), 3.62 (s, 2H, -$CH_2CO_2H$), 3.94 (t, J = 7.0 Hz, 2H, -$NCH_2CH_2CH_2CH_3$), 7.61 (m, 2H, Ar-H), 7.74 (dt, J = 8.0 Hz, 1.4 Hz, 1H, Ar-H), 8.18 (dd, J = 8.0 Hz, 1.6 Hz, 1H, Ar-H)
IR (KBr, $cm^{-1}$): 3500-2500 (brs), 1750 (s), 1710 (s), 1640 (s), 1420 (m), 745 (m)
MS (m/e): 333 ($M^+$), 274 ($M^+$-$CH_2CO_2H$)

| | | | |
|---|---|---|---|
| Anal. Calcd.: | C, 61.25; | H, 5.75; | N, 4.20 |
| Found: | C, 61.09; | H, 5.67; | N, 4.23 |

M.P. 133-134 °C.

6-Bromo-1,2,3,4-tetrahydro-4-(methoxycarbonyl)-2-methyl-1,3-dioxo-4-isoquinolineacetic Acid

NMR (DMSO-$d_6$, 200 MHz): δ 3.26 (s, 3H, N-$CH_3$), 3.53 (d, J = 17.2 Hz, 1H, -$CH_2CO_2H$), 3.58 (s, 3H, -$CO_2CH_3$), 3.74 (d, J = 17.2 Hz, 1H, -$CH_2CO_2H$), 7.77 (dd, J = 8.2 Hz, 2.2 Hz, 1H, Ar-H), 7.87 (d, J = 2.2 Hz, 1H, Ar-H), 8.0 (d, J = 8.2 Hz, 1H, Ar-H), 12.64 (s, 1H, -$CO_2H$)
IR (KBr, $cm^{-1}$): 3450-2600 (br), 1735 (s), 1700 (s), 1660 (s)
MS (m/e): 369 ($M^+$), 324 ($M^+$-$CO_2H$)

| | | | |
|---|---|---|---|
| Anal. Calcd.: | C, 45.43; | H, 3.27; | N, 3.78 |
| Found: | C, 45.04; | H, 3.16; | N, 3.62 |

M.P. 194-195 °C.

**Step e)** Preparation of 4-(2-Amino-2-oxoethyl)-2-[(3,4-dichlorophenyl)methyl]-1,2,3,4-tetrahydro-1,3-dioxo-4-isoquinolinecarboxylic Acid Methyl Ester

To a solution of 2-[(3,4-dichlorophenyl)methyl]-1,2,3,4-tetrahydro-4-(methoxycarbonyl)-1,3-dioxo-4-isoquinolineacetic acid (3.6 g, 8.26 mmol) in DMF (50 mL) were added 1-(3-dimethylaminopropyl)-3-ethylcarbodiimide hydrochloride (2.06 g, 10.74 mmol), and 1-hydroxybenzotriazole hydrate (1.67 g, 12.38 mmol) and the mixture was stirred for 2 hours. A freshly prepared tetrahydrofuran ammonium solution was added dropwise with continuous monitoring of the reaction by thin layer chromatography (TLC). After the reaction was completed the mixture was poured into $H_2O$, acidified with HCl (2N), extracted with EtOAc and dried over MgSO4. The crude product was recrystallized from ether/hexane (at 0 °C) to yield a white solid, m.p. 193-194 °C (3.4 g, 94.7%).
NMR (DMSO-$d_6$, 400 MHz): δ 3.55 (s, 3H, -$CO_2CH_3$), 3.56 (q, J = 16.54 Hz, 2H, -$CH_2CONH_2$), 5.1 (q, J = 15.37 Hz, 2H, -$NCH_2$-), 6.87 (s, 1H, -$CONH_2$), 7.33 (dd, J = 8.37 Hz, 1.87 Hz, 1H, Ar-H), 7.46 (d, J = 7.84 Hz, 1H, Ar-H), 7.52 (s, 1H, -$CONH_2$), 7.57-7.60 (m, 3H, Ar-H), 7.77 (dt, J = 7.65 Hz, 1.1 Hz, 1H, Ar-H), 8.11 (dd, J = 7.6 Hz, 1.74 Hz, 1H, Ar-H)
IR (KBr, $cm^{-1}$): 3450 (s), 3340 (m), 1750 (s), 1720 (s), 1670 (s), 1360 (s), 740 (m)
MS (m/e): 435$(M+H)^+$, 377 ($M^+$-$CH_2CONH_2$)

| | | | |
|---|---|---|---|
| Anal. Calcd.: | C, 55.19; | H, 2.71; | N, 6.44 |
| Found: | C, 55.17; | H, 3.69; | N, 6.62. |

The following compounds were obtained in substantially the same manner as that of Example 1, Step e).

4-(2-Amino-2-oxoethyl)-2-[(4-bromo-2-fluorophenyl)methyl]-1,2,3,4-tetrahydro-1,3-dioxo-4-isoquinolinecarboxylic Acid Methyl Ester

NMR (DMSO-$d_6$, 400 MHz): δ 3.53 (s, 3H, $-CO_2CH_3$), 3.51 (q, J = 16.6 Hz, 2H, $-CH_2CONH_2$), 5.1 (q, J = 15.4 Hz, 2H, $-NCH_2-$), 6.88 (s, 1H, $-CONH_2$), 7.23 (t, J = 8.0 Hz, 1H, Ar-H), 7.3 (dd, J = 8.3 Hz, 1.84 Hz, 1H, Ar-H), 7.46 (d, J = 7.98 Hz, 1H, Ar-H), 7.52 (s, 1H, $-CONH_2$), 7.54-7.60 (m, 2H, Ar-H), 7.75 (dt, J = 7.76 Hz, 1.39 Hz, 1H, Ar-H), 8.1 (dd, J = 7.87 Hz, 1.22 Hz, 1H, Ar-H)
IR (KBr, $cm^{-1}$): 3450 (s), 1730 (s), 1720 (s), 1670 (s), 1350 (s), 875 (m)
MS (m/e): 463 $(M+H)^+$, 446 ($M^+$-OH)

| | | | |
|---|---|---|---|
| Anal. Calcd.: | C, 52.08; | H, 3.06; | N, 6.07 |
| Found: | C, 51.91; | H, 3.36; | N, 6.09 |

M.P. 180-181 °C.

4-(2-Amino-2-oxoethyl)-2-[(4-bromo-2-fluorophenyl)methyl]-7-chloro-1,2,3,4-tetrahydro-1,3-dioxo-4-isoquinolinecarboxylic Acid Methyl Ester

NMR (DMSO-$d_6$, 200 MHz): δ 3.56 (s, 5H, $-CO_2CH_3$, $CH_2CONH_2$), 5.12 (q, J = 17.2 Hz, 2H, $-NCH_2C_6H_3BrF$), 6.95 (s, 1H, $-CONH_2$), 7.2-7.35 (m, 2H, Ar-H), 7.5-7.6 (m, 2H, Ar-H), 7.84 (dd, J = 8.6 Hz, 1.8 Hz, 1H, Ar-H), 8.1 (d, J = 2.2 Hz, 1H, Ar-H)
IR (KBr, $cm^{-1}$): 3450 (m), 3370 (m), 1755 (m), 1720 (m), 1675 (s), 1430 (m), 765 (m)
MS (m/e): 497 $(M+H)^+$

| | | | |
|---|---|---|---|
| Anal. Calcd.: | C, 48.27; | H, 3.04; | N, 5.63 |
| Found: | C, 48.27; | H, 3.12; | N, 5.46 |

M.P. 94-96 °C.

4-(2-Amino-2-oxoethyl)-2-[(4-bromo-2-fluorophenyl)methyl]-6-chloro-1,2,3,4-tetrahydro-1,3-dioxo-4-isoquinolinecarboxylic Acid Methyl Ester

NMR (DMSO-$d_6$, 200 MHz): δ 3.62 (m, 5H, $-CO_2CH_3$, $-CH_2CONH_2$), 5.11 (q, J = 17.6 Hz, 2H, $-NCH_2C_6H_3BrF$), 6.95 (brs, 1H, $-CONH_2$), 7.20-7.35 (m, 2H, Ar-H), 7.5-7.7 (m, 4H, Ar-H, $-CONH_2$), 8.12 (d, J = 7.8 Hz, 1H, Ar-H)
IR (KBr, $cm^{-1}$): 3450 (m), 1725 (m), 1675 (s), 1490 (m)
MS (m/e): 497 $(M+H)^+$

| | | | |
|---|---|---|---|
| Anal. Calcd.: | C, 48.26; | H, 3.04; | N, 5.63 |
| Found: | C, 48.53; | H, 3.22; | N, 5.53. |

4-(2-Amino-2-oxoethyl)-2-[(4-bromo-2-fluorophenyl)methyl]-6-fluoro-1,2,3,4-tetrahydro-1,3-dioxo-4-isoquinolinecarboxylic Acid Methyl Ester

NMR (DMSO-$d_6$, 400 MHz): δ 3.49 (d, J = 16.65 Hz, 1H, $-CH_2CONH_2$), 3.56 (s, 3H, $-CO_2CH_3$), 3.59 (d, J = 16.65 Hz, 1H, $-CH_2CONH_2$), 5.08 (dd, J = 15.48 Hz, 2H, $-NCH_2-$), 6.94 (s, 1H, $-CONH_2$), 7.21 (t, J = 8.22 Hz, 1H, Ar-H), 7.30 (dd, J = 8.27 Hz, 1.64 Hz, 1H, Ar-H), 7.38-7.46 (m, 2H, Ar-H), 7.51 (s, 1H, $-CONH_2$), 7.54

(dd, J = 9.81 Hz, 1.83 Hz, 1H, Ar-H), 8.20 (dd, J-8.74 Hz, 5.84 Hz, 1H, Ar-H)
IR (KBr, $cm^{-1}$): 3440 (s), 3350 (s), 1740 (s), 1710 (s), 1670 (s), 1660 (s), 765 (m)
MS (m/e): 480 ($M^+$), 463 ($M^+$-$NH_3$)

| | | | |
|---|---|---|---|
| Anal. Calcd: | C, 49.91; | H, 3.14; | N, 5.82 |
| Found: | C, 49.56; | H, 3.09; | N, 5.73 |

M.P. 203-204.5 ° C.

4-(2-Amino-2-oxoethyl)-1,2,3,4-tetrahydro-2-methyl-1,3-dioxo-4-isoquinoline-carboxylic Acid Methyl Ester

NMR (DMS0-$d_6$, 200 MHz): $\delta$ 3.27 (s, 3H, -N$CH_3$), 3.49 (s, 2H, -$CH_2CO_2H$), 3.56 (s, 3H, -$CO_2CH_3$), 6.78 (s, 1H, -CON$H_2$), 7.4-7.6 (m, 3H, Ar-H, -CON$H_2$), 7.69 (dt, J = 7.6 Hz, 2Hz, 1H, Ar-H), 8.16 (d, J = 8.2 Hz, 1H, Ar-H)
IR (KBr, $cm^{-1}$): 3420 (m), 3320 (m), 1760 (s), 1660 (s), 1420 (m), 755 (m)
MS (m/e): 290 ($M^+$)

| | | | |
|---|---|---|---|
| Anal. Calcd.: | C, 57.93; | H, 4.86; | N, 9.65 |
| Found: | C, 57.73; | H, 4.95; | N, 9.56 |

M.P. 230-231 ° C.

4-(2-Amino-2-oxoethyl)-7-chloro-1,2,3,4-tetrahydro-2-methyl-1,3-dioxo-4-isoquinolinecarboxylic Acid Methyl Ester

NMR (DMSO-$d_6$, 200 MHz): $\delta$ 3.27 (s, 3H, -N$CH_3$), 3.48 (s, 2H, -$CH_2CONH_2$), 3.58 (s, 3H, -$CO_2CH_3$), 6.85 (s, 1H, -CON$H_2$), 7.45 (d, J = 8.6 Hz, 1H, Ar-H), 7.49 (s, 1H, -CON$H_2$), 7.80 (dd, J = 8.2 Hz, 2.2Hz, 1H, Ar-H), 8.08 (d, J = 2.2 Hz, 1H, Ar-H)
IR (KBr, $cm^{-1}$): 3420 (m), 1760 (s), 1715 (m), 1665 (s), 1440 (m), 770 (m)
MS (m/e): 324 ($M^+$)

| | | | |
|---|---|---|---|
| Anal. Calcd.: | C, 51.78; | H, 4.03; | N, 8.63 |
| Found: | C, 51.82; | H, 3.98; | N, 8.54 |

M.P. 215-216 ° C.

4-(2-Amino-2-oxoethyl)-6-chloro-1,2,3,4-tetrahydro-2-methyl-1,3-dioxo-4-isoquinolinecarboxylicAcid Methyl Ester

NMR (DMSO-$d_6$, 200 MHz): $\delta$ 3.26 (s, 3H, -N$CH_3$), 3.51 (q, J = 17.5 Hz, 2H, -$CH_2CONH_2$), 3.59 (s, 3H, -$CO_2CH_3$), 6.85 (s, 1H, -CON$H_2$), 7.5 (s, 1H, -CON$H_2$), 7.53 (d, J = 2.0 Hz, 1H, Ar-H), 7.62 (dd, J = 8.6 Hz, 2.0 Hz, 1H, Ar-H), 8.16 (d, J = 8.0 Hz, 1H, Ar-H)
IR (KBr, $cm^{-1}$): 3420 (s), 1760 (s), 1710 (m), 1660 (s), 1235 (m), 770 (m)
MS (m/e): 324 ($M^+$)

| | | | |
|---|---|---|---|
| Anal. Calcd.: | C, 51.78; | H, 4.03; | N, 8.63 |
| Found: | C, 51.67; | H, 3.95; | N, 8.42 |

M.P. 221-222 ° C.

4-(2-Amino-2-oxoethyl)-2-ethyl-1,2,3,4-tetrahydro-1,3-dioxo-4-isoquinolinecarboxylic Acid Methyl Ester

NMR (DMSO-$d_6$, 400 MHz): $\delta$ 1.09 (t, J = 6.94 Hz, 3H, -$NCH_2CH_3$), 3.46 (q, J = 16.57 Hz, 2H, -$CH_2CONH_2$), 3.54 (s, 3H, -$CO_2CH_3$), 3.94 (q, J = 6.73 Hz, 2H, -N$CH_2CH_3$), 6.76 (brs, 1H, -CON$H_2$), 7.38 (d, J = 7.8 Hz, 1H,

Ar-H), 7.44 (brs, 1H, $-CONH_2$), 7.53 (t, J = 7.56 Hz, 1H, Ar-H), 7.7 (dt, J = 7.86 Hz, 1.28 Hz, 1H, Ar-H), 8.11 (d, J = 7.82 Hz, 1H, Ar-H)
IR (KBr, $cm^{-1}$): 3450 (s), 1750 (s), 1710 (s), 1680 (s), 1650 (s), 1420 (m), 780 (m)
MS (m/e): 304 ($M^+$), 246 ($M^+$-$CH_2CONH_2$), 245 ($M^+$-$CO_2CH_3$)

| | | | |
|---|---|---|---|
| Anal. Calcd.: | C, 59.21; | H, 5.3; | N, 9.21 |
| Found: | C, 59.17; | H, 5.46; | N, 9.23 |

M.P. 190-191 °C.

4-(2-Amino-2-oxoethyl)-1,2,3,4-tetrahydro-2-propyl-1,3-dioxo-4-isoquinoline-carboxylic Acid Methyl Ester

NMR (DMSO-$d_6$, 200 MHz): δ 0.87 (t, J = 7.0 Hz, 3H, $-NCH_2CH_2CH_3$), 1.51-1.62 (m, 2H, $-NCH_2CH_2CH_3$), 3.49 (s, 2H, $-CH_2CONH_2$), 3.55 (s, 3H, $-CO_2CH_3$), 3.89 (t, J = 7.0 Hz, 2H, $-NCH_2CH_2CH_3$), 6.78 (s, 1H, $-CONH_2$), 7.43 (d, J = 7.2 Hz, 1H, Ar-H), 7.46 (s, 1H, $-CONH_2$), 7.58 (dd, J = 7.6 Hz, 1.6 Hz, 1H, Ar-H), 7.7 (dt, J = 7.6 Hz, 2.0Hz, 1H, Ar-H), 8.15 (dd, J = 8.2 Hz, 1.8 Hz, 1H, Ar-H)
IR (KBr, $cm^{-1}$): 3420 (s), 3200 (s), 1765 (s), 1710 (s), 1670 (s), 1430 (m), 750 (m)
MS (m/e): 318 ($M^+$), 260 ($M^+$-$CH_2CONH_2$), 259 ($M^+$-$CO_2CH_3$)

| | | | |
|---|---|---|---|
| Anal. Calcd.: | C, 60.37; | H, 5.70; | N, 8.80 |
| Found: | C, 60.24; | H, 5.60; | N, 8.72 |

M.P. 181-182 °C.

4-(2-Amino-2-oxoethyl)-2-butyl-1,2,3,4-tetrahydro-1,3-dioxo-4-isoquinoline-carboxylic Acid Methyl Ester

NMR (DMSO-$d_6$, 200 MHz): δ 0.89 (t, J = 7.6 Hz, 3H, $-NCH_2CH_2CH_2CH_3$), 1.2-1.4 (m, 2H, $-NCH_2CH_2CH_2CH_3$), 1.4--1.62 (m, 2H, $-NCH_2CH_2CH_2CH_3$), 3.49 (s, 2H, $-CH_2CONH_2$), 3.55 (s, 3H, $-CO_2CH_3$), 3.92 (t, J = 7.6 Hz, 2H, $-NCH_2CH_2CH_2CH_3$), 6.79 (s, 1H, $-CONH_2$), 7.43 (d, J = 8.0 Hz, 1H, Ar-H), 7.46 (s, 1H, $-CONH_2$), 7.58 (dt, J = 7.6 Hz, 1.2 Hz, 1H, Ar-H), 8.15 (dd, J = 8.0 Hz, 1.0 Hz, 1H, Ar-H)
IR (KBr, $cm^{-1}$): 3400 (s), 3200 (s), 1750 (s), 1710 (s), 1660 (s), 1410 (m), 750 (m)
MS (m/e): 332 ($M^+$), 274 ($M^+$-$CH_2CONH_2$), 273 ($M^+$-$CO_2CH_3$)

| | | | |
|---|---|---|---|
| Anal. Calcd.: | C, 61.44; | H, 6.07; | N, 8.43 |
| Found: | C, 61.23; | H, 5.99; | N, 8.33 |

M.P. 142-143 °C.

4-(2-Amino-2-oxoethyl)-6-bromo-1,2,3,4-tetrahydro-2-methyl-1,3-dioxo-4-isoquinolinecarboxylic Acid Methyl Ester

NMR (DMSO-$d_6$, 400 MHz): δ 3.24 (s, 3H, N-$CH_3$), 3.44 (dd, J = 6.54 Hz, 2H, $-CH_2CONH_2$), 3.58 (s, 3H, $-CO_2CH_3$), 6.84 (s, 1H, -CONH-), 7.48 (s, 1H, -CONH-), 7.64 (d, J = 1.76 Hz, 1H, Ar-H), 7.76 (dd, J = 8.4 Hz, 1.83 Hz, 1H, Ar-H), 8.03 (d, J = 8.42 Hz, 1H, Ar-H)
IR (KBr, $cm^{-1}$): 3420 (s), 1740 (s), 1715 (s), 1665 (s)

| | | | |
|---|---|---|---|
| Anal. Calcd.: | C, 45.54; | H, 3.55; | N, 7.59 |
| Found: | C, 45.26; | H, 3.34; | N, 7.46 |

M.P. 212-213 °C.

**Step f)** Preparation of 2-[(3,4-Dichlorophenyl)methyl]spiro[isoquinoline-4(1H)3'-pyrrolidine]-1,2',3,5'(2H)-tetrone

To a solution of 4-(2-amino-2-oxoethyl)-2-[(3,4-dichlorophenyl)methyl]-1,2,3,4-tetrahydro-1,3-dioxo-isoquinolinecarboxylic acid methyl ester (2.5 g, 5.75 mmol) in DMF (20 mL) was added NaH (80% dispersion in oil, 172.4 mg, 5.75 mmol) portionwise, over a 10 minute period. After stirring for 30 minutes, the mixture was poured into $H_2O$, acidified with HCl (2N), extracted with EtOAc and dried over $MgSO_4$. The crude product was purified by flash chromatography on acid washed silica gel (5% $H_3PO_4$ in MeOH) to yield a white solid, m.p. 118-120 °C (1.82 g, 78.8 %).
NMR (DMSO-$d_6$, 400 MHz): δ 3.5 (q, J = 18.34 Hz, 2H, -$\underline{CH_2}$CONH-), 5.06 (q, J = 15.26 Hz, 2H, -N$\underline{CH_2}$-), 7.27 (dd, J = 8.33 Hz, 2.0 Hz, 1H, Ar-$\underline{H}$), 7.52 (d, J = 1.93 Hz, 1H, Ar-$\underline{H}$), 7.57 (d, J = 8.3 Hz, 1H, Ar-$\underline{H}$), 7.61 (t, J = 7.6 Hz, 1H, Ar-$\underline{H}$), 7.68 (d, J = 7.72 Hz, 1H, Ar-$\underline{H}$), 7.79 (dt, J = 7.76 Hz, 1.2 Hz, 1H, Ar-$\underline{H}$), 8.17 (dd, J = 7.8 Hz, 0.9 Hz, 1H, Ar-$\underline{H}$), 12.02 (s, 1H, -CONHCO-)
IR (KBr, $cm^{-1}$): 3250 (s), 1730 (s), 1670 (s), 1340 (s), 970 (m), 760 (m)
MS (m/e): 403 $(M + H)^+$

| | | | |
|---|---|---|---|
| Anal. Calcd. for $C_{19}H_{12}Cl_2N_2O_4$ x 1/3$H_2O$: | C, 55.74; | H, 3.09; | N, 6.84 |
| Found: | C, 55.94; | H, 3.04; | N, 6.61. |

The following compounds were obtained in substantially the same manner as that of Example 1, Step f).

2-[(4-Bromo-2-fluorophenyl)methyl]spiro[isoquinoline-4(1H),3'-pyrrolidine) -1,2',3,5'(2H)-tetrone

NMR (DMSO-$d_6$, 400 MHz): δ 3.47 (q, J = 18.24 Hz, 2H, -$\underline{CH_2}$CONH-), 5.06 (s, 2H, -N$\underline{CH_2}$-), 7.14 (t, J = 8.2 Hz, 1H, Ar-$\underline{H}$), 7.33 (dd, J = 8.28 Hz, 1.71 Hz, 1H, Ar-$\underline{H}$), 7.55 (dd, J = 9.9 Hz, 1.8 Hz, 1H, Ar-$\underline{H}$), 7.62 (t, J = 7.6 Hz, 1H, Ar-$\underline{H}$), 7.68 (d, J = 7.78 Hz, 1H, Ar-$\underline{H}$), 7.78 (dt, J = 8.85 Hz, 1.12 Hz, 1H, Ar-$\underline{H}$), 8.15 (dd, J = 7.86 Hz, 1.3 Hz, 1H, Ar-$\underline{H}$), 12.01 (s, 1H, -CO$\underline{NH}$CO-)
IR (KBr, $cm^{-1}$): 3450 (m), 3250 (m), 1730 (s), 1680 (s), 1490 (m), 1345 (m), 1180 (m), 875 (m)
MS (m/e): 430 ($M^+$), 387 ($M^+$-CONH), 200 ($M^+$-CONH, -$CH_2C_6H_3$BrF)

| | | | |
|---|---|---|---|
| Anal. Calcd.: | C, 52.92; | H, 2.80; | H, 6.50 |
| Found: | C, 52.61; | H, 2.70; | N, 6.46 |

M.P. 112-114 °C.

[2-[(4-Bromo-2-fluorophenyl)methyl]-7-chloro]spiro[isoquinoline-4(1H),3'-pyrrolidine]-1,2',3,5'(2H)-tetrone

NMR (DMSO-$d_6$, 400 MHz): δ 3.41 (q, J = 18.29 Hz, 2H, -$CH_2$CONH-), 5.05 (s, 2H, -N$\underline{CH_2}C_6H_3$BrF), 7.15 (t, J = 8.2 Hz, 1H, Ar-$\underline{H}$), 7.32 (dd, J = 8.3 Hz, 1.76 Hz, 1H, Ar-$\underline{H}$), 7.52 (dd, J = 9.78 Hz, 1.94 Hz, 1H, Ar-$\underline{H}$), 7.76 (d, J = 8.5 Hz, 1H, Ar-$\underline{H}$), 7.85 (dd, J = 8.55 Hz, 2.43 Hz, 1H, Ar-$\underline{H}$), 8.11 (d, J = 2.3 Hz, 1H, Ar-$\underline{H}$), 12.04 (s, 1H, -CO$\underline{NH}$CO-)
IR (KBr, $cm^{-1}$): 3450 (m), 3620 (s), 1720 (s), 1670 (s), 1430 (m), 775 (m)
MS (m/e): 465 $(M + H)^+$

| | | | |
|---|---|---|---|
| Anal. Calcd.: | C, 49.01; | H, 2.38; | N, 6.02 |
| Found: | C, 48.88; | H, 2.37; | N, 5.92 |

M.P. 187-188 °C.

[2-[(4-Bromo-2-fluorophenyl)methyl]-6-chloro]spiro[isoquinoline-4(1H),3'-pyrrolidine]-1,2',3,5'(2H)-tetrone

NMR (DMSO-$d_6$, 200 MHz): δ 3.46 (q, J = 18.3 Hz, 2H, -$\underline{CH_2}$CONH-), 5.06 (s, 2H, -N$\underline{CH_2}C_6H_3$BrF), 7.14 (t, J = 8.0 Hz, 1H, Ar-$\underline{H}$), 7.36 (d, J = 8.8 Hz, 1H, Ar-$\underline{H}$), 7.57 (dd, J = 8.4 Hz, 1.8 Hz, 1H, Ar-$\underline{H}$), 7.68 (dd, J = 8.6 Hz, 1.2 Hz, 1H, Ar-$\underline{H}$), 7.98 (s, 1H, Ar-$\underline{H}$), 8.18 (d, J = 8.4 Hz, 1H, Ar-$\underline{H}$), 12.0 (s, 1H, -CO$\underline{NH}$CO)

IR (KBr, $cm^{-1}$): 3310 (s), 1745 (s), 1715 (m), 1675 (s), 1430 (m), 780 (m)
MS (m/e): 465 $(M+H)^+$

| | | | |
|---|---|---|---|
| Anal. Calcd.: | C, 49.01; | H, 2.38; | N, 6.02 |
| Found: | C, 48.93; | H, 2.37; | N, 5.97 |

M.P. 196-197 ° C.

[2-[(4-Bromo-2-fluorophenyl)methyl]-5-fluoro]spiro[isoquinoline-4(1H),3'-pyrrolidine]-1,2',3,5'(2H)-tetrone

[5]NMR (DMSO-$d_6$, 400 MHz): δ 3.20 (dd, J = 18.56 Hz, 1.97 Hz, 1H, -$CH_2$CONH-), 3.33 (dd, J = 18.56 Hz, 2.74 Hz, 1H, -$CH_2$CONH-), 5.05 (dd, J = 15.3 Hz, 2H, -N$CH_2$-), 7.16 (t, J = 8.27 Hz, 1H, Ar-H), 7.34 (dd, J = 8.32 Hz, 1.72 Hz, 1H, Ar-H), 7.54 (dd, J = 9.85 Hz, 1.88 Hz, 1H, Ar-H), 7.67-7.75 (m, 2H, Ar-H), 8.03 (m, 1H, Ar-H), 12.2 (s, 1H, -CONHCO-)
IR (KBr, $cm^{-1}$): 3325 (s), 1730 (s), 1700 (s), 1670 (s), 760 (m)
MS (m/e): 448 ($M^+$)

| | | | |
|---|---|---|---|
| Anal. Calcd.: | C, 50.80; | H, 2.47; | N, 6.24 |
| Found: | C, 50.61; | H, 2.46; | N, 6.28 |

M.P. 202.5-203.5 ° C.

[2-[(4-Bromo-2-fluorophenyl)methyl] -6-fluoro]spiro[isoquinoline-4(1H),3'-pyrrolidine]-1,2',3,5'(2H)-tetrone

NMR (DMSO-$d_6$, 400 MHz): δ 3.44 (s, 2H, -$CH_2$CONH-), 5.05 (s, 2H, -N$CH_2$-), 7.14 (t, J = 8.42 Hz, 1H, Ar-H), 7.32 (dd, J = 7.58 Hz, 1.26 Hz, 1H, Ar-H), 7.48 (dt, J = 8.64 Hz, 2.1 Hz, 1H, Ar-H), 7.53 (dd, 9.89 Hz, 1.89 Hz, 1H, Ar-H), 7.75 (dd, 9.69 Hz, 2.32 Hz, 1H, Ar-H), 8.22 (dd, J = 8.84 Hz, 5.89 Hz, 1H, Ar-H), 12.0 (s, 1H, -CONHCO-)
IR (KBr, $cm^{-1}$): 3400 (m), 3260 (s), 1735 (s), 1680 (s)
MS (m/e): 448 ($M^+$), 405 ($M^+$-CONH)

| | | | |
|---|---|---|---|
| Anal. Calcd.: | C, 50.80; | H, 2.47; | N, 6.24 |
| Found: | C, 50.73; | H, 2.52; | N, 6.16 |

M.P. 231-232 ° C.

[2-[(4-Bromo-2-fluorophenyl)methyl]-7-fluoro]spiro[isoquinoline-4(1H),3'-pyrrolidine]-1,2'3,5'(2H)-tetrone

NMR (DMSO-$d_6$, 400 MHz): δ 3.42 (dd, J = 18.3 Hz, 2H, -$CH_2$CONH-), 5.06 (s, 2H, N$CH_2$-), 7.15 (t, J = 8.21 Hz, 1H, Ar-H), 7.32 (dd, J = 8.21 Hz, J = 1.9 Hz, 1H, Ar-H), 7.53 (dd, J = 9.9 Hz, 1.9 Hz, 1H, Ar-H), 7.67 (dt, J = 8.42 Hz, 2.74 Hz, 1H, Ar-H), 7.81 (dd, J = 8.64 Hz, 4.84 Hz, 1H, Ar-H), 7.89 (dd, J = 8.84 Hz, 2.73 Hz, 1H, Ar-H), 12.01 (s, 1H, -CONHCO-)
IR (KBr, $cm^{-1}$): 3400 (m), 3270 (s), 1725 (s), 1680 (s)
MS (m/e): 449 $(M+H)^+$

| | | | |
|---|---|---|---|
| Anal. Calcd.: | C, 50.80; | H, 2.47; | N, 6.24 |
| Found: | C, 50.52; | H, 2.53; | N, 6.06 |

M.P. 189-190 ° C.

[2[(4-Bromo-2-fluorophenyl)methyl] -8-fluoro] spiro[isoquinoline-4(1H),3'-pyrrolidine]-1,2',3,5'(2H)-tetrone

NMR (DMSO-$d_6$, 400 MHz): δ 3.5 (dd, J = 18.32 Hz, 2H, -$CH_2$CONH-), 5.03 (dd, J = 15.34 Hz, 2H, -N$CH_2$-), 7.17 (t, J = 8.21 Hz, 1H, Ar-H), 7.33 (dd, J = 8.21 Hz, 1.47 Hz, 1H, Ar-H), 7.43 (d, J = 8.42 Hz, 1H, Ar-H), 7.46

(d, J = 8.42 Hz, 1H, Ar-H), 7.52-7.56 (m, 2H, Ar-H), 7.79 (m, 1H, Ar-H), 12.04 (s, 1H, -CONHCO-)
IR (KBr, $cm^{-1}$): 3460 (m), 3380 (s), 1740 (s), 1690 (s)
MS (m/e): 449 $(M+H)^+$

| | | | |
|---|---|---|---|
| Anal. Calcd.: | C, 50.80; | H, 2.47; | N, 6.24 |
| Found: | C, 50.44; | H, 2.81; | N, 5.87. |

[2-[(4-Bromo-2-fluorophenyl)methyl]-7-methoxy]spiro[isoquinoline-4(1H),3'-pyrrolidine]-1,2',3,5'(2H)-tetrone

NMR (DMSO-$d_6$, 400 MHz): δ 3.31 (dd, J = 18.43 Hz, 2H, -$CH_2$CONH-), 3.85 (s, 3H, -O$CH_3$), 5.06 (s, 2H, -N$CH_2$-), 7.13 (t, J = 8.3 Hz, 1H, Ar-H), 7.31-7.36 (m, 2H, Ar-H), 7.52 (dd, J = 9.8 Hz, 1.9 Hz, 1H, Ar-H), 7.6-7.61 (m, 2H, Ar-H), 11.95 (s, 1H, -CONHCO-)
IR (KBr, $cm^{-1}$): 3250 (s), 1735 (s), 1680 (s)
MS (m/e): 461 $(M+H)^+$

| | | | |
|---|---|---|---|
| Anal. Calcd.: | C, 52.08; | H, 3.06; | N, 6.07 |
| Found: | C, 51.91; | H, 3.23; | N, 5.86 |

M.P. 160-161 °C.

[6-Bromo-2-[(4-bromo-2-fluorophenyl)methyl]-7-methoxy]spiro[isoquinoline-4(1H),3'-pyrrolidine]-1,2',3,5'(2H)-tetrone

NMR (DMSO-$d_6$, 400 MHz): δ 3.38 (dd, J = 18.11 Hz, 2H, -$CH_2$CONH-), 3.96 (s, 3H, -O$CH_3$), 5.05 (s, 2H, -N$CH_2$-), 7.12 (t, J = 8.24 Hz, 1H, Ar-H), 7.32 (dd, J = 8.08 Hz, 1.65 Hz, 1H, Ar-H), 7.52 (dd, J = 9.8 Hz, 1.95 Hz, 1H, Ar-H), 7.68 (s, 1H, Ar-H), 8.09 (s, 1H, Ar-H), 11.93 (s, 1H, -CONHCO-)
IR (KBr, $cm^{-1}$): 3400 (m), 3360 (s), 1730 (s), 1680 (s)
MS (m/e): 538 $(M+H)^+$, 495 ($M^+$ + H-CONH), 467 ($M^+$ + H-CONHCO)

| | | | |
|---|---|---|---|
| Anal. Calcd.: | C, 44.56; | H, 2.24; | N, 5.20 |
| Found: | C, 44.26; | H, 2.82; | N, 4.97 |

M.P. 134-136 °C.

2-Methylspiro[isoquinoline-4(1H),3'pyrrolidine]-1,2',3,5'(2H)-tetrone

NMR (DMSO-$d_6$, 400 MHz): δ 3.24 (s, 3H, -N-$CH_3$), 3.43 (q, J = 18.38 Hz, 2H, -$CH_2$CONH-), 7.58-7.64 (m, 2H, Ar-H), 7.74 (dt, J = 7.64 Hz, 1.2 Hz, 1H, Ar-H), 8.15 (dd, J = 7.72 Hz, 0.94 Hz, 1H, Ar-H), 12.0 (2, 1H, -CONHCO-)
IR (KBr, $cm^{-1}$): 3340 (s), 1720 (s), 1660 (s), 1310 (s), 765 (m)
MS (m/e): 258 ($M^+$)

| | | | |
|---|---|---|---|
| Anal. Calcd.: | C, 60.47; | H, 3.90; | N, 10.85 |
| Found: | C, 60.27; | H, 4.03; | N, 10.82 |

M.P. 224-225 °C.

2-Ethylspiro[isoquinoline-4(1H),3'-pyrrolidine]-1,2',3,5'(2H)-tetrone

NMR (DMSO-$d_6$, 400 MHz): δ 1.09 (t, J = 7.0 Hz, 3H, -N$CH_2$$CH_3$), 3.45 (q, J = 18.35 Hz, 2H, -$CH_2$CONH-), 4.9 (q, J = 6.9 Hz, 2H, -N$CH_2$$CH_3$), 7.58-7.64 (m, 2H, Ar-H), 7.75 (dt, J = 7.45 Hz, 1.46 Hz, 1H, Ar-H), 8.16 (dd, J = 7.88 Hz, 1.19 Hz, 1H, Ar-H), 11.98 (s, 1H, -CONHCO-)
IR (KBr, $cm^{-1}$): 3300 (s), 1730 (s), 1710 (s), 1670 (s), 1465 (m), 760 (m)

MS (m/e): 273 $(M+H)^+$

| | | | |
|---|---|---|---|
| Anal. Calcd.: | C, 61.76; | H, 4.44; | N, 10.29 |
| Found: | C, 61.45; | H, 4.44; | N, 10.22 |

M.P. 183-185 °C.

2-Propylspiro[isoquinoline-4(1H),3'-pyrrolidine]-1,2',3,5'(2H)-tetrone

NMR (DMSO-$d_6$, 400 MHz): δ 0.84 (t, J=7.4 Hz, 3H, $-NCH_2CH_2\underline{CH_3}$), 1.51-1.61 (m, 2H, $-NCH_2\underline{CH_2}CH_3$), 3.35 (q, J=18.3 Hz, 2H, $-\underline{CH_2}CONH-$), 3.85 (t, J=7.2 Hz, 2H, $-N\underline{CH_2}CH_2CH_3$), 7.58-7.65 (m, 2H, Ar-H), 7.73 (dt, J=7.64 Hz, 0.97 Hz, 1H, Ar-H), 8.16 (d, J=7.84 Hz, 1H, Ar-H), 11.98 (s, 1H, -CONHCO)
IR (KBr, $cm^{-1}$): 3320 (s), 1730 (s), 1710 (s), 1460 (m), 755 (m)
MS (m/e): 287 $(M+H)^+$, 257 $(M^+-CH_2CH_3)$, 243 $(M^+-CH_2CH_2-CH_3)$

| | | | |
|---|---|---|---|
| Anal. Calcd.: | C, 62.93; | H, 4.93; | N, 9.78 |
| Found: | C, 62.79; | H, 5.06; | N, 9.77 |

M.P. 190-192 °C.

2-Butylspiro[isoquinoline-4(1H),3'-pyrrolidine]1,2',3,5'(2H)-tetrone

NMR (DMSO-$d_6$, 400 MHz): δ 0.87 (t, J=7.27 Hz, 3H, $-NCH_2CH_2CH_2\underline{CH_3}$), 1.21-1.31 (m, 2H, $-NCH_2CH_2\underline{CH_2}CH_3$), 1.42-1.52 (m, 2H, $-NCH_2\underline{CH_2}CH_2CH_3$), 3.44 (q, J=18.26 Hz, 2H, $-\underline{CH_2}CONH-$), 3.87 (t, J=7.3 Hz, 2H, $-N\underline{CH_2}CH_2CH_2CH_3$), 7.58-7.64 (m, 2H, Ar-H), 7.75 (dt, J=7.86 Hz, 1.4 Hz, 1H, Ar-H), 8.15 (dd, J=7.82 Hz, 1.27 Hz, 1H, Ar-H), 11.97 (s, 1H, -CONHCO)
IR (KBr, $cm^{-1}$): 3250 (s), 1730 (s), 1670 (s), 1465 (m), 760 (m)
MS (m/e): 301 $(M+H)^+$, 271 $(M^+-CH_2CH_3)$, 257 $(M^+-CH_2CH_2CH_3)$

| | | | |
|---|---|---|---|
| Anal. Calcd.: | C, 63.99; | H, 5.37; | N, 9.33 |
| Found: | C, 63.87; | H, 5.31; | N, 9.25 |

M.P. 143-144 °C.

**EXAMPLE 2**

**2-Methyl-1,3(2H, 4H)-isoquinolinedione**

Anhydrous monomethylamine was passed through a solution of homophthalic anhydride (10.0 g, 61.73 mmol) in anhydrous THF (200 mL) for 10 minutes. The formed suspension was stirred for 1 hour and the volatiles were removed in vacuo. The residue was taken in DMF (200 mL) and the suspension was stirred at 180 °C for 10 hours. After cooling, the brownish solution was poured into $H_2O$, extracted with EtOAc and dried over $MgSO_4$. The crude product was recrystallised from acetone/ether/hexane (at O° C) to yield a yellow solid, m.p. 120-121 °C (7.9 g, 73.1%).
NMR (DMSO-$d_6$, 400 MHz): δ 3.18 (s, 3H, $-N\underline{CH_3}$), 4.12 (s, 2H, $-\underline{CH_2}CONCH_3$), 7.36 (d, J=7.67 Hz, 1H, Ar-H), 7.47 (t, J=7.37 Hz, 1H, Ar-H), 7.64 (t, J=7.45 Hz, 1H, Ar-H), 8.02 (d, J=7.87 Hz, 1H, Ar-H)
IR (KBr, $cm^{-1}$): 3420 (m), 1720 (s), 1665 (s), 1460 (m), 735 (m)
MS (m/e): 175 $(M^+)$, 118 $(M^+-CONCH_3)$

| | | | |
|---|---|---|---|
| Anal. Calcd.: | C, 68.56; | H, 5.18; | N, 8.00 |
| Found: | C, 68.57; | H, 5.40; | N, 8.01. |

The following compound was obtained in substantially the same manner as that of Example 2.

2-Ethyl-1,3(2H, 4H)-isoquinolinedione

NMR (DMSO-$d_6$, 200 MHz): δ 1.1 (t, J = 7.0 Hz, 3H, -$NCH_2CH_3$), 3.89 (q, J = 6.8 Hz, 2H, -$NCH_2CH_3$), 4.11 (s, 2H, -$CH_2CO$), 7.34-7.49 (m, 2H, Ar-H), 7.63 (dt, J = 7.8 Hz, 2.0 Hz, 1H, Ar-H), 8.05 (d, J = 7.6 Hz, 1H, Ar-H)
IR (KBr, $cm^{-1}$): 3420 (m), 1710 (s), 1650 (s), 1450 (s), 740 (m)
MS (m/e): 189 ($M^+$), 174 ($M^+$-$CH_3$), 146 ($M^+$-$CH_3$,-CO), 118 ($M^+$-$CONCH_2CH_3$), 90 ($M^+$-$CON(CN_2CH_3)CO$)

| | | | |
|---|---|---|---|
| Anal. Calcd.: | C, 69.83; | H, 5.86; | N, 7.40 |
| Found: | C, 69.64; | H, 5.73; | N, 7.36 |

M.P. 103-104 °C.

**EXAMPLE 3**

2-Methylspiro[isoquinoline-4(1H),3'-pyrrolidine]-1,2',3,5'(2H)-tetrone

To a cold (-78 °C) solution of 4-(2-amino-2-oxoethyl)-1,2,3,4-tetrahydro-1,3-dioxo-4-isoquinolinecarboxylic acid methyl ester (2.5 g, 8.62 mmol) in anhydrous THF (80 mL) was added lithium bis(trimethylsilyl)-amide (17.24mL, 17.24 mmol, 1.0 M in THF) over a 10 minute period. After stirring at -78 °C for 10 hours, the mixture was poured into $H_2O$, acidified with HCl (2N), extracted with EtOAc, and dried over $MgSO_4$. The crude product was purified by flash chromatography (hexane/EtOAc 1/1) to yield a white solid, m.p. 224-225 °C (1.25 g, 56.8%).
NMR (DMSO-$d_6$, 400 MHz): δ 3.23 (s, 3H, -N-$CH_3$), 3.43 (q, J = 18.3 Hz, 2H, -$CH_2$CONHCO-), 7.60-7.62 (m, 2H, Ar-H), 7.74 (dt, J = 7.6 Hz, 1.22 Hz, 1H, Ar-H), 8.14 (dd, J = 7.72 Hz, 0.95 Hz, 1H, Ar-H), 12.0 (s, 1H, -CONHCO-)
IR (KBr, $cm^{-1}$): 3340 (s), 1725 (s), 1660 (s), 1310 (s), 765 (m)
MS (m/e): 259 $(M+H)^+$

| | | | |
|---|---|---|---|
| Anal. Calcd. for $C_{13}H_{10}N_2O_4$x1/4$H_2O$: | C, 59.42; | H, 3.91; | N, 10.66 |
| Found: | C, 59.44; | H, 3.97; | N, 10.54. |

The following compounds were obtained in substantially the same manner as that of Example 3.

7-Chloro-2-methylspiro[isoquinoline-4(1H)3'-pyrrolidine]-1,2',3,5'(2H)-tetrone

NMR (DMSO-$d_6$, 400 MHz): δ 3.23 (s, 3H, -$NCH_3$), 3.4 (q, J = 18.26 Hz, 2H, -$CH_2$CONH-), 7.7 (d, J = 8.45 Hz, 1H, Ar-H), 7.82 (dd, J = 8.51 Hz, 2.38 Hz, 1H, Ar-H), 8.09 (d, J = 2.31 Hz, 1H, Ar-H), 12.02 (brs, 1H, -CONHCO-)
IR (KBr, $cm^{-1}$): 3440(m), 1720(s), 1660(s), 1310(m), 1180(m), 690(m)
MS (m/e): 293 $(M+H)^+$

| | | | |
|---|---|---|---|
| Anal. Calcd. for $C_{13}H_9N_2ClO_4$x 1/3 $H_2O$: | C, 52.56; | H, 3.13; | N, 9.38 |
| Found: | C, 52.72; | H, 3.14; | N, 9.33 |

M.P. 234-236 °C.

6-Chloro-2-methylspiro[isoquinoline-4(1H)3'-pyrrolidine]-1,2',3,5'(2H)-tetrone

NMR (DMSO-$d_6$, 400 MHz): δ 3.22 (s, 3H, -$NCH_3$), 3.38 (s, 2H, $CH_2$CONH-), 7.66 (dd, J = 8.55 Hz, 2.02 Hz, 1H, Ar-H), 7.92 (d, J = 1.97 Hz, 1H, Ar-H), 8.13 (d, J = 8.52 Hz, 1H, Ar-H), 11.99 (s, 1H, -CONHCO-)
IR (KBr, $cm^{-1}$): 3350 (m), 1750 (s), 1730 (m), 1660 (s), 1310 (s), 690 (m)
MS (m/e): 293 $(M+H)^+$

| | | | |
|---|---|---|---|
| Anal. Calcd.: | C, 53.35; | H, 3.10; | N, 9.57 |
| Found: | C, 53.43; | H, 3.09; | N, 9.38 |

M.P. 213-214 ° C.

6-Bromo-2-methylspiro[isoquinoline-4-(1H),3'-pyrrolidine]-1,2',3,5'(2H)-tetrone

NMR (DMSO-$d_6$, 400 MHz): δ 3.22 (s, 3H, -NCH$_3$), 3.37 (s, 2H, -CH$_2$CONH-), 7.80 (dd, J = 8.42 Hz, 1.67 Hz, 1H, Ar-H), 8.05 (m, 2H, Ar-H), 11.98 (s, 1H, -CONHCO-)
IR (KBr, $cm^{-1}$): 3260 (s), 1720 (s), 1660 (s)
MS (m/e): 337 $(M+H)^+$

| | | | |
|---|---|---|---|
| Anal. Calcd.: | C, 46.31; | H, 2.69; | N, 8.31 |
| Found: | C, 46.04; | H, 2.66; | N, 8.01 |

M.P. 245-247 ° C.

**EXAMPLE 4**

6-Chloro-2-Methyl-1,3(2H, 4H)-isoquinolinedione

In a pressure glass vessel were placed 5-chloro-2-(methoxycarbonyl)-benzeneacetic acid methyl ester (20.0 g, 82.47 mmol) and MeOH (150 mL). Anhydrous monomethylamine was passed through the mixture for 30 minutes. The vessel was introduced into an oil bath and heated at 75 ° C for 24 hours. After cooling to room temperature, the mixture was poured into $H_2O$, acidified with HCl (2N), extracted with EtOAc, and dried over $MgSO_4$. The crude product was purified by flash chromatography (hexane/EtOAc 2/1) to yield a yellow solid, m.p. 167-180 ° C (11.1 g, 64.2%).
NMR (DMSO-$d_6$, 200 MHz): δ 3.18 (s, 3H, -NCH$_3$), 4.12 (s, 2H, -CH$_2$CO-), 7.52 (m, 2K, Ar-H), 8.04 (d, J = 8.8 Hz, 1H, Ar-H)
IR (KBr, $cm^{-1}$): 3440 (m), 1665 (s), 1300 (s), 770 (m)
MS (m/e): 209 ($M^+$), 152 ($M^+$-CONCH$_3$), 124[$M^+$-CON(CH$_3$)CO-]

| | | | |
|---|---|---|---|
| Anal. Calcd.: | C, 57.30; | H, 3.85; | N, 6.68 |
| Found: | C, 57.10; | H, 3.79; | N, 6.69 |

The following compound was prepared in substantially the same manner as that of Example 4.

7-Chloro-2-methyl-1,3(2H,4H)-isoquinolinedione

NMR (DMSO-$d_6$, 200 MHz): δ 3.19 (s, 3H, -NCH$_3$), 4.11 (s, 2H, -CH$_2$CO-), 7.41 (d, J = 8.6 Hz, 1H, Ar-H), 7.73 (dd, J = 8.2 Hz, 2.2 Hz, 1H, Ar-H), 7.96 (d, J = 1.8 Hz, 1H, Ar-H)
IR (KBr, $cm^{-1}$): 3420 (m), 1710 (m), 1635 (s), 1295 (m), 770 (m)
MS (m/e): 209 ($M^+$), 152 ($M^+$-CONCH$_3$), 124[$M^+$-CON(CH$_3$)CO-]

| | | | |
|---|---|---|---|
| Anal. Calcd.: | C, 57.30; | H, 3.85; | N, 6.68 |
| Found: | C, 57.34; | H, 3.84; | N, 6.60 |

M.P. 158-160 ° C.

## EXAMPLE 5

(2-Carboxyphenyl)propanedioic Acid Dimethyl Ester

To a rapidly stirred cold suspension (0° C) of 2-bromobenzoic acid (30.0 g, 149.32 mmol), cuprous bromide (2.14 g, 14.93 mmol) and dimethyl malonate (300 mL) was added NaH (80% in mineral oil, 10.75 g, 358.37 mmol) over a 30 minute period, while a stream of dry $N_2$ was passed over the mixture. After the addition of the NaH had been completed, the mixture was stirred for 10 minutes at room temperature and 30 minutes at 70° C (external oil bath temperature). At this point, the suspension had turned to a solid mass, which was dissolved in $H_2O$ (1000 mL). The aqueous layer was extracted with diethyl ether (3x500 mL) and was acidified with HCl (2N). The mixture was extracted with EtOAc and dried over $MgSO_4$. Evaporation gave an off-white solid which was recrystallised from $Et_2O$/hexane (-20°C) to give a white solid (34.2 g).

NMR (DMSO-$d_6$, 400 MHz): δ 3.67 [s, 6H, -CH($CO_2\underline{CH_3}$)$_2$], 5.72 [s, 1H, -$\underline{CH}$($CO_2CH_3$)$_2$], 7.3 (d, J=7.76 Hz, 1H, Ar-$\underline{H}$), 7.45 (dt, J=7.66 Hz, 1.12 Hz, 1H, Ar-$\underline{H}$), 7.6 (dt, J=7.66 Hz, 1.45 Hz, 1H, Ar-$\underline{H}$), 7.94 (dd, J=7.8 Hz, 1.33 Hz, 1H, Ar-$\underline{H}$), 13.2 (s, 1H, -$CO_2\underline{H}$)
IR (KBr, $cm^{-1}$): 3300-2700 (br), 1750 (s), 1730 (s), 1680 (s), 1430 (m), 730 (m)
MS (m/e): 252 ($M^+$), 220 ($M^+$-$CH_3OH$), 188 ($M^+$-2x$CH_3OH$)

| | | |
|---|---|---|
| Anal. Calcd.: | C, 57.14; | H, 4.80 |
| Found: | C, 57.05; | H, 4.78 |

M.P. 119-120°C.

The following compounds were prepared in substantially the same manner as that of Example 5.

(2-Carboxy-6-fluorophenyl)propanedioic Acid Dimethyl Ester

NMR (DMSO-$d_6$, 400 MHz): δ 3.68 [s, 6H, (-$CO_2\underline{Me}$)$_2$], 5.79 (s, 1H, Ar-$\underline{CH}$-), 7.12 (dd, J=10.06 Hz, 2.61 Hz, 1H, Ar-$\underline{H}$), 7.33 (dt, J=8.48 Hz, 2.64 Hz, 1H,, Ar-$\underline{H}$), 8.03 (dd, 8.77 Hz, 6.17 Hz, 1H, Ar-$\underline{H}$)
IR (KBr, $cm^{-1}$): 3400-2700 (br), 1730 (s), 1680 (s), 750 (m)
MS (m/e): 270 ($M^+$), 238 ($M^+$ -$CH_3OH$), 210 ($M^+$-$CH_3OH$, -CO), 151 ($M^+$-$CH_3OH$-CO-$CO_2CH_3$)
M.P. 121.5-123.0°C.

(2-Carboxy-6-methoxyphenyl)propanedioic Acid Diethyl Ester

NMR (DMSO-$d_6$, 400 MHz): δ 1.16 [t, J=7.02 Hz, 6H, (-$CO_2CH_2\underline{CH_3}$)$_2$], 3.79 (s, 3H, -O$\underline{CH_3}$), 4.13 [q, J=7.02 Hz, 4H, (-$CO_2\underline{CH_2}CH_3$)$_2$], 5.56 (s, 1H, Ar$\underline{CH}$-), 7.16 (dd, J=8.63 Hz, 2.81 Hz, 1H, Ar-$\underline{H}$), 7.22 (d, J=8.62 Hz, 1H, Ar-$\underline{H}$), 7.42 (d, J=2.79 Hz, 1H, Ar-$\underline{H}$)
IR (KBr, $cm^{-1}$): 3450-2550 (br), 1750 (s), 1730 (s)
MS (m/e): 310 ($M^+$), 264 ($M^+$-EtOH), 218 ($M^+$-2EtOH)

| | | |
|---|---|---|
| Anal. Calcd.: | C, 58.06; | H, 5.85 |
| Found: | C, 57.88; | H, 5.67 |

M.P. 94.5-96.0° C.

(2-Carboxy-6-bromophenyl)propanedioic Acid Dimethyl Ester

NMR (DMSO-$d_6$, 400 MHz): δ 3.68 [s, 6H, -($CO_2\underline{CH_3}$)$_2$], 5.74 (s, 1H, Ar-$\underline{CH}$-), 7.5 (d, J=2.02 Hz, 1H, Ar-$\underline{H}$), 7.70 (dd, J=8.4 Hz, 1.98 Hz, 1H, Ar-$\underline{H}$), 7.87 (d, J=8.41 Hz, 1H, Ar-$\underline{H}$)
IR (KBr, $cm^{-1}$): 3400-2300 (br), 1745 (s), 1720 (s), 1695 (s)
MS (m/e): 330 ($M^+$), 298 ($M^+$-$CH_3OH$)

| | | |
|---|---|---|
| Anal. Calcd.: | C, 43,53; | H, 3.35 |
| Found: | C, 43.56; | H, 3.23 |

M.P. 127-128 °C.

**EXAMPLE 6**

2-[(4-Bromo-2-fluorophenyl)methyl]-1,2,3,4-tetrahydro-1,3-dioxo-4-isoquinolinecarboxylic Acid Methyl Ester

A mixture of (2-carboxyphenyl)propanedioic acid dimethyl ester (5.0 g, 19.84 mmol) and $SOCl_2$ (20 g) was refluxed for 1 and 1/2 hours. The volatiles were removed in vacuo and the acid chloride was dissolved in THF (20 mL). In a second flask were placed 4-bromo-2-fluorobenzylamine (4.67 g, 22.91 mmol), triethylamine (15.96 mL, 114.55 mmol) and THF (150 mL). The contents of the first flask were added to the second flask and the mixture was stirred for 30 minutes. The formed suspension was poured into $H_2O$ (1000 mL), stirred for 10 minutes and acidified with HCl (2N). The mixture was extracted with EtOAc and the organic layer was dried over $MgSO_4$. Evaporation gave a yellowish solid which was recrystallised from acetone/ether/hexane (at -20 ° C) to yield a white solid (6.91 g, m.p. 149-150 °C).

NMR (DMSO-$d_6$, 400 MHz): δ [3.67, 3.99 (s, 3H, -$CO_2CH_3$, tautomeric)], [5.06 (q, J = 15.4 Hz), 5.29 (s) 2H, N-$CH_2$-, tautomeric], 5.03 (s, 1H, -$CHCO_2CH_3$, tautomeric), 7.07-8.44 (m, 7H, Ar-H , tautomeric)
IR (KBr, $cm^{-1}$): 1675 (s), 1610 (s), 1490 (s), 795 (m)
MS (m/e): 405 ($M^+$), 373 ($M^+$-MeOH)

| | | | |
|---|---|---|---|
| Anal. Calcd.: | C, 53.22; | H, 3.23; | N, 3.45 |
| Found: | C, 52.91; | H, 3.20; | N, 3.27 |

M.P. 149-150 °C.

The following compounds were prepared in substantially the same manner as that of Example 6.

2-[(4-Bromo-2-fluorophenyl)methyl-6-fluoro-1,2,3,4-tetrahydro-1,3-dioxo-4-isoquinolineacetic Acid Methyl Ester

NMR (DMSO-$d_6$, 400 MHz): δ 3.98 (s, 3H, -$CO_2CH_3$), 5.27 (s, 2H, -N$CH_2$-), 7.08 (t, J = 7.95 Hz, 1H, Ar-H), 7.2 (m, 1H, Ar-H), 7.34 (m, 2H, Ar-H, -OH), 7.54 (m, 1H, Ar-H), 8.1-8.26 (m, 2H, Ar-H)
IR (KBr, $cm^{-1}$): 1680 (s), 1660 (s), 1610 (s), 785 (m)
MS (m/e): 423 ($M^+$), 391 ($M^+$-$CH_3OH$)

| | | | |
|---|---|---|---|
| Anal. Calcd.: | C, 50.97; | H, 2.85; | N, 3.30 |
| Found: | C, 50.86; | H, 2.86; | N, 3.33 |

M.P. 157-158 °C.

2-[(4-Bromo-2-fluorophenyl)methyl]-7-methoxy-1,2,3,4-tetrahydro-1,3-dioxo-4-isoquinolineacetic Acid Ethyl Ester

NMR (DMSO-$d_6$, 400 MHz): δ [1.07 (t), 1.4 (t), 3H, -$CO_2CH_2CH_3$, tautomeric], [3.82 (s), 3.83 (s), 3H, -O$CH_3$, tautomeric], [4.1 (q), 4.46 (q), 2H, -$CO_2CH_2CH_3$, tautomeric], [5.05 (dd), 5.3 (s), tautomeric, -N$CH_2$-], [5.25 (s), 8.2 (brs), 1H, Ar-CH-, ArCH = C-OH], [7.1 (t), 7.2 (t), tautomeric, 1H, Ar-H], 7.35-7.4 (m, 3H, Ar-H), [7.58 (d), 7.62 (d), tautomeric, 1H, Ar-H], [7.56 (d), 8.41 (d) tautomeric, 1H, Ar-H]
IR (KBr, $cm^{-1}$): 3450 (s), 1680 (s), 1620 (s), 1610 (s)
MS (m/e): 449 ($M^+$), 403 ($M^+$-EtOH)
M.P. 139-140 °C.

6-Bromo-1,2,3,4-tetrahydro-2-methyl-1,3-dioxo-4-isoquinoline-carboxylic Acid Methyl Ester

NMR (DMSO-$d_6$, 400 MHz): δ [3.2 (s), 3.42 (s), 3H, tautomeric, N-$\underline{CH_3}$],[3.7 (s), 4.01(s), 3H, tautomeric, -$CO_2\underline{CH_3}$], [5.33 (s), 1H, tautomeric, Ar-$\underline{CH}$-], [7.5 (dd), 7.8 (dd), tautomeric, 1H, Ar-$\underline{H}$], [8.0 (d), 8.08 (d), tautomeric, 1H, Ar-$\underline{H}$], [8.51(d), 7.63 (d), tautomeric, 1H, Ar-$\underline{H}$]
IR (KBr, $cm^{-1}$): 1665 (s), 1590 (s), 780 (m)
MS (m/e): 311 ($M^+$)

| | | | |
|---|---|---|---|
| Anal. Calcd.: | C, 46.18; | H, 3.23; | N, 4.49 |
| Found: | C, 45.83; | H, 2.77; | N, 4.38 |

M.P. 190-191 °C.

**EXAMPLE 7**

(R)-(+)-2-[(4-Bromo-2-fluorophenyl)methyl]spiro[isoquinoline-4(1H),3'-pyrrolidine]-1,2',3,5'-tetrone;

(S)-(-)-2-[(4-Bromo-2-fluorophenyl)methyl]spiro[isoquinoline-4(1H),3'-pyrrolidine-1,2',3,5'-tetrone

**Step k)** Preparation of 2-[(4-Bromo-2-fluorophenyl)methyl]-1,2,3,4-tetrahydro-1,3-dioxo-4-isoquinolinecarboxylic Acid Menthyl Ester

To a cold (-78 °C) solution of 2-[(4-bromo-2-fluorophenyl)methyl]-1,3(2H, 3H)-isoquinolinedione (9.0 g, 25.86 mmol) in anhydrous THF (120 mL) was added lithium bis(trimethylsilyl)amide (1.0 M solution in THF, 56.91 mL, 56.91 mmol), dropwise over a 10 minute period. After stirring for 3 hours, (-)menthyl chloroformate (8.48 mL, 38.79 mmol) was added and the reaction was allowed to warm up to room temperature. The mixture during that period turned a dark color. It was stirred an additional 30 minutes and quenched with $H_2O$. The dark solution was poured into $H_2O$, acidified with HCl (2N) and extracted with EtOAc. The organic extracts were dried over $MgSO_4$. Evaporation and purification by flash chromatography on acid-washed silica gel (5% $H_3PO_4$ in MeOH) gave a clear oil (10.2 g).
NMR (DMSO-$d_6$, 200 MHz): δ 0.3-1.9 (m, 18H, menthyl), 4.3-4.6 (m, 1H, -$CO_2\underline{CH}$-), 5.0-5.35 (m, 3H, -N$\underline{CH_2}$-, Ar$\underline{CH}CO_2$-), 7.1-7.8 (m, 6H, Ar-$\underline{H}$), 8.2-8.5 (m, 1H, Ar-$\underline{H}$)
IR ($cm^{-1}$): 1780 (s), 1740 (s), 1690 (s), 835 (m)
MS (m/e): 529 ($M^+$), 391 ($M^+$-menthyl).

The following compound was prepared in substantially the same manner as that of Example 7, Step k).

2-[(4-Bromo-2-fluorophenyl)methyl-6-fluoro-1,2,3,4-tetrahydro-**1,3-dioxo-4-isoquinolinecarboxylic Acid Menthyl Ester**

NMR (DMSO-$d_6$, 200 MHz): δ 0.3-2.2 (m, 18H, menthyl), 4.4-4.7 (m, 1H, -$CO_2\underline{CH}$-), 5.0-5.4 (m, 2H, -$NCH_2$-), 7.1-7.6 (m, 4H, Ar-$\underline{H}$), 8.1-8.3 (m, 2H, Ar-$\underline{H}$)
IR (KBr, $cm^{-1}$): 3440 (m), 1690 (s), 1620 (s)
MS (m/e): 547 ($M^+$), 409 ($M^+$-menthyl).

**Step l)** Preparation of 2-[(4-Bromo-2-fluorophenyl)methyl]-1,2,3,4-tetrahydro-4-(menthoxycarbonyl)-1,3-dioxo-4-isoquinolineacetic Acid 1,1-Dimethylethyl Ester

To a mixture of 2-[(4-bromo-2-fluorphenyl)methyl]-1,2,3,4-tetrahydro-1,3-dioxo-4-isoquinoline carboxylic acid menthyl ester (7.62 g, 14.37 mmol), $K_2CO_3$ (3.96 g, 28.74 mmol) in DMF (100 mL) was added tert-butyl bromoacetate (4.64 mL, 28.74 mmol). The mixture was stirred at 85 °C for 2 hours, poured into $H_2O$ (1000 mL) and extracted with EtOAc. The organic extracts were dried over $MgSO_4$. Evaporation and purification by flash chromatography (hexane/EtOAc:4/1) gave a clear oil (6.9 g, diastereomeric mixture). The two diastereomers were separated by preparative HPLC on prep. Pac silica column, (μ poraSi/125 Å, 15-20 μsilica) by elution with hexane/$CH_2Cl_2$ to give the less polar A-diastereomer as a white solid (2.4 g) and the more polar diastereomer B as a clear oil (2.2 g). The diastereomeric purity was determined by analytical HPLC, on Dynamax silica 25 cmx4.1 mm and was found to be greater than 99% for both diastereomers.

Spectroscopic Data: Less Polar A-Diastereomer

NMR (DMSO-$d_6$, 400 MHz): δ 0.42 (m, 1H, menthyl), 0.56 (d, J = 7.4 Hz, 3H, -CHCH$_3$), 0.64 (d, J = 7.4 Hz, 3H, -CHCH$_3$), 0.72 (d, J = 6.2 Hz, 3H, -CHCH$_3$),0.92-0.97 (m, 2H, menthyl), 1.05 [s, 9H, -C(CH$_3$)$_3$], 1.22-1.39 (m, 3H, menthyl), 2.5-2.6 (m, 2H, menthyl), 3.55 (dd, J = 17.02 Hz, 2H, -CH$_2$CO$_2$-), 4.46 (m, 1H, -OCH-), 5.10 (dd, J = 15.02 Hz, 2H, -NCH$_2$-), 7.2 (t, J = 8.16 Hz, 1H, Ar-H), 7.33 (dd, J = 8.44 Hz, 1.7 Hz, 1H, Ar-H), 7.53 (dd, J = 9.7 Hz, 1.85 Hz, 1H, Ar-H), 7.6-7.77 (m, 2H, Ar-H), 7.79 (dt, J = 7.67 Hz, 1.35 Hz, 1H, Ar-H), 8.2 (dd, J = 7.77 Hz, 1.0 Hz, 1H, Ar-H)

IR (KBr, $cm^{-1}$): 1715 (s), 1670 (s), 735 (m)

MS (m/e): 644 $(M+H)^+$, 587 $[M+H-C(CH_3)_3]^+$

M.P. 50-51 °C.

Spectroscopic Data: More Polar B-Diastereomer

NMR (DMSO-$d_6$, 400 MHz): δ 0.1 (d, J = 6.6 Hz, 3H, -CHCH$_3$), 0.15 (m, 1H, menthyl), 0.42 (d, J = 6.6 Hz, 3H, -CHCH$_3$), 0.58-0.61 (m, 1H, menthyl), 0.64-0.9 (m, 3H, menthyl), 0.78 (d, J = 6.2 Hz, 3H, -CHCH$_3$), 1.04 [s, 9H, -C(CH$_3$)$_3$], 1.2-1.38 (m, 1H, menthyl), 1.4 (m, 1H, menthyl), 1.43-1.47 (m, 2H, menthyl), 3.54 (dd, J = 17.0 Hz, 2H, -CH$_2$CO$_2$-), 4.24 (m, 1H, -OCH-), 4.96 (d, J = 14.56 Hz, 1H, -NCH-), 5.32 (d, J = 14.56 Hz, 1H, -NCH-), 7.29 (t, J = 7.96 Hz, 1H, Ar-H), 7.35 (dd, J = 8.31 Hz, 1.8 Hz, 1H, Ar-H), 7.53-7.55 (m, 2H, Ar-H), 7.61 (dt, J = 8.29 Hz, 0.9 Hz, 1H, Ar-H), 7.76 (dt, J = 7.65 Hz, 1.4 Hz, 1H, Ar-H), 8.22 (dd, J = 7.85 Hz, 1.2 Hz, 1H, Ar-H)

IR ($CHCl_3$, $cm^{-1}$): 1715 (s), 1670 (s)

MS (m/e): 643 $(M^+)$, $[M^++H-C(CH_3)_3]$, 449 $[M^++H-C(CH_3)_3$-menthyl].

The following compounds were prepared in substantially the same manner as that of Example 7, Step 1).

**2-[(4-Bromo-2-fluorophenyl)methyl]-6-fluoro-1,2,3,4-tetrahydro-4-(menthoxycarbonyl)-1,3-dioxo-4-isoquinoline Acetic Acid 1,1-Dimethylethyl Ester**

Less Polar Diastereomer: A-Diastereomer

NMR (DMSO-$d_6$, 400 MHz): δ 0.54 (d, J = 6.86 Hz, 3H, -CHCH$_3$), 0.55 (m, 1H, menthyl), 0.65 (d, J = 6.97 Hz, 3H, -CHCH$_3$), 0.74 (d, J = 6.38 Hz, 3H, -CHCH$_3$),0.8-0.96 (m, 3H, menthyl), 1.09 [s, 9H, -C(CH$_3$)$_3$], 1.23-1.4 (m, 3H, menthyl), 1.5 (m, 2H, menthyl), 3.5 (d, J = 17.4 Hz, 1H, -CH$_2$CO$_2$-), 3.73 (d, J = 17.4 Hz, 1H, -CH$_2$CO$_2$-), 4.46 (dt, J = 10.7 Hz, 4.23 Hz, 1H, -OCH-), 5.1 (dd, J = I5.0 Hz, 2H, -NCH$_2$-), 7.2 (t, J = 8.15 Hz, 1H, Ar-H), 7.33 (dd, J = 8.3 Hz, 1.76 Hz, 1H, Ar-H), 7.48 (dt, J = 8.52 Hz, 2.4 Hz, 1H, Ar-H), 7.54 (dd, J = 9.75 Hz, 1.85 Hz, 1H, Ar-H), 7.61 (dd, J = 9.58 Hz, 2.46 Hz, 1H, Ar-H), 8.27 (dd, J = 8.81 Hz, 5.8 Hz, 1H, Ar-H)

IR (KBr, $cm^{-1}$): 1750 (s), 1730 (s), 1680 (s), 1620 (s)

MS (m/e): 662 $(M+H)^+$, 606 $[M+H-C(CH_3)_3]$, 468 ($[M+H-C(CH_3)_3$-menthyl], 424 $[M+H-C(CH_3)_3$-menthyl-$CO_2]$

| | | | |
|---|---|---|---|
| Anal. Calcd.: | C, 59.82; | H, 5.78; | N, 2.11 |
| Found: | C, 60.19; | H, 5.72; | N, 2.13 |

M.P. 54-55 °C.

More Polar Diastereomer: B-Diastereomer

NMR (DMSO-$d_6$, 400 MHz): δ 0.15 (m, 1H, menthyl), 1.73 (d, J = 6.87 Hz, 3H, -CHCH$_3$), 0.45 (d, J = 6.92 Hz, 3H, -CHCH$_3$), 0.6-0.75 (m, 2H, menthyl), 0.77 (d, J = 6.55 Hz, 3H, -CHCH$_3$), 0.8-0.95 (m, 2H), 1.09 [s, 9H, C-(CH$_3$)$_3$], 1.2-1.35 (m, 1H), 1.4-1.56 (m, 3H, Ar-H), 3.5 (d, J = 17.36 Hz, 1H, -CH$_2$CO$_2$-), 3.7 (d, J = 17.36 Hz, 1H, -CH$_2$CO$_2$-), 4.27 (dt, J = 10.54 Hz, 6.61 Hz, 1H, -OCH-), 5.0 (d, J = 14.67 Hz, 1H, -NCH$_2$-), 5.3 (d, J = 14.67 Hz, 1H, -NCH$_2$-), 7.28 (t, J = 8.0 Hz, 1H, Ar-H), 7.36 (dd, J = 8.34 Hz, 1.75 Hz, 1H, Ar-H), 7.50 (dt, J = 8.5 Hz, 2.5 Hz, 1H, Ar-H), 7.54 (dd, J = 9.61 Hz, 1.85 Hz, 1H, Ar-H), 7.6 (dd, J = 9.57 Hz, 2.38 Hz, 1H, Ar-H), 8.3 (dd, J = 8.83 Hz, 5.8 Hz, 1H, Ar-H)

IR (KBr, $cm^{-1}$): 1745 (s), 1725 (s), 1680 (s), 1610 (s)

MS (m/e): 662 $(M+H)^+$, 606 $[M+H-C(CH_3)_3]$, 468 $[M+H-C(CH_3)_3$-menthyl], 424 $[M+H-C(CH_3)_3$-menthyl-$CO_2]$

| | | | |
|---|---|---|---|
| Anal. Calcd.: | C, 59.82; | H, 5.78; | N, 2.11 |
| Found: | C, 59.91; | H, 5.55; | N, 2.20 |

M.P. 49-50 ° C.

**Step m)** Preparation of 2-[(4-Bromo-2-fluorophenyl)methyl]-1,2,3,4-tetrahydro-4-(menthoxycarbonyl)-1,3-dioxo-4-isoquinolineacetic Acid (A-Diastereomer)

A mixture of 2-[(4-bromo-2-fluorophenyl)methyl]-1,2,3,4-tetrahydro-4-(menthoxycarbonyl)-1,3-dioxo-4-isoquinolineacetic acid 1,1-dimethylethyl ester (less polar, A-diastereomer, 7.0 g, 10.87 mmol), $CH_2Cl_2$ (60 mL) and $CF_3CO_2H$ (15 mL) was stirred at room temperature for 5 hours. The volatiles were removed in vacuo and the residue was purified by flash chromatography on acid-washed silica gel (5% $H_3PO_4$ in MeOH), to give a white solid (6.0 g).
NMR (DMSO-$d_6$, 400 MHz): δ 0.5 (q, J = 7.4 Hz, 1H, -CHCH$_3$), 0.58 (d, J = 6.6 Hz, 3H, -CHCH$_3$), 0.66 (d, J = 6.6 Hz, 3H, -CHCH$_3$), 0.71 (d, J = 7.4 Hz, 3H, -CHCH$_3$), 0.82-1.05 (m, 3H, menthyl), 1.2-1.56 (m, 5H, menthyl), 3.6 (dd, J = 17.65 Hz, 2H, -CH$_2$CO$_2$H), 4.46 (dt, 10.67 Hz, 3.9 Hz, 1H, -OCH-), 5.1 (dd, J = 15.2 Hz, 2H, -NCH$_2$-), 7.17 (t, J = 8.2 Hz, 1H, Ar-H), 7.3 (dd, J = 8.28 Hz, 1.7 Hz, 1H, Ar-H), 7.53 (dd, J = 9.8 Hz 1.9 Hz, 1H, Ar-H), 7.6 (m, 2H, Ar-H), 7.77 (dt, J = 7.64 Hz, 1.4 Hz, 1H, Ar-H), 8.17 (dd, J = 8.16 Hz, 1.5 Hz, 1H, Ar-H)
IR (KBr, $cm^{-1}$): 3350-2700 (br), 1745 (s), 1720 (s), 1675 (s), 745 (m)
MS (m/e): 587 ($M^+$), 449 ($M^+$-menthyl)

| | | | |
|---|---|---|---|
| Anal. Calcd.: | C, 59.19; | H, 5.31; | N, 2.38 |
| Found: | C, 59.30; | H, 4.99; | N, 2.28 |

M.P. 79-80 ° C.

The following compound was prepared in substantially the same manner as that of Example 7, Step m).

**2-[(4-Bromo-2-fluorophenyl)methyl]-6-fluoro-1,2,3,4-tetrahydro-4-(menthoxycarbonyl)-1,3-dioxo-4-isoquinolineacetic Acid (A-Diastereomer)**

NMR (DMSO-$d_6$, 400 MHz): δ 0.51 (m, 1H, menthyl), 5.5 (d, J = 6.92 Hz, 3H, -CHCH$_3$), 0.68 (d, J = 6.96 Hz, 3H, -CHCH$_3$), 0.69 (m, 1H, menthyl), 0.74 (d, J = 6.46 Hz, 3H, -CHCH$_3$), 0.8-1.0 (m, 2H, menthyl), 1.25-1.4 (m, 3H, menthyl), 1.5 (m, 2H, menthyl), 3.55 (d, J = 17.9 Hz, 1H, -CH$_2$CO$_2$H), 3.77 (d, J = 17.9 Hz, 1H, -CH$_2$CO$_2$H), 4.46 (dt, J = 10.81 Hz, 4.51 Hz, 1H, -OCH-), 5.07 (dd, J = 15.2 Hz, 2H, -NCH$_2$-), 7.15 (t, J = 8.24 Hz, 1H, Ar-H), 7.31 (dd, J = 8.35 Hz, 1.82 Hz, 1H, Ar-H), 7.46 (dt, J = 8.49 Hz, 2.49 Hz, 1H, Ar-H), 7.54 (dd, J = 9.77 Hz, 1.87 Hz, 1H, Ar-H), 7.61 (dd, J = 9.65 Hz, 2.45 Hz, 1H, Ar-H), 8.25 (dd, J = 8.8 Hz, 5.87 Hz, 1H, Ar-H), 12.75 (brs, 1H, -CO$_2$H)
IR (KBr, $cm^{-1}$): 3450-2700 (br), 1745 (s), 1720 (s), 1680 (s), 1610 (s)
MS (m/e): 606 $(M+H)^+$, 468 (M + H-menthyl)

| | | | |
|---|---|---|---|
| Anal. Calcd.: | C, 57.43; | H, 4.99; | N, 2.31 |
| Found: | C, 57.40; | H, 4.76; | N, 2.35 |

M.P. 84-85 ° C.

**Step n)** Preparation of 2-[(4-Bromo-2-fluorophenyl)methyl]-1,2,3,4-tetrahydro-4-(menthoxycarbonyl)-1,3-dioxo-4-isoquinolineacetic Acid (B-Diastereomer)

A mixture of 2-[(4-bromo-2-fluorophenyl)methyl]-1,2,3,4-tetrahydro-4-(menthoxycarbonyl)-1,3-dioxo-4-isoquinolineacetic acid 1,1-dimethylethyl ester (more polar, B-diastereomer, 6.8 g, 10.56 mmol), $CH_2Cl_2$ (80 mL) and $CF_3CO_2H$ (20 mL) was stirred at room temperature for 5 hours. The volatiles were removed in vacuo and the residue was purified by flash chromatography on acid-washed silica gel (5% $H_3PO_4$ in MeOH) to give a white solid (5.81 g).
NMR (DMSO-$d_6$, 400 MHz): δ 0.16 (d, J = 6.6 Hz, 3H, -CHCH$_3$), 0.22 (q, J = 7.2 Hz, 1H, -CHCH$_3$), 0.42 (d, J = 6.6 Hz, 3H, -CHCH$_3$), 0.6 (m, 1H, menthyl), 0.8 (d, J = 7.2 Hz, 3H, -CHCH$_3$), 0.7-0.95 (m, 4H, menthyl), 1.25 (m, 1H, menthyl), 1.42 (m, 1H, Ar-H), 1.45-1.48 (m, 2H, menthyl), 3.6 (dd, J = 17.75 Hz, 2H, -CH$_2$CO$_2$H), 4.25 (dt, J = 10.7 Hz, 3.2 Hz, 1H, -OCH-), 5.0 (dd, J = 14.7 Hz, 2H, -NCH$_2$-), 7.27 (t, J = 8.05 Hz, 1H, Ar-H), 7.34 (dd, J = 8.28 Hz, 1.7 Hz, 1H, Ar-H), 7.53-7.61 (m, 3H, Ar-H), 7.74 (dt, J = 7.65 Hz, 1.3 Hz, 1H,

Ar-H), 8.2 (dd, J = 7.86 Hz, 1.16 Hz, 1H, Ar-H)
IR (KBr, $cm^{-1}$): 3350-2700 (br), 1745 (s), 1720 (s), 1675 (s), 745 (m)
MS (m/e): 588 $(M + H)^+$, 450 (M + H-menthyl)

| | | | |
|---|---|---|---|
| Anal. Calcd.: | C, 59.19; | H, 5.31; | N, 2.38 |
| Found: | C, 59.40; | H, 5.08; | N, 2.22 |

M.P. 132-133 ° C.

The following compound was prepared in substantially the same manner as that of Example 7, Step n).

**2-[(4-Bromo-2-fluorophenyl)methyl]-6-fluoro-1,2,3,4-tetrahydro-4-(menthoxycarbonyl)-1,3-dioxo-4-isoquinolineacetic Acid (B-Diastereomer)**

NMR (DMSO-$d_6$, 400 MHz): δ 0.19 (d, J = 6.89 Hz, 3H, $-CHCH_3$), 0.23 (m, 1H, menthyl), 0.45 (d, J = 6.92 Hz, 3H, $-CHCH_3$), 0.6 (m, 1H, menthyl), 0.72 (m, 1H, menthyl), 0.77 (d, J = 6.52 Hz, 3H, $-CHCH_3$), 0.8-0.9 (m, 2H, menthyl), 1.3 (m, 1H, menthyl), 1.42 (m, 1H, menthyl), 1.5-1.54 (m, 2H, menthyl), 3.52 (d, J = 17.92 Hz, 1H, $-CH_2CO_2H$), 3.75 (d, J = 17.92 Hz, 1H, $-CH_2CO_2H$), 4.28 (dt, J = 10.6 Hz, 4.24 Hz, 1H, -OCH-), 4.93 (d, J = 14.75 Hz, 1H, $-NCH_2-$), 5.29 (d, J = 14.75 Hz, 1H, $-NCH_2-$), 7.25 (t, J = 8.09 Hz, 1H, ArH), 7.34 (dd, 8.27 Hz, 1.74 Hz, 1H, Ar-H), 7.46 (dt, J = 8.57 Hz, 2.4 Hz, 1H, Ar-H), 7.53 (dd, J = 9.62 Hz, 1.84 Hz, 1H, Ar-H), 7.58 (dd, J = 9.6 Hz, 2.42 Hz, 1H, Ar-H), 8.26 (dd, J = 8.85 Hz, 5.87 Hz, 1H, Ar-H), 12.75 (brs, 1H, $-CO_2H$)
IR (KBr, $cm^{-1}$): 3450-2700 (br), 1750 (s), 1725 (s), 1680 (s), 1615 (s)
MS (m/e): 606 $(M + H)^+$, 468 (M + H-menthyl), 424 (M + H-menthyl-$CO_2$)

| | | | |
|---|---|---|---|
| Anal. Calcd.: | C, 57.43; | H, 4.99; | N, 2.31 |
| Found: | C, 57.09; | H, 4.72; | N, 2.34 |

M.P. 79-80 ° C.

**Step o)** Preparation of 4-(2-Amino-2-oxoethyl)-2-[(4-bromo-2-fluorophenyl)-methyl]-1,2,3,4-tetrahydro-1,3-dioxo-4-isoquinolinecarboxylic Acid Menthyl Ester (A-Diastereomer)

A mixture of 2-[(4-bromo-2-fluorophenyl)methyl]-1,2,3,4-tetrahydro-4-(menthoxycarbonyl)-1,3-dioxo-4-isoquinolineacetic acid (A-diastereomer, 5.5 g, 9.35 mmol) and $SOCl_2$ (30 mL) was refluxed for 2 hours. The volatiles were removed in vacuo and the acid chloride was dissolved in THF (20 mL). In a second flask was placed a freshly prepared, saturated $NH_3$/THF solution (100 mL) and the contents of the first flask were added slowly. After the addition, the mixture was stirred for 15 minutes, poured into $H_2O$ (500 mL), acidified with HCl (2N) and extracted with EtOAc. The organic extracts were dried over $MgSO_4$. Evaporation and purification by flash chromatography (hexane/EtOAc, 1/1) gave a white solid (4.86 g).
NMR (DMSO-$d_6$, 400 MHz): δ 0.44 (q, J = 7.4 Hz, IH, $-CHCH_3$), 0.57 (d, J = 6.6 Hz, 3H, $-CHCH_3$), 0.64 (d, J = 6.6 Hz, 3H, $-CHCH_3$), 0.71 (d, J = 7.4 Hz, 3H, $-CHCH_3$), 0.84-1.04 (m, 3H, menthyl), 1.2-1.4 (m, 2H, menthyl), 1.42-1.58 (m, 3H, menthyl), 3.47 (dd, J = 16.57 Hz, 2H, $-CH_2CONH_2$), 4.46 (dt, J = 10.7 Hz, 3.9 Hz, 1H, -OCH-), 5.05 (dd, J = 15.4 Hz, 2H, $-NCH_2-$), 6.87 (s, 1H, -CONH-), 7.27 (m, 2H, Ar-H), 7.43 (d, J = 7.8 Hz, 1H, Ar-H), 7.55 (m, 3H, Ar-H, -CONH-), 7.76 (dt, J = 7.6 Hz, 1.1 Hz, 1H, Ar-H), 8.13 (dd, J = 7.85 Hz, 1.1 Hz, 1H, Ar-H)
IR (KBr, $cm^{-1}$): 3450 (s), 3320 (s), 1730 (s), 1715 (s), 1670 (s), 745 (m)
MS (m/e): 587 $(M + H)^+$, 449 (M + H-menthyl)

| | | | |
|---|---|---|---|
| Anal. Calcd.: | C, 59.29; | H, 5.49; | N, 4.77 |
| Found: | C, 59.29; | H, 5.40; | N, 4.74 |

M.P. 94-95 ° C.

The following compound was prepared in substantially the same manner as that of Example 7, Step o).

**4-(2-Amino-2-oxoethyl)-2-[(4-bromo-2-fluorophenyl)methyl]-6-fluoro-1,2,3,4-tetrahydro-1,3-dioxo-4-isoquinolinecarboxylic Acid Menthyl Ester (A-Diastereomer)**

NMR (DMSO-$d_6$, 400 MHz): δ 0.48 (m, 1H, menthyl), 0.55 (d, J = 6.89 Hz, 3H, -CHCH$_3$), 0.68 (d, J = 6.99 Hz, 3H, -CHCH$_3$), 0.7 (m, 1H, menthyl), 0.74 (d, J = 6.44 Hz, 3H, -CHCH$_3$), 0.84-1.0 (m, 2H, menthyl), 1.25-1.4 (m, 3H, menthyl), 1.45-1.49 (m, 2H, menthyl), 3.45 (d, J = 16.6 Hz, 1H, -CH$_2$CONH$_2$), 3.57 (d, J = 16.6 Hz, 1H, -CH$_2$CONH$_2$), 4.47 (dt, J = 10.71 Hz, 4.2 Hz, 1H, -OCH-), 5.1 (dd, J = 15.35 Hz, 2H, -NCH$_2$-), 6.93 (s, 1H, -CONH$_2$), 7.22-7.3 (m, 2H, Ar-H), 7.35 (dd, J = 9.48 Hz, 2.43 Hz, 1H, Ar-H), 7.44 (dt, J = 8.55 Hz, 2.49 Hz, 1H, Ar-H), 7.51 (s, 1H, -CH$_2$CONH$_2$), 7.53 (dd, J = 9.66 Hz, 1.6 Hz, 1H, Ar-H), 8.21 (dd, J = 8.79 Hz, 5.85 Hz, 1H, Ar-H)
IR (KBr, cm$^{-1}$): 3450 (s), 3380 (s), 1750 (s), 1725 (s), 1675 (s), 1610 (s)
MS (m/e): 605 (M + H)$^+$, 467 (M + H-menthyl), 423 (M + H-menthyl-CO$_2$)

| | | | |
|---|---|---|---|
| Anal. Calcd.: | C, 57.53; | H, 5.16; | N, 4.63 |
| Found: | C, 57.52; | H, 4.99; | N, 4.58 |

M.P. 93-94 °C.

**Step p)** Preparation of 4-(2-Amino-2-oxoethyl)-2-[(4-bromo-2-fluorophenyl)-methyl]-1,2,3,4-tetrahydro-1,3-dioxo-4-isoquinolinecarboxylic Acid Menthyl Ester (B-Diastereomer)

A mixture of 2-[(4-bromo-2-fluorophenyl)methyl]-1,2,3,4-tetrahydro-4-(menthoxycarbonyl)-1,3-dioxo-4-isoquinolineacetic acid (B-diastereomer, 5.5 g, 9.35 mmol) and SOCl$_2$ (30 mL) was refluxed for 2 hours. The volatiles were removed in vacuo and the residue was dissolved in THF (20 mL). In a second flask was placed a freshly prepared, saturated NH$_3$/THF solution (100 mL), and the contents of the first flask were added slowly. After the additon, the mixture was stirred for 15 minutes, poured into H$_2$O (500 mL), acidified with HCl (2N) and extracted with EtOAc. The organic extracts were dried over MgSO$_4$. Evaporation and purification by flash chromatography (hexane/EtOAc, 1/1) gave a white solid (4.75 g).
NMR (DMSO-$d_6$, 200 MHz): δ 0.14 (d, J = 6.6 Hz, 3H, -CHCH$_3$), 0.25 (m, 1H, menthyl), 0.4 (d, J = 6.8 Hz, 3H, -CHCH$_3$), 0.6 (m, 3H, menthyl), 0.77 (d, J = 7.0 Hz, 3H, -CHCH$_3$), 1.3-1.6 (m, 5H, menthyl), 3.49 (dd, J = 16.6 Hz, 2H, -CH$_2$CONH$_2$), 4.3 (dt, J = 10.6 Hz, 3.8 Hz, 1H, -OCH-), 4.9 (d, J = 14.6 Hz, 1H, -NCH-), 5.26 (d, J = 14.6 Hz, 1H, -NCH-), 6.85 (s, 1H, -CONH), 7.31 (m, 2H, Ar-H), 7.4 (d, J = 7.4 Hz, 1H, Ar-H), 7.5-7.6 (m, 3H, Ar-H, -CONH-), 7.7 (dt, J = 7.4 Hz, 2.0 Hz, 1H, Ar-H), 8.14 (dd, J = 7.4 Hz, 1.6 Hz, 1H, Ar-H)
IR (KBr, cm$^{-1}$): 3460 (s), 3315 (s), 1735 (s), 1715 (s), 1675 (s), 745 (s)
MS (m/e): 587 (M + H)$^+$, 449 (M + H-menthyl)

| | | | |
|---|---|---|---|
| Anal. Calcd.: | C, 59.29; | H, 5.49; | N, 4.77 |
| Found: | C, 59.40; | H, 5.39; | N, 4.76 |

M.P. 151-152 °C.

The following compound was prepared in substantially the same manner as that of Example 7, Step p).

**4-(2-Amino-2-oxoethyl)-2-[(4-bromo-2-fluorophenyl)methyl]-6-fluoro-1,2,3,4-tetrahydro-1,3-dioxo-4-isoquinolinecarboxylic Acid Menthyl Ester (B-Diastereomer)**

NMR (DMSO-$d_6$, 400 MHz): δ 0.19 (d, J = 6.89 Hz, 3H, -CHCH$_3$), 0.21 (m, 1H, menthyl), 0.45 (d, J = 6.95 Hz, 3H, -CHCH$_3$), 0.6 (m, 1H, menthyl), 0.7 (m, 1H, menthyl), 0.78 (d, J = 6.52 Hz, 3H, -CHCH$_3$), 0.8-0.9 (m, 2H, menthyl), 1.3 (m, 1H, menthyl), 1.43 (m, 1H, menthyl), 1.5-1.57 (m, 2H, menthyl), 3.43 (d, J = 16.67 Hz, 1H, -CH$_2$CONH$_2$), 3.55 (d, J = 16.67 Hz, 1H, -CH$_2$CONH$_2$), 4.3 (dt, J = 10.49 Hz, 6.42 Hz, 1H, -OCH-), 4.9 (d, J = 14.78 Hz, 1H, -NCH$_2$-), 5.3 (d, J = 14.78 Hz, 1H, -NCH$_2$-), 6.09 (s, 1H, -CONH$_2$), 7.26-7.34 (m, 3H, Ar-H), 7.43 (dt, J = 8.57 Hz, 2.43 Hz, 1H, Ar-H), 7.50 (s, 1H, -CONH$_2$), 7.53 (dd, J = 9.6 Hz, 1.73 Hz, 1H, Ar-H ), 8.24 (dd, J = 8.81 Hz, 5.9 Hz, 1H, Ar-H)
IR (KBr, cm$^{-1}$): 3485 (s), 3360 (s), 1740 (s), 1730 (s), 1685 (s), 1675 (s), 1615 (s)
MS (m/e): 605 (M + H)$^+$, 467 (M + H-menthyl), 423 (M + H-menthyl-CO$_2$)

| | | | |
|---|---|---|---|
| Anal. Calcd.: | C, 57.53; | H, 5.16; | N, 4.63 |
| Found: | C, 57.62; | H, 4.89; | N, 4.58 |

M.P. 111-112 °C.

**Step q)** Preparation of (S)-(-)-2-[(4-Bromo-2-fluorophenyl)methyl]spiro-[isoquinoline-4(1H),3'-pyrrolidine]-1,2',3,5'(2H)-tetrone

To a cold (-78 ° C) solution of 4-(2-amino-2-oxoethyl)-2-[(4-bromo-2-fluorophenyl)methyl]-1,2,3,4-tetrahydro-1,3-dioxo-4-isoquinolinecarboxylic acid menthyl ester (A-diastereomer, 3.5 g, 5.96 mmol) in anhydrous THF (80 mL) was added lithium bis(trimethylsilyl)amide (1.0M in THF, 5.96 mL, 5.96 mmol) dropwise over a 5 minute period. After stirring at -78 ° C for 20 minutes, the reaction was quenched with HCl (2N) and the mixture was poured into $H_2O$ and extracted with EtOAc. The organic extracts were dried over $MgSO_4$. Evaporation and purification by flash chromatography on acid-washed silica gel (5% $H_3PO_4$ in MeOH) gave a white solid, which was recrystallised once from ethyl ether (at -20 °C) to yield a crystalline white solid (1.1 g). The enantiomeric purity was determined by analytical HPLC (Chiralcel OB) and was found to be greater than 99% (99.6% ee).

NMR (DMSO-$d_6$, 400 MHz): δ 3.4 (dd, J = 18.35 Hz, 2H, -$CH_2$CONH-), 5.06 (s, 2H, -N$CH_2$-), 7.14 (t, J = 8.21 Hz, 1H, Ar-H), 7.32 (dd, J = 8.34 Hz, 1.67 Hz, 1H, Ar-H), 7.53 (dd, J = 9.83 Hz, 1.87 Hz, 1H, Ar-H), 7.6 (dt, J = 7.85 Hz, 1.03 Hz, 1H, Ar-H), 7.68 (d, J = 7.76 Hz, 1H, Ar-H), 7.77 (dt, J = 7.53 Hz, 1.34 Hz, 1H, Ar-H), 8.15 (dd, J = 7.88 Hz, 1.25 Hz, 1H, Ar-H), 12.01 (s, 1H, -CONHCO-)

IR (KBr, $cm^{-1}$): 3340 (s), 1735 (s), 1710 (s), 1675 (s), 1490 (m), 1340 (s), 810 (m), 755 (m)

MS (m/e): 430 ($M^+$), 387 ($M^+$-CONH)

| | | | |
|---|---|---|---|
| Anal. Calcd.: | C, 52.92; | H, 2.80; | N, 6.50 |
| Found: | C, 53.01; | H, 2.75; | N, 6.45 |

$[\alpha]_D^{25}$ = -63.3 (c = 1.02, EtOAc)

M.P. 175-176 °C.

The following compound was prepared in substantially the same manner as that of Example 7, Step q).

**(S)-(-)-[2-[(4-Bromo-2-fluorophenyl)methyl]-6-fluoro]spiro[isoquinoline-4(1H),3'-pyrrolidine]-1,2',3,5'-(2H)-tetrone**

NMR (DMSO-$d_6$, 400 MHz): δ 3.43 (s, 2H, -$CH_2$CONH-), 5.05 (s, 2H, -N$CH_2$-), 7.13 (t, J = 8.26 Hz, 1H, Ar-H), 7.33 (dd, J = 8.3 Hz, 1.81 Hz, 1H, Ar-H), 7.47 (dt, J = 8.57 Hz, 2.4 Hz, 1H, Ar-H), 7.53 (dd, J = 9.8 Hz, 1.9 Hz, 1H, Ar-H), 7.75 (dd, J = 9.8 Hz, 2.4 Hz, 1H, Ar-H), 8.22 (dd, J = 8.81 Hz, 5.83 Hz, 1H, Ar-H), 12.0 (s, 1H, -CONHCO-)

IR (KBr, $cm^{-1}$): 3325 (s), 1740 (s), 1715 (s), 1675 (s), 1335 (s), 710 (m)

MS (m/e): 449 $(M + H)^+$

| | | | |
|---|---|---|---|
| Anal. Calcd.: | C, 50.80; | H, 2.47; | N, 6.24 |
| Found: | C, 51.00; | H, 2.64; | N, 6.11 |

$[\alpha]_D^{25}$ = -41.9 (c = 1.0, EtOAc)

M.P. 247-248 °C.

**Step r)** Preparation of (R)-(+)-2-[(4-Bromo-2-fluorophenyl)methyl]spiro-[isoquinoline-4(1H),3'-pyrrolidine]-1,2',3,5'(2H)-tetrone

To a cold (-78 ° C) solution of 4-(2-amino-2-oxoethyl)-2-[(4-bromo-2-fluorophenyl)methyl]-1,2,3,4-tetrahydro-1,3-dioxo-4-isoquinolinecarboxylic acid menthyl ester (B-diastereomer, 3.0 g, 5.11 mmol) in anhydrous THF (60 mL) was added lithium bis(trimethylsilyl)amide (1.0M in THF, 5.11 mL, 5.11 mmol) dropwise over a 5 minute period. After stirring at -78 ° C for 20 minutes, the reaction was quenched with HCl (2N) and the mixture was poured into $H_2O$ and extracted with EtOAc. The organic extracts were dried

over $MgSO_4$. Evaporation and purification by flash chromatography on acid-washed silica gel (5% $H_3PO_4$ in MeOH) gave a white solid, which was recrystallised once from ethyl ether (at -20 °C) to yield a crystalline white solid (1.2 g). The enantiomeric purity was determined by analytical HPLC (Chiralcel OB) and was found to be greater than 99% (99.2% ee).

NMR (DMSO-$d_6$, 400 MHz): $\delta$ 3.4 (dd, J = 18.35 Hz, 2H, -$\underline{CH_2}$CONH-), 5.06 (s, 2H, -N$\underline{CH_2}$-), 7.14 (t, J = 8.21 Hz, 1H, Ar-$\underline{H}$), 7.32 (dd, J = 8.34 Hz, 1.67 Hz, 1H, Ar-$\underline{H}$), 7.5 (dd, J = 9.83 Hz, 1.87 Hz, 1H, Ar-$\underline{H}$), 7.6 (dt, J = 7.85 Hz, 1.03 Hz, 1H, Ar-$\underline{H}$), 7.68 (d, J = 7.76 Hz, 1H, Ar-$\underline{H}$), 7.77 (dt, J = 7.53 Hz, 1.34 Hz, 1H, Ar-$\underline{H}$), 8.15 (dd, J = 7.88 Hz, 1.25 Hz, 1H, Ar-$\underline{H}$), 12.01 (s, 1H, -CON$\underline{H}$CO-)

IR (KBr, $cm^{-1}$): 3340 (s), 1735 (s), 1710 (s), 1675 (s), 1490 (m), 1340 (s), 810 (m), 755 (m)

MS (m/e): 430 (M+), 387 (M+-COHN)

| | | | |
|---|---|---|---|
| Anal. Calcd.: | C, 52.92; | H, 2.80; | N, 6.50 |
| Found: | C, 52.75; | H, 2.54; | N, 6.26 |

$[\alpha]_D^{25}$ = +62.3 (c = 0.8, EtOAc)

M.P. 175-176 °C.

The following compound was prepared in substantially the same manner as that of Example 7, Step r).

**(R)-(+)-[2-(4-Bromo-2-fluorophenyl)methyl]-6-fluoro]spiro[isoquinoline-4(1H),3'-pyrrolidine]-1,2',3,5'-(2H)-tetrone**

NMR (DMSO-$d_6$, 400 MHz): $\delta$ 3.43 (s, 2H, -$\underline{CH_2}$CONH-), 5.05 (s, 2H, -N$\underline{CH_2}$-), 7.13 (t, J = 8.26 Hz, 1H, Ar-$\underline{H}$), 7.33 (dd, J = 8.3 Hz, 1.81 Hz, 1H, Ar-$\underline{H}$), 7.47 (dt, J = 8.57 Hz, 2.4 Hz, 1H, Ar-$\underline{H}$), 7.53 (dd, J = 9.8 Hz, 1.9 Hz, 1H, Ar-$\underline{H}$), 7.75 (dd, J = 9.8 Hz, 2.4 Hz, 1H, Ar-$\underline{H}$), 8.22 (dd, J = 8.81 Hz, 5.83 Hz, 1H, Ar-$\underline{H}$), 12.0 (s, 1H, -CON$\underline{H}$CO-)

IR (KBr, $cm^{-1}$): 3325 (s), 1740 (s), 1715 (s), 1675 (s), 1335 (s), 710 (m)

MS (m/e): 449 $(M+H)^+$

| | | | |
|---|---|---|---|
| Anal. Calcd.: | C, 50.80; | H, 2.47; | N, 6.24 |
| Found: | C, 51.06; | H, 2.64; | N, 6.12 |

$[\alpha]_D^{25}$ = +40.4 (c = 1.01, EtOAc)

M.P. 247-248 ° C.

**EXAMPLE 8**

The crystals of (-)-2-[(4-bromo-2-fluorophenyl)methyl]spiro[isoquinoline-4(1H),3'-pyrrolidine]-1,2',3,5'-(2H)-tetrone, were obtained from ethyl ether. The data were measured at room ternperature on a Nicolet R3m diffractometer using a crystal of dimensions 0.35x0.42x0.50 mm and Cuka (λ = 1.54178Å) radiation. An n refinement was carried out to determine the absolute configuration. The value for n refined to 1.04 for the structure with C(4)S stereochemistry indicating that to be the correct absolute stereochemistry [D. Rogers; Acta. Cryst. A37, 734, (1981)].

## Claims

### Claims for the following Contracting States : AT, BE, CH, DE, FR, GB, IT, LI, LU, NL, SE

**1.** A compound of formula (I)

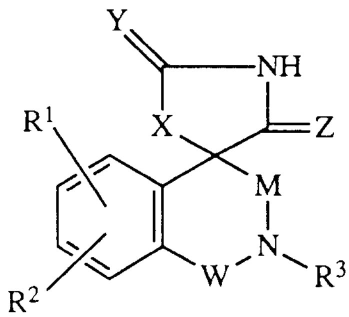

(I)

and analogues thereof wherein the fused benzene ring is replaced by a fused thiophene, pyridine or furan ring, wherein $R^1$ and $R^2$ are independently hydrogen, alkyl containing 1 to 6 carbon atoms, halogen, lower alkoxy containing 1 to 6 carbon atoms, trifluoromethyl, lower alkylthio wherein lower alkyl contains 1 to 6 carbon atoms, dialkylamino wherein alkyl contains 1 to 6 carbon atoms, nitro, aryl, or aryl (lower alkyl) oxy wherein aryl contains 6 to 10 carbon atoms and lower alkyl contains 1 to 6 carbon atoms; $R^3$ is hydrogen, lower alkyl containing 1 to 6 carbon atoms, aryl (lower alkyl) or halogen substituted aryl (lower alkyl) wherein aryl contains 6 to 10 carbon atoms and lower alkyl contains 1 to 6 carbon atoms or heterocyclic (lower alkyl) of structural formula

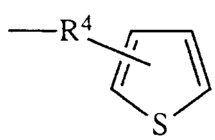

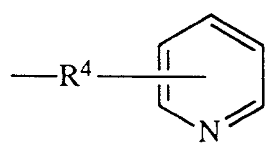

wherein $R^4$ is lower alkylene containing 1 to 3 carbon atoms; X is lower alkylene containing 1 to 3 carbon atoms, oxygen, sulfur or NH; Y and Z are independently oxygen or sulfur, M and W are independently carbonyl, thiocarbonyl, sulfonyl, sulfoxo or lower alkylene containing 1 to 2 carbon atoms with the provisos that (i) M and W are not both lower alkylene and (ii) when M is $CH_2$, W is other than sulfonyl, or a pharmaceutically acceptable salt thereof.

**2.** A compound according to claim 1 of formula (II)

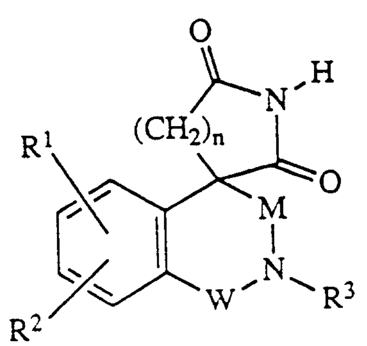

(II)

wherein $R^1$ and $R^2$ are independently hydrogen, alkyl containing 1 to 6 carbon atoms, halogen, lower alkoxy containing 1 to 6 carbon atoms, trifluoromethyl, lower alkylthio wherein lower alkyl contains 1 to 6 carbon atoms; $R^3$ is hydrogen, lower alkyl containing 1 to 6 carbon atoms; M and W are carbonyl or thiocarbonyl; n is 1 to 3, or a pharmaceutically acceptable salt thereof.

**3.** A compound according to claim 1 of formula (II)

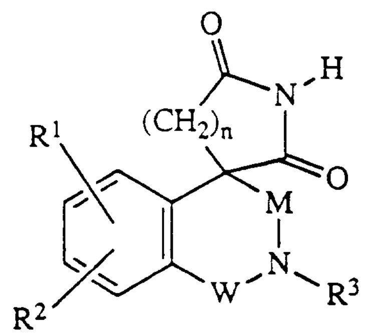

(II)

wherein $R^1$ and $R^2$ are independently hydrogen, alkyl containing 1 to 6 carbon atoms, halogen, lower alkoxy containing 1 to 6 carbon atoms, trifluoromethyl, lower alkythio wherein lower alkyl contains 1 to 6 carbon atoms; $R^3$ is aryl-(lower alkyl) or dihalogen substituted aryl(lower alkyl) wherein aryl contains 6 to 10 carbon atoms and lower alkyl contains 1 to 6 carbon atoms; M and W are carbonyl or thiocarbonyl; n is 1 to 3, or a pharmaceutically acceptable salt thereof.

**4.** A compound according to claim 1 of formula (III)

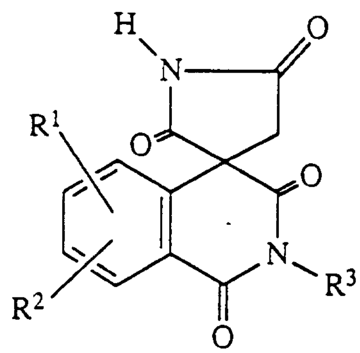

(III)

wherein $R^1$ and $R^2$ are hydrogen or halogen; $R^3$ is lower alkyl containing 1 to 6 carbon atoms, benzyl or dihalogen substituted benzyl, or a pharmaceutically acceptable salt thereof.

**5.** A compound according to Claim 1 which is one of the following:
2-[(4-bromo-2-fluorophenyl)methyl]spiro[isoquinoline-4 (1H), 3'-pyrrolidine]-1,2',3,5'(2H)-tetrone,
[2-[(4-bromo-2-fluorphenyl)methyl]-7-chloro]spiro[isoquinoline-4(1H),3'pyrrolidine]-1,2',3,5'(2H)-tetrone,
[2-[(4-bromo-2-fluorophenyl)methyl]-6-chloro]spiro[iso quinoline-4(1H),3'-pyrrolidine]-1,2',3,5'(2H)-tetrone,
2-[(3,4-dichlorophenyl)methyl]spiro[isoquinoline-4(1H), 3'-pyrrolidine]-1,2',3,5'(2H)-tetrone,
2-[(4-bromo-2-fluorophenyl)methyl]-5-fluoro]spiro[isoquinoline-4(1H),3'-pyrrolidine]-1,2',3,5'(2H)-tetrone,
[2-[(4-bromo-2-fluorophenyl)methyl]-6-fluoro]spiro[isoquinoline-4(1H),3'-pyrrolidine]-1,2',3,5'(2H)-tetrone,
[2-[(4-bromo-2-fluorophenyl)methyl]-7-fluoro]spiro[isoquinoline-4-(1H),3'-pyrrolidine]1,2',3,5'(2H)-tetrone,
[2-[(4-bromo-2-fluorophenyl)methyl]-8-fluoro]spiro[isoquinoline-4(1H),3'-pyrrolidine]-1,2',3,5'(2H)-tetrone,
[6-bromo-2-[(4-bromo-2-fluorophenyl)methyl]-7-methoxy]-spiro[isoquinoline-4(1H),3'-pyrrolidine]-1,2',3,5'(2H)-tetrone,
[2-[(4-bromo-2-fluorophenyl)methyl]-7-methoxy]spiro[isoquinoline-4(1H),3'-pyrrolidine]-1,2',3,5'(2H)-tetrone,
(R)-(+)-2-[(4-bromo-2-fluorophenyl)methyl]spiro[isoquinoline-4(1H)3'-pyrrolidine]-1,2',3,5'(2H)-tetrone,
(S)-(-)-2-[(4-bromo-2-fluorophenyl)methyl]spiro[isoquinoline-4(1H),3'-pyrrolidine]-1,2',3,5'(2H)-tetrone,
(R)-(+)-[(4-bromo-2-fluorophenyl)methyl]-6-fluoro]-spiro[isoquinoline-4(1H),3'-pyrrolidine]-1,2',3,5'(2H)-tetrone,
(S)-(-)-[(2-[(4-bromo-2-fluorophenyl)methyl]-6-fluoro]-spiro[isoquinoline-4(1H),3'-pyrrolidine]-1,2'3,5'(2H)-tetrone,
2-methylspiro[isoquinoline-4(1H),3'pyrrolidine]-1,2',3,5'(2H)-tetrone,
6-chloro-2-methylspiro[isoquinoline-4(1H),3'-pyrrolidine]-1,2',3,5'(2H)-tetrone,
6-bromo-2-methylspiro[isoquinoline-4(1H),3'-pyrrolidine]-1,2',3,5'(2H)-tetrone,
7-chloro-2-methylspiro[isoquinoline-4(1H),3'-pyrrolidine]-1,2',3,5'(2H)-tetrone,
2-ethylspiro[isoquinoline-4(1H),3'-pyrrolidine]-1,2'3,5'(2H)-tetrone,
2-propylspiro[isoquinoline-4(1H),3'-pyrrolidine]-1,2',3,5'(2H)-tetrone,
2-butylspiro[isoquinoline-4(1H),3'-pyrrolidine]-1,2',3, 5'(2H)-tetrone; or a pharmaceutically acceptable salt thereof.

**6.** A compound as claimed in any one of Claims 1 to 5 when in the form of a pharmaceutically acceptable ammonium alkali metal or alkaline earth metal salt.

**7.** A process for preparing a compound of formula I as claimed in Claim 1 which comprises one of the following:

a)cyclising an amide of formula (A)

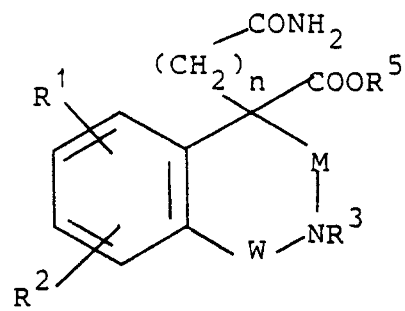

(A)

wherein $R^1$, $R^2$, $R^3$, n, $M^1$ and W are as defined above and $COOR^5$ is an ester group, using a suitable base to give a compound of formula I wherein Y and Z are both oxygen and X is $-(CH_2)_n-$ wherein n is 1 to 3;

b) reacting a compound of formula (B)

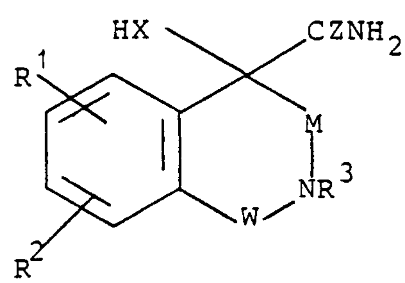

(B)

wherein $R^1$, $R^2$, $R^3$,M and W are as defined above and X is O, S or NH and Z is O or S, with $CYCl_2$ wherein Y is O or S to give a compound of formula I wherein X is O, S or NH and Y and Z independently represent O or S;

or (c) reacting a compound of formula (C)

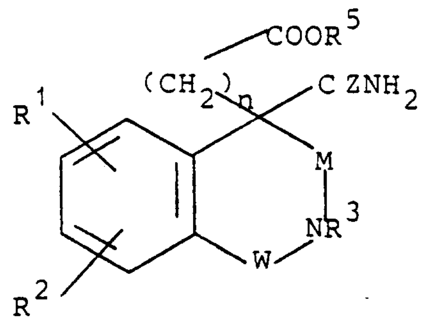

(C)

wherein $R^1$, $R^2$, $R^3$,M, W, n, Z and $COOR^5$ are as defined above using a suitable base to give a compound of formula I wherein Y is O, Z is O or S and n is 1 to 3;
or (d) reacting a compound of formula (D)

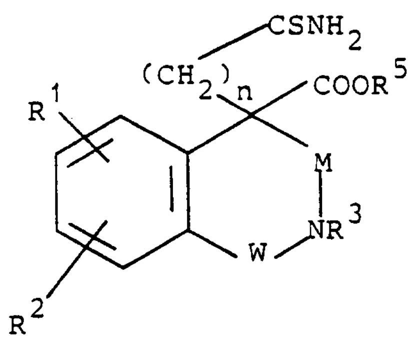

(D)

wherein $R^1$, $R^2$, $R^3$,M, W, n, and $COOR^5$ are as defined above with strong base to give a compound of formula I wherein X is $-(CH_2)_n-$
in which n is 1 to 3, and Y is S and Z is O;
or
(e) reacting a compound of formula

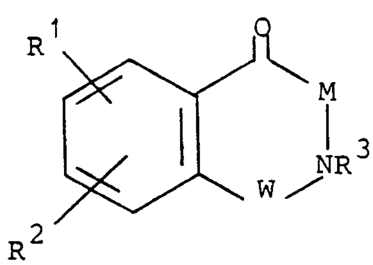

(F)

wherein $R^1$, $R^2$, $R^3$, M and W are as defined above with ammonium carbonate and an alkali metal cyanide to give a compound of formula I wherein X is -NH- and Y and Z are both oxygen;
or

(f) reacting a compound of formula (F) with successively hydrogen cyanide, thionyl chloride and thiourea to give a compound of formula (I) wherein X is S and Y and Z are both oxygen;
or
(g) reacting a compound of formula (F) with trimethylsilyl cyanide followed by HCl/alkanol and phosgene or thiophosgene to give a compound of formula (I) wherein X is O or S and Y and Z are both oxygen.

**8.** A process for preparing a compound of formula (XI)

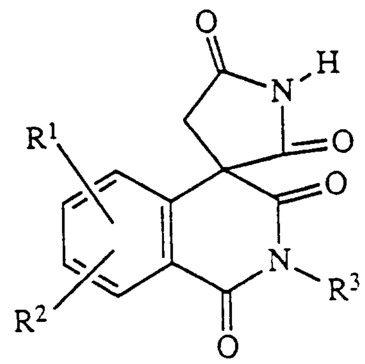
(XI)

wherein $R^1$ and $R^2$ are hydrogen or halogen; $R^3$ is alkyl containing 1 to 6 carbon atoms, aryl (lower alkyl) or dihalogen substituted aryl (lower alkyl) wherein aryl contains 6 to 10 carbon atoms and lower alkyl contains 1 to 6 carbon atoms, which comprises:
a) reacting the compound of formula (VI)

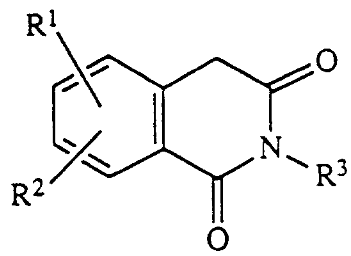
(VI)

wherein $R^1$, $R^2$, and $R^3$ areas defined above, with a base in a conventional solvent and subsequently adding a reactive carbomethoxylating agent to produce the compound of formula (VII)

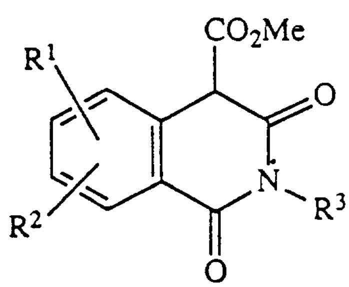
(VII)

wherein $R^1$, $R^2$, and $R^3$ are as defined above;
b) reacting said compound of formula (VII) with an inorganic base in a conventional solvent and subsequently adding tert-butyl bromoacetate to produce the compound of the formula (VIII)

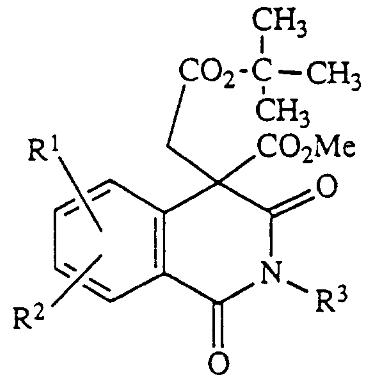

(VIII)

wherein $R^1$, $R^2$, and $R^3$ are as defined above;

c) hydrolyzing said compound of formula (VIII) with an organic acid in a conventional solvent to produce the compound of formula (IX)

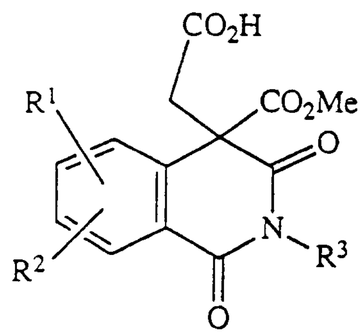

(IX)

wherein $R^1$, $R^2$, and $R^3$ are as defined above;

d) reacting said compound of formula (IX) with a coupling agent in a conventional solvent and subsequently adding tetrahydrofuran ammonium solution to produce the compound of formula (X)

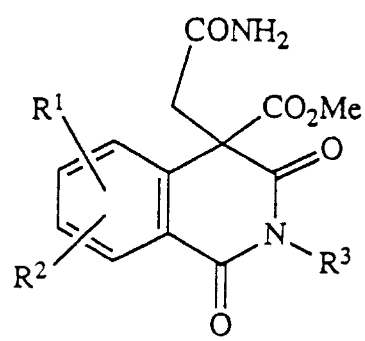

(X)

wherein $R^1$, $R^2$, and $R^3$ are as defined above;

e) reacting said compound of formula (X) with a base in a conventional solvent to produce the compound of formula (XI).

**9.** A process according to claim 8 for the production of compounds of formula (XI), wherein $R^1$ and $R^2$ are as defined in claim 8, and $R^3$ is alkyl containing 1 to 6 carbon atoms, wherein the compound of formula (X), wherein $R^1$ and $R^2$ are as defined above, $R^3$ is an alkyl, as defined above, is reacted with a base, such as lithium bis(trimethylsilyl)amide, in a conventional solvent to produce the compound of the formula (XI), wherein $R^1$ and $R^2$ are as defined above, and $R^3$ is an alkyl, as defined above.

**10.** A process for preparing a compound of formula (VII)

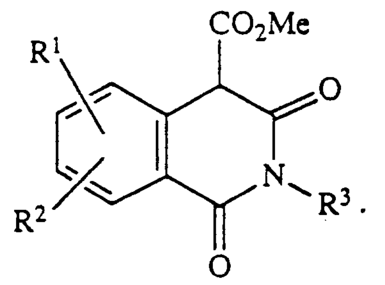

(VII)

wherein $R^1$ and $R^2$ are hydrogen or halogen; $R^3$ is alkyl containing 1 to 6 carbon atoms, aryl(lower alkyl) or dihalogen substituted aryl(lower alkyl) wherein aryl contains 6 to 10 carbon atoms and lower alkyl contains 1 to 6 carbon atoms which comprises reacting the compound of formula (XII)

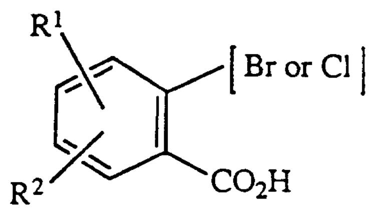

(XII)

wherein $R^1$ and $R^2$ are as defined above with dimethyl malonate and NaH in the presence of a catalytic amount of CuBr to produce the compound of formula (XIII)

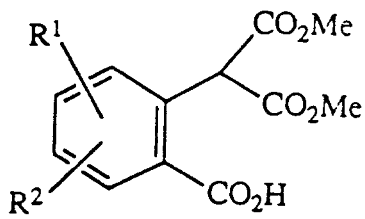

(XIII)

wherein $R^1$ and $R^2$ are as defined above and reacting said compound of formula (XIII) with thionyl chloride and subsequently adding an inert solvent, triethylamine and an amine of formula $R^3NH_2$ to produce the compound of formula (VII).

**11.** A pharmaceutical composition comprising a compound of formula I or a pharmaceutically acceptable salt thereof as defined in any one of Claims 1 to 5 and a pharmaceutically acceptable carrier.

**12.** A compound according to Claim 1 for use in the manufacture of a medicament for preventing or relieving neuropathy, nephropathy, retinopathy, or cataracts in a diabetic mammal.

**13.** A compound of formula

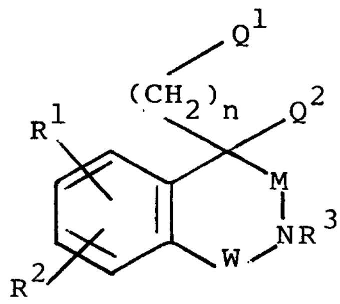

XXIV

wherein one of $Q^1$ and $Q^2$ is $-CZNH_2$ or CN; the other one of $Q^1$ and $Q^2$ is $-COOR^5$; Z, $R^1$,$R^2$, $R^3$, M and W are as defined in Claim 1, $-COOR^5$ is an ester group and n is 1 to 3.

**14.** A compound as claimed in Claim 13 wherein $Q^1$ is $-CONH_2$, $R^1$ and $R^2$ are hydrogen or halogen; n is 1; M and W are both carbonyl and $R^3$ is lower alkyl containing 1 to 6 carbon atoms, benzyl or dihalogen substituted benzyl.

**15.** A compound as claimed in Claim 13 which is one of following:
4-(2-Amino-2-oxoethyl)-2-[(3,4-dichlorophenyl)methyl]-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-7-chloro-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-6-chloro-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-6-fluoro-1,2,3,4-tetrahydro-1,3-dioxo-4-isoquinolinecarboxylic Acid Methyl Ester;
4-(2-Amino-2-oxoethyl)-1,2,3,4-tetrahydro-2-methyl-1,3-dioxo-4-isoquinoline-carboxylic Acid Methyl Ester;
4-(2-Amino-2-oxoethyl)-7-chloro-1,2,3,4-tetrahydro-2-methyl-1,3-dioxo-4-isoquinolinecarboxylic Acid Methyl Ester;
4-(2-amino-2-oxoethyl)-6-chloro-1,2,3,4-tetrahydro-2-methyl-1,3-dioxo-4-isoquinolinecarboxylic Acid Methyl Ester;
4-(2-Amino-2-oxoethyl)-2-ethyl-1,2,3,4-tetrahydro-1,3-dioxo-4-isoquinolinecarboxylic Acid Methyl Ester;
4-(2-Amino-2-oxoethyl)-1,2,3,4-tetrahydro-2-propyl-1,3-dioxo-4-isoquinoline-carboxylic Acid Methyl Ester;
4-(2-Amino-2-oxoethyl)-2-butyl-1,2,3,4-tetrahydro-1,3-dioxo-4-isoquinoline-carboxylic Acid Methyl Ester;
4-(2-Amino-2-oxoethyl)-6-bromo-1,2,3,4-tetrahydro-2-methyl-1,3-dioxo-4-isoquinolinecarboxylic Acid Methyl Ester;
4-(2-Amino-2-oxoethyl)-2-[(4-bromo-2-fluorophenyl)methyl]-1,2,3,4-tetrahydro-1,3-dioxo-4-isoquinolinecarboxylic Acid Menthyl Ester; or
4-(2-Amino-2-oxoethyl)-2-[(4-bromo-2-fluorophenyl) methyl]-6-fluoro-1,2,3,4-tetrahydro-1,3-dioxo-4-isoquinolinecarboxylic Acid Menthyl Ester.

**16.** A process for preparing a compound of formula

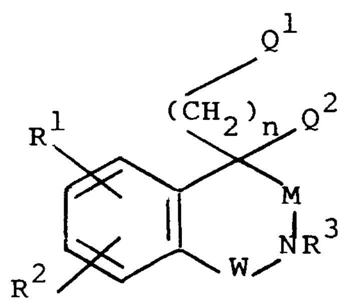

XXIV

wherein one of $Q^1$ and $Q^2$ is $CZNH_2$ or CN and the other of $Q^1$ and $Q^2$ is $COOR^5$; n is 1 to 3 and M, W, Z, $R^1$, $R^2$, $R^3$ and $COOR^5$ are as defined in Claim 1, which comprises

a) reacting an acid of formula

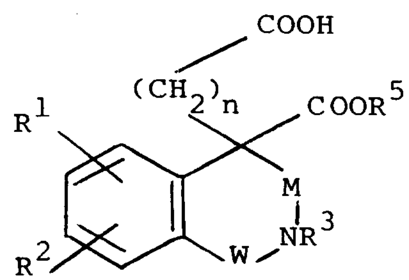

XXV

wherein $R^1$ $R^2$, $R^3$, M, W and $COOR^5$ are as defined above with a coupling agent and ammonia to give a corresponding compound of formula I wherein $Q^1$ is $CONH_2$;

or

b) reacting a compound of formula L

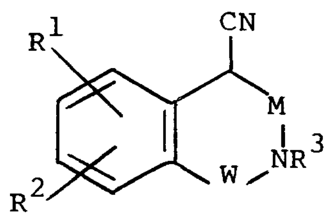

(L)

wherein $R^1$, $R^2$, M, W and $R^3$ are as defined in Claim 1 with a compound of formula

$Br(CH_2)_nCOOR^5$

wherein n is 1 to 3 and $COOR^5$ is an ester group to give a compound of formula XXIV wherein $Q^1$ is $COOR^5$ and $Q^2$ is CN;

c) reacting a compound of formula G

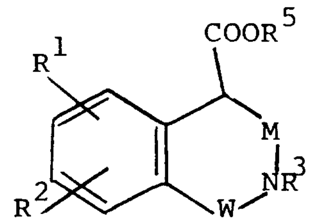

(G)

wherein $R^1$, $R^2$, $R^3$, M, W and $COOR^5$ are as defined in Claim 1 with a compound of formula

$Br(CH_2)_nCN$

wherein n is as defined above to give a compound of formula XXIV wherein $Q^1$ is CN and $Q^2$ is $COOR^5$;

or

d) converting nitrile of formula XXIV wherein $Q^2$ is CN to an amide or thioamide of formula XXIV wherein $Q^2$ is $CONH_2$ or $CSNH_2$ in known manner;

or

e) converting an nitrile of formula XXIV wherein $Q^1$ is CN to thioamide of formula XXIV wherein $Q^1$ is $CSNH_2$ using $HSP(=S)(OEt)_2/HCl$.

**Claims for the following Contracting State : ES**

**1.** A process for preparing a compound of formula (I)

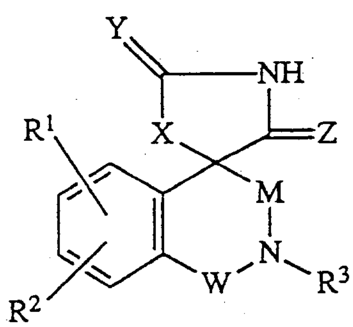

(I)

and analogues thereof wherein the fused benzene ring is replaced by a fused thiophene, pyridine or furan ring, wherein $R^1$ and $R^2$ are independently hydrogen, alkyl containing 1 to 6 carbon atoms, halogen, lower alkoxy containing 1 to 6 carbon atoms, trifluoromethyl, lower alkylthio wherein lower alkyl contains 1 to 6 carbon atoms, dialkylamino wherein alkyl contains 1 to 6 carbon atoms, nitro, aryl, or aryl (lower alkyl) oxy wherein aryl contains 6 to 10 carbon atoms and lower alkyl contains 1 to 6 carbon atoms; $R^3$ is hydrogen, lower alkyl containing 1 to 6 carbon atoms, aryl (lower alkyl) or halogen substituted aryl (lower alkyl) wherein aryl contains 6 to 10 carbon atoms and lower alkyl contains 1 to 6 carbon atoms or heterocyclic (lower alkyl) of structural formula

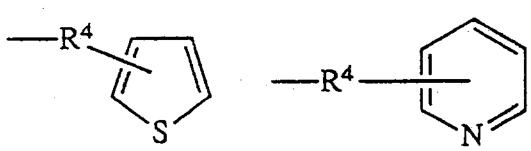

wherein $R^4$ is lower alkylene containing 1 to 3 carbon atoms; X is lower alkylene containing 1 to 3 carbon atoms, oxygen, sulfur or NH; Y and Z are independently oxygen or sulfur, M and W are independently carbonyl, thiocarbonyl, sulfonyl, sulfoxo or lower alkylene containing 1 to 2 carbon atoms with the provisos (i) that M and W are not both lower alkylene and (ii) when M is $CH_2$, W is other than sulfonyl or a pharmaceutically acceptable salt thereof; which comprises one of the following:

a)cyclising an amide of formula (A)

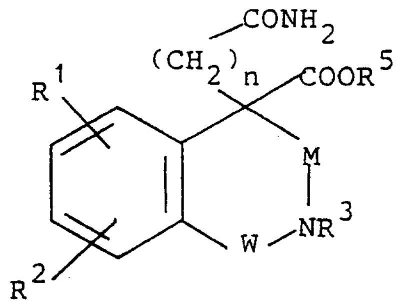

(A)

wherein $R^1$, $R^2$, $R^3$, n, $M^1$ and W are as defined above and $COOR^5$ is an ester group, using a suitable base to give a compound of formula I wherein Y and Z are both oxygen and X is $-(CH_2)_n-$ wherein n is 1 to 3;

b) reacting a compound of formula (B)

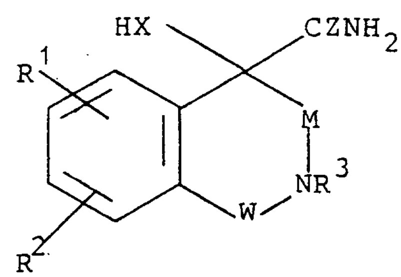

(B)

wherein $R^1$, $R^2$, $R^3$,M and W are as defined above and X is O, S or NH and Z is O or S, with $CYCl_2$ wherein Y is O or S to give a compound of formula I wherein X is O, S or NH and Y and Z independently represent O or S;

or (c) reacting a compound of formula (C)

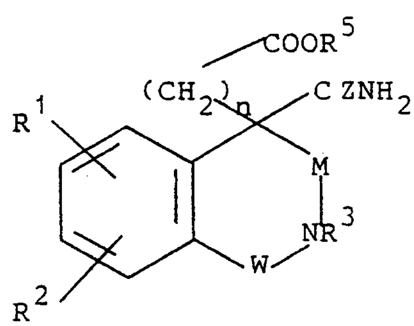

(C)

wherein $R^1$, $R^2$, $R^3$,M, W, n, Z and $COOR^5$ are as defined above using a suitable base to give a compound of formula I wherein Y is O, Z is O or S and n is 1 to 3;
or (d) reacting a compound of formula (D)

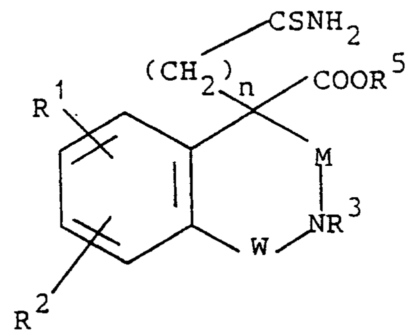

(D)

wherein $R^1$, $R^2$, $R^3$,M, W, n, and $COOR^5$ are as defined above with strong base to give a compound of formula I wherein X is $-(CH_2)_n-$
in which n is 1 to 3, and Y is S and Z is O;
or
(e) reacting a compound of formula

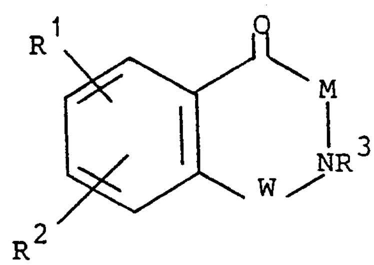

(F)

wherein $R^1$, $R^2$, $R^3$, M and W are as defined above with ammonium carbonate and an alkali metal cyanide to give a compound of formula I wherein X is -NH- and Y and Z are both oxygen;
or
(f) reacting a compound of formula (F) with successively hydrogen cyanide, thionyl chloride and thiourea to give a compound of formula (I) wherein X is S and Y and Z are
both oxygen;
or
(g) reacting a compound of formula (F) with trimethylsilyl cyanide followed by HCl/alkanol and phosgene or thiophosgene to give a compound of formula (I) wherein X is O or S and Y and Z are both oxygen.

**2.** A process according to claim 1 in which the compound prepared has the formula II

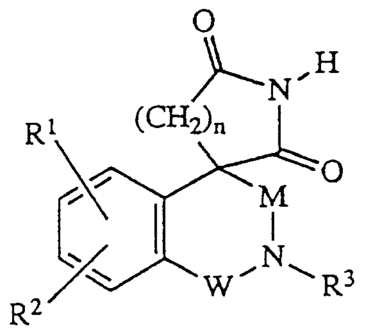

(II)

wherein $R^1$ and $R^2$ are independently hydrogen, alkyl containing 1 to 6 carbon atoms, halogen, lower alkoxy containing 1 to 6 carbon atoms, trifluoromethyl, lower alkylthio wherein lower alkyl contains 1 to 6 carbon atoms; $R^3$ is hydrogen, lower alkyl containing 1 to 6 carbon atoms; M and W are carbonyl or thiocarbonyl; n is 1 to 3, or a pharmaceutically acceptable salt thereof.

**3.** A process according to claim 1 in which the compound prepared has the formula

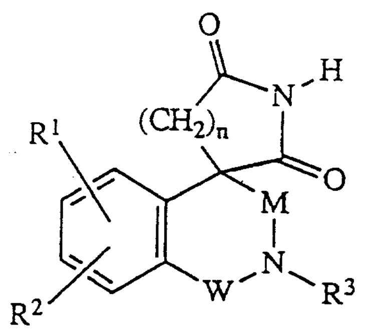

(II)

wherein $R^1$ and $R^2$ are independently hydrogen, alkyl containing 1 to 6 carbon atoms, halogen, lower alkoxy containing 1 to 6 carbons atoms, trifluoromethyl, lower alkythio wherein lower alkyl contains 1 to 6 carbon atoms; $R^3$ is aryl(lower alkyl) or dihalogen substituted aryl(lower alkyl) wherein aryl contains 6 to 10 carbon atoms and lower alkyl contains 1 to 6 carbon atoms; M and W are carbonyl or thiocarbonyl; n is 1 to 3, or a pharmaceutically acceptable salt thereof.

**4.** A process according to claim 1 in which the compound prepared has formula (III)

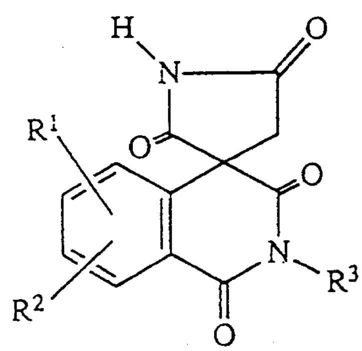

(III)

wherein $R^1$ and $R^2$ are hydrogen or halogen; $R^3$ is lower alkyl containing 1 to 6 carbon atoms, benzyl or dihalogen substituted benzyl, or a pharmaceutically acceptable salt thereof.

5. A process according to Claim 1 which the compound of formula I prepared is one of the following:
2-[(4-bromo-2-fluorophenyl)methyl]spiro[isoquinoline-4 (1H),3'-pyrrolidine]-1,2',3,5'(2H)-tetrone,
[2-[(4-bromo-2-fluorophenyl)methyl]-7-chloro]spiro[isoquinoline-4(1H),3'-pyrrolidine]-1,2',3,5'(2H)-tetrone,
[2-[(4-bromo-2-fluorophenyl)methyl]-6-chloro]spiro[iso quinoline-4(1H),3'-pyrrolidine]-1,2',3,5'(2H)-tetrone,
2-[(3,4-dichlorophenyl)methyl]spiro[isoquinoline-4(1H), 3'-pyrrolidine]-1,2',3,5'(2H)-tetrone,
2-[(4-bromo-2-fluorophenyl)methyl]-5-fluoro]spiro[isoquinoline-4(1H),3'-pyrrolidine]-1,2',3,5'(2H)-tetrone,
[2-[(4-bromo-2-fluorophenyl)methyl]-6-fluoro]spiro[isoquinoline-4(1H),3'-pyrrolidine]-1,2',3,5'(2H)-tetrone,
[2-[(4-bromo-2-fluorophenyl)methyl]-7-fluoro]spiro[isoquinoline-4-(1H),3'-pyrrolidine]-1,2',3,5'(2H)-tetrone,
[2-[(4-bromo-2-fluorophenyl)methyl]-8-fluoro]spiro[isoquinoline-4(1H),3'-pyrrolidine]-1,2',3,5'(2H)-tetrone,
[6-bromo-2-[(4-bromo-2-fluorophenyl)methyl]-7-methoxy]spiro[isoquinoline-4(1H),3'-pyrrolidine]-1,28,3,5'(2H)-tetrone,
[2-[(4-bromo-2-fluorophenyl)methyl]-7-methoxy]spiro[iso quinoline-4(1H),3'-pyrrolidine]-1,2',3,5'(2H)-tetrone,
(R)-(+)-2-[(4-bromo-2-fluorophenyl)methyl]spiro[isoquinoline-4(1H)3'-pyrrolidine]-1,2',3,5'(2H)-tetrone,
(S)-(-)-2-[(4-bromo-2-fluorophenyl)methyl]spiro[isoquinoline-4(1H),3'-pyrrolidine]-1,2',3,5'(2H)-tetrone,
(R)-(+)-[(4-bromo-2-fluorophenyl)methyl]-6-fluoro]spiro[isoquinoline-4(1H),3'-pyrrolidine]-1,2',3,5'(2H)-tetrone,
(S)-(-)-[(2-[(4-bromo-2-fluorophenyl)methyl]-6-fluoro]spiro[isoquinoline-4(1H),3'-pyrrolidine]-1,2'3,5'(2H)-tetrone,
2-methylspiro[isoquinoline-4(1H),3'-pyrrolidine]-1,2',3,5'(2H)-tetrone,
6-chloro-2-methylspiro[isoquinoline-4(1H),3'-pyrrolidine]-1,2',3,5'(2H)-tetrone,
6-bromo-2-methylspiro[isoquinoline-4(1H),3'-pyrrolidine]-1,2',3,5'(2H)-tetrone,
7-chloro-2-methylspiro[isoquinoline-4(1H),3'-pyrrolidine]-1,2',3,5'(2H)-tetrone,
2-ethylspiro[isoquinoline-4(1H),3'-pyrrolidine]-1,2'3,5'(2H)-tetrone,
2-propylspiro[isoquinoline-4(1H),3'-pyrrolidine]-1,2',3,5'(2H)-tetrone,
2-butylspiro[isoquinoline-4(1H),3'-pyrrolidine]-1,2',3, 5'(2H)-tetrone; or a pharmaceutically acceptable salt thereof.

6. A process for preparing a compound of formula (XI)

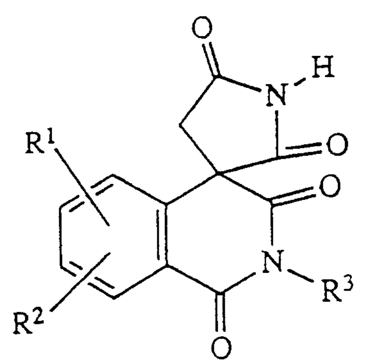

(XI)

wherein $R^1$ and $R^2$ are hydrogen or halogen; $R^3$ is alkyl containing 1 to 6 carbon atoms, aryl (lower alkyl) or dihalogen substituted aryl (lower alkyl) wherein aryl contains 6 to 10 carbon atoms and lower alkyl contains 1 to 6 carbon atoms, which comprises:

a) reacting the compound of formula (VI)

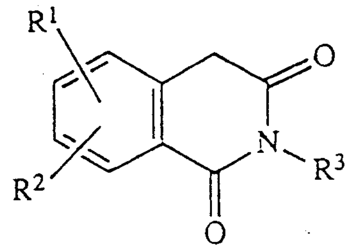

(VI)

wherein $R^1$, $R^2$, and $R^3$ are as defined above, with a base in a conventional solvent and subsequently adding a reactive carbomethoxylating agent to produce the compound of formula (VII)

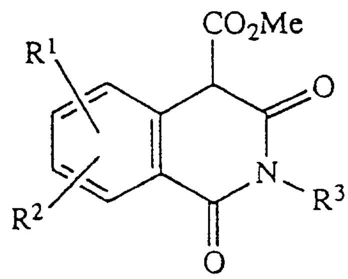

(VII)

wherein $R^1$, $R^2$, and $R^3$ are as defined above;
b) reacting said compound of formula (VII) with an inorganic base in a conventional solvent and subsequently adding tert-butyl bromoacetate to produce the compound of the formula (VIII)

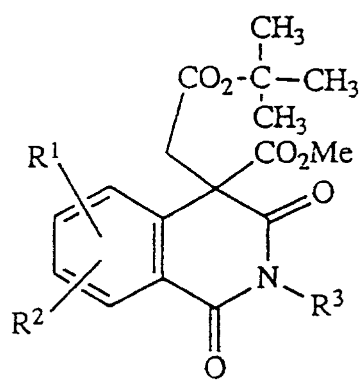

(VIII)

wherein $R^1$, $R^2$, and $R^3$ are as defined above;
c) hydrolyzing said compound of formula (VIII) with an organic acid in a conventional solvent to produce the compound of formula (IX)

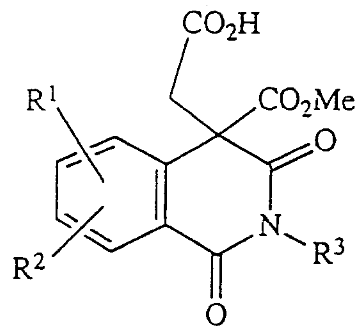

(IX)

wherein $R^1$, $R^2$, and $R^3$ are as defined above;

d) reacting said compound of formula (IX) with a coupling agent in a conventional solvent and subsequently adding tetrahydrofuran ammonium solution to produce the compound of formula (X)

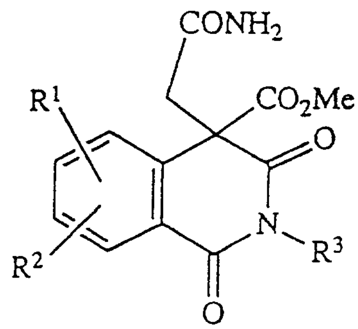

(X)

wherein $R^1$, $R^2$, and $R^3$ are as defined above;

e) reacting said compound of formula (X) with a base in a conventional solvent to produce the compound of formula (XI).

**7.** A process according to claim 6 for the production of compounds of formula (XI), wherein $R^1$ and $R^2$ are as defined in claim 6, and $R^3$ is alkyl containing 1 to 6 carbon atoms, wherein the compound of formula (X), wherein $R^1$ and $R^2$ are as defined above, $R^3$ is an alkyl, as defined above, is reacted with a base, such as lithium bis(trimethylsilyl)amide, in a conventional solvent to produce the compound of the formula (XI), wherein $R^1$ and $R^2$ are as defined above, and $R^3$ is an alkyl, as defined above.

**8.** A process for preparing a compound of formula (VII)

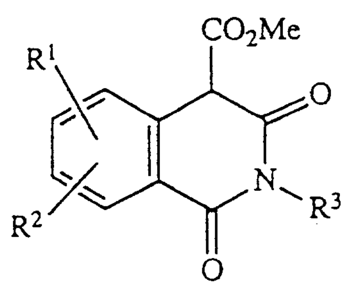

(VII)

wherein $R^1$ and $R^2$ are hydrogen or halogen; $R^3$ is alkyl containing 1 to 6 carbon atoms, aryl(lower alkyl) or dihalogen substituted aryl(lower alkyl) wherein aryl contains 6 to 10 carbon atoms and lower alkyl contains 1 to 6 carbon atoms which comprises reacting the compound of formula (XII)

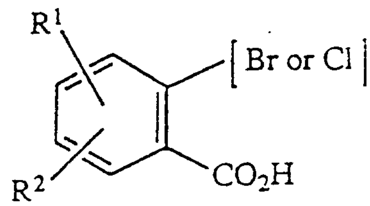

(XII)

wherein $R^1$ and $R^2$ are as defined above with dimethyl malonate and NaH in the presence of a catalytic amount of CuBr to produce the compound of formula (XIII)

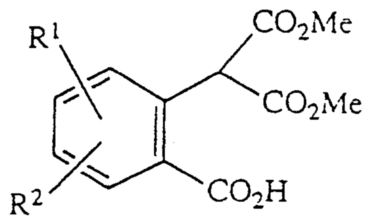

(XIII)

wherein $R^1$ and $R^2$ are as defined above and reacting said compound of formula (XIII) with thionyl chloride and subsequently adding and inert solvent, triethylamine and an amine of formula $R^3NH_2$ to produce the compound of formula (VII)

**9.** A process for preparing a pharmaceutical composition which comprises bringing a compound of formula I or analogue or pharmaceutically acceptable salt thereof as defined in Claim 1 into association with a pharmaceutically acceptable carrier.

**10.** A process for preparing a compound of formula

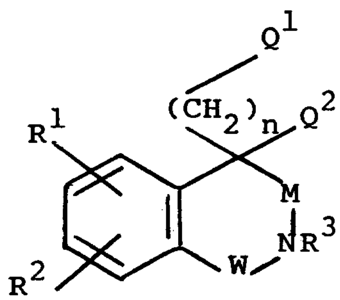

XXIV

wherein one of $Q^1$ and $Q^2$ is $CZNH_2$ or CN and the other of $Q^1$ and $Q^2$ is $COOR^5$; n is 1 to 3 and M, W, Z, $R^1$, $R^2$, $R^3$ and $COOR^5$ are as defined in Claim 1, which comprises

a) reacting an acid of formula

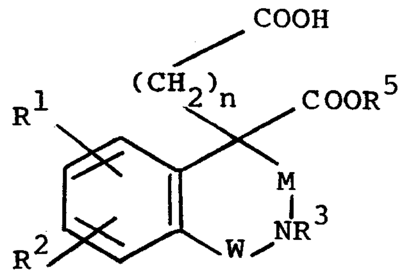

XXV

wherein $R^1$, $R^2$, $R^3$, M, W and $COOR^5$ are as defined above with a coupling agent and ammonia to give a corresponding compound of formula I wherein $Q^1$ is $CONH_2$;
or
b) reacting a compound of formula L

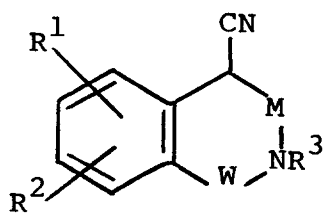

(L)

wherein $R^1$, $R^2$, M, W and $R^3$ are as defined in Claim 1 with a compound of formula

$Br(CH_2)_nCOOR^5$

wherein n is 1 to 3 and $COOR^5$ is an ester group to give a compound of formula XXIV wherein $Q^1$ is $COOR^5$ and $Q^2$ is CN;
c) reacting a compound of formula G

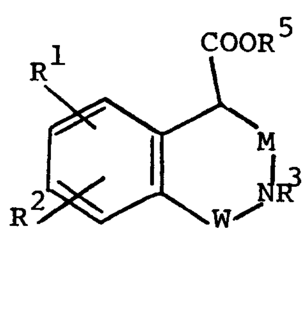

(G)

wherein $R^1$, $R^2$, $R^3$, M, W and $COOR^5$ are as defined in Claim 1 with a compound of formula

$Br(CH_2)_nCN$

wherein n is as defined above to give a compound of formula XXIV wherein $Q^1$ is CN and $Q^2$ is

$COOR^5$;

or

d) converting nitrile of formula XXIV wherein $Q^2$ is CN to an amide or thioamide of formula XXIV wherein $Q^2$ is $CONH_2$ or $CSNH_2$ in known manner;

or

e) converting an nitrile of formula XXIV wherein $Q^1$ is CN to thioamide of formula XXIV wherein $Q^1$ is $CSNH_2$ using $HSP(=S)(OEt)_2/HCl$.

**11.** A process as claimed in Claim 10 in which the product is one of the following:
4-(2-Amino-2-oxoethyl)-2-[(3,4-dichlorophenyl)methyl]-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-7-chloro-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-6-chloro-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-6-fluoro-1,2,3,4-tetrahydro-1,3-dioxo-4-isoquinolinecarboxylic Acid Methyl Ester;
4-(2-Amino-2-oxoethyl)-1,2,3,4-tetrahydro-2-methyl-1,3-dioxo-4-isoquinoline-carboxylic Acid Methyl Ester;
4-(2-Amino-2-oxoethyl)-7-chloro-1,2,3,4-tetrahydro-2-methyl-1,3-dioxo-4-isoquinolinecarboxylic Acid Methyl Ester;
4-(2-amino-2-oxoethyl)-6-chloro-1,2,3,4-tetrahydro-2-methyl-1,3-dioxo-4-isoquinolinecarboxylic Acid Methyl Ester;
4-(2-Amino-2-oxoethyl)-2-ethyl-1,2,3,4-tetrahydro-1,3-dioxo-4-isoquinolinecarboxylic Acid Methyl Ester;
4-(2-Amino-2-oxoethyl)-1,2,3,4-tetrahydro-2-propyl-1,3-dioxo-4-isoquinoline-carboxylic Acid Methyl Ester;
4-(2-Amino-2-oxoethyl)-2-butyl-1,2,3,4-tetrahydro-1,3-dioxo-4-isoquinoline-carboxylic Acid Methyl Ester;
4-(2-Amino-2-oxoethyl)-6-bromo-1,2,3,4-tetrahydro-2-methyl-1,3-dioxo-4-isoquinolinecarboxylic Acid Methyl Ester;
4-(2-Amino-2-oxoethyl)-2-[(4-bromo-2-fluorophenyl)methyl]-1,2,3,4-tetrahydro-1,3-dioxo-4-isoquinolinecarboxylic Acid Menthyl Ester; or
4-(2-Amino-2-oxoethyl)-2-[(4-bromo-2-fluorophenyl) methyl]-6-fluoro-1,2,3,4-tetrahydro-1,3-dioxo-4-isoquinolinecarboxylic Acid Menthyl Ester.

**Claims for the following Contracting State : GR**

**1.** A process for preparing a compound of formula (I)

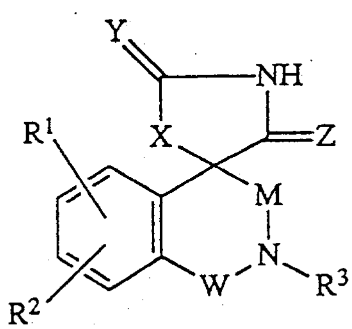

(I)

and analogues thereof wherein the fused benzene ring is replaced by a fused thiophene, pyridine or furan ring, wherein $R^1$ and $R^2$ are independently hydrogen, alkyl containing 1 to 6 carbon atoms, halogen, lower alkoxy containing 1 to 6 carbon atoms, trifluoromethyl, lower alkylthio wherein lower alkyl contains 1 to 6 carbon atoms, dialkylamino wherein alkyl contains 1 to 6 carbon atoms, nitro, aryl, or aryl (lower alkyl) oxy wherein aryl contains 6 to 10 carbon atoms and lower alkyl contains 1 to 6 carbon atoms; $R^3$ is hydrogen, lower alkyl containing 1 to 6 carbon atoms, aryl (lower alkyl) or halogen

substituted aryl (lower alkyl) wherein aryl contains 6 to 10 carbon atoms and lower alkyl contains 1 to 6 carbon atoms or heterocyclic (lower alkyl) of structural formula

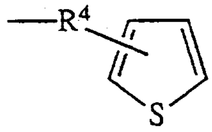

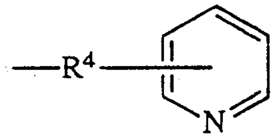

wherein $R^4$ is lower alkylene containing 1 to 3 carbon atoms; X is lower alkylene containing 1 to 3 carbon atoms, oxygen, sulfur or NH; Y and Z are independently oxygen or sulfur, M and W are independently carbonyl, thiocarbonyl, sulfonyl, sulfoxo or lower alkylene containing 1 to 2 carbon atoms with the provisos (i) that M and W are not both lower alkylene and (ii) when M is $CH_2$, W is other than sulfonyl or a pharmaceutically acceptable salt thereof; which comprises one of the following:

a)cyclising an amide of formula (A)

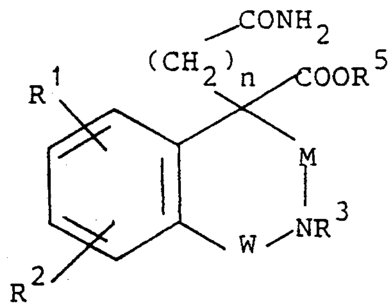

(A)

wherein $R^1$, $R^2$, $R^3$, n, $M^1$ and W are as defined above and $COOR^5$ is an ester group, using a suitable base to give a compound of formula I wherein Y and Z are both oxygen and X is $-(CH_2)_n-$ wherein n is 1 to 3;

b) reacting a compound of formula (B)

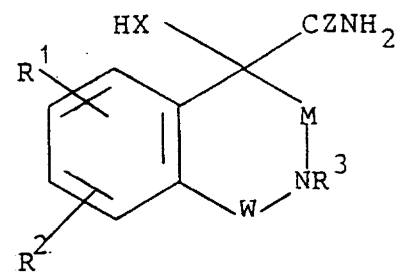

(B)

wherein $R^1$, $R^2$, $R^3$,M and W are as defined above and X is O, S or NH and Z is O or S, with $CYCl_2$ wherein Y is O or S to give a compound of formula I wherein X is O, S or NH and Y and Z independently represent O or S;

or (c) reacting a compound of formula (C)

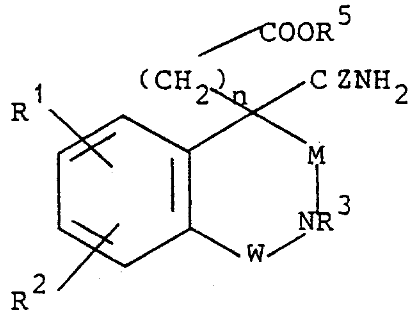

(C)

wherein $R^1$, $R^2$, $R^3$,M, W, n, Z and $COOR^5$ are as defined above using a suitable base to give a compound of formula I wherein Y is O, Z is O or S and n is 1 to 3;
or (d) reacting a compound of formula (D)

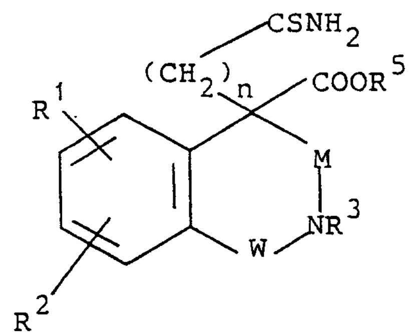

(D)

wherein $R^1$, $R^2$, $R^3$,M, W, n, and $COOR^5$ are as defined above with strong base to give a compound of formula I wherein X is $-(CH_2)_n-$
in which n is 1 to 3, and Y is S and Z is O;
or
(e) reacting a compound of formula

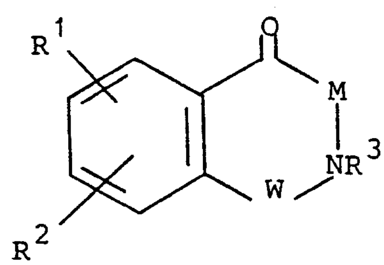

(F)

wherein $R^1$, $R^2$, $R^3$, M and W are as defined above with ammonium carbonate and an alkali metal cyanide to give a compound of formula I wherein X is -NH- and Y and Z are both oxygen;
or
(f) reacting a compound of formula (F) with successively hydrogen cyanide, thionyl chloride and thiourea to give a compound of formula (I) wherein X is S and Y and Z are

both oxygen;
or
(g) reacting a compound of formula (F) with trimethylsilyl cyanide followed by HCl/alkanol and phosgene or thiophosgene to give a compound of formula (I) wherein X is O or S and Y and Z are both oxygen.

2. A process according to claim 1 in which the compound prepared has the formula II

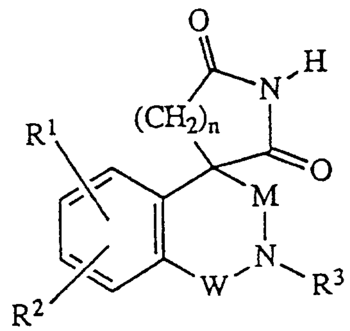

(II)

wherein $R^1$ and $R^2$ are independently hydrogen, alkyl containing 1 to 6 carbon atoms, halogen, lower alkoxy containing 1 to 6 carbon atoms, trifluoromethyl, lower alkylthio wherein lower alkyl contains 1 to 6 carbon atoms; $R^3$ is hydrogen, lower alkyl containing 1 to 6 carbon atoms; M and W are carbonyl or thiocarbonyl; n is 1 to 3, or a pharmaceutically acceptable salt thereof.

3. A process according to claim 1 in which the compound prepared has the formula

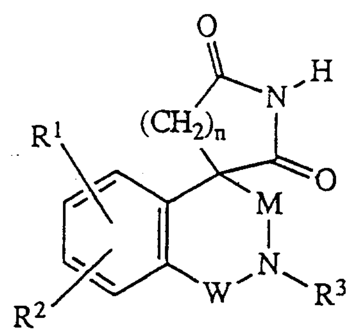

(II)

wherein $R^1$ and $R^2$ are independently hydrogen, alkyl containing 1 to 6 carbon atoms, halogen, lower alkoxy containing 1 to 6 carbon atoms, trifluoromethyl, lower alkythio wherein lower alkyl contains 1 to 6 carbon atoms; $R^3$ is aryl(lower alkyl) or dihalogen substituted aryl(lower alkyl) wherein aryl contains 6 to 10 carbon atoms and lower alkyl contains 1 to 6 carbon atoms; M and W are carbonyl or thiocarbonyl; n is 1 to 3, or a pharmaceutically acceptable salt thereof.

**4.** A process according to claim 1 in which the compound prepared has formula (III)

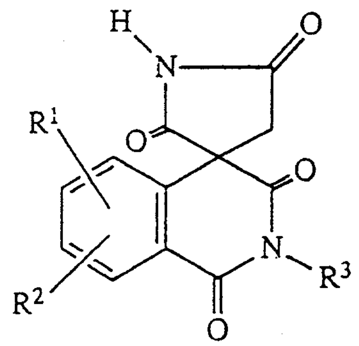

(III)

wherein $R^1$ and $R^2$ are hydrogen or halogen; $R^3$ is lower alkyl containing 1 to 6 carbon atoms, benzyl or dihalogen substituted benzyl, or a pharmaceutically acceptable salt thereof.

**5.** A process according to Claim 1 which the compound of formula I prepared is one of the following:
2-[(4-bromo-2-fluorophenyl)methyl]spiro[isoquinoline-4 (1H),3'-pyrrolidine]-1,2',3,5'(2H)-tetrone,
[2-[(4-bromo-2-fluorophenyl)methyl]-7-chloro]spiro[isoquinoline-4(1H),3'-pyrrolidine]-1,2',3,5'(2H)-tetrone,
[2-[(4-bromo-2-fluorophenyl)methyl]-6-chloro]spiro[iso quinoline-4(1H),3'-pyrrolidine]-1,2',3,5'(2H)-tetrone,
2-[(3,4-dichlorophenyl)methyl]spiro[isoquinoline-4(1H), 3'-pyrrolidine]-1,2',3,5'(2H)-tetrone,
2-[(4-bromo-2-fluorophenyl)methyl]-5-fluoro]spiro[isoquinoline-4(1H),3'-pyrrolidine]-1,2',3,5'(2H)-tetrone,
[2-[(4-bromo-2-fluorophenyl)methyl]-6-fluoro]spiro[isoquinoline-4(1H),3'-pyrrolidine]-1,2',3,5'(2H)-tetrone,
[2-[(4-bromo-2-fluorophenyl)methyl]-7-fluoro]spiro[isoquinoline-4-(1H),3'-pyrrolidine]-1,2',3,5'(2H)-tetrone,
[2-[(4-bromo-2-fluorophenyl)methyl]-8-fluoro]spiro[isoquinoline-4(1H),3'-pyrrolidine]-1,2',3,5'(2H)-tetrone,
[6-bromo-2-[(4-bromo-2-fluorophenyl)methyl]-7-methoxy]spiro[isoquinoline-4(1H),3'-pyrrolidine]-1,28,3,5'(2H)-tetrone,
[2-[(4-bromo-2-fluorophenyl)methyl]-7-methoxy]spiro[iso quinoline-4(1H),3'-pyrrolidine]-1,2',3,5'(2H)-tetrone,
(R)-(+)-2-[(4-bromo-2-fluorophenyl)methyl]spiro[isoquinoline-4(1H)3'-pyrrolidine]-1,2',3,5'(2H)-tetrone,
(S)-(-)-2-[(4-bromo-2-fluorophenyl)methyl]spiro[isoquinoline-4(1H),3'-pyrrolidine]-1,2',3,5'(2H)-tetrone,
(R)-(+)-[(4-bromo-2-fluorophenyl)methyl]-6-fluoro]spiro[isoquinoline-4(1H),3'-pyrrolidine]-1,2',3,5'(2H)-tetrone,
(S)-(-)-[(2-[(4-bromo-2-fluorophenyl)methyl]-6-fluoro]spiro[isoquinoline-4(1H),3'-pyrrolidine]-1,2'3,5(2H)-tetrone,
2-methylspiro[isoquinoline-4(1H),3'-pyrrolidine]-1,2',3 ,5'(2H)-tetrone,
6-chloro-2-methylspiro[isoquinoline-4(1H),3'-pyrrolidine]-1,2',3,5'(2H)-tetrone,
6-bromo-2-methylspiro[isoquinoline-4(1H),3'-pyrrolidine]-1,2',3,5'(2H)-tetrone,
7-chloro-2-methylspiro[isoquinoline-4(1H),3'-pyrrolidine]-1,2',3,5'(2H)-tetrone,
2-ethylspiro[isoquinoline-4(1H),3'-pyrrolidine]-1,2'3,5 '(2H)-tetrone,
2-propylspiro[isoquinoline-4(1H),3'-pyrrolidine]-1,2',3 ,5'(2H)-tetrone,
2-butylspiro[isoquinoline-4(1H),3'-pyrrolidine]-1,2',3, 5'(2H)-tetrone; or a pharmaceutically acceptable salt thereof.

**6.** A process for preparing a compound of formula (XI)

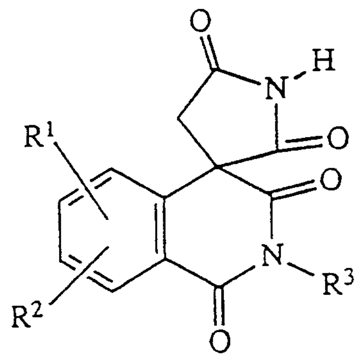

(XI)

wherein $R^1$ and $R^2$ are hydrogen or halogen; $R^3$ is alkyl containing 1 to 6 carbon atoms, aryl (lower alkyl) or dihalogen substituted aryl (lower alkyl) wherein aryl contains 6 to 10 carbon atoms and lower alkyl contains 1 to 6 carbon atoms, which comprises:

a) reacting the compound of formula (VI)

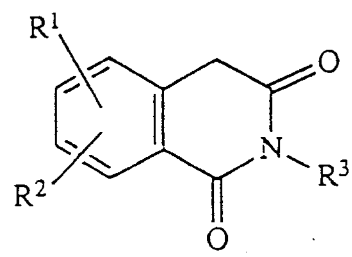

(VI)

wherein $R^1$, $R^2$, and $R^3$ are as defined above, with a base in a conventional solvent and subsequently adding a reactive carbomethoxylating agent to produce the compound of formula (VII)

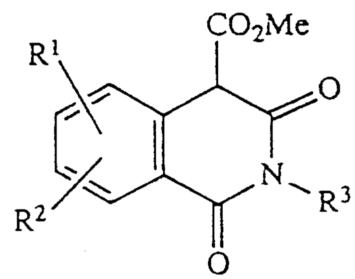

(VII)

wherein $R^1$, $R^2$, and $R^3$ are as defined above;

b) reacting said compound of formula (VII) with an inorganic base in a conventional solvent and subsequently adding tert-butyl bromoacetate to produce the compound of the formula (VIII)

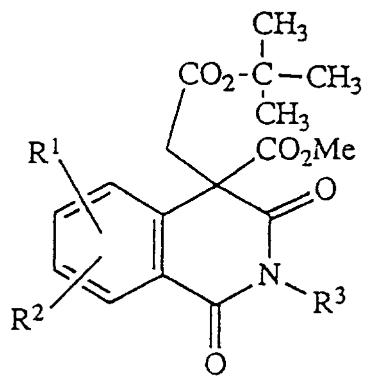

(VIII)

wherein $R^1$, $R^2$, and $R^3$ are as defined above;

c) hydrolyzing said compound of formula (VIII) with an organic acid in a conventional solvent to produce the compound of formula (IX)

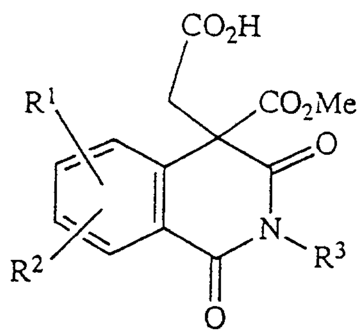

(IX)

wherein $R^1$, $R^2$, and $R^3$ are as defined above;

d) reacting said compound of formula (IX) with a coupling agent in a conventional solvent and subsequently adding tetrahydrofuran ammonium solution to produce the compound of formula (X)

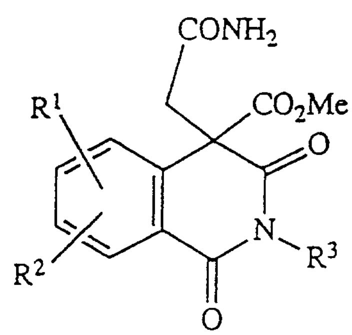

(X)

wherein $R^1$, $R^2$, and $R^3$ are as defined above;

e) reacting said compound of formula (X) with a base in a conventional solvent to produce the compound of formula (XI).

**7.** A process according to claim 6 for the production of compounds of formula (XI), wherein $R^1$ and $R^2$ are as defined in claim 6, and $R^3$ is alkyl containing 1 to 6 carbon atoms, wherein the compound of formula (X), wherein $R^1$ and $R^2$ are as defined above, $R^3$ is an alkyl, as defined above, is reacted with a base, such as lithium bis(trimethylsilyl)amide, in a conventional solvent to produce the compound of the formula (XI), wherein $R^1$ and $R^2$ are as defined above, and $R^3$ is an alkyl, as defined above.

8. A process for preparing a compound of formula (VII)

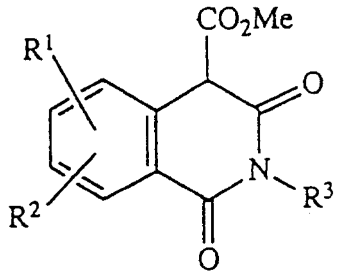

(VII)

wherein $R^1$ and $R^2$ are hydrogen or halogen; $R^3$ is alkyl containing 1 to 6 carbon atoms, aryl(lower alkyl) or dihalogen substituted aryl(lower alkyl) wherein aryl contains 6 to 10 carbon atoms and lower alkyl contains 1 to 6 carbon atoms which comprises reacting the compound of formula (XII)

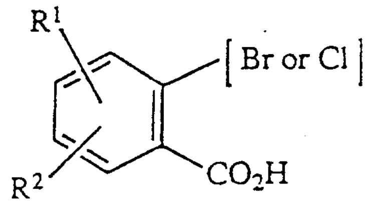

(XII)

wherein $R^1$ and $R^2$ are as defined above with dimethyl malonate and NaH in the presence of a catalytic amount of CuBr to produce the compound of formula (XIII)

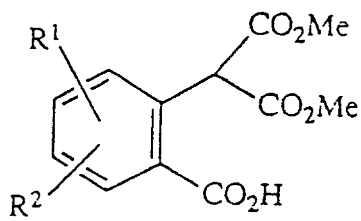

(XIII)

wherein $R^1$ and $R^2$ are as defined above and reacting said compound of formula (XIII) with thionyl chloride and subsequently adding an inert solvent, triethylamine and an amine of formula $R^3NH_2$ to produce the compound of formula (VII)

9. A process for preparing a pharmaceutical composition which comprises bringing a compound of formula I or analogue or pharmaceutically acceptable salt thereof as defined in Claim 1 into association with a pharmaceutically acceptable carrier.

**10.** A process for preparing a compound of formula

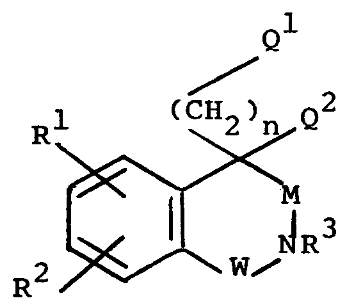

XXIV

wherein one of $Q^1$ and $Q^2$ is $CZNH_2$ or CN and the other of $Q^1$ and $Q^2$ is $COOR^5$; n is 1 to 3 and M, W, Z, $R^1$, $R^2$, $R^3$ and $COOR^5$ are as defined in Claim 1, which comprises

a) reacting an acid of formula

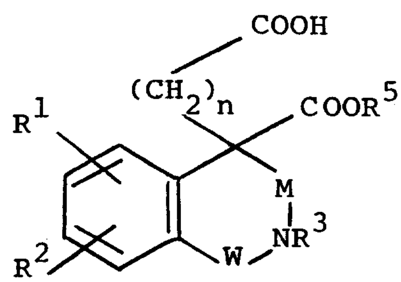

XXV

wherein $R^1$, $R^2$, $R^3$, M, W and $COOR^5$ are as defined above with a coupling agent and ammonia to give a corresponding compound of formula I wherein $Q^1$ is $CONH_2$;

or

b) reacting a compound of formula L

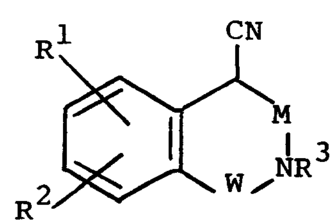

(L)

wherein $R^1$, $R^2$, M, W and $R^3$ are as defined in Claim 1 with a compound of formula

$Br(CH_2)_nCOOR^5$

wherein n is 1 to 3 and $COOR^5$ is an ester group to give a compound of formula XXIV wherein $Q^1$ is $COOR^5$ and $Q^2$ is CN;

c) reacting a compound of formula G

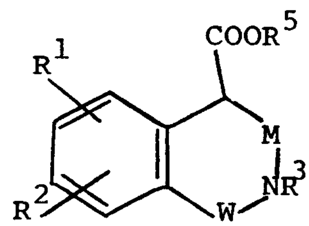

(G)

wherein $R^1$, $R^2$, $R^3$, M, W and $COOR^5$ are as defined in Claim 1 with a compound of formula

$Br(CH_2)_nCN$

wherein n is as defined above to give a compound of formula XXIV wherein $Q^1$ is CN and $Q^2$ is $COOR^5$;
or
d) converting nitrile of formula XXIV wherein $Q^2$ is CN to an amide or thioamide of formula XXIV wherein $Q^2$ is $CONH_2$ or $CSNH_2$ in known manner;
or
e) converting an nitrile of formula XXIV wherein $Q^1$ is CN to thioamide of formula XXIV wherein $Q^1$ is $CSNH_2$ using $HSP(=S)(OEt)_2/HCl$.

**11.** A compound of formula

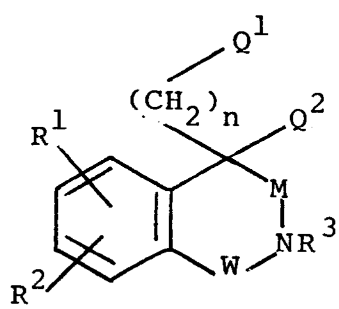

XXIV

wherein one of $Q^1$ and $Q^2$ is $-CZNH_2$ or CN; the other one of $Q^1$ and $Q^2$ is $-COOR^5$; Z, $R^1$,$R^2$, $R^3$, M and W are as defined in Claim 1, $-COOR^5$ is an ester group and n is 1 to 3.

**12.** A compound as claimed in Claim 11 wherein $Q^1$ is $-CONH_2$, $R^1$ and $R^2$ are hydrogen or halogen; n is 1; M and W are both carbonyl and $R^3$ is lower alkyl containing 1 to 6 carbon atoms, benzyl or dihalogen substituted benzyl.

**13.** A compound as claimed in Claim 11 which is one of following:
4-(2-Amino-2-oxoethyl)-2-[(3,4-dichlorophenyl)methyl]-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-7-chloro-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-6-chloro-1,2,3,4-tetrahydro-1,3-dioxo-4-isoquinolinecarboxylic Acid Methyl Ester;
4-(2-Amino-2-oxoethyl)-2-[(4-bromo-2-fluorophenyl)methyl]-6-fluoro-1,2,3,4-tetrahydro-1,3-dioxo-4-isoquinolinecarboxylic Acid Methyl Ester;
4-(2-Amino-2-oxoethyl)-1,2,3,4-tetrahydro-2-methyl-1,3-dioxo-4-isoquinoline-carboxylic Acid Methyl Ester;
4-(2-Amino-2-oxoethyl)-7-chloro-1,2,3,4-tetrahydro-2-methyl-1,3-dioxo-4-isoquinolinecarboxylic Acid Methyl Ester;
4-(2-amino-2-oxoethyl)-6-chloro-1,2,3,4-tetrahydro-2-methyl-1,3-dioxo-4-isoquinolinecarboxylic Acid Methyl Ester;
4-(2-Amino-2-oxoethyl)-2-ethyl-1,2,3,4-tetrahydro-1,3-dioxo-4-isoquinolinecarboxylic Acid Methyl Ester;
4-(2-Amino-2-oxoethyl)-1,2,3,4-tetrahydro-2-propyl-1,3-dioxo-4-isoquinoline-carboxylic Acid Methyl Ester;
4-(2-Amino-2-oxoethyl)-2-butyl-1,2,3,4-tetrahydro-1,3-dioxo-4-isoquinoline-carboxylic Acid Methyl Ester;
4-(2-Amino-2-oxoethyl)-6-bromo-1,2,3,4-tetrahydro-2-methyl-1,3-dioxo-4-isoquinolinecarboxylic Acid Methyl Ester;
4-(2-Amino-2-oxoethyl)-2-[(4-bromo-2-fluorophenyl)methyl]-1,2,3,4-tetrahydro-1,3-dioxo-4-isoquinolinecarboxylic Acid Menthyl Ester; or
4-(2-Amino-2-oxoethyl)-2-[(4-bromo-2-fluorophenyl) methyl]-6-fluoro-1,2,3,4-tetrahydro-1,3-dioxo-4-isoquinolinecarboxylic Acid Menthyl Ester.

## Patentansprüche

## Patentansprüche für folgende Vertragsstaaten : AT, BE, CH, DE, FR, GB, IT, LI, LU, NL, SE

**1.** Verbindung der Formel (I)

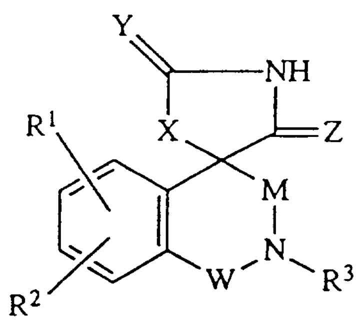

(I)

und Analoga hievon, wobei der kondensierte Benzolring durch einen kondensierten Thiophen-, Pyridin- oder Furanring ersetzt ist, worin $R^1$ und $R^2$ unabhängig Wasserstoff, Alkyl mit 1 bis 6 Kohlenstoffatomen, Halogen, nied.Alkoxy mit 1 bis 6 Kohlenstoffatomen, Trifluormethyl, nied.Alkylthio, wobei nied.Alkyl 1 bis 6 Kohlenstoffatome enthält, Dialkylamino, wobei Alkyl 1 bis 6 Kohlenstoffatome enthält, Nitro, Aryl oder Aryl-(nied.alkyl)-oxy, wobei Aryl 6 bis 10 Kohlenstoffatome enthält, und nied.Alkyl 1 bis 6 Kohlenstoffatome enthält, bedeuten; $R^3$ Wasserstoff, nied.Alkyl mit 1 bis 6 Kohlenstoffatomen, Aryl-(nied.alkyl) oder Halogen-substituiertes Aryl-(nied.alkyl), wobei Aryl 6 bis 10 Kohlenstoffatome enthält, und nied.Alkyl 1 bis 6 Kohlenstoffatome enthält, oder einen (nied.Alkyl)-Heterocyclus der Strukturformel

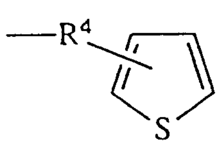 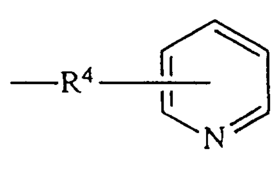 ,

wobei $R^4$ nied.Alkylen mit 1 bis 3 Kohlenstoffatomen ist, darstellt; X nied.Alkylen mit 1 bis 3 Kohlenstoffatomen, Sauerstoff, Schwefel oder NH bedeutet; Y und Z unabhängig Sauerstoff oder Schwefel sind, M und W unabhängig Carbonyl, Thiocarbonyl, Sulfonyl, Sulfoxo oder nied.Alkylen mit 1 bis 2 Kohlenstoffatomen darstellen, mit den Maßgaben, daß (i) M und W nicht beide nied.Alkylen sind, und (ii), wenn M die Bedeutung $CH_2$ hat, W von Sulfonyl verschieden ist; oder ein pharmazeutisch

annehmbares Salz hievon.

2. Verbindung nach Anspruch 1 der Formel (II)

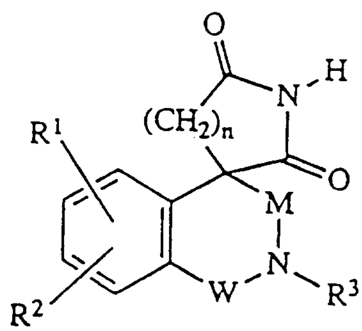

(II),

worin $R^1$ und $R^2$ unabhängig Wasserstoff, Alkyl mit 1 bis 6 Kohlenstoffatomen, Halogen, nied.Alkoxy mit 1 bis 6 Kohlenstoffatomen, Trifluormethyl, nied.Alkylthio, wobei nied.Alkyl 1 bis 6 Kohlenstoffatome enthält, bedeuten; $R^3$ Wasserstoff, nied.Alkyl mit 1 bis 6 Kohlenstoffatomen darstellt; M und W Carbonyl oder Thiocarbonyl sind; n 1 bis 3 ist; oder ein pharmazeutisch annehmbares Salz hievon.

3. Verbindung nach Anspruch 1 der Formel (II)

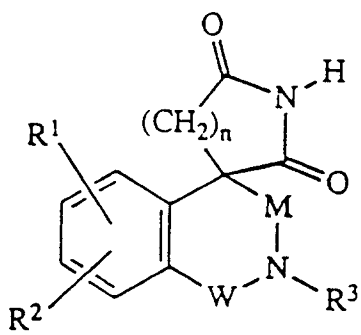

(II),

worin $R^1$ und $R^2$ unabhängig Wasserstoff, Alkyl mit 1 bis 6 Kohlenstoffatomen, Halogen, nied.Alkoxy mit 1 bis 6 Kohlenstoffatomen, Trifluormethyl, nied.Alkylthio, wobei nied.Alkyl 1 bis 6 Kohlenstoffatome enthält, bedeuten; $R^3$ Aryl-(nied.alkyl) oder Dihalogen-substituiertes Aryl-(nied.alkyl), wobei Aryl 6 bis 10 Kohlenstoffatome enthält, und nied.Alkyl 1 bis 6 Kohlenstoffatome enthält, darstellt; M und W Carbonyl oder Thiocarbonyl sind; n 1 bis 3 ist; oder ein pharmazeutisch annehmbares Salz hievon.

4. Verbindung nach Anspruch 1 der Formel (III)

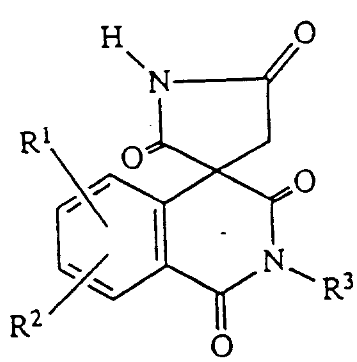

(III),

worin $R^1$ und $R^2$ Wasserstoff oder Halogen bedeuten; $R^3$ nied.Alkyl mit 1 bis 6 Kohlenstoffatomen,

Benzyl oder Dihalogen-substituiertes Benzyl darstellt; oder ein pharmazeutisch annehmbares Salz hievon.

**5.** Verbindung nach Anspruch 1, welche eine der folgenden ist:
2-[(4-Brom-2-fluorphenyl)-methyl]-spiro-[isochinolin-4(1H),3'-pyrrolidin]-1,2',3,5'(2H)-tetron,
[2-[(4-Brom-2-fluorphenyl)-methyl]-7-chlor]-spiro-[isochinolin-4(1H),3'-pyrrolidin]-1,2',3,5'(2H)-tetron,
[2-[(4-Brom-2-fluorphenyl)-methyl]-6-chlor]-spiro-[isochinolin-4(1H),3'-pyrrolidin]-1,2',3,5'(2H)-tetron,
2-[(3,4-Dichlorphenyl)-methyl]-spiro-[isochinolin-4(1H),3'-pyrrolidin]-1,2',3,5'(2H)-tetron,
[2-[(4-Brom-2-fluorphenyl)-methyl]-5-fluor]-spiro-[isochinolin-4(1H),3'-pyrrolidin]-1,2',3,5'(2H)-tetron,
[2-[(4-Brom-2-fluorphenyl)-methyl]-6-fluor]-spiro-[isochinolin-4(1H),3'-pyrrolidin]-1,2',3,5'(2H)-tetron,
[2-[(4-Brom-2-fluorphenyl)-methyl]-7-fluor]-spiro-[isochinolin-4(1H),3'-pyrrolidin]-1,2',3,5'(2H)-tetron,
[2-[(4-Brom-2-fluorphenyl)-methyl]-8-fluor]-spiro-[isochinolin-4(1H),3'-pyrrolidin]-1,2',3,5'(2H)-tetron,
[6-Brom-2-[(4-brom-2-fluorphenyl)-methyl]-7-methoxy]-spiro-[isochinolin-4(1H),3'-pyrrolidin]-1,2',3,5'-(2H)-tetron,
[2-[(4-Brom-2-fluorphenyl)-methyl]-7-methoxy]-spiro-[isochinolin-4(1H),3'-pyrrolidin]-1,2',3,5'(2H)-tetron,
(R)-(+)-2-[(4-Brom-2-fluorphenyl)-methyl]-spiro-[isochinolin-4(1H),3'-pyrrolidin]-1,2',3,5'(2H)-tetron,
(S)-(-)-2-[(4-Brom-2-fluorphenyl)-methyl]-spiro-[isochinolin-4(1H),3'-pyrrolidin]-1,2',3,5'(2H)-tetron,
(R)-(+)-[2-[(4-Brom-2-fluorphenyl)-methyl]-6-fluor]-spiro-[isochinolin-4(1H),3'-pyrrolidin]-1,2',3,5'(2H)-tetron,
(S)-(-)-[2-[(4-Brom-2-fluorphenyl)-methyl]-6-fluor]-spiro-[isochinolin-4(1H),3'-pyrrolidin]-1,2',3,5'(2H)-tetron,
2-Methylspiro-[isochinolin-4(1H),3'-pyrrolidin]-1,2',3,5'(2H)-tetron,
6-Chlor-2-methylspiro-[isochinolin-4(1H),3'-pyrrolidin]-1,2',3,5'(2H)-tetron,
6-Brom-2-methylspiro-[isochinolin-4(1H),3'-pyrrolidin]-1,2',3,5'(2H)-tetron,
7-Chlor-2-methylspiro-[isochinolin-4(1H),3'-pyrrolidin]-1,2',3,5'(2H)-tetron,
2-Ethylspiro-[isochinolin-4(1H),3'-pyrrolidin]-1,2',3,5'(2H)-tetron,
2-Propylspiro-[isochinolin-4(1H),3'-pyrrolidin]-1,2',3,5'(2H)-tetron,
2-Butylspiro-[isochinolin-4(1H),3'-pyrrolidin]-1,2',3,5'(2H)-tetron; oder ein pharmazeutisch annehmbares Salz hievon.

**6.** Verbindung nach einem der Ansprüche 1 bis 5, wenn in Form eines pharmazeutisch annehmbaren Ammonium-Alkalimetall- oder -Erdalkalimetallsalzes.

**7.** Verfahren zur Herstellung einer Verbindung der Formel (I) nach Anspruch 1, welches eines der folgenden umfaßt:
a) Cyclisieren eines Amids der Formel (A)

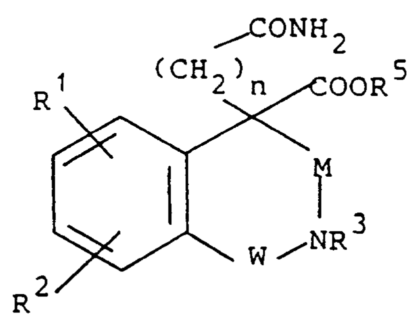

(A),

worin $R^1$, $R^2$, $R^3$, n, M und W wie oben definiert sind, und $COOR^5$ eine Estergruppe bedeutet, unter Verwendung einer geeigneten Base, wobei eine Verbindung der Formel (I), worin Y und Z beide Sauerstoff darstellen und X die Bedeutung $-(CH_2)_n-$, wobei n 1 bis 3 ist, hat, erhalten wird;

b) Umsetzen einer Verbindung der Formel (B)

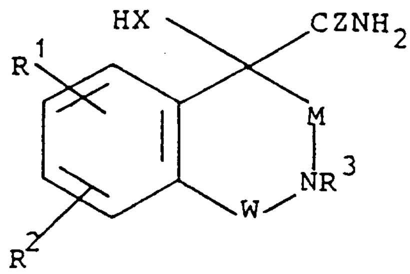

(B),

worin $R^1$, $R^2$, $R^3$, M und W wie oben definiert sind, und X die Bedeutung O, S oder NH hat, und Z O oder S ist, mit $CYCl_2$, worin Y O oder S darstellt, wobei eine Verbindung der Formel (I), worin X O, S oder NH bedeutet, und Y und Z unabhängig O oder S sind, erhalten wird; oder

c) Umsetzen einer Verbindung der Formel (C)

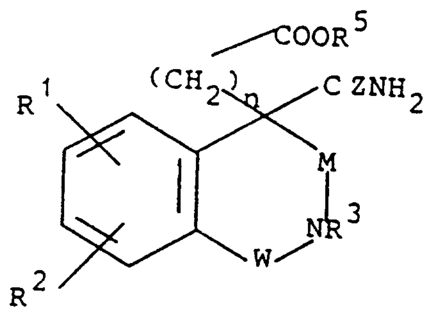

(C),

worin $R^1$, $R^2$, $R^3$, M, W, n, Z und $COOR^5$ wie oben definiert sind, unter Verwendung einer geeigneten Base, wobei eine Verbindung der Formel (I), worin Y die Bedeutung O hat, Z O oder S darstellt, und n 1 bis 3 ist, erhalten wird; oder

d) Umsetzen einer Verbindung der Formel D

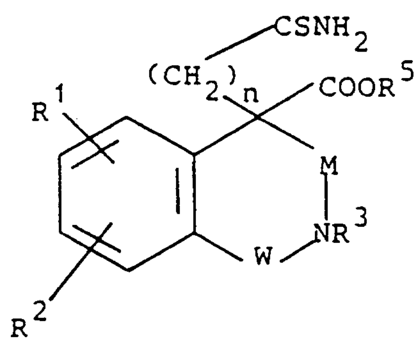

(D),

worin $R^1$, $R^2$, $R^3$, M, W, n und $COOR^5$ wie oben definiert sind, mit einer starken Base, wobei eine Verbindung der Formel (I), worin X die Bedeutung $-(CH_2)_n-$, wobei n 1 bis 3 ist, hat, und Y S darstellt, und Z O bedeutet, erhalten wird; oder

e) Umsetzen einer Verbindung der Formel

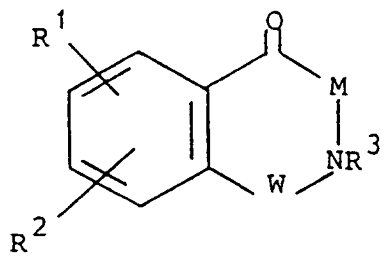

(F),

worin $R^1$, $R^2$, $R^3$, M und W wie oben definiert sind, mit Ammoniumcarbonat und einem Alkalimetallcyanid, wobei eine Verbindung der Formel (I), worin X die Bedeutung -NH- hat, und Y und Z beide Sauerstoff darstellen, erhalten wird; oder

f) Umsetzen einer Verbindung der Formel (F) aufeinanderfolgend mit Cyanwasserstoff, Thionylchlorid und Thioharnstoff, wobei eine Verbindung der Formel (I), worin X die Bedeutung S hat, und Y und Z beide Sauerstoff darstellen, erhalten wird; oder

g) Umsetzen einer Verbindung der Formel (F) mit Trimethylsilylcyanid, gefolgt von HCl/Alkanol und Phosgen oder Thiophosgen, wobei eine Verbindung der Formel (I), worin X die Bedeutung O oder S hat, und Y und Z beide Sauerstoff darstellen, erhalten wird.

**8.** Verfahren zur Herstellung einer Verbindung der Formel (XI)

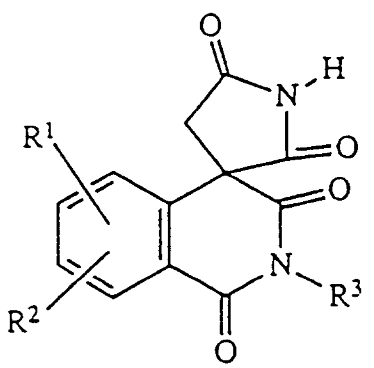

(XI),

worin $R^1$ und $R^2$ Wasserstoff oder Halogen bedeuten; $R^3$ Alkyl mit 1 bis 6 Kohlenstoffatomen, Aryl-(nied.alkyl) oder Dihalogen-substituiertes Aryl-(nied.alkyl), wobei Aryl 6 bis 10 Kohlenstoffatome enthält, und nied.Alkyl 1 bis 6 Kohlenstoffatome enthält, darstellt, welches Verfahren umfaßt:

a) Umsetzen der Verbindung der Formel (VI)

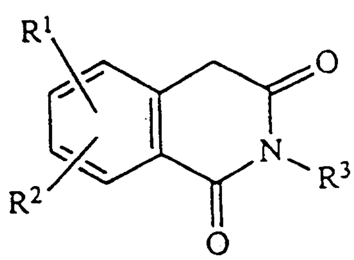

(VI),

worin $R^1$, $R^2$ und $R^3$ wie oben definiert sind, mit einer Base in einem herkömmlichen Lösungsmittel und anschließend Zusetzen eines reaktiven Carbomethoxylierungsmittels, wobei die Verbindung der Formel (VII)

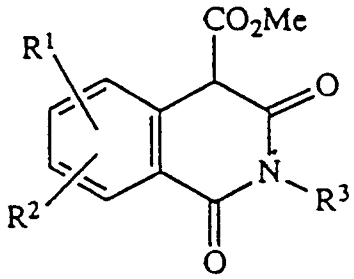

(VII),

worin $R^1$, $R^2$ und $R^3$ wie oben definiert sind, erhalten wird;
b) Umsetzen der Verbindung der Formel (VII) mit einer anorganischen Base in einem herkömmlichen Lösungsmittel und anschließend Zusetzen von tert.Butylbromacetat, wobei die Verbindung der Formel (VIII)

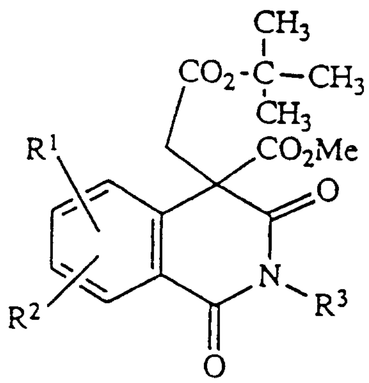

(VIII),

worin $R^1$, $R^2$ und $R^3$ wie oben definiert sind, erhalten wird;
c) Hydrolysieren der Verbindung der Formel (VIII) mit einer organischen Säure in einem herkömmlichen Lösungsmittel, wobei die Verbindung der Formel (IX)

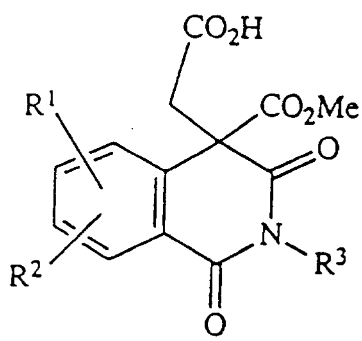

(IX),

worin $R^1$, $R^2$ und $R^3$ wie oben definiert sind, erhalten wird;
d) Umsetzen der Verbindung der Formel (IX) mit einem Kopplungsmittel in einem herkömmlichen Lösungsmittel und anschließend Zusetzen von Tetrahydrofuranammonium-Lösung, wobei die Verbindung der Formel (X)

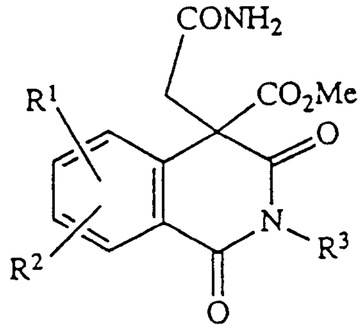

(X),

worin $R^1$, $R^2$ und $R^3$ wie oben definiert sind, erhalten wird;
e) Umsetzen der Verbindung der Formel (X) mit einer Base in einem herkömmlichen Lösungsmittel, wobei die Verbindung der Formel (XI) erhalten wird.

**9.** Verfahren nach Anspruch 8 zur Herstellung von Verbindungen der Formel (XI), worin $R^1$ und $R^2$ wie in Anspruch 8 definiert sind, und $R^3$ Alkyl mit 1 bis 6 Kohlenstoffatomen ist, bei welchem die Verbindung der Formel (X), worin $R^1$ und $R^2$ wie oben definiert sind, $R^3$ ein Alkyl darstellt, wie oben definiert, mit einer Base, wie Lithiumbis-(trimethylsilyl)-amid, in einem herkömmlichen Lösungsmittel umgesetzt wird, wobei die Verbindung der Formel (XI), worin $R^1$ und $R^2$ wie oben definiert sind, und $R^3$ ein Alkyl darstellt, wie oben definiert, erhalten wird.

**10.** Verfahren zur Herstellung einer Verbindung der Formel (VII)

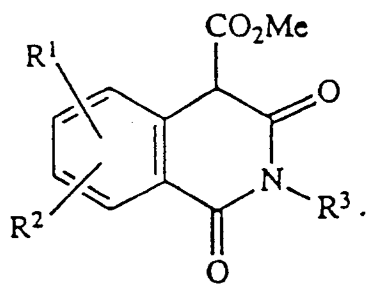

(VII),

worin $R^1$ und $R^2$ Wasserstoff oder Halogen bedeuten; $R^3$ Alkyl mit 1 bis 6 Kohlenstoffatomen, Aryl-(nied.alkyl) oder Dihalogen-substituiertes Aryl-(nied.alkyl), wobei Aryl 6 bis 10 Kohlenstoffatome enthält, und nied.Alkyl 1 bis 6 Kohlenstoffatome enthält, darstellt; welches Verfahren das Umsetzen der Verbindung der Formel (XII)

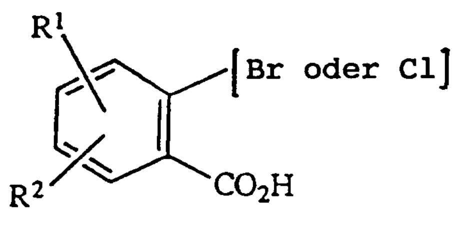

(XII),

worin $R^1$ und $R^2$ wie oben definiert sind, mit Dimethylmalonat und NaH in Anwesenheit einer katalytischen Menge an CuBr, wobei die Verbindung der Formel (XIII)

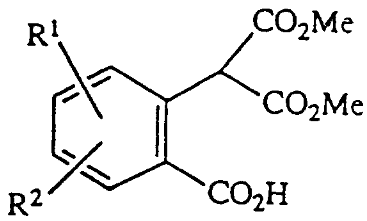

(XIII),

worin $R^1$ und $R^2$ wie oben definiert sind, erhalten wird, und Umsetzen der Verbindung der Formel (XIII) mit Thionylchlorid und anschließend Zusetzen eines inerten Lösungsmittels, von Triethylamin und eines Amins der Formel $R^3NH_2$, wobei die Verbindung der Formel (VII) erhalten wird, umfaßt.

**11.** Pharmazeutische Zusammensetzung, welche eine Verbindung der Formel (I) oder ein pharmazeutisch annehmbares Salz hievon, wie in einem der Ansprüche 1 bis 5 definiert, und einen pharmazeutisch annehmbaren Träger umfaßt.

**12.** Verbindung nach Anspruch 1, zur Verwendung bei der Herstellung eines Medikaments zur Prävention oder Linderung von Neuropathie, Nephropathie, Retinopathie oder Katarakten bei einem diabetischen Säuger.

**13.** Verbindung der Formel

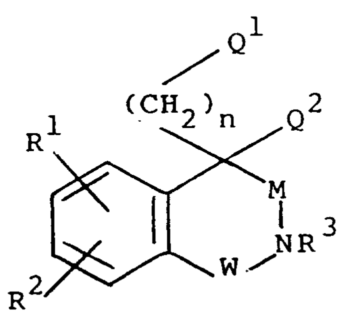

(XXIV),

worin eines von $Q^1$ und $Q^2$ die Bedeutung $-CZNH_2$ oder CN hat; das andere von $Q^1$ und $Q^2$ $-COOR^5$ darstellt; Z, $R^1$, $R^2$, $R^3$, M und W wie in Anspruch 1 definiert sind, $-COOR^5$ eine Estergruppe bedeutet, und n 1 bis 3 ist.

**14.** Verbindung nach Anspruch 13, worin $Q^1$ die Bedeutung $-CONH_2$ hat, $R^1$ und $R^2$ Wasserstoff oder Halogen darstellen; n 1 ist; M und W beide Carbonyl sind, und $R^3$ nied.Alkyl mit 1 bis 6 Kohlenstoffatomen, Benzyl oder Dihalogen-substituiertes Benzyl bedeutet.

**15.** Verbindung nach Anspruch 13, welche eine der folgenden ist:
4-(2-Amino-2-oxoethyl)-2-[(3,4-dichlorphenyl)-methyl]-1,2,3,4-tetrahydro-1,3-dioxo-4-isochinolincarbonsäuremethylester;
4-(2-Amino-2-oxoethyl)-2-[(4-brom-2-fluorphenyl)-methyl]-1,2,3,4-tetrahydro-1,3-dioxo-4-isochinolincarbonsäuremethylester;
4-(2-Amino-2-oxoethyl)-2-[(4-brom-2-fluorphenyl)-methyl]-7-chlor-1,2,3,4-tetrahydro-1,3-dioxo-4-isochinolincarbonsäuremethylester;
4-(2-Amino-2-oxoethyl)-2-[(4-brom-2-fluorphenyl)-methyl]-6-chlor-1,2,3,4-tetrahydro-1,3-dioxo-4-isochinolincarbonsäuremethylester;
4-(2-Amino-2-oxoethyl)-2-[(4-brom-2-fluorphenyl)-methyl]-6-fluor-1,2,3,4-tetrahydro-1,3-dioxo-4-isochinolincarbonsäuremethylester;
4-(2-Amino-2-oxoethyl)-1,2,3,4-tetrahydro-2-methyl-1,3-dioxo-4-isochinolincarbonsäuremethylester;
4-(2-Amino-2-oxoethyl)-7-chlor-1,2,3,4-tetrahydro-2-methyl-1,3-dioxo-4-isochinolincarbonsäuremethylester;
4-(2-Amino-2-oxoethyl)-6-chlor-1,2,3,4-tetrahydro-2-methyl-1,3-dioxo-4-isochinolincarbonsäuremethylester;

4-(2-Amino-2-oxoethyl)-2-ethyl-1,2,3,4-tetrahydro-1,3-dioxo-4-isochinolincarbonsäuremethylester;
4-(2-Amino-2-oxoethyl)-1,2,3,4-tetrahydro-2-propyl-1,3-dioxo-4-isochinolincarbonsäuremethylester;
4-(2-Amino-2-oxoethyl)-2-butyl-1,2,3,4-tetrahydro-1,3-dioxo-4-isochinolincarbonsäuremethylester;
4-(2-Amino-2-oxoethyl)-6-brom-1,2,3,4-tetrahydro-2-methyl-1,3-dioxo-4-isochinolincarbonsäuremethylester;
4-(2-Amino-2-oxoethyl)-2-[(4-brom-2-fluorphenyl)-methyl]-1,2,3,4-tetrahydro-1,3-dioxo-4-isochinolincarbonsäurementhylester; oder
4-(2-Amino-2-oxoethyl)-2-[(4-brom-2-fluorphenyl)-methyl]-6-fluor-1,2,3,4-tetrahydro-1,3-dioxo-4-isochinolincarbonsäurementhylester.

**16.** Verfahren zur Herstellung einer Verbindung der Formel

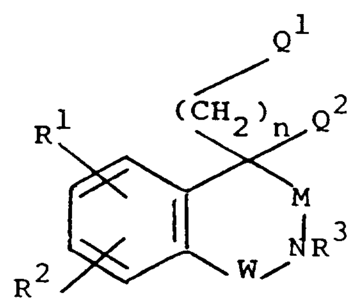

(XXIV),

worin eines von $Q^1$ und $Q^2$ die Bedeutung $-CZNH_2$ oder CN hat, und das andere von $Q^1$ und $Q^2$ $-COOR^5$ darstellt; n 1 bis 3 ist, und M, W, Z, $R^1$, $R^2$, $R^3$ und $-COOR^5$ wie in Anspruch 1 definiert sind, welches Verfahren umfaßt:

a) Umsetzen einer Säure der Formel

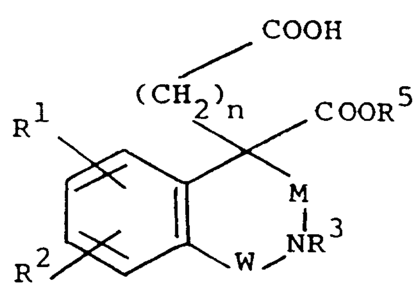

(XXV),

worin $R^1$, $R^2$, $R^3$, M, W und $-COOR^5$ wie oben definiert sind, mit einem Kopplungsmittel und Ammoniak, wobei eine entsprechende Verbindung der Formel (I), worin $Q^1$ die Bedeutung $CONH_2$ hat, erhalten wird; oder

b) Umsetzen einer Verbindung der Formel (L)

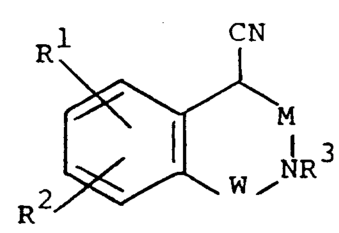

(L),

worin $R^1$, $R^2$, M, W und $R^3$ wie in Anspruch 1 definiert sind, mit einer Verbindung der Formel

$Br(CH_2)_nCOOR^5$,

worin n 1 bis 3 ist, und $COOR^5$ eine Estergruppe darstellt, wobei eine Verbindung der Formel

(XXIV), worin $Q^1$ die Bedeutung $COOR^5$ hat, und $Q^2$ CN ist, erhalten wird;
c) Umsetzen einer Verbindung der Formel G

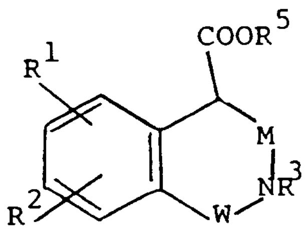

(G),

worin $R^1$, $R^2$, $R^3$, M, W und $-COOR^5$ wie in Anspruch 1 definiert sind, mit einer Verbindung der Formel

$Br(CH_2)_nCN$,

worin n wie oben definiert ist, wobei eine Verbindung der Formel (XXIV), worin $Q^1$ die Bedeutung CN hat, und $Q^2$ $COOR^5$ darstellt, erhalten wird; oder
d) Überführen von Nitril der Formel (XXIV), worin $Q^2$ die Bedeutung CN hat, in ein Amid oder Thioamid der Formel (XXIV), worin $Q^2$ $CONH_2$ oder $CSNH_2$ darstellt, auf bekannte Weise; oder
e) Überführen eines Nitrils der Formel (XXIV), worin $Q^1$ die Bedeutung CN hat, in Thioamid der Formel (XXIV), worin $Q^1$ $CSNH_2$ darstellt, unter Verwendung von $HSP(=S)(OEt)_2/HCl$.

**Patentansprüche für folgenden Vertragsstaat : ES**

**1.** Verfahren zur Herstellung einer Verbindung der Formel (I)

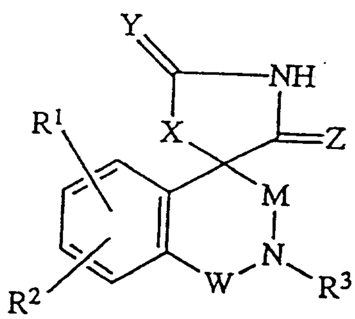

(I)

und von Analoga hievon, wobei der kondensierte Benzolring durch einen kondensierten Thiophen-, Pyridin- oder Furanring ersetzt ist, worin $R^1$ und $R^2$ unabhängig Wasserstoff, Alkyl mit 1 bis 6 Kohlenstoffatomen, Halogen, nied.Alkoxy mit 1 bis 6 Kohlenstoffatomen, Trifluormethyl, nied.Alkylthio, wobei nied.Alkyl 1 bis 6 Kohlenstoffatome enthält, Dialkylamino, wobei Alkyl 1 bis 6 Kohlenstoffatome enthält, Nitro, Aryl oder Aryl-(nied.alkyl)-oxy, wobei Aryl 6 bis 10 Kohlenstoffatome enthält, und nied.Alkyl 1 bis 6 Kohlenstoffatome enthält, bedeuten; $R^3$ Wasserstoff, nied.Alkyl mit 1 bis 6 Kohlenstoffatomen, Aryl-(nied.alkyl) oder Halogen-substituiertes Aryl-(nied.alkyl), wobei Aryl 6 bis 10 Kohlenstoffatome enthält, und nied.Alkyl 1 bis 6 Kohlenstoffatome enthält, oder einen (nied.Alkyl)-Heterocyclus der Strukturformel

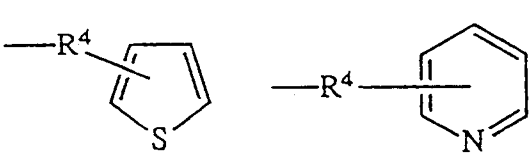

,

wobei $R^4$ nied.Alkylen mit 1 bis 3 Kohlenstoffatomen ist, darstellt; X nied.Alkylen mit 1 bis 3

Kohlenstoffatomen, Sauerstoff, Schwefel oder NH bedeutet; Y und Z unabhängig Sauerstoff oder Schwefel sind, M und W unabhängig Carbonyl, Thiocarbonyl, Sulfonyl, Sulfoxo oder nied.Alkylen mit 1 bis 2 Kohlenstoffatomen darstellen, mit den Maßgaben, daß (i) M und W nicht beide nied.Alkylen sind, und (ii), wenn M die Bedeutung $CH_2$ hat, W von Sulfonyl verschieden ist; oder eines pharmazeutisch annehmbaren Salzes hievon, welches Verfahren eines der folgenden umfaßt:

a) Cyclisieren eines Amids der Formel (A)

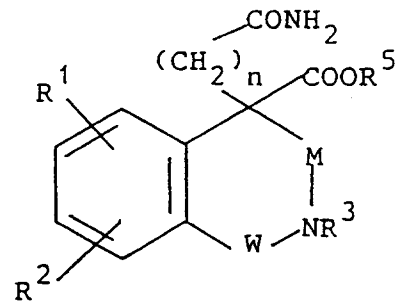

(A),

worin $R^1$, $R^2$, $R^3$, n, M und W wie oben definiert sind, und $COOR^5$ eine Estergruppe bedeutet, unter Verwendung einer geeigneten Base, wobei eine Verbindung der Formel (I), worin Y und Z beide Sauerstoff darstellen und X die Bedeutung $-(CH_2)_n-$, wobei n 1 bis 3 ist, hat, erhalten wird;

b) Umsetzen einer Verbindung der Formel (B)

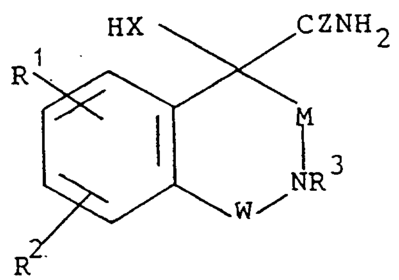

(B),

worin $R^1$, $R^2$, $R^3$, M und W wie oben definiert sind, und X die Bedeutung O, S oder NH hat, und Z O oder S ist, mit $CYCl_2$, worin Y O oder S darstellt, wobei eine Verbindung der Formel (I), worin X O, S oder NH bedeutet, und Y und Z unabhängig O oder S sind, erhalten wird; oder

c) Umsetzen einer Verbindung der Formel (C)

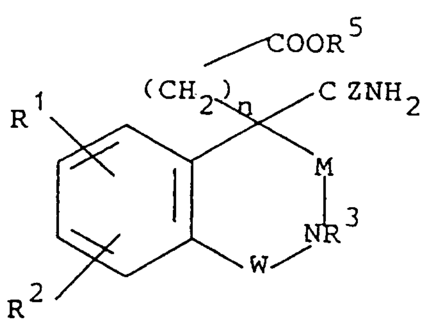

(C),

worin $R^1$, $R^2$, $R^3$, M, W, n, Z und $COOR^5$ wie oben definiert sind, unter Verwendung einer geeigneten Base, wobei eine Verbindung der Formel (I), worin Y die Bedeutung O hat, Z O oder S darstellt, und n 1 bis 3 ist, erhalten wird; oder

d) Umsetzen einer Verbindung der Formel D

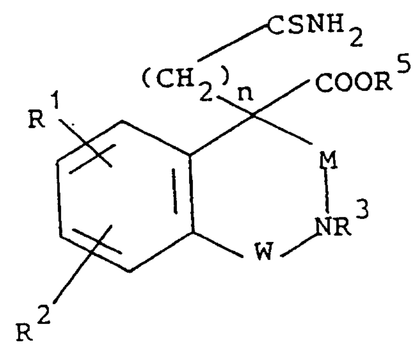

(D),

worin $R^1$, $R^2$, $R^3$, M, W, n und $COOR^5$ wie oben definiert sind, mit einer starken Base, wobei eine Verbindung der Formel (I), worin X die Bedeutung $-(CH_2)_n-$, wobei n 1 bis 3 ist, hat, und Y S darstellt, und Z O bedeutet, erhalten wird; oder

e) Umsetzen einer Verbindung der Formel

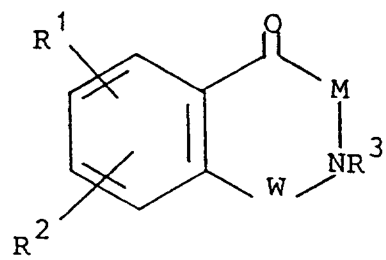

(F),

worin $R^1$, $R^2$, $R^3$, M und W wie oben definiert sind, mit Ammoniumcarbonat und einem Alkalimetallcyanid, wobei eine Verbindung der Formel (I), worin X die Bedeutung -NH- hat, und Y und Z beide Sauerstoff darstellen, erhalten wird; oder

f) Umsetzen einer Verbindung der Formel (F) aufeinanderfolgend mit Cyanwasserstoff, Thionylchlorid und Thioharnstoff, wobei eine Verbindung der Formel (I), worin X die Bedeutung S hat, und Y und Z beide Sauerstoff darstellen, erhalten wird; oder

g) Umsetzen einer Verbindung der Formel (F) mit Trimethylsilylcyanid, gefolgt von HCl/Alkanol und Phosgen oder Thiophosgen, wobei eine Verbindung der Formel (I), worin X die Bedeutung O oder S hat, und Y und Z beide Sauerstoff darstellen, erhalten wird.

**2.** Verfahren nach Anspruch 1, bei welchem die hergestellte Verbindung die Formel (II)

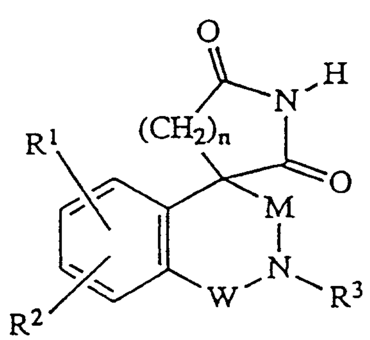

(II),

worin $R^1$ und $R^2$ unabhängig Wasserstoff, Alkyl mit 1 bis 6 Kohlenstoffatomen, Halogen, nied.Alkoxy mit 1 bis 6 Kohlenstoffatomen, Trifluormethyl, nied.Alkylthio, wobei nied.Alkyl 1 bis 6 Kohlenstoffatome enthält, bedeuten; $R^3$ Wasserstoff, nied.Alkyl mit 1 bis 6 Kohlenstoffatomen darstellt; M und W Carbonyl oder Thiocarbonyl sind; n 1 bis 3 ist; aufweist, oder ein pharmazeutisch annehmbares Salz

hievon.

3. Verfahren nach Anspruch 1, bei welchem die hergestellte Verbindung die Formel (II)

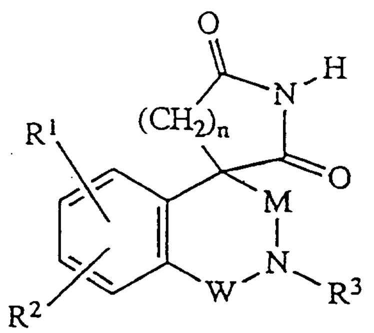

(II),

worin $R^1$ und $R^2$ unabhängig Wasserstoff, Alkyl mit 1 bis 6 Kohlenstoffatomen, Halogen, nied.Alkoxy mit 1 bis 6 Kohlenstoffatomen, Trifluormethyl, nied.Alkylthio, wobei nied.Alkyl 1 bis 6 Kohlenstoffatome enthält, bedeuten; $R^3$ Aryl-(nied.alkyl) oder Dihalogen-substituiertes Aryl-(nied.alkyl), wobei Aryl 6 bis 10 Kohlenstoffatome enthält, und nied.Alkyl 1 bis 6 Kohlenstoffatome enthält, darstellt; M und W Carbonyl oder Thiocarbonyl sind; n 1 bis 3 ist; aufweist, oder ein pharmazeutisch annehmbares Salz hievon.

4. Verfahren nach Anspruch 1, bei welchem die hergestellte Verbindung die Formel (III)

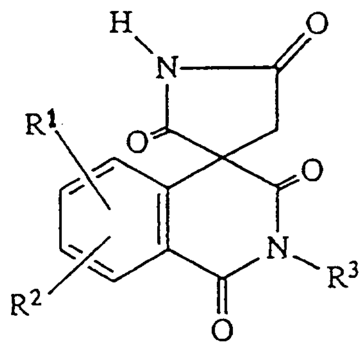

(III),

worin $R^1$ und $R^2$ Wasserstoff oder Halogen bedeuten; $R^3$ nied.Alkyl mit 1 bis 6 Kohlenstoffatomen, Benzyl oder Dihalogen-substituiertes Benzyl darstellt; aufweist, oder ein pharmazeutisch annehmbares Salz hievon.

5. Verfahren nach Anspruch 1, bei welchem die hergestellte Verbindung der Formel (I) eine der folgenden ist:
2-[(4-Brom-2-fluorphenyl)-methyl]-spiro-[isochinolin-4(1H),3'-pyrrolidin]-1,2',3,5'(2H)-tetron,
[2-[(4-Brom-2-fluorphenyl)-methyl]-7-chlor]-spiro-[isochinolin-4(1H),3'-pyrrolidin]-1,2',3,5'(2H)-tetron,
[2-[(4-Brom-2-fluorphenyl)-methyl]-6-chlor]-spiro-[isochinolin-4(1H),3'-pyrrolidin]-1,2',3,5'(2H)-tetron,
2-[(3,4-Dichlorphenyl)-methyl]-spiro-[isochinolin-4(1H),3'-pyrrolidin]-1,2',3,5'(2H)-tetron,
[2-[(4-Brom-2-fluorphenyl)-methyl]-5-fluor]-spiro-[isochinolin-4(1H),3'-pyrrolidin]-1,2',3,5'(2H)-tetron,
[2-[(4-Brom-2-fluorphenyl)-methyl]-6-fluor]-spiro-[isochinolin-4(1H),3'-pyrrolidin]-1,2',3,5'(2H)-tetron,
[2-[(4-Brom-2-fluorphenyl)-methyl]-7-fluor]-spiro-[isochinolin-4(1H),3'-pyrrolidin]-1,2',3,5'(2H)-tetron,
[2-[(4-Brom-2-fluorphenyl)-methyl]-8-fluor]-spiro-[isochinolin-4(1H),3'-pyrrolidin]-1,2',3,5'(2H)-tetron,
[6-Brom-2-[(4-brom-2-fluorphenyl)-methyl]-7-methoxy]-spiro-[isochinolin-4(1H),3'-pyrrolidin]-1,2',3,5'-(2H)-tetron,
[2-[(4-Brom-2-fluorphenyl)-methyl]-7-methoxy]-spiro-[isochinolin-4(1H),3'-pyrrolidin]-1,2',3,5'(2H)-tetron,
(R)-(+)-2-[(4-Brom-2-fluorphenyl)-methyl]-spiro-[isochinolin-4(1H),3'-pyrrolldin]-1,2',3,5'(2H)-tetron,
(S)-(-)-2-[(4-Brom-2-fluorphenyl)-methyl]-spiro-[isochinolin-4(1H),3'-pyrrolidin]-1,2',3,5'(2H)-tetron,
(R)-(+)-[2-[(4-Brom-2-fluorphenyl)-methyl]-6-fluor]-spiro-[isochinolin-4(1H),3'-pyrrolidin]-1,2',3,5'(2H)-tetron,

(S)-(-)-[2-[(4-Brom-2-fluorphenyl)-methyl]-6-fluor]-spiro-[isochinolin-4(1H),3'-pyrrolidin]-1,2',3,5'(2H)-tetron,
2-Methylspiro-[isochinolin-4(1H),3'-pyrrolldin]-1,2',3,5'(2H)-tetron,
6-Chlor-2-methylspiro-[isochinolin-4(1H),3'-pyrrolidin]-1,2',3,5'(2H)-tetron,
6-Brom-2-methylspiro-[isochinolin-4(1H),3'-pyrrolidin]-1,2',3,5'(2H)-tetron,
7-Chlor-2-methylspiro-[isochinolin-4(1H),3'-pyrrolidin]-1,2',3,5'(2H)-tetron,
2-Ethylspiro-[isochinolin-4(1H),3'-pyrrolidin]-1,2',3,5'(2H)-tetron,
2-Propylspiro-[isochinolin-4(1H),3'-pyrrolidin]-1,2',3,5'(2H)-tetron,
2-Butylspiro-[isochinolin-4(1H),3'-pyrrolidin]-1,2',3,5'(2H)-tetron; oder ein pharmazeutisch annehmbares Salz hievon ist.

**6.** Verfahren zur Herstellung einer Verbindung der Formel (XI)

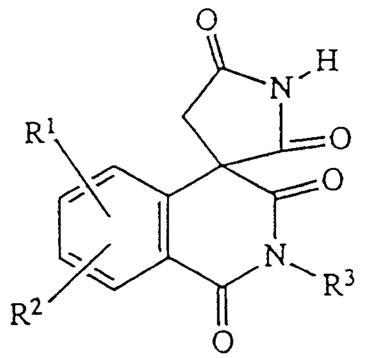

(XI),

worin $R^1$ und $R^2$ Wasserstoff oder Halogen bedeuten; $R^3$ Alkyl mit 1 bis 6 Kohlenstoffatomen, Aryl-(nied.alkyl) oder Dihalogen-substituiertes Aryl-(nied.alkyl), wobei Aryl 6 bis 10 Kohlenstoffatome enthält, und nied.Alkyl 1 bis 6 Kohlenstoffatome enthält, darstellt, welches Verfahren umfaßt:

a) Umsetzen der Verbindung der Formel (VI)

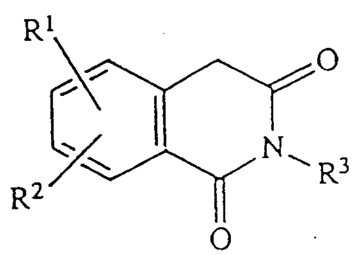

(VI),

worin $R^1$, $R^2$ und $R^3$ wie oben definiert sind, mit einer Base in einem herkömmlichen Lösungsmittel und anschließend Zusetzen eines reaktiven Carbomethoxylierungsmittels, wobei die Verbindung der Formel (VII)

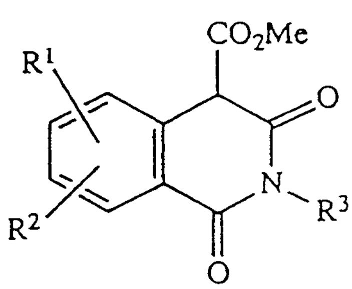

(VII),

worin $R^1$, $R^2$ und $R^3$ wie oben definiert sind, erhalten wird;

b) Umsetzen der Verbindung der Formel (VII) mit einer anorganischen Base in einem herkömmlichen Lösungsmittel und anschließend Zusetzen von tert.Butylbromacetat, wobei die Verbindung der

Formel (VIII)

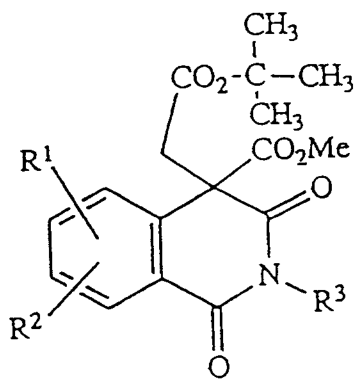

(VIII),

worin $R^1$, $R^2$ und $R^3$ wie oben definiert sind, erhalten wird;

c) Hydrolysieren der Verbindung der Formel (VIII) mit einer organischen Säure in einem herkömmlichen Lösungsmittel, wobei die Verbindung der Formel (IX)

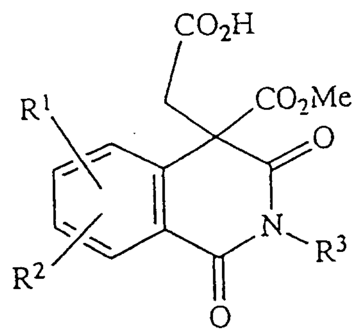

(IX),

worin $R^1$, $R^2$ und $R^3$ wie oben definiert sind, erhalten wird;

d) Umsetzen der Verbindung der Formel (IX) mit einem Kopplungsmittel in einem herkömmlichen Lösungsmittel und anschließend Zusetzen von Tetrahydrofuranammonium-Lösung, wobei die Verbindung der Formel (X)

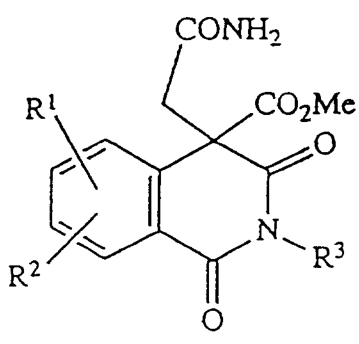

(X),

worin $R^1$, $R^2$ und $R^3$ wie oben definiert sind, erhalten wird;

e) Umsetzen der Verbindung der Formel (X) mit einer Base in einem herkömmlichen Lösungsmittel, wobei die Verbindung der Formel (XI) erhalten wird.

**7.** Verfahren nach Anspruch 6 zur Herstellung von Verbindungen der Formel (XI), worin $R^1$ und $R^2$ wie in Anspruch 6 definiert sind, und $R^3$ Alkyl mit 1 bis 6 Kohlenstoffatomen ist, bei welchem die Verbindung der Formel (X), worin $R^1$ und $R^2$ wie oben definiert sind, $R^3$ ein Alkyl darstellt, wie oben definiert, mit einer Base, wie Lithiumbis-(trimethylsilyl)-amid, in einem herkömmlichen Lösungsmittel umgesetzt wird, wobei die Verbindung der Formel (XI), worin $R^1$ und $R^2$ wie oben definiert sind, und $R^3$ ein Alkyl

darstellt, wie oben definiert, erhalten wird.

**8.** Verfahren zur Herstellung einer Verbindung der Formel (VII)

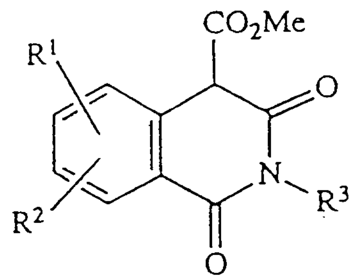

(VII),

worin $R^1$ und $R^2$ Wasserstoff oder Halogen bedeuten; $R^3$ Alkyl mit 1 bis 6 Kohlenstoffatomen, Aryl-(nied.alkyl) oder Dihalogen-substituiertes Aryl-(nied.alkyl), wobei Aryl 6 bis 10 Kohlenstoffatome enthält, und nied.Alkyl 1 bis 6 Kohlenstoffatome enthält, darstellt; welches Verfahren das Umsetzen der Verbindung der Formel (XII)

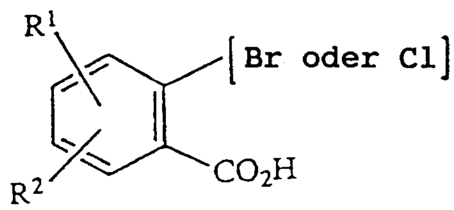

(XII),

worin $R^1$ und $R^2$ wie oben definiert sind, mit Dimethylmalonat und NaH in Anwesenheit einer katalytischen Menge an CuBr, wobei die Verbindung der Formel (XIII)

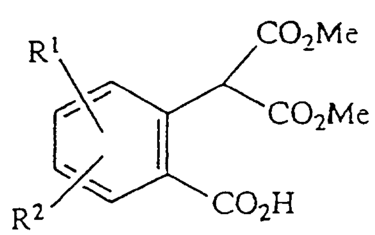

(XIII),

worin $R^1$ und $R^2$ wie oben definiert sind, erhalten wird, und Umsetzen der Verbindung der Formel (XIII) mit Thionylchlorid und anschließend Zusetzen eines inerten Lösungsmittels, von Triethylamin und eines Amins der Formel $R^3NH_2$, wobei die Verbindung der Formel (VII) erhalten wird, umfaßt.

**9.** Verfahren zur Herstellung einer pharmazeutischen Zusammensetzung, welches das Vereinigen einer Verbindung der Formel (I) oder eines Analogons oder eines pharmazeutisch annehmbaren Salzes hievon, wie in Anspruch 1 definiert, mit einem pharmazeutisch annehmbaren Träger umfaßt.

**10.** Verfahren zur Herstellung einer Verbindung der Formel

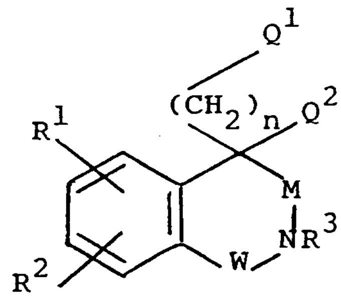

(XXIV),

worin eines von $Q^1$ und $Q^2$ die Bedeutung $-CZNH_2$ oder CN hat; das andere von $Q^1$ und $Q^2$ $-COOR^5$ darstellt; n 1 bis 3 ist, und M, W, Z, $R^1$, $R^2$, $R^3$ und $-COOR^5$ wie in Anspruch 1 definiert sind, welches Verfahren umfaßt:

a) Umsetzen einer Säure der Formel

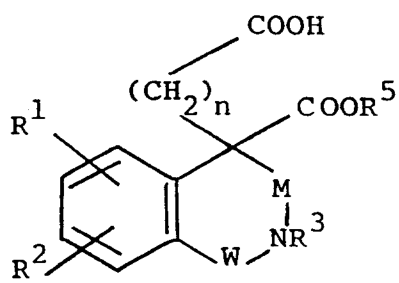

(XXV),

worin $R^1$, $R^2$, $R^3$, M, W und $-COOR^5$ wie oben definiert sind, mit einem Kopplungsmittel und Ammoniak, wobei eine entsprechende Verbindung der Formel (I), worin $Q^1$ die Bedeutung $CONH_2$ hat, erhalten wird; oder

b) Umsetzen einer Verbindung der Formel (L)

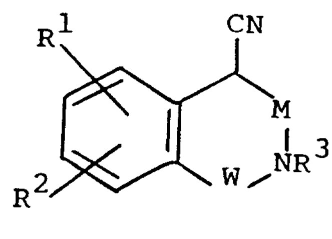

(L),

worin $R^1$, $R^2$, M, W und $R^3$ wie in Anspruch 1 definiert sind, mit einer Verbindung der Formel

$Br(CH_2)_nCOOR^5$,

worin n 1 bis 3 ist, und $COOR^5$ eine Estergruppe darstellt, wobei eine Verbindung der Formel (XXIV), worin $Q^1$ die Bedeutung $COOR^5$ hat, und $Q^2$ CN ist, erhalten wird;

c) Umsetzen einer Verbindung der Formel G

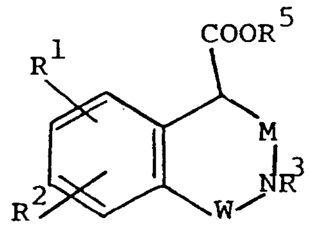

(G),

worin $R^1$, $R^2$, $R^3$, M, W und -$COOR^5$ wie in Anspruch 1 definiert sind, mit einer Verbindung der Formel

$Br(CH_2)_nCN$,

worin n wie oben definiert ist, wobei eine Verbindung der Formel (XXIV), worin $Q^1$ die Bedeutung CN hat, und $Q^2$ $COOR^5$ darstellt, erhalten wird; oder

d) Überführen von Nitril der Formel (XXIV), worin $Q^2$ die Bedeutung CN hat, in ein Amid oder Thioamid der Formel (XXIV), worin $Q^2$ $CONH_2$ oder $CSNH_2$ darstellt, auf bekannte Weise; oder

e) Überführen eines Nitrils der Formel (XXIV), worin $Q^1$ die Bedeutung CN hat, in Thioamid der Formel (XXIV), worin $Q^1$ $CSNH_2$ darstellt, unter Verwendung von $HSP(=S)(OEt)_2/HCl$.

**11.** Verfahren nach Anspruch 10, bei welchem das Produkt eines der folgenden ist:
4-(2-Amino-2-oxoethyl)-2-[(3,4-dichlorphenyl)-methyl]-1,2,3,4-tetrahydro-1,3-dioxo-4-isochinolincarbonsäuremethylester;
4-(2-Amino-2-oxoethyl)-2-[(4-brom-2-fluorphenyl)-methyl]-1,2,3,4-tetrahydro-1,3-dioxo-4-isochinolincarbonsäuremethylester;
4-(2-Amino-2-oxoethyl)-2-[(4-brom-2-fluorphenyl)-methyl]-7-chlor-1,2,3,4-tetrahydro-1,3-dioxo-4-isochinolincarbonsäuremethylester;
4-(2-Amino-2-oxoethyl)-2-[(4-brom-2-fluorphenyl)-methyl]-6-chlor-1,2,3,4-tetrahydro-1,3-dioxo-4-isochinolincarbonsäuremethylester;
4-(2-Amino-2-oxoethyl)-2-[(4-brom-2-fluorphenyl)-methyl]-6-fluor-1,2,3,4-tetrahydro-1,3-dioxo-4-isochinolincarbonsäuremethylester;
4-(2-Amino-2-oxoethyl)-1,2,3,4-tetrahydro-2-methyl-1,3-dioxo-4-isochinolincarbonsäuremethylester;
4-(2-Amino-2-oxoethyl)-7-chlor-1,2,3,4-tetrahydro-2-methyl-1,3-dioxo-4-isochinolincarbonsäuremethylester;
4-(2-Amino-2-oxoethyl)-6-chlor-1,2,3,4-tetrahydro-2-methyl-1,3-dioxo-4-isochinolincarbonsäuremethylester;
4-(2-Amino-2-oxoethyl)-2-ethyl-1,2,3,4-tetrahydro-1,3-dioxo-4-isochinolincarbonsäuremethylester;
4-(2-Amino-2-oxoethyl)-1,2,3,4-tetrahydro-2-propyl-1,3-dioxo-4-isochinolincarbonsäuremethylester;
4-(2-Amino-2-oxoethyl)-2-butyl-1,2,3,4-tetrahydro-1,3-dioxo-4-isochinolincarbonsäuremethylester;
4-(2-Amino-2-oxoethyl)-6-brom-1,2,3,4-tetrahydro-2-methyl-1,3-dioxo-4-isochinolincarbonsäuremethylester;
4-(2-Amino-2-oxoethyl)-2-[(4-brom-2-fluorphenyl)-methyl]-1,2,3,4-tetrahydro-1,3-dioxo-4-isochinolincarbonsäurementhylester; oder
4-(2-Amino-2-oxoethyl)-2-[(4-brom-2-fluorphenyl)-methyl]-6-fluor-1,2,3,4-tetrahydro-1,3-dioxo-4-isochinolincarbonsäurementhylester.

**Patentansprüche für folgenden Vertragsstaat : GR**

1. Verfahren zur Herstellung einer Verbindung der Formel (I)

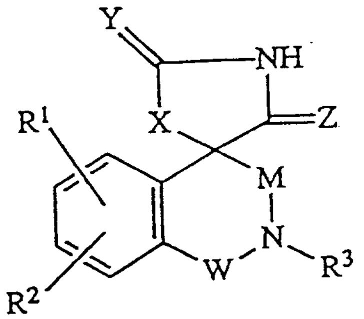

(I)

und von Analoga hievon, wobei der kondensierte Benzolring durch einen kondensierten Thiophen-, Pyridin- oder Furanring ersetzt ist, worin $R^1$ und $R^2$ unabhängig Wasserstoff, Alkyl mit 1 bis 6 Kohlenstoffatomen, Halogen, nied.Alkoxy mit 1 bis 6 Kohlenstoffatomen, Trifluormethyl, nied.Alkylthio, wobei nied.Alkyl 1 bis 6 Kohlenstoffatome enthält, Dialkylamino, wobei Alkyl 1 bis 6 Kohlenstoffatome enthält, Nitro, Aryl oder Aryl-(nied.alkyl)-oxy, wobei Aryl 6 bis 10 Kohlenstoffatome enthält, und nied.Alkyl 1 bis 6 Kohlenstoffatome enthält, bedeuten; $R^3$ Wasserstoff, nied.Alkyl mit 1 bis 6 Kohlenstoffatomen, Aryl-(nied.alkyl) oder Halogen-substituiertes Aryl-(nied.alkyl), wobei Aryl 6 bis 10 Kohlenstoffatome enthält, und nied.Alkyl 1 bis 6 Kohlenstoffatome enthält, oder einen (nied.Alkyl)-Heterocyclus der Strukturformel

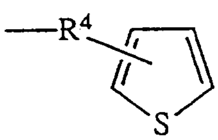

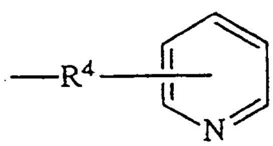

,

wobei $R^4$ nied.Alkylen mit 1 bis 3 Kohlenstoffatomen ist, darstellt; X nied.Alkylen mit 1 bis 3 Kohlenstoffatomen, Sauerstoff, Schwefel oder NH bedeutet; Y und Z unabhängig Sauerstoff oder Schwefel sind, M und W unabhängig Carbonyl, Thiocarbonyl, Sulfonyl, Sulfoxo oder nied.Alkylen mit 1 bis 2 Kohlenstoffatomen darstellen, mit den Maßgaben, daß (i) M und W nicht beide nied.Alkylen sind, und (ii), wenn M die Bedeutung $CH_2$ hat, W von Sulfonyl verschieden ist; oder eines pharmazeutisch annehmbaren Salzes hievon, welches Verfahren eines der folgenden umfaßt:

a) Cyclisieren eines Amids der Formel (A)

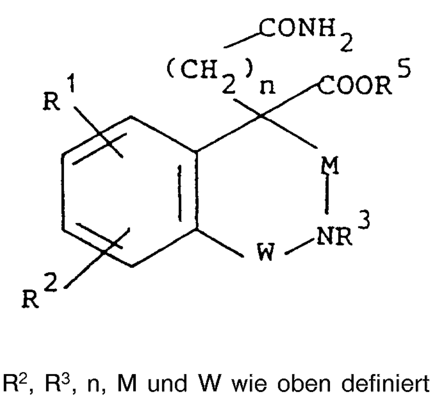

(A),

worin $R^1$, $R^2$, $R^3$, n, M und W wie oben definiert sind, und $COOR^5$ eine Estergruppe bedeutet, unter Verwendung einer geeigneten Base, wobei eine Verbindung der Formel (I), worin Y und Z beide Sauerstoff darstellen und X die Bedeutung $-(CH_2)_n-$, wobei n 1 bis 3 ist, hat, erhalten wird;

b) Umsetzen einer Verbindung der Formel (B)

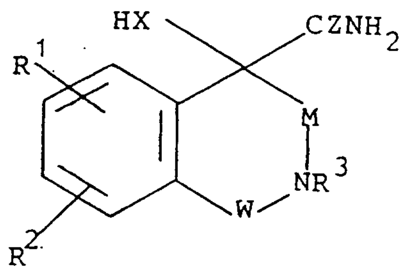

(B),

worin $R^1$, $R^2$, $R^3$, M und W wie oben definiert sind, und X die Bedeutung O, S oder NH hat, und Z O oder S ist, mit $CYCl_2$, worin Y O oder S darstellt, wobei eine Verbindung der Formel (I), worin X O, S oder NH bedeutet, und Y und Z unabhängig O oder S sind, erhalten wird; oder

c) Umsetzen einer Verbindung der Formel (C)

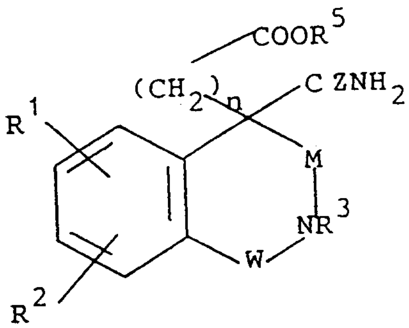

(C),

worin $R^1$, $R^2$, $R^3$, M, W, n, Z und $COOR^5$ wie oben definiert sind, unter Verwendung einer geeigneten Base, wobei eine Verbindung der Formel (I), worin Y die Bedeutung O hat, Z O oder S darstellt, und n 1 bis 3 ist, erhalten wird; oder

d) Umsetzen einer Verbindung der Formel D

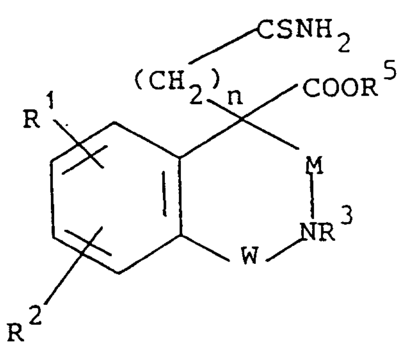

(D),

worin $R^1$, $R^2$, $R^3$, M, W, n und $COOR^5$ wie oben definiert sind, mit einer starken Base, wobei eine Verbindung der Formel (I), worin X die Bedeutung $-(CH_2)_n-$, wobei n 1 bis 3 ist, hat, und Y S darstellt, und Z O bedeutet, erhalten wird; oder

e) Umsetzen einer Verbindung der Formel

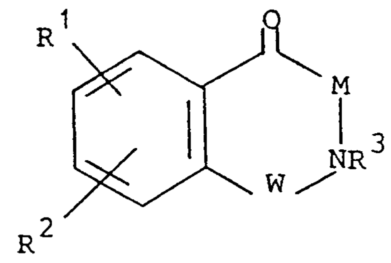

(F),

worin $R^1$, $R^2$, $R^3$, M und W wie oben definiert sind, mit Ammoniumcarbonat und einem Alkalimetallcyanid, wobei eine Verbindung der Formel (I), worin X die Bedeutung -NH- hat, und Y und Z beide Sauerstoff darstellen, erhalten wird; oder

f) Umsetzen einer Verbindung der Formel (F) aufeinanderfolgend mit Cyanwasserstoff, Thionylchlorid und Thioharnstoff, wobei eine Verbindung der Formel (I), worin X die Bedeutung S hat, und Y und Z beide Sauerstoff darstellen, erhalten wird; oder

g) Umsetzen einer Verbindung der Formel (F) mit Trimethylsilylcyanid, gefolgt von HCl/Alkanol und Phosgen oder Thiophosgen, wobei eine Verbindung der Formel (I), worin X die Bedeutung O oder S hat, und Y und Z beide Sauerstoff darstellen, erhalten wird.

**2.** Verfahren nach Anspruch 1, bei welchem die hergestellte Verbindung die Formel (II)

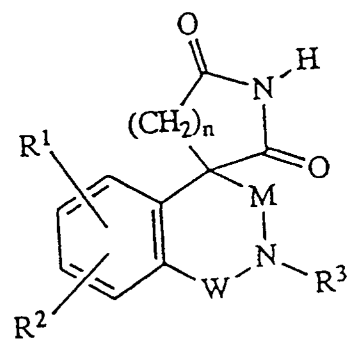

(II),

worin $R^1$ und $R^2$ unabhängig Wasserstoff, Alkyl mit 1 bis 6 Kohlenstoffatomen, Halogen, nied.Alkoxy mit 1 bis 6 Kohlenstoffatomen, Trifluormethyl, nied.Alkylthio, wobei nied.Alkyl 1 bis 6 Kohlenstoffatome enthält, bedeuten; $R^3$ Wasserstoff, nied.Alkyl mit 1 bis 6 Kohlenstoffatomen darstellt; M und W Carbonyl oder Thiocarbonyl sind; n 1 bis 3 ist; aufweist, oder ein pharmazeutisch annehmbares Salz hievon.

**3.** Verfahren nach Anspruch 1, bei welchem die hergestellte Verbindung die Formel

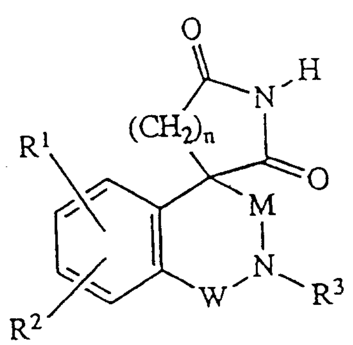

(II),

worin $R^1$ und $R^2$ unabhängig Wasserstoff, Alkyl mit 1 bis 6 Kohlenstoffatomen, Halogen, nied.Alkoxy mit

1 bis 6 Kohlenstoffatomen, Trifluormethyl, nied.Alkylthio, wobei nied.Alkyl 1 bis 6 Kohlenstoffatome enthält, bedeuten; $R^3$ Aryl-(nied.alkyl) oder DIhalogen-substituiertes Aryl-(nied.alkyl), wobei Aryl 6 bis 10 Kohlenstoffatome enthält, und nied.Alkyl 1 bis 6 Kohlenstoffatome enthält, darstellt; M und W Carbonyl oder Thiocarbonyl sind; n 1 bis 3 ist; aufweist, oder ein pharmazeutisch annehmbares Salz hievon.

**4.** Verfahren nach Anspruch 1, bei welchem die hergestellte Verbindung die Formel (III)

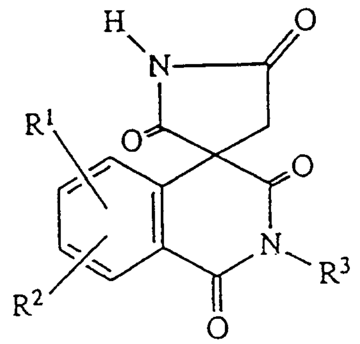

(III),

worin $R^1$ und $R^2$ Wasserstoff oder Halogen bedeuten; $R^3$ nied.Alkyl mit 1 bis 6 Kohlenstoffatomen, Benzyl oder Dihalogen-substituiertes Benzyl darstellt; aufweist, oder ein pharmazeutisch annehmbares Salz hievon.

**5.** Verfahren nach Anspruch 1, bei welchem die hergestellte Verbindung der Formel (I) eine der folgenden ist:
2-[(4-Brom-2-fluorphenyl)-methyl]-spiro-[isochinolin-4(1H),3'-pyrrolidin]-1,2',3,5'(2H)-tetron,
[2-[(4-Brom-2-fluorphenyl)-methyl]-7-chlor]-spiro-[isochinolin-4(1H),3'-pyrrolidin]-1,2',3,5'(2H)-tetron,
[2-[(4-Brom-2-fluorphenyl)-methyl]-6-chlor]-spiro-[isochinolin-4(1H),3'-pyrrolidin]-1,2',3,5'(2H)-tetron,
2-[(3,4-Dichlorphenyl)-methyl]-spiro-[isochinolin-4(1H),3'-pyrrolidin]-1,2',3,5'(2H)-tetron,
[2-[(4-Brom-2-fluorphenyl)-methyl]-5-fluor]-spiro-[isochinolin-4(1H),3'-pyrrolidin]-1,2',3,5'(2H)-tetron,
[2-[(4-Brom-2-fluorphenyl)-methyl]-6-fluor]-spiro-[isochinolin-4(1H),3'-pyrrolidin]-1,2',3,5'(2H)-tetron,
[2-[(4-Brom-2-fluorphenyl)-methyl]-7-fluor]-spiro-[isochinolin-4(1H),3'-pyrrolidin]-1,2',3,5'(2H)-tetron,
[2-[(4-Brom-2-fluorphenyl)-methyl]-8-fluor]-spiro-[isochinolin-4(1H),3'-pyrrolidin]-1,2',3,5'(2H)-tetron,
[6-Brom-2-[(4-brom-2-fluorphenyl)-methyl]-7-methoxy]-spiro-[isochinolin-4(1H),3'-pyrrolidin]-1,2',3,5'-(2H)-tetron,
[2-[(4-Brom-2-fluorphenyl)-methyl]-7-methoxy]-spiro-[isochinolin-4(1H),3'-pyrrolidin]-1,2',3,5'(2H)-tetron,
(R)-(+)-2-[(4-Brom-2-fluorphenyl)-methyl]-spiro-[isochinolin-4(1H),3'-pyrrolidin]-1,2',3,5'(2H)-tetron,
(S)-(-)-2-[(4-Brom-2-fluorphenyl)-methyl]-spiro-[isochinolin-4(1H),3'-pyrrolidin]-1,2',3,5'(2H)-tetron,
(R)-(+)-[2-[(4-Brom-2-fluorphenyl)-methyl]-6-fluor]-spiro-[isochinolin-4(1H),3'-pyrrolidin]-1,2',3,5'(2H)-tetron,
(S)-(-)-[2-[(4-Brom-2-fluorphenyl)-methyl]-6-fluor]-spiro-[isochinolin-4(1H),3'-pyrrolidin]-1,2',3,5'(2H)-tetron,
2-Methylspiro-[isochinolin-4(1H),3'-pyrrolidin]-1,2',3,5'(2H)-tetron,
6-Chlor-2-methylspiro-[isochinolin-4(1H),3'-pyrrolidin]-1,2',3,5'(2H)-tetron,
6-Brom-2-methylspiro-[isochinolin-4(1H),3'-pyrrolidin]-1,2',3,5'(2H)-tetron,
7-Chlor-2-methylspiro-[isochinolin-4(1H),3'-pyrrolidin]-1,2',3,5'(2H)-tetron,
2-Ethylspiro-[isochinolin-4(1H),3'-pyrrolidin]-1,2',3,5'(2H)-tetron,
2-Propylspiro-[isochinolin-4(1H),3'-pyrrolidin]-1,2',3,5'(2H)-tetron,
2-Butylspiro-[isochinolin-4(1H),3'-pyrrolidin]-1,2',3,5'(2H)-tetron; oder ein pharmazeutisch annehmbares Salz hievon ist.

**6.** Verfahren zur Herstellung einer Verbindung der Formel (XI)

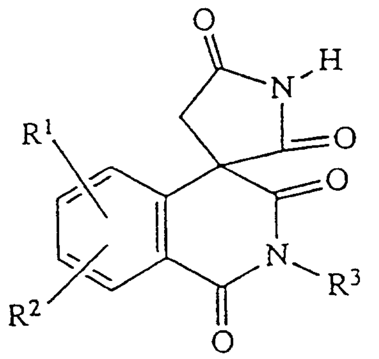

(XI),

worin $R^1$ und $R^2$ Wasserstoff oder Halogen bedeuten; $R^3$ Alkyl mit 1 bis 6 Kohlenstoffatomen, Aryl-(nied.alkyl) oder Dihalogen-substituiertes Aryl-(nied.alkyl), wobei Aryl 6 bis 10 Kohlenstoffatome enthält, und nied.Alkyl 1 bis 6 Kohlenstoffatome enthält, darstellt, welches Verfahren umfaßt:

a) Umsetzen der Verbindung der Formel (VI)

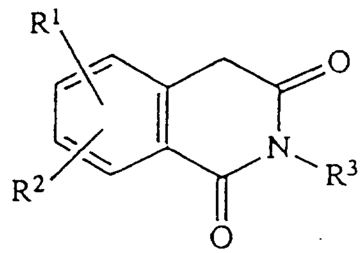

(VI),

worin $R^1$, $R^2$ und $R^3$ wie oben definiert sind, mit einer Base in einem herkömmlichen Lösungsmittel und anschließend Zusetzen eines reaktiven Carbomethoxylierungsmittels, wobei die Verbindung der Formel (VII)

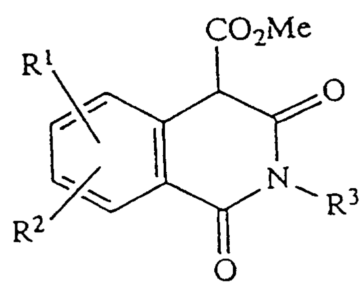

(VII),

worin $R^1$, $R^2$ und $R^3$ wie oben definiert sind, erhalten wird;

b) Umsetzen der Verbindung der Formel (VII) mit einer anorganischen Base in einem herkömmlichen Lösungsmittel und anschließend Zusetzen von tert.Butylbromacetat, wobei die Verbindung der Formel (VIII)

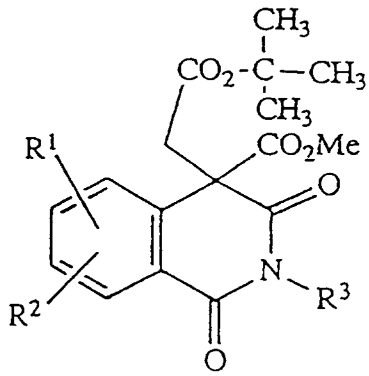

(VIII),

worin $R^1$, $R^2$ und $R^3$ wie oben definiert sind, erhalten wird;
c) Hydrolysieren der Verbindung der Formel (VIII) mit einer organischen Säure in einem herkömmlichen Lösungsmittel, wobei die Verbindung der Formel (IX)

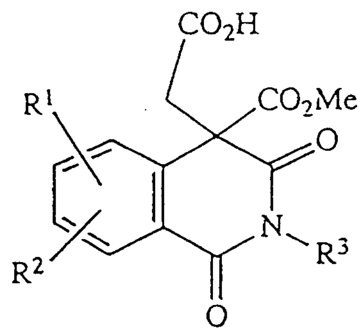

(IX),

worin $R^1$, $R^2$ und $R^3$ wie oben definiert sind, erhalten wird;
d) Umsetzen der Verbindung der Formel (IX) mit einem Kopplungsmittel in einem herkömmlichen Lösungsmittel und anschließend Zusetzen von Tetrahydrofuranammonium-Lösung, wobei die Verbindung der Formel (X)

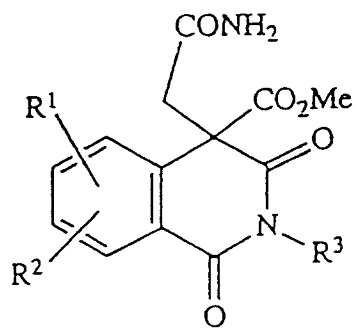

(X),

worin $R^1$, $R^2$ und $R^3$ wie oben definiert sind, erhalten wird;
e) Umsetzen der Verbindung der Formel (X) mit einer Base in einem herkömmlichen Lösungsmittel, wobei die Verbindung der Formel (XI) erhalten wird.

7. Verfahren nach Anspruch 6 zur Herstellung von Verbindungen der Formel (XI), worin $R^1$ und $R^2$ wie in Anspruch 6 definiert sind, und $R^3$ Alkyl mit 1 bis 6 Kohlenstoffatomen ist, bei welchem die Verbindung der Formel (X), worin $R^1$ und $R^2$ wie oben definiert sind, $R^3$ ein Alkyl darstellt, wie oben definiert, mit einer Base, wie Lithiumbis-(trimethylsilyl)-amid, in einem herkömmlichen Lösungsmittel umgesetzt wird, wobei die Verbindung der Formel (XI), worin $R^1$ und $R^2$ wie oben definiert sind, und $R^3$ ein Alkyl darstellt, wie oben definiert, erhalten wird.

**8.** Verfahren zur Herstellung einer Verbindung der Formel (VII)

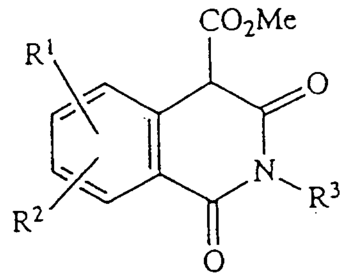

(VII),

worin $R^1$ und $R^2$ Wasserstoff oder Halogen bedeuten; $R^3$ Alkyl mit 1 bis 6 Kohlenstoffatomen, Aryl-(nied.alkyl) oder Dihalogen-substituiertes Aryl-(nied.alkyl), wobei Aryl 6 bis 10 Kohlenstoffatome enthält, und nied.Alkyl 1 bis 6 Kohlenstoffatome enthält, darstellt; welches Verfahren das Umsetzen der Verbindung der Formel (XII)

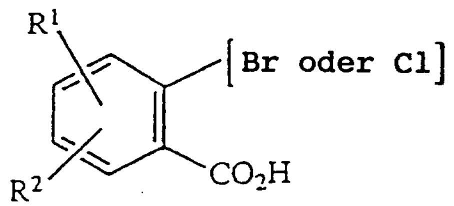

(XII),

worin $R^1$ und $R^2$ wie oben definiert sind, mit Dimethylmalonat und NaH in Anwesenheit einer katalytischen Menge an CuBr, wobei die Verbindung der Formel (XIII)

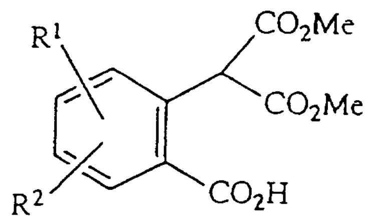

(XIII),

worin $R^1$ und $R^2$ wie oben definiert sind, erhalten wird, und Umsetzen der Verbindung der Formel (XIII) mit Thionylchlorid und anschließend Zusetzen eines inerten Lösungsmittels, von Triethylamin und eines Amins der Formel $R^3NH_2$, wobei die Verbindung der Formel (VII) erhalten wird, umfaßt.

**9.** Verfahren zur Herstellung einer pharmazeutischen Zusammensetzung, welches das Vereinigen einer Verbindung der Formel (I) oder eines Analogons oder eines pharmazeutisch annehmbaren Salzes hievon, wie in Anspruch 1 definiert, mit einem pharmazeutisch annehmbaren Träger umfaßt.

**10.** Verfahren zur Herstellung einer Verbindung der Formel

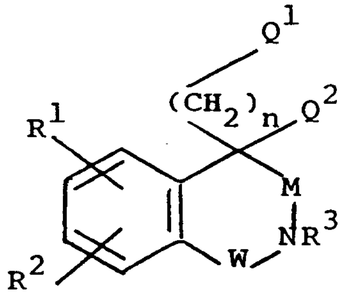

(XXIV),

worin eines von $Q^1$ und $Q^2$ die Bedeutung $-CZNH_2$ oder CN hat; das andere von $Q^1$ und $Q^2$ $-COOR^5$ darstellt; n 1 bis 3 ist, und M, W, Z, $R^1$, $R^2$, $R^3$ und $-COOR^5$ wie in Anspruch 1 definiert sind, welches Verfahren umfaßt:

a) Umsetzen einer Säure der Formel

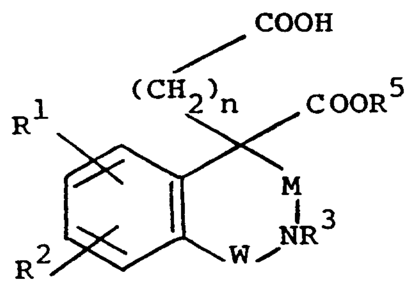

(XXV),

worin $R^1$, $R^2$, $R^3$, M, W und $-COOR^5$ wie oben definiert sind, mit einem Kopplungsmittel und Ammoniak, wobei eine entsprechende Verbindung der Formel (I), worin $Q^1$ die Bedeutung $CONH_2$ hat, erhalten wird; oder

b) Umsetzen einer Verbindung der Formel (L)

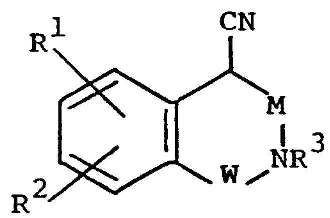

(L),

worin $R^1$, $R^2$, M, W und $R^3$ wie in Anspruch 1 definiert sind, mit einer Verbindung der Formel

$Br(CH_2)_nCOOR^5$,

worin n 1 bis 3 ist, und $COOR^5$ eine Estergruppe darstellt, wobei eine Verbindung der Formel (XXIV), worin $Q^1$ die Bedeutung $COOR^5$ hat, und $Q^2$ CN ist, erhalten wird;

c) Umsetzen einer Verbindung der Formel G

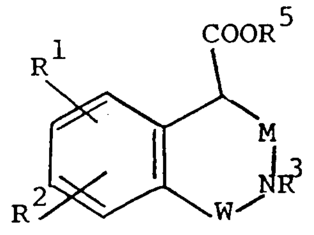

(G),

worin $R^1$, $R^2$, $R^3$, M, W und $-COOR^5$ wie in Anspruch 1 definiert sind, mit einer Verbindung der Formel

$Br(CH_2)_nCN$,

worin n wie oben definiert ist, wobei eine Verbindung der Formel (XXIV), worin $Q^1$ die Bedeutung CN hat, und $Q^2$ $COOR^5$ darstellt, erhalten wird; oder

d) Überführen von Mitril der Formel (XXIV), worin $Q^2$ die Bedeutung CN hat, in ein Amid oder Thioamid der Formel (XXIV), worin $Q^2$ $CONH_2$ oder $CSNH_2$ darstellt, auf bekannte Weise; oder

e) Überführen eines Nitrils der Formel (XXIV), worin $Q^1$ die Bedeutung CN hat, in Thioamid der Formel (XXIV), worin $Q^1$ $CSNH_2$ darstellt, unter Verwendung von $HSP(=S)(OEt)_2/HCl$.

**11.** Verbindung der Formel

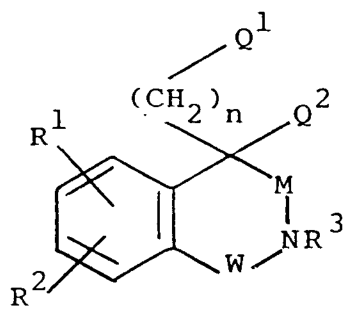

(XXIV),

worin eines von $Q^1$ und $Q^2$ die Bedeutung $-CZNH_2$ oder CN hat; das andere von $Q^1$ und $Q^2$ $-COOR^5$ darstellt; Z, $R^1$, $R^2$, $R^3$, M und W wie in Anspruch 1 definiert sind, $-COOR^5$ eine Estergruppe bedeutet, und n 1 bis 3 ist.

**12.** Verbindung nach Anspruch 11, worin $Q^1$ die Bedeutung $-CONH_2$ hat, $R^1$ und $R^2$ Wasserstoff oder Halogen darstellen; n 1 ist; M und W beide Carbonyl sind, und $R^3$ nied.Alkyl mit 1 bis 6 Kohlenstoffatomen, Benzyl oder Dihalogen-substituiertes Benzyl bedeutet.

**13.** Verbindung nach Anspruch 11, welche eine der folgenden ist:
4-(2-Amino-2-oxoethyl)-2-[(3,4-dichlorphenyl)-methyl]-1,2,3,4-tetrahydro-1,3-dioxo-4-isochinolincarbonsäuremethylester;
4-(2-Amino-2-oxoethyl)-2-[(4-brom-2-fluorphenyl)-methyl]-1,2,3,4-tetrahydro-1,3-dioxo-4-isochinolincarbonsäuremethylester;
4-(2-Amino-2-oxoethyl)-2-[(4-brom-2-fluorphenyl)-methyl]-7-chlor-1,2,3,4-tetrahydro-1,3-dioxo-4-isochinolincarbonsäuremethylester;
4-(2-Amino-2-oxoethyl)-2-[(4-brom-2-fluorphenyl)-methyl]-6-chlor-1,2,3,4-tetrahydro-1,3-dioxo-4-isochinolincarbonsäuremethylester;
4-(2-Amino-2-oxoethyl)-2-[(4-brom-2-fluorphenyl)-methyl]-6-fluor-1,2,3,4-tetrahydro-1,3-dioxo-4-isochinolincarbonsäuremethylester;
4-(2-Amino-2-oxoethyl)-1,2,3,4-tetrahydro-2-methyl-1,3-dioxo-4-isochinolincarbonsäuremethylester;
4-(2-Amino-2-oxoethyl)-7-chlor-1,2,3,4-tetrahydro-2-methyl-1,3-dioxo-4-isochinolincarbonsäuremethylester;
4-(2-Amino-2-oxoethyl)-6-chlor-1,2,3,4-tetrahydro-2-methyl-1,3-dioxo-4-isochinolincarbonsäuremethylester;
4-(2-Amino-2-oxoethyl)-2-ethyl-1,2,3,4-tetrahydro-1,3-dioxo-4-isochinolincarbonsäuremethylester;
4-(2-Amino-2-oxoethyl)-1,2,3,4-tetrahydro-2-propyl-1,3-dioxo-4-isochinolincarbonsäuremethylester;
4-(2-Amino-2-oxoethyl)-2-butyl-1,2,3,4-tetrahydro-1,3-dioxo-4-isochinolincarbonsäuremethylester;
4-(2-Amino-2-oxoethyl)-6-brom-1,2,3,4-tetrahydro-2-methyl-1,3-dioxo-4-isochinolincarbonsäuremethylester;
4-(2-Amino-2-oxoethyl)-2-[(4-brom-2-fluorphenyl)-methyl]-1,2,3,4-tetrahydro-1,3-dioxo-4-isochinolincarbonsäurementhylester; oder
4-(2-Amino-2-oxoethyl)-2-[(4-brom-2-fluorphenyl)-methyl]-6-fluor-1,2,3,4-tetrahydro-1,3-dioxo-4-isochinolincarbonsäurementhylester.

**Revendications**

**Revendications pour les Etats contractants suivants : AT, BE, CH, DE, FR, GB, IT, LI, LU, NL, SE**

**1.** Composé de formule (I) :

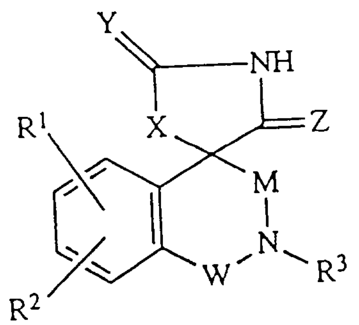

(I)

et les analogues de celui-ci où le cycle benzène condensé est remplacé par un cycle thiophène, pyridine ou furanne condensé, dans laquelle $R^1$ et $R^2$ sont indépendamment un atome d'hydrogène, un radical alcoyle contenant 1 à 6 atomes de carbone, un atome d'halogène, un radical alcoxy inférieur contenant 1 à 6 atomes de carbone, un radical trifluorométhyle, un radical alcoylthio inférieur, où la partie alcoyle inférieur contient 1 à 6 atomes de carbone, un radical dialcoylamino où la partie alcoyle contient 1 à 6 atomes de carbone, un radical nitro, un radical aryle ou (alcoyle inférieur)aryloxy où la partie aryle contient 6 à 10 atomes de carbone et la partie alcoyle inférieur contient 1 à 6 atomes de carbone; $R^3$ est un atome d'hydrogène, un radical alcoyle inférieur contenant 1 à 6 atomes de carbone, un radical (alcoyle inférieur)aryle ou un radical (alcoyle inférieur)aryle substitué par un halogène où la partie aryle contient 6 à 10 atomes de carbone et l'alcoyle inférieur contient 1 à 6 atomes de carbone ou (alcoyle inférieur)aryle hétérocyclique de formule de structure :

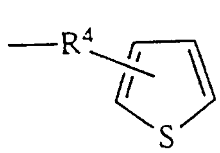

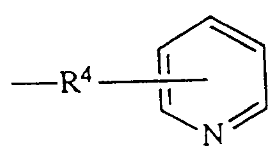

dans laquelle $R^4$ est un radical alcoylène inférieur contenant 1 à 3 atomes de carbone; X est un radical alcoylène inférieur contenant 1 à 3 atomes de carbone, un atome d'oxygène, de soufre ou un radical -NH-; Y et Z sont indépendamment un atome d'oxygène ou de soufre; M et W sont indépendamment un radical carbonyle, un radical thiocarbonyle, un radical sulfonyle, un radical sulfoxo ou un radical alcoylène inférieur contenant 1 à 2 atomes de carbone, avec la condition que (i) M et W ne peuvent pas être ensemble alcoylène inférieur et que (ii) quand M est $-CH_2-$, W est autre que sulfonyle ou un sel pharmaceutiquement acceptable de celui-ci.

**2.** Composé suivant la revendication 1, de formule (II) :

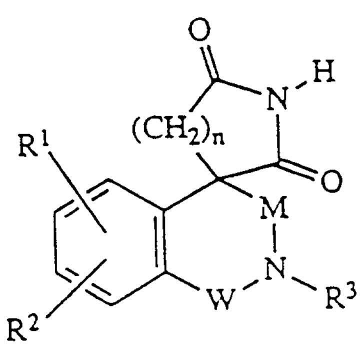

(II)

dans laquelle $R^1$ et $R^2$ sont indépendamment un atome d'hydrogène, un radical alcoyle contenant 1 à 6 atomes de carbone, un atome d'halogène, un radical alcoxy inférieur contenant 1 à 6 atomes de carbone, un radical trifluorométhyle, un radical acoylthio inférieur où la partie alcoyle inférieur contient 1 à 6 atomes de carbone; $R^3$ est un atome d'hydrogène, un radical alcoyle inférieur contenant 1 à 6 atomes de carbone; M et W sont un radical carbonyle ou thiocarbonyle; n est un nombre de 1 à 3 ou un sel pharmaceutiquement acceptable de celui-ci.

**3.** Composé suivant la revendication 1, de formule (II) :

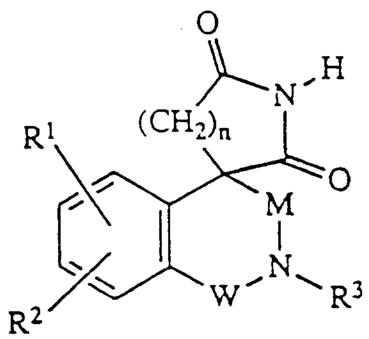

(II)

dans laquelle $R^1$ et $R^2$ sont indépendamment un atome d'hydrogène, un radical alcoyle contenant 1 à 6 atomes de carbone, un atome d'halogène, un radical alcoxy inférieur contenant 1 à 6 atomes de carbone, un radical trifluorométhyle, un radical alcoylthio inférieur où la partie alcoyle inférieur contient 1 à 6 atomes de carbone; $R^3$ est un radical (alcoyle inférieur)aryle ou (alcoyle inférieur)aryle dihalogéno-substitué où la partie aryle contient 6 à 10 atomes de carbone et la partie alcoyle inférieur contient 1 à 6 atomes de carbone; M et W sont un radical carbonyle ou thiocarbonyle; n est un nombre de 1 à 3, ou un sel pharmaceutiquement acceptable de celui-ci.

**4.** Composé suivant la revendication 1, de formule (III) :

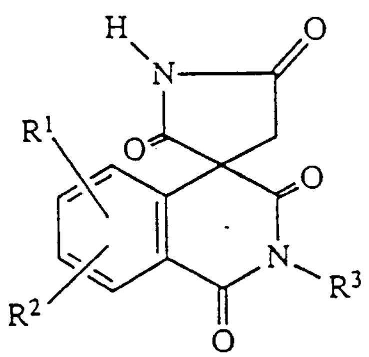

(III)

dans laquelle $R^1$ et $R^2$ sont un atome d'hydrogène ou d'halogène; $R^3$ est un radical alcoyle inférieur contenant 1 à 6 atomes de carbone, un radical benzyle ou un radical benzyle dihalogéno-substitué, ou un sel pharmaceutiquement acceptable de celui-ci.

**5.** Composé suivant la revendication 1, qui est l'un des suivants :

la 2-[(4-bromo-2-fluorophényl)méthyl]spiro[isoquinoléine-4(1H),3'-pyrrolidine]-1,2',3,5'(2H)-tétrone;

la [2-[(4-bromo-2-fluorophényl)méthyl]-7-chloro]spiro[isoquinoléine-4(1H),3'-pyrrolidine]-1,2',3,5'-(2H)-tétrone;

la [2-[(4-bromo-2-fluorophényl)méthyl]-6-chloro]spiro[isoquinoléine-4(1H),3'-pyrrolidine]-1,2',3,5'-(2H)-tétrone;

la 2-[(3,4 dichlorophényl)méthyl]spiro[isoquinoléine-4(1H),3'-pyrrolidine]-1,2',3-5'(2H)-tétrone;

la [2-[(4-bromo-2-fluorophényl)méthyl]-5-fluoro]spiro[isoquinoléine-4(1H),3'-pyrrolidine]-1,2',3,5'(2H)-tétrone;

la [2-[(4-bromo-2-fluorophényl)méthyl]-6-fluoro]spiro[isoquinoléine-4(1H),3'-pyrrolidine]-1,2',3,5'(2H)-tétrone;

la [2-[(4-bromo-2-fluorophényl)méthyl]-7-fluoro]spiro[isoquinoléine-4(1H),3'-pyrrolidine]-1,2',3,5'(2H)-tétrone;

la [2-[(4-bromo-2-fluorophényl)méthyl]-8-fluoro]spiro[isoquinoléine-4(1H),3'-pyrrolidine]-1,2',3,5'(2H)-tétrone;

la [6-bromo-2-[(4-bromo-2-fluorophényl)méthyl]-7-méthoxy]spiro[isoquinoléine-4(1H),3'-pyrrolidine]-1,2',3,5'(2H)-tétrone;

la [2-[(4-bromo-2-fluorophényl)méthyl]-7-méthoxy]spiro[isoquinoléine-4(1H),3'-pyrrolidine]-1,2',3,5'-(2H)-tétrone;

la (R)-(+)-2-[(4-bromo-2-fluorophényl)méthyl]-spiro[isoquinoléine-4(1H),3'-pyrrolidine]-1,2',3,5'(2H)-tétrone;

la (S)-(-)-2-[(4-bromo-2-fluorophényl)méthyl]spiro[isoquinoléine-4(1H),3'-pyrrolidine]-1,2',3,5'(2H)-tétrone;

la (R)-(+)-[2-[(4-bromo-2-fluorophényl)méthyl]-6-fluoro]spiro[isoquinoléine-4(1H),3'-pyrrolidine]-1,2',3,5'(2H)-tétrone;

la (S)-(-)-[2-[(4-bromo-2-fluorophényl)méthyl]-6-fluoro]spiro[isoquinoléine-4(1H),3'-pyrrolidine]-1,2',3,5'(2H)-tétrone;

la 2-méthylspiro[isoquinoléine-4(1H),3'-pyrrolidine]-1,2',3,5'(2H)-tétrone;

la 6-chloro-2-méthylspiro[isoquinoléine-4(1H),3'-pyrrolidine]-1,2',3,5'(2H)-tétrone;

la 6-bromo-2-méthylspiro[isoquinoléine-4(1H),3'-pyrrolidine]-1,2',3,5'(2H)-tétrone;

la 7-chloro-2-méthylspiro[isoquinoléine-4(1H),3'-pyrrolidine]-1,2',3,5'(2H)-tétrone;

la 2-éthylspiro[isoquinoléine-4(1H),3'-pyrrolidine]-1,2',3,5'(2H)-tétrone;

la 2-propylspiro[isoquinoléine-4(1H),3'-pyrrolidine]-1,2',3,5'(2H)-tétrone;

la 2-butylspiro[isoquinoiéine-4(1H),3'-pyrrolidine]-1,2',3,5'(2H)-tétrone,

ou un sel pharmaceutiquement acceptable de celui-ci.

**6.** Composé suivant l'une quelconque des revendications 1 à 5, sous la forme d'un sel pharmaceutiquement acceptable d'ammonium, d'un métal alcalin ou alcalino-terreux.

**7.** Procédé de préparation d'un composé de formule (I) suivant la revendication 1, qui comprend une des étapes suivantes :

a) la cyclisation d'un amide de formule (A) :

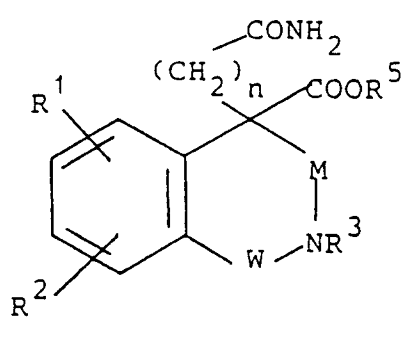

(A)

dans laquelle $R^1$, $R^2$, $R^3$, n, M et W sont tels que définis ci-dessus et $COOR^5$ est un radical ester, en utilisant une base adéquate pour donner un composé de formule (I) dans laquelle Y et Z sont, tous deux, un atome d'oxygène et X est un radical $-(CH_2)_n-$ dans lequel n est un nombre de 1 à 3;

b) la réaction d'un composé de formule (B) :

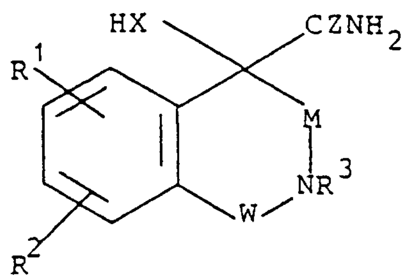

(B)

dans laquelle $R^1$, $R^2$, $R^3$, M et W sont tels que définis ci-dessus et X est -O-, -S- ou -NH- et Z est -O- ou -S-, avec $CYCl_2$ où Y est -O- ou -S- pour donner un composé de formule (I) dans laquelle X est -O-, -S- ou -NH- et Y et Z indépendamment représentent -O- ou -S-; ou

c) la réaction d'un composé de formule (C) :

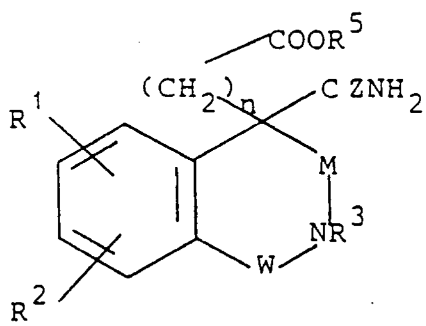

(C)

dans laquelle $R^1$, $R^2$, $R^3$, M, W, n, Z et $COOR^5$ sont tels que définis ci-dessus en utilisant une base adéquate pour donner un composé de la formule (I) où Y est un -O-, Z est -O-, ou -S- et n est un nombre de 1 à 3; ou

d) la réaction d'un composé de formule (D) :

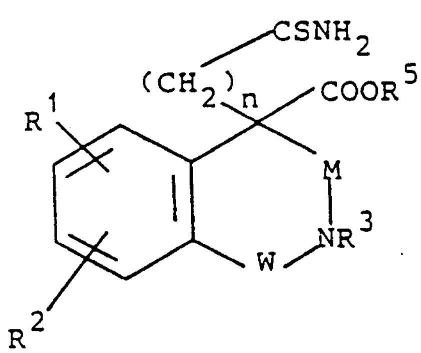

(D)

dans laquelle $R^1$, $R^2$, $R^3$, M, W, n et $COOR^5$ sont tels que définis ci-dessus avec une base forte pour donner un composé de formule (I) dans laquelle X est $-(CH_2)_n-$, où n est un nombre de 1 à 3 et Y est -S- et Z est -O-; ou

e) la réaction d'un composé de formule (F) :

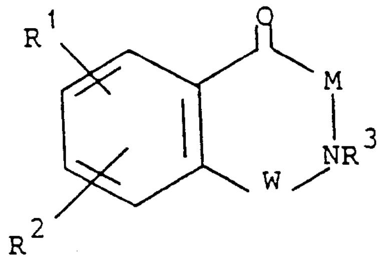

(F)

dans laquelle $R^1$, $R^2$, $R^3$, M et W sont tels que définis ci-dessus avec un carbonate d'ammonium et un cyanure de métal alcalin pour donner un composé de formule (I) dans laquelle X est -NH- et Y et Z sont tous deux oxygène; ou

f) la réaction d'un composé de formule (F) successivement avec le cyanure d'hydrogène, le chlorure de thionyle et la thiourée pour donner un composé de formule (I) dans laquelle X est -S- et Y et Z sont tous deux oxygène; ou

g) la réaction d'un composé de formule (F) avec le cyanure de triméthylsilyle, puis le HCl/alcanol et le phosgène ou le thiophosgène pour donner un composé de formule (I) dans laquelle X est -O- ou -S- et Y et Z sont tous deux oxygène.

**8.** Procédé de préparation d'un composé de formule (XI) :

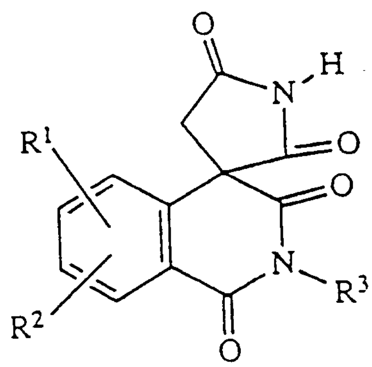

(XI)

dans laquelle $R^1$ et $R^2$ sont des atomes d'hydrogène ou d'halogène; $R^3$ est un radical alcoyle contenant 1 à 6 atomes de carbone, un radical (alcoyle inférieur)aryle ou un radical (alcoyle inférieur)aryle dihalogéno-substitué où le radical aryle contient 6 à 10 atomes de carbone et le radical alcoyle inférieur contient 1 à 6 atomes de carbone, qui comprend :

a) la réaction d'un composé de formule (VI) :

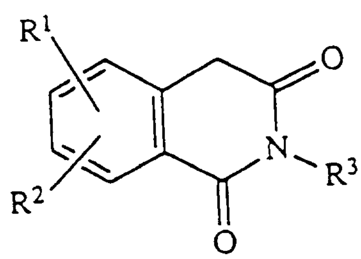

(VI)

dans laquelle $R^1$, $R^2$ et $R^3$ sont tels que définis ci-dessus, avec une base dans un solvant classique et l'addition subséquente d'un agent réactif carbométhoxylant pour produire le composé de formule (VII) :

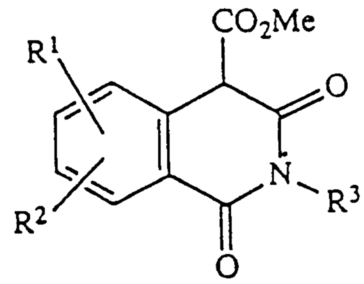

(VII)

dans laquelle $R^1$, $R^2$ et $R^3$ sont tels que définis ci-dessus;

b) la réaction dudit composé de formule (VII) avec une base inorganique dans un solvant classique et l'addition subséquente de bromacétate de t-butyle pour produire le composé de formule (VIII) :

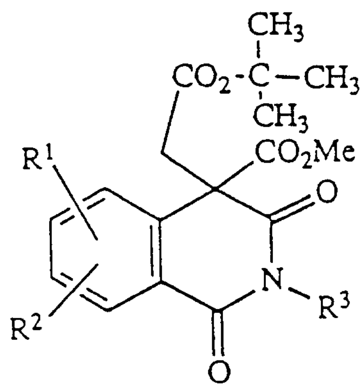

(VIII)

dans laquelle $R^1$, $R^2$ et $R^3$ sont tels que définis ci-dessus;

c) l'hydrolyse dudit composé de formule (VIII) avec un acide organique dans un solvant classique pour produire le composé de formule (IX) :

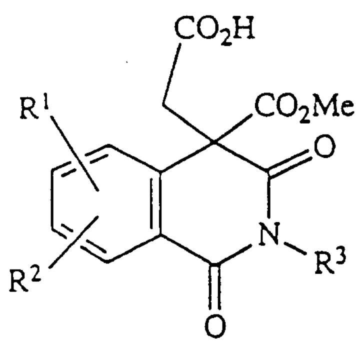

(IX)

dans laquelle $R^1$, $R^2$ et $R^3$ sont tels que définis ci-dessus;

d) la réaction dudit composé de formule (IX) avec un agent de couplage dans un solvant classique et l'addition subséquente d'une solution d'ammonium dans du tétrahydrofuranne pour produire le composé de formule (X) :

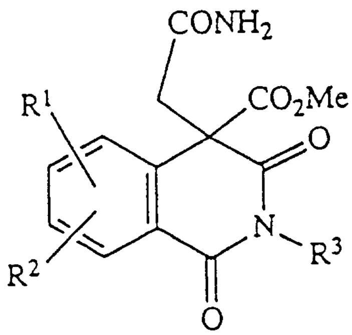

(X)

dans laquelle $R^1$, $R^2$ et $R^3$ sont tels que définis ci-dessus;
e) la réaction dudit composé de formule (X) avec une base dans un solvant classique pour produire le composé de formule (XI).

**9.** Procédé suivant la revendication 8, pour la production de composés de formule (XI), dans laquelle $R^1$ et $R^2$ sont tels que définis dans la revendication 8 et $R^3$ est un radical alcoyle contenant 1 à 6 atomes de carbone, dans laquelle le composé de formule (X), où $R^1$ et $R^2$ sont tels que définis ci-dessus, $R^3$ est un radical alcoyle tel que défini ci-dessus, est mis à réagir avec une base telle que le bis-(triméthylsilyl)amidure de lithium, dans un solvant classique pour produire le composé de formule (XI), où $R^1$ et $R^2$ sont tels que définis ci-dessus, et $R^3$ est un radical alcoyle tel que défini ci-dessus.

**10.** Procédé de préparation d'un composé de formule (VII) :

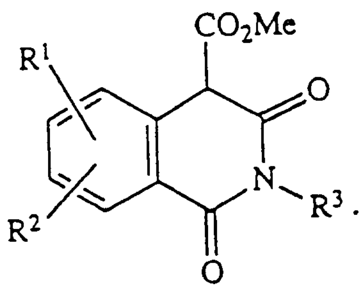

(VII)

dans laquelle $R^1$ et $R^2$ sont des atomes d'hydrogène ou d'halogène; $R^3$ est un radical alcoyle contenant 1 à 6 atomes de carbone, un radical (alcoyle inférieur)aryle ou (alcoyle inférieur)aryle dihalogéno-substitué où le radical aryle contient 6 à 10 atomes de carbone et le radical alcoyle inférieur contient 1 à 6 atomes de carbone qui comprend la réaction d'un composé de formule (XII) :

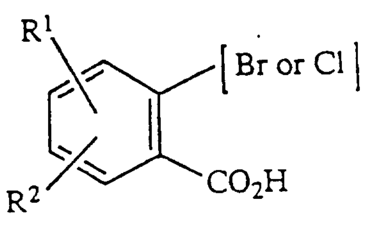

(XII)

dans laquelle $R^1$ et $R^2$ sont tels que définis ci-dessus avec le malonate de diméthyle et du NaH en présence d'une quantité catalytique de CuBr pour produire le composé de formule (XIII) :

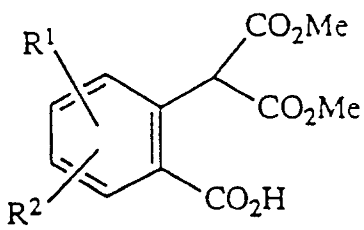

(XIII)

dans laquelle $R^1$ et $R^2$ sont tels que définis ci-dessus et la réaction dudit composé de formule (XIII) avec le chlorure de thionyle et l'addition subséquente d'un solvant inerte, de triéthylamine et d'une amine de formule $R^3NH_2$ pour produire le composé de formule (VII).

**11.** Composition pharmaceutique comprenant un composé de formule (I) ou un sel pharmaceutiquement acceptable de celui-ci tel que défini dans l'une quelconque des revendications 1 à 5 et un excipient pharmaceutiquement acceptable.

**12.** Composé suivant la revendication 1, pour l'utilisation dans la fabrication d'un médicament pour la prévention ou le traitement de la neuropathie, de la néphropathie, de la rétinopathie ou des cataractes chez des mammifères diabétiques.

**13.** Composé de formule (XXIV) :

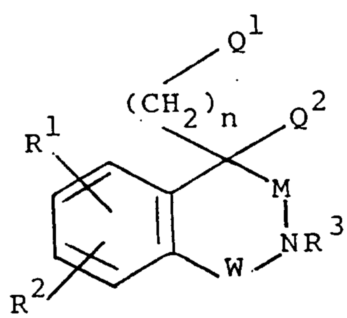

(XXIV)

dans laquelle un des $Q^1$ et $Q^2$ est $-CZNH_2$ ou -CN; l'autre des $Q^1$ et $Q^2$ est $-COOR^5$; Z, $R^1$, $R^2$, $R^3$, M et w sont tels que définis dans la revendication 1, $-COOR^5$ est un radical ester et n est un nombre de 1 à 3.

**14.** Composé suivant la revendication 13, où $Q^1$ est $-CONH_2$, $R^1$ et $R^2$ sont des atomes d'hydrogène ou d'halogène; n est 1; M et W sont tous deux des radicaux carbonyle et $R^3$ est un radical alcoyle inférieur contenant 1 à 6 atomes de carbone, un radical benzyle ou un radical benzyle dihalogéno-substitué.

**15.** Composé suivant la revendication 13, parmi lesquels :

l'ester méthylique de l'acide 4-(2-amino-2-oxoéthyl)-2-[(3,4-dichlorophényl)méthyl]-1,2,3,4-tétrahydro-1,3-dioxo-4-isoquinoléinecarboxylique;

l'ester méthylique de l'acide 4-(2-amino-2-oxoéthyl)-2-[(4-bromo-2-fluorophényl)méthyl]-1,2,3,4-tétrahydro-1,3-dioxo-4-isoquinoléinecarboxylique;

l'ester méthylique de l'acide 4-(2-amino-2-oxoéthyl)-2-[(4-bromo-2-fluorophényl)méthyl)-7-chloro-1,2,3,4-tétrahydro-1,3-dioxo-4-isoquinoléinecarboxylique;

l'ester méthylique de l'acide 4-(2-amino-2-oxoéthyl)-2-[(4-bromo-2-fluorophényl)méthyl]-6-chloro-1,2,3,4-tétrahydro-1,3-dioxo-4-isoquinoléinecarboxylique;

l'ester méthylique de l'acide 4-(2-amino-2-oxoéthyl)-2-[(4-bromo-2-fluorophényl)méthyl]-6-fluoro-1,2,3,4-tétrahydro-1,3-dioxo-4-isoquinoléinecarboxylique;

l'ester méthylique de l'acide 4-(2-amino-2-oxoéthyl)-1,2,3,4-tétrahydro-2-méthyl-1,3-dioxo-4-isoquinoléinecarboxylique;

l'ester méthylique de l'acide 4-(2-amino-2-oxoéthyl)-7-chloro-1,2,3,4-tétrahydro-2-méthyl-1,3-dioxo-4-isoquinoléinecarboxylique;

l'ester méthylique de l'acide 4-(2-amino-2-oxoéthyl)-6-chloro-1,2,3,4-tétrahydro-2-méthyl-1,3-dioxo-4-isoquinoléinecarboxylique;

l'ester méthylique de l'acide 4-(2-amino-2-oxoéthyl)-2-éthyl-1,2,3,4-tétrahydro-1,3-dioxo-4-isoquinoléinecarboxylique;

l'ester méthylique de l'acide 4-(2-amino-2-oxoéthyl)-1,2,3,4-tétrahydro-2-propyl-1,3-dioxo-4-isoquinoléinecarboxylique;

l'ester méthylique de l'acide 4-(2-amino-2-oxoéthyl)-2-butyl-1,2,3,4-tétrahydro-1,3-dioxo-4-isoquinoléinecarboxylique;

l'ester méthylique de l'acide 4-(2-amino-2-oxoéthyl)-6-bromo-1,2,3,4-tétrahydro-2-méthyl-1,3-dioxo-4-isoquinoléinecarboxylique;

l'ester menthylique de l'acide 4-(2-amino-2-oxoéthyl)-2-[(4-bromo-2-fluorophényl)méthyl]-1,2,3,4-tétrahydro-1,3-dioxo-4-isoquinoléinecarboxylique, ou

l'ester menthylique de l'acide 4-(2-amino-2-oxoéthyl)-2-[(4-bromo-2-fluorophényl)méthyl]-6-fluoro-1,2,3,4-tétrahydro-1,3-dioxo-4-isoquinoléinecarboxylique;

**16.** Procédé de préparation d'un composé de formule (XXIV) :

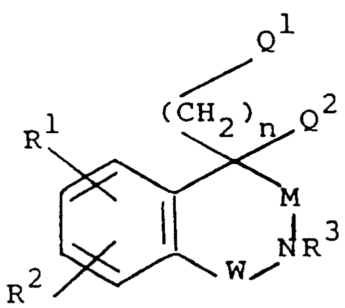

(XXIV)

dans laquelle un des $Q^1$ et $Q^2$ est $-CZNH_2$ ou -CN et l'autre des $Q^1$ et $Q^2$ est $-COOR^5$; n est un nombre de 1 à 3 et M, W, Z, $R^1$, $R^2$, $R^3$ et $-COOR^5$ sont tels que définis dans la revendication 1, qui comprend :

a) la réaction d'un acide de formule (XXV) :

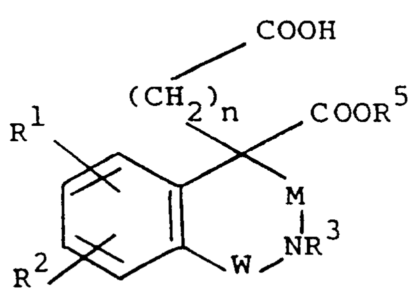

(XXV)

dans laquelle $R^1$, $R^2$, $R^3$, M, W et $-COOR^5$ sont tels que définis ci-dessus avec un agent de couplage et l'ammoniac pour donner un composé correspondant de formule (I) dans laquelle $Q^1$ est $-CONH_2$, ou

b) la réaction d'un composé de formule (L) :

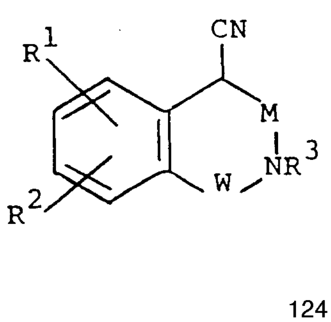

(L)

dans laquelle $R^1$, $R^2$, M, W et $R^3$ sont tels que définis dans la revendication 1 avec un composé de formule :

$Br(CH_2)_nCOOR^5$

dans laquelle n est un nombre de 1 à 3 et $COOR^5$ est un radical ester pour donner un composé de formule (XXIV) où $Q^1$ est $-COOR^5$ et $Q^2$ est -CN;
c) la réaction d'un composé de formule (G) :

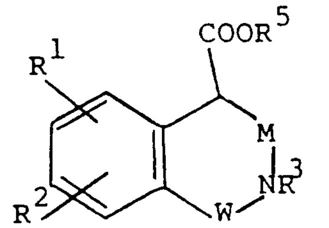

(G)

dans laquelle $R^1$, $R^2$, $R^3$, M, W et $-COOR^5$ sont tels que définis dans la revendication 1 avec un composé de formule :

$Br(CH_2)_nCN$

où n est tel que défini ci-dessus pour donner un composé de formule (XXIV) où $Q^1$ est -CN et $Q^2$ est $-COOR^5$-, ou
d) la conversion du nitrile de formule (XXIV) où $Q^2$ est -CN en un amide ou un thioamide de formule (XXIV) où $Q^2$ est $-CONH_2$ ou $-CSNH_2$ de manière connue, ou
e) la conversion d'un nitrile de formule (XXIV) où $Q^1$ est -CN en un thioamide de formule (XXIV) où $Q^1$ est $-CSNH_2$ en utilisant $HSP(=S)(OEt)_2/HCl$.

**Revendications pour l'Etat contractant suivant : ES**

1. Procédé de préparation d'un composé de formule (I) :

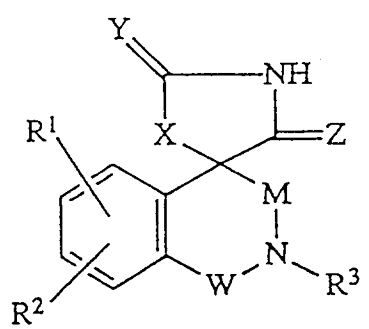

(I)

et les analogues de celui-ci où le cycle benzène condensé est remplacé par un cycle thiophène, pyridine ou furanne condensé, dans laquelle $R^1$ et $R^2$ sont indépendamment un atome d'hydrogène, un radical alcoyle contenant 1 à 6 atomes de carbone, un atome d'halogène, un radical alcoxy inférieur contenant 1 à 6 atomes de carbone, un radical trifluorométhyle, un radical alcoylthio inférieur où la partie alcoyle inférieur contient 1 à 6 atomes de carbone, un radical dialcoylamino où la partie alcoyle contient 1 à 6 atomes de carbone, un radical nitro, un radical aryle ou (alcoyle inférieur)aryloxy où la partie aryle contient 6 à 10 atomes de carbone et la partie alcoyle inférieur contient 1 à 6 atomes de carbone; $R^3$ est un atome d'hydrogène, un radical alcoyle inférieur contenant 1 à 6 atomes de carbone, un radical (alcoyle inférieur)aryle ou un radical (alcoyle inférieur)aryle substitué par un halogène où la partie aryle contient 6 à 10 atomes de carbone et l'alcoyle inférieur contient 1 à 6 atomes de carbone ou (alcoyle inférieur)aryle hétérocyclique de formule de structure :

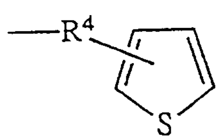

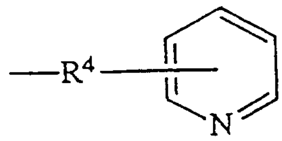

dans laquelle $R^4$ est un radical alcoylène inférieur contenant 1 à 3 atomes de carbone; X est un radical alcoylène inférieur contenant 1 à 3 atomes de carbone, un atome d'oxygène, de soufre ou un radical -NH-; Y et Z sont indépendamment un atome d'oxygène ou de soufre; M et W sont indépendamment un radical carbonyle, un radical thiocarbonyle, un radical sulfonyle, un radical sulfoxo ou un radical alcoylène inférieur contenant 1 à 2 atomes de carbone, avec la condition (i) que M et W ne peuvent pas être ensemble alcoylène inférieur et (ii) que quand M est $-CH_2-$, W est autre que sulfonyle ou un sel pharmaceutiquement acceptable de celui-ci; qui comprend une des étapes suivantes :

a) la cyclisation d'un amide de formule (A) :

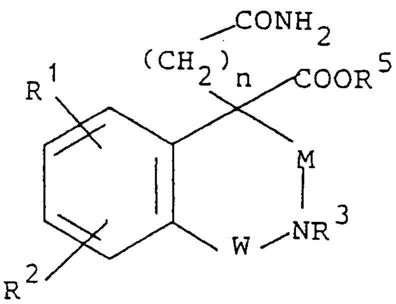

(A)

dans laquelle $R^1$, $R^2$, $R^3$, n, M et W sont tels que définis ci-dessus et $-COOR^5$ est un radical ester, en utilisant une base adéquate pour donner un composé de formule (I) dans laquelle Y et Z sont tous deux un atome d'oxygène et X est un radical $-(CH_2)_n-$ dans lequel n est un nombre de 1 à 3;

b) la réaction d'un composé de formule (B) :

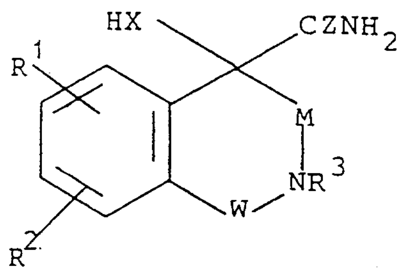

(B)

dans laquelle $R^1$, $R^2$, $R^3$, M et W sont tels que définis ci-dessus et X est -O-, -S- ou -NH- et Z est -O- ou -S-, avec $CYCl_2$ où Y est -O- ou -S- pour donner un composé de formule (I) dans laquelle X est -O-, -S- ou -NH- et Y et Z indépendamment représentent -O- ou -S-;

ou c) la réaction d'un composé de formule (C) :

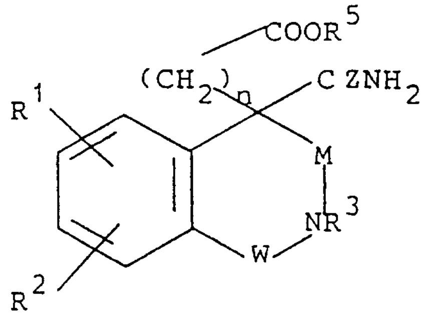

(C)

dans laquelle $R^1$, $R^2$, $R^3$, M, W, n, Z et $-COOR^5$ sont tels que définis ci-dessus en utilisant une base adéquate pour donner un composé de formule (I) où Y est -O-, Z est -O- ou -S- et n est un nombre de 1 à 3;
ou d) la réaction d'un composé de formule (D) :

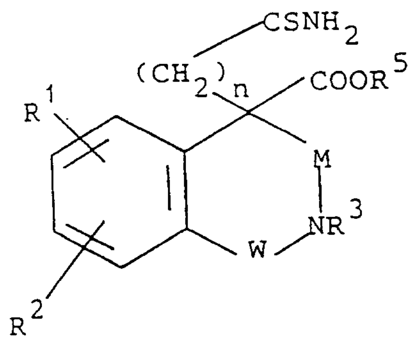

(D)

dans laquelle $R^1$, $R^2$, $R^3$, M, W, n et $-COOR^5$ sont tels que définis ci-dessus avec une base forte pour donner un composé de formule (I) dans laquelle X est un radical $-(CH_2)_n-$ où n est un nombre de 1 à 3 et Y est -S- et Z est -O-;
ou e) la réaction d'un composé de formule (F) :

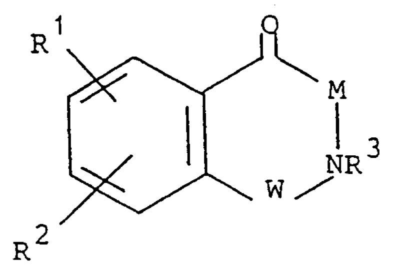

(F)

dans laquelle $R^1$, $R^2$, $R^3$, M et W sont tels que définis ci-dessus avec un carbonate d'ammonium et un cyanure de métal alcalin pour donner un composé de formule (I) dans laquelle X est un radical -NH- et Y et Z sont tous deux oxygène;
ou f) la réaction d'un composé de formule (F) successivement avec le cyanure d'hydrogène, le chlorure de thionyle et la thiourée pour donner un composé de formule (I) dans laquelle X est -S- et Y et Z sont tous deux oxygène;
ou g) la réaction d'un composé de formule (F) avec le cyanure de triméthylsilyle, puis le HCl/alcanol et le phosgène ou le thiophosgène pour donner un composé de formule (I) dans laquelle X est -O- ou -S- et Y et Z sont tous deux oxygène.

**2.** Procédé suivant la revendication 1 dans lequel le composé préparé a la formule (II) :

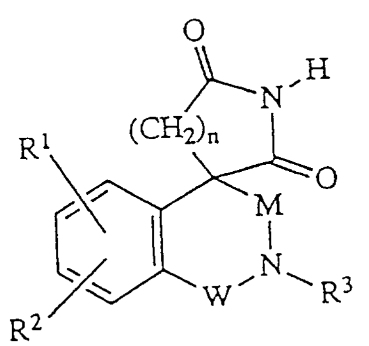

(II)

dans laquelle $R^1$ et $R^2$ sont indépendamment un atome d'hydrogène, un radical alcoyle contenant 1 à 6

atomes de carbone, un atome d'halogène, un radical alcoxy inférieur contenant 1 à 6 atomes de carbone, un radical trifluorométhyle, un radical alcoylthio inférieur où la partie alcoyle inférieur contient 1 à 6 atomes de carbone; $R^3$ est un atome d'hydrogène, un radical alcoyle inférieur contenant 1 à 6 atomes de carbone; M et W sont un radical carbonyle ou thiocarbonyle; n est un nombre de 1 à 3 ou un sel pharmaceutiquement acceptable de celui-ci.

**3.** Procédé suivant la revendication 1, dans lequel le composé préparé a la formule (II) :

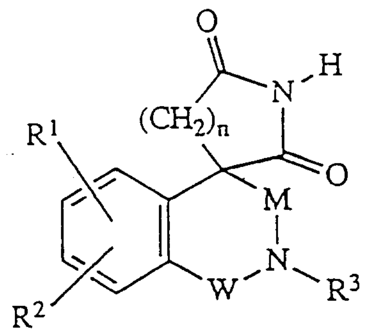

(II)

dans laquelle $R^1$ et $R^2$ sont indépendamment un atome d'hydrogène, un radical alcoyle contenant 1 à 6 atomes de carbone, un atome d'halogène, un radical alcoxy inférieur contenant 1 à 6 atomes de carbone, un radical trifluorométhyle, un radical alcoylthio inférieur où la partie alcoyle inférieur contient 1 à 6 atomes de carbone; $R^3$ est un radical (alcoyle inférieur)aryle ou (alcoyle inférieur)aryle dihalogéno-substitué où la partie aryle contient 6 à 10 atomes de carbone et la partie alcoyle inférieur contient 1 à 6 atomes de carbone; M et W sont un radical carbonyle ou thiocarbonyle; n est un nombre de 1 à 3, ou un sel pharmaceutiquement acceptable de celui-ci.

**4.** Procédé suivant la revendication 1, dans lequel le composé préparé a la formule (III) :

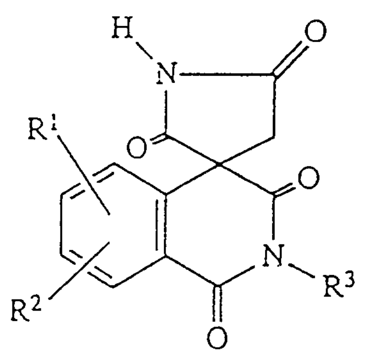

(III)

dans laquelle $R^1$ et $R^2$ sont un atome d'hydrogène ou d'halogène; $R^3$ est un radical alcoyle inférieur contenant 1 à 6 atomes de carbone, un radical benzyle ou un radical benzyle dihalogéno-substitué, ou un sel pharmaceutiquement acceptable de celui-ci.

**5.** Procédé suivant la revendication 1, dans lequel le composé de formule (I) préparé est :

la 2-[(4-bromo-2-fluorophényl)méthyl]spiro[isoquinoléine-4(1H),3'-pyrrolidine]-1,2',3,5'(2H)-tétrone;

la [2-[(4-bromo-2-fluorophényl)méthyl]-7-chloro]spiro[isoquinoléine-4(1H),3'-pyrrolidine]-1,2',3,5'-(2H)-tétrone;

la [2-[(4-bromo-2-fluorophényl)méthyl]-6-chloro]spiro[isoquinoléine-4(1H),3'-pyrrolidine]-1,2',3,5'-(2H)-tétrone;

la 2-[(3,4-dichlorophényl)méthyl]spiro[isoquinoléine-4(1H),3'-pyrrolidine]-1,2',3,5'(2H)-tétrone;

la [2-[(4-bromo-2-fluorophényl)méthyl]-5-fluoro]spiro[isoquinoléine-4(1H),3'-pyrrolidine]-1,2',3,5'(2H)-tétrone;

la [2-[(4-bromo-2-fluorophényl)méthyl]-6-fluoro]spiro[isoquinoléine-4(1H),3'-pyrrolidine]-1,2',3,5'(2H)-tétrone;

la [2-[(4-bromo-2-fluorophényl)méthyl]-7-fluoro]spiro[isoquinoléine-4(1H),3'-pyrrolidine]-1,2',3,5'(2H)-tétrone;
la [2-[(4-bromo-2-fluorophényl)méthyl]-8-fluoro]spiro[isoquinoléine-4(1H),3'-pyrrolidine]-1,2',3,5'(2H)-tétrone;
la [6-bromo-2[(4-bromo-2-fluorophényl)méthyl]-7-méthoxy]spiro[isoquinoléine-4(1H),3'-pyrrolidine]-1,2',3,5'(2H)-tétrone;
la [2-[(4-bromo-2-fluorophényl)méthyl]-7-méthoxy]spiro[isoquinoléine-4(1H),3'-pyrrolidine]-1,2',3,5'-(2H)-tétrone;
la (R)-(+)-2-[(4-bromo-2-fluorophényl)méthyl]spiro[isoquinoléine-4(1H),3'-pyrrolidine]-1,2',3,5'(2H)-tétrone;
la (S)-(-)-2-[(4-bromo-2-fluorophényl)méthyl]spiro[isoquinoléine-4(1H),3'-pyrrolidine]-1,2',3,5'(2H)-tétrone;
la (R)-(+)-[2-[(4-bromo-2-fluorophényl)méthyl]-6-fluoro]spiro[isoquinoléine-4(1H),3'-pyrrolidine]-1,2',3,5'(2H)-tétrone;
la (S)-(-)-[2-[(4-bromo-2-fluorophényl)méthyl]-6-fluoro]spiro[isoquinoléine-4(1H),3'-pyrrolidine]-1,2',3,5'(2H)-tétrone;
la 2-méthylspiro[isoquinoléine-4(1H),3'-pyrrolidine]-1,2',3,5'(2H)-tétrone;
la 6-chloro-2-méthylspiro[isoquinoléine-4(1H),3'-pyrrolidine]-1,2',3,5'(2H)-tétrone;
la 6-bromo-2-méthylspiro[isoquinoléine-4(1H),3'-pyrrolidine]-1,2',3,5'(2H)-tétrone;
la 7-chloro-2-méthylspiro[isoquinoléine-4(1H),3'-pyrrolidine]-1,2',3,5'(2H)-tétrone;
la 2-éthylspiro[isoquinoléine-4(1H),3'-pyrrolidine]-1,2',3,5'(2H)-tétrone;
la 2-propylspiro[isoquinoléine-4(1H),3'-pyrrolidine]-1,2',3,5'(2H)-tétrone;
la 2-butylspiro[isoquinoléine-4(1H),3'-pyrrolidine]-1,2',3,5'(2H)-tétrone;
ou un sel pharmaceutiquement acceptable de celui-ci.

**6.** Procédé de préparation d'un composé de formule (XI) :

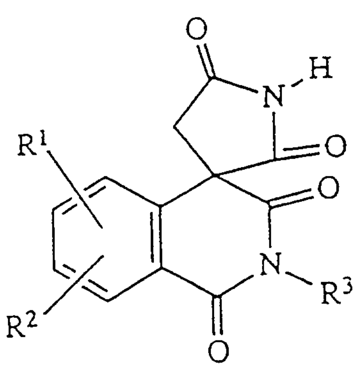

(XI)

dans laquelle $R^1$ et $R^2$ sont des atomes d'hydrogène ou d'halogène; $R^3$ est un radical alcoyle contenant 1 à 6 atomes de carbone, un radical (alcoyle inférieur)aryle ou un radical (alcoyle inférieur)aryle dihalogéno-substitué où le radical aryle contient 6 à 10 atomes de carbone et le radical alcoyle inférieur contient 1 à 6 atomes de carbone, qui comprend :

a) la réaction d'un composé de formule (VI) :

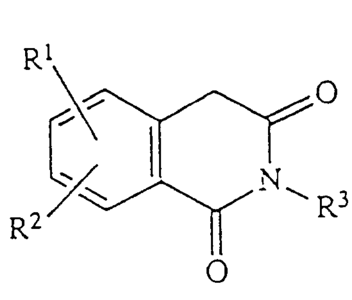

(VI)

dans laquelle $R^1$, $R^2$ et $R^3$ sont tels que définis ci-dessus, avec une base dans un solvant classique et l'addition subséquente d'un agent réactif carbométhoxylant pour produire le composé de formule

(VII) :

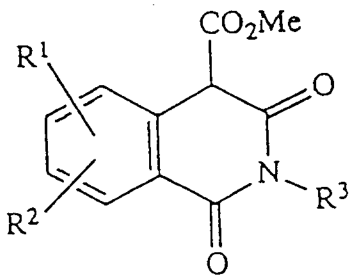

(VII)

dans laquelle $R^1$, $R^2$ et $R^3$ sont tels que définis ci-dessus;
b) la réaction dudit composé de formule (VII) avec une base inorganique dans un solvant classique et l'addition subséquente de bromacétate de t-butyle pour produire le composé de formule (VIII) :

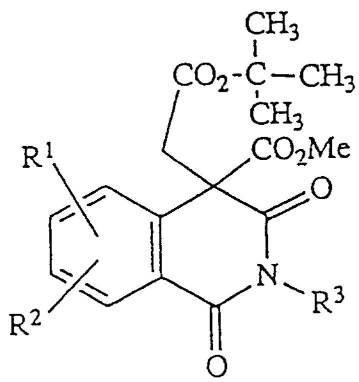

(VIII)

dans laquelle $R^1$, $R^2$ et $R^3$ sont tels que définis ci-dessus;
c) l'hydrolyse dudit composé de formule (VIII) avec un acide organique dans un solvant classique pour produire le composé de formule (IX) :

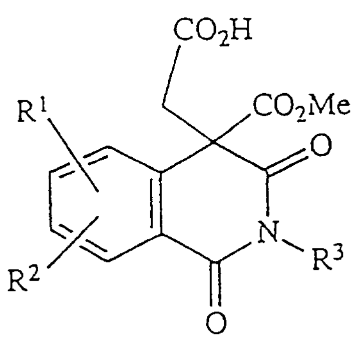

(IX)

dans laquelle $R^1$, $R^2$ et $R^3$ sont tels que définis ci-dessus;
d) la réaction dudit composé de formule (IX) avec un agent de couplage et l'addition subséquente d'une solution d'ammonium dans du tétrahydrofuranne pour produire le composé de formule (X) :

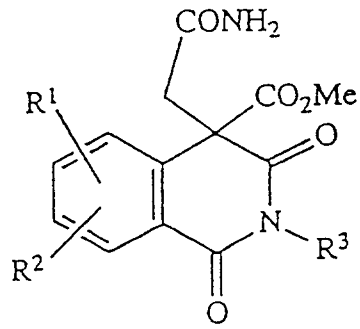

(X)

dans laquelle $R^1$, $R^2$ et $R^3$ sont tels que définis ci-dessus;

e) la réaction dudit composé de formule (X) avec une base dans un solvant classique pour produire le composé de formule (XI).

**7.** Procédé suivant la revendication 6, pour la production de composés de formule (XI) dans laquelle $R^1$ et $R^2$ sont tels que définis dans la revendication 6 et $R^3$ est un radical alcoyle contenant 1 à 6 atomes de carbone, dans laquelle le composé de formule (X), où $R^1$ et $R^2$ sont tels que définis ci-dessus, $R^3$ est un radical alcoyle tel que défini ci-dessus, est mis à réagir avec une base telle que le bis-(triméthylsilyl)amidure de lithium, dans un solvant classique pour produire le composé de formule (XI), où $R^1$ et $R^2$ sont tels que définis ci-dessus, $R^3$ est un radical alcoyle tel que défini ci-dessus.

**8.** Procédé de préparation d'un composé de formule (VII) :

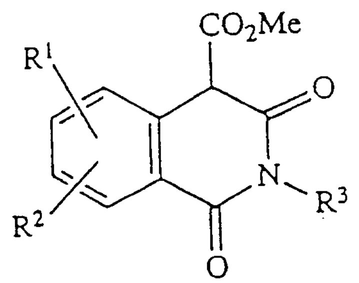

(VII)

dans laquelle $R^1$ et $R^2$ sont des atomes d'hydrogène ou d'halogène; $R^3$ est un radical alcoyle contenant 1 à 6 atomes de carbone, un radical (alcoyle inférieur)aryle ou (alcoyle inférieur)aryle dihalogéno-substitué où le radical aryle contient 6 à 10 atomes de carbone et le radical alcoyle inférieur contient 1 à 6 atomes de carbone qui comprend la réaction du composé de formule (XII) :

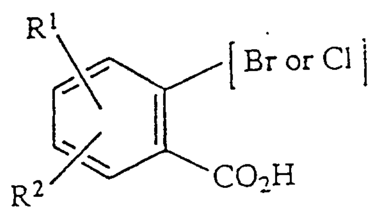

(XII)

dans laquelle $R^1$ et $R^2$ sont tels que définis ci-dessus avec le malonate de diméthyle et du NaH en présence d'une quantité catalytique de CuBr pour produire le composé de formule (XIII) :

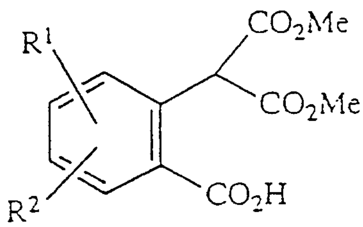

(XIII)

dans laquelle $R^1$ et $R^2$ sont tels que définis ci-dessus et la réaction dudit composé de formule (XIII) avec le chlorure de thionyle et l'addition subséquente d'un solvant inerte, de triéthylamine et d'une amine de formule $R^3NH_2$ pour produire le composé de formule (VII).

**9.** Procédé de préparation d'une composition pharmaceutique qui comprend la mise en association d'un composé de formule (I) ou d'un analogue ou d'un sel pharmaceutiquement acceptable de celui-ci tel que défini dans la revendication 1 avec un excipient pharmaceutiquement acceptable.

**10.** Procédé de préparation d'un composé de formule (XXIV) :

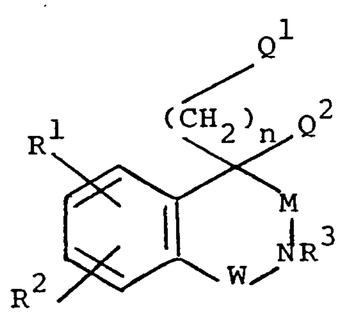

(XXIV)

dans laquelle un des $Q^1$ et $Q^2$ est $-CZNH_2$ ou -CN et l'autre des $Q^1$ et $Q^2$ est $-COOR^5$; n est un nombre de 1 à 3 et M, W, Z, $R^1$, $R^2$, $R^3$ et $-COOR^5$ sont tels que définis dans la revendication 1, qui comprend :

a) la réaction d'un acide de formule (XXV) :

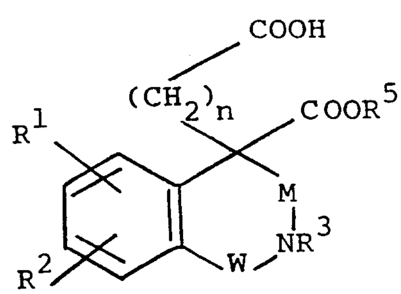

(XXV)

dans laquelle $R^1$, $R^2$, $R^3$, M, W et $-COOR^5$ sont tels que définis ci-dessus avec un agent de couplage et de l'ammoniac pour donner un composé correspondant de formule (I) dans laquelle $Q^1$ est $-CONH_2$; ou

b) la réaction d'un composé de formule (L) :

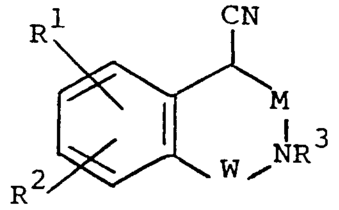

(L)

dans laquelle $R^1$, $R^2$, M, W et $R^3$ sont tels que définis dans la revendication 1 avec un composé de formule :

$Br(CH_2)_nCOOR^5$

dans laquelle n est un nombre de 1 à 3 et $-COOR^5$ est un radical ester pour donner un composé de formule (XXIV), où $Q^1$ est $-COOR^5$ et $Q^2$ est -CN;
c) la réaction d'un composé de formule (G) :

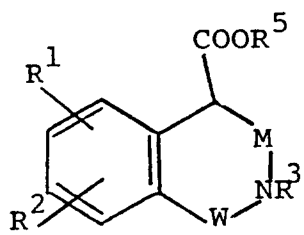

(G)

dans laquelle $R^1$, $R^2$, $R^3$, M, W et $-COOR^5$ sont tels que définis dans la revendication 1 avec un composé de formule :

$Br(CH_2)_nCN$

où n est tel que défini ci-dessus pour donner un composé de formule (XXIV), où $Q^1$ est -CN et $Q^2$ est $-COOR^5$; ou
d) la conversion d'un nitrile de formule (XXIV), où $Q^2$ est -CN en un amide ou un thioamide de formule (XXIV), où $Q^2$ est $-CONH_2$ ou $-CSNH_2$ de manière connue; ou
e) la conversion d'un nitrile de formule (XXIV) où $Q^1$ est -CN en un thioamide de formule (XXIV), où $Q^1$ est $-CSNH_2$ en utilisant $HSP(=S)(OEt)_2/HCl$.

**11.** Procédé suivant la revendication 10, dans lequel le produit est :
l'ester méthylique de l'acide 4-(2-amino-2-oxoéthyl)-2-[(3,4-dichlorophényl)méthyl]-1,2,3,4-tétrahydro-1,3-dioxo-4-isoquinoléinecarboxylique;
l'ester méthylique de l'acide 4-(2-amino-2-oxoéthyl)-2-[(4-bromo-2-fluorophényl)méthyl]-1,2,3,4-tétrahydro-1,3-dioxo-4-isoquinoléinecarboxylique;
l'ester méthylique de l'acide 4-(2-amino-2-oxoéthyl)-2-[(4-bromo-2-fluorophényl)méthyl]-7-chloro-1,2,3,4-tétrahydro-1,3-dioxo-4-isoquinoléinecarboxylique;
l'ester méthylique de l'acide 4-(2-amino-2-oxoéthyl)-2-[(4-bromo-2-fluorophényl)méthyl]-6-chloro-1,2,3,4-tétrahydro-1,3-dioxo-4-isoquinoléinecarboxylique;
l'ester méthylique de l'acide 4-(2-amino-2-oxoéthyl)-2-[(4-bromo-2-fluorophényl)méthyl]-6-fluoro-1,2,3,4-tétrahydro-1,3-dioxo-4-isoquinoléinecarboxylique;
l'ester méthylique de l'acide 4-(2-amino-2-oxoéthyl)-1,2,3,4-tétrahydro-2-méthyl-1,3-dioxo-4-isoquinoléinecarboxylique;
l'ester méthylique de l'acide 4-(2-amino-2-oxoéthyl)-7-chloro-1,2,3,4-tétrahydro-2-méthyl-1,3-dioxo-4-isoquinoléinecarboxylique;
l'ester méthylique de 1'acide 4-(2-amino-2-oxoéthyl)-6-chloro-1,2,3,4-tétrahydro-2-méthyl-1,3-dioxo-4-isoquinoléinecarboxylique;

l'ester méthylique de l'acide 4-(2-amino-2-oxoéthyl)-2-éthyl-1,2,3,4-tétrahydro-1,3-dioxo-4-isoquinoléinecarboxylique;

l'ester méthylique de l'acide 4-(2-amino-2-oxoéthyl)-1,2,3,4-tétrahydro-2-propyl-1,3-dioxo-4-isoquinoléinecarboxylique;

l'ester méthylique de l'acide 4-(2-amino-2-oxoéthyl)-2-butyl-1,2,3,4-tétrahydro-1,3-dioxo-4-isoquinoléinecarboxylique;

l'ester méthylique de l'acide 4-(2-amino-2-oxoéthyl)-6-bromo-1,2,3,4-tétrahydro-2-méthyl-1,3-dioxo-4-isoquinoléinecarboxylique;

l'ester menthylique de l'acide 4-(2-amino-2-oxoéthyl)-2-[(4-bromo-2-fluorophényl)méthyl]-1,2,3,4-tétrahydro-1,3-dioxo-4-isoquinoléinecarboxylique;

l'ester menthylique de l'acide 4-(2-amino-2-oxoéthyl)-2-[(4-bromo-2-fluorophényl)méthyl]-6-fluoro-1,2,3,4-tétrahydro-1,3-dioxo-4-isoquinoléinecarboxylique.

**Revendications pour l'Etat contractant suivant : GR**

**1.** Procédé de préparation d'un composé de formule (I) :

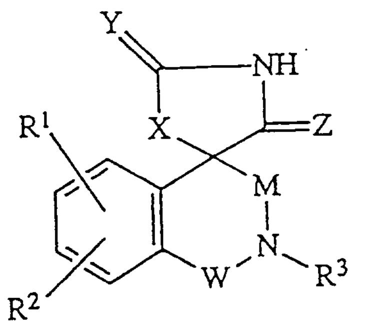

(I)

et les analogues de celui-ci où le cycle benzène condensé est remplacé par un cycle thiophène, pyridine ou furanne condensé, dans laquelle $R^1$ et $R^2$ sont indépendamment un atome d'hydrogène, un radical alcoyle contenant 1 à 6 atomes de carbone, un atome d'halogène, un radical alcoxy inférieur contenant 1 à 6 atomes de carbone, un radical trifluorométhyle, un radical alcoylthio inférieur où la partie alcoyle inférieur contient 1 à 6 atomes de carbone, un radical dialcoylamino où la partie alcoyle contient 1 à 6 atomes de carbone, un radical nitro, un radical aryle ou (alcoyle inférieur)aryloxy où la partie aryle contient 6 à 10 atomes de carbone et la partie alcoyle inférieur contient 1 à 6 atomes de carbone; $R^3$ est un atome d'hydrogène, un radical alcoyle inférieur contenant 1 à 6 atomes de carbone, un radical (alcoyle inférieur)aryle ou un radical (alcoyle inférieur)aryle substitué par un halogène où la partie aryle contient 6 à 10 atomes de carbone et l'alcoyle inférieur contient 1 à 6 atomes de carbone ou (alcoyle inférieur)aryle hétérocyclique de formule de structure :

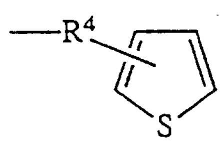

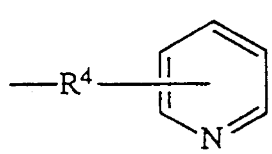

dans laquelle $R^4$ est un radical alcoylène inférieur contenant 1 à 3 atomes de carbone; X est un radical alcoylène inférieur contenant 1 à 3 atomes de carbone, un atome d'oxygène, de soufre ou un radical -NH-; Y et Z sont indépendamment un atome d'oxygène ou de soufre; M et W sont indépendamment un radical carbonyle, un radical thiocarbonyle, un radical sulfonyle, un radical sulfoxo ou un radical alcoylène inférieur contenant 1 à 2 atomes de carbone, avec la condition (i) que M et W ne peuvent pas être ensemble alcoylène inférieur et (ii) que quand M est $-CH_2-$, W est autre que sulfonyle ou un sel pharmaceutiquement acceptable de celui-ci; qui comprend une des étapes suivantes :

a) la cyclisation d'un amide de formule (A) :

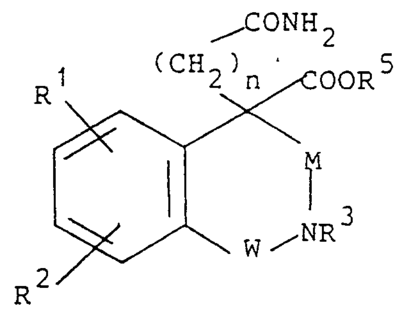

(A)

dans laquelle $R^1$, $R^2$, $R^3$, n, M et W sont tels que définis ci-dessus et $-COOR^5$ est un radical ester, en utilisant une base adéquate pour donner un composé de formule (I) dans laquelle Y et Z sont tous deux un atome d'oxygène et X est un radical $-(CH_2)_n-$ dans lequel n est un nombre de 1 à 3;

b) la réaction d'un composé de formule (B) :

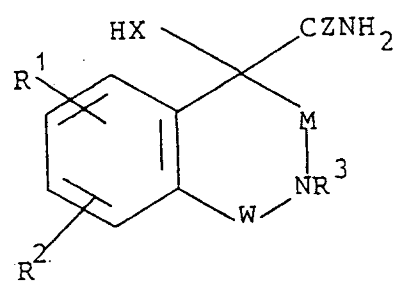

(B)

dans laquelle $R^1$, $R^2$, $R^3$, M et W sont tels que définis ci-dessus et X est -O-, -S- ou -NH- et Z est -O- ou -S-, avec $CYCl_2$, où Y est -O- ou -S- pour donner un composé de formule (I) dans laquelle X est -O-, -S- ou -NH- et Y et Z indépendamment représentent -O- ou -S-; ou

c) la réaction d'un composé de formule (C) :

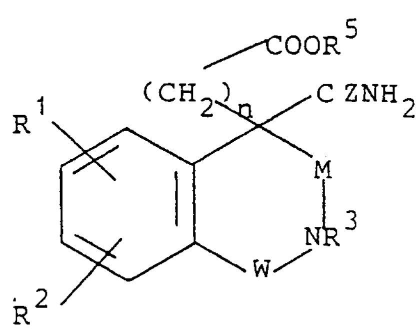

(C)

dans laquelle $R^1$, $R^2$, $R^3$, M, W, n, Z et $-COOR^5$ sont tels que définis ci-dessus en utilisant une base adéquate pour donner un composé de formule (I), où Y est -O-, Z est -O- ou -S- et n est un nombre de 1 à 3; ou

d) la réaction d'un composé de formule (D) :

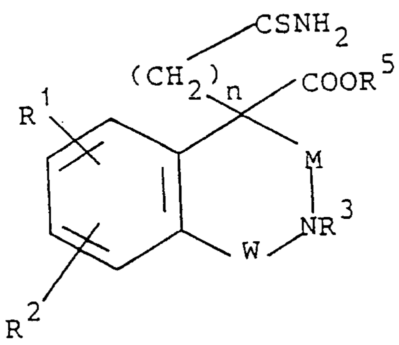

(D)

dans laquelle $R^1$, $R^2$, $R^3$, M, W, n et $-COOR^5$ sont tels que définis ci-dessus avec une base forte pour donner un composé de formule (I) dans laquelle X est un radical $-(CH_2)_n-$ où n est un nombre de 1 à 3 et Y est -S- et Z est -O-; ou

e) la réaction d'un composé de formule (F) :

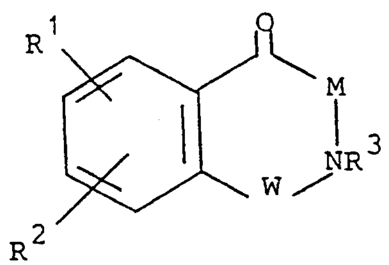

(F)

dans laquelle $R^1$, $R^2$, $R^3$, M et W sont tels que définis ci-dessus avec du carbonate d'ammonium et un cyanure de métal alcalin pour donner un composé de formule (I) dans laquelle X est un radical -NH- et Y et Z sont tous deux oxygène; ou

f) la réaction d'un composé de formule (F) successivement avec du cyanure d'hydrogène, du chlorure de thionyle et de la thiourée pour donner un composé de formule (I) dans laquelle X est -S- et Y et Z sont tous deux oxygène; ou

g) la réaction d'un composé de formule (F) avec le cyanure de triméthylsilyle, puis le HCl/alcanol et le phosgène ou le thiophosgène pour donner un composé de formule (I) dans laquelle X est -O- ou -S- et Y et Z sont tous deux oxygène.

**2.** Procédé suivant la revendication 1, dans lequel le composé préparé a la formule (II) :

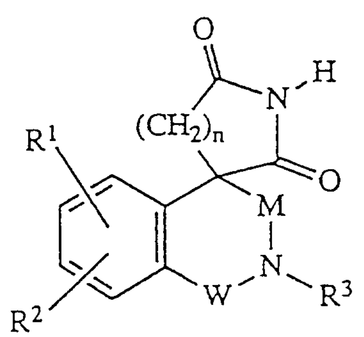

(II)

dans laquelle $R^1$ et $R^2$ sont indépendamment un atome d'hydrogène, un radical alcoyle contenant 1 à 6 atomes de carbone, un atome d'halogène, un radical alcoxy inférieur contenant 1 à 6 atomes de carbone, un radical trifluorométhyle, un radical alcoylthio inférieur où la partie alcoyle inférieur contient 1 à 6 atomes de carbone; $R^3$ est un atome d'hydrogène, un radical alcoyle inférieur contenant 1 à 6

atomes de carbone; M et W sont un radical carbonyle ou thiocarbonyle; n est un nombre de 1 à 3 ou un sel pharmaceutiquement acceptable de celui-ci.

3. Procédé suivant la revendication 1, dans lequel le composé préparé a la formule (II) :

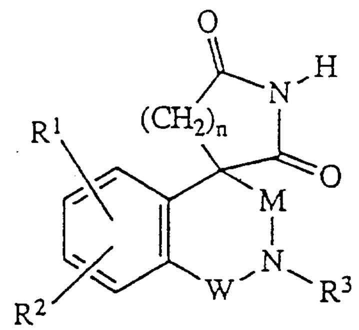

(II)

dans laquelle $R^1$ et $R^2$ sont indépendamment un atome d'hydrogène, un radical alcoyle contenant 1 à 6 atomes de carbone, un atome d'halogène, un radical alcoxy inférieur contenant 1 à 6 atomes de carbone, un radical trifluorométhyle, un radical alcoylthio inférieur où la partie alcoyle inférieur contient 1 à 6 atomes de carbone; $R^3$ est un radical (alcoyle inférieur)aryle ou (alcoyle inférieur)aryle dihalogéno-substitué où la partie aryle contient 6 à 10 atomes de carbone et la partie alcoyle inférieur contient 1 à 6 atomes de carbone; M et W sont un radical carbonyle ou thiocarbonyle; n est un nombre de 1 à 3 ou un sel pharmaceutiquement acceptable de celui-ci.

4. Procédé suivant la revendication 1, dans lequel le composé préparé a la formule (III) :

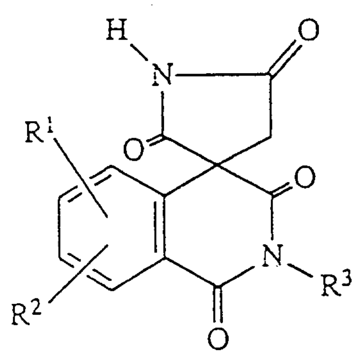

(III)

dans laquelle $R^1$ et $R^2$ sont un atome d'hydrogène ou d'halogène; $R^3$ est un radical alcoyle inférieur contenant 1 à 6 atomes de carbone, un radical benzyle ou un radical benzyle dihalogéno-substitué, ou un sel pharmaceutiquement acceptable de celui-ci.

5. Procédé suivant la revendication 1, dans lequel le composé de formule (I) préparé est :
la 2-[(4-bromo-2-fluorophényl)méthyl]spiro[isoquinoléine-4(1H),3'-pyrrolidine]-1,2',3,5'(2H)-tétrone;
la [2-[(4-bromo-2-fluorophényl)méthyl]-7-chloro]spiro[isoquinoléine-4(1H),3'-pyrrolidine]-1,2',3,5'-(2H)-tétrone;
la [2-[(4-bromo-2-fluorophényl)méthyl]-6-chloro]spiro[isoquinoléine-4(1H),3'-pyrrolidine]-1,2',3,5'-(2H)-tétrone;
la 2-[(3,4-dichlorophényl)méthyl]spiro[isoquinoléine-4(1H),3'-pyrrolidine]-1,2',3,5'(2H)-tétrone;
la [2-[(4-bromo-2-fluorophényl)méthyl]-5-fluoro]spiro[isoquinoléine-4(1H),3'-pyrrolidine]-1,2',3,5'(2H)-tétrone;
la [2-[(4-bromo-2-fluorophényl)méthyl]-6-fluoro]spiro[isoquinoléine-4(1H),3'-pyrrolidine]-1,2',3,5'(2H)-tétrone;
la [2-[(4-bromo-2-fluorophényl)méthyl]-7-fluoro]spiro[isoquinoléine-4(1H),3'-pyrrolidine]-1,2',3,5'(2H)-tétrone;
la [2-[(4-bromo-2-fluoropnényl)méthyl]-8-fluoro]spiro[isoquinoléine-4(1H),3'-pyrrolidine]-1,2',3,5'(2H)-tétrone;

la [6-bromo-2-[(4-bromo-2-fluorophényl)méthyl]-7-méthoxy]spiro[isoquinoléine-4(1H),3'-pyrrolidine]-1,2',3,5'(2H)-tétrone;

la [2-[(4-bromo-2-fluorophényl)méthyl]-7-méthoxy]spiro[isoquinoléine-4(1H),3'-pyrrolidine]-1,2',3,5'-(2H)-tétrone;

la (R)-(+)-2-[(4-bromo-2-fluorophényl)méthyl]spiro[isoquinoléine-4(1H),3'-pyrrolidine]-1,2',3,5'(2H)-tétrone;

la (S)-(-)-2-[(4-bromo-2-fluorophényl)méthyl]spiro[isoquinoléine-4(1H),3'-pyrrolidine]-1,2',3,5'(2H)-tétrone;

la (R)-(+)-[2-[(4-bromo-2-fluorophényl)méthyl]-6-fluoro]spiro[isoquinoléine-4(1H),3'-pyrrolidine]-1,2',3,5'(2H)-tétrone;

la (S)-(-)-[2-[(4-bromo-2-fluorophényl)méthyl]-6-fluoro]spiro[isoquinoléine-4(1H),3'-pyrrolidine]-1,2',3,5'(2H)-tétrone;

la 2-méthylspiro[isoquinoléine-4(1H),3'-pyrrolidine]-1,2',3,5'(2H)-tétrone;

la 6-chloro-2-méthylspiro[isoquinoléine-4(1H),3'-pyrrolidine]-1,2',3,5'(2H)-tétrone;

la 6-bromo-2-méthylspiro[isoquinoléine-4(1H),3'-pyrrolidine]-1,2',3,5'(2H)-tétrone;

la 7-chloro-2-méthylspiro[isoquinoléine-4(1H),3'-pyrrolidine]-1,2',3,5'(2H)-tétrone;

la 2-éthylspiro[isoquinoléine-4(1H),3'-pyrrolidine]-1,2',3,5'(2H)-tétrone;

la 2-propylspiro[isoquinoléine-4(1H),3'-pyrrolidine]-1,2',3,5'(2H)-tétrone;

la 2-butylspiro[isoquinoléine-4(1H),3'-pyrrolidine]-1,2',3,5'(2H)-tétrone;

ou un sel pharmaceutiquement acceptable de celui-ci.

**6.** Procédé de préparation d'un composé de formule (XI) :

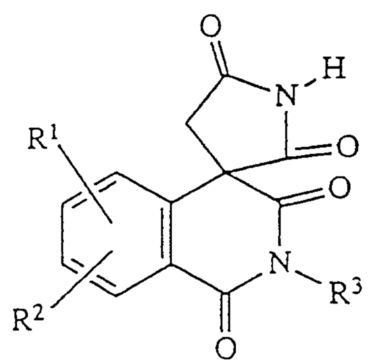

(XI)

dans laquelle $R^1$ et $R^2$ sont des atomes d'hydrogène ou d'halogène; $R^3$ est un radical alcoyle contenant 1 à 6 atomes de carbone, un radical (alcoyle inférieur)aryle ou un radical (alcoyle inférieur)aryle dihalogéno-substitué où le radical aryle contient 6 à 10 atomes de carbone et le radical alcoyle inférieur contient 1 à 6 atomes de carbone, qui comprend :

a) la réaction d'un composé de formule (VI) :

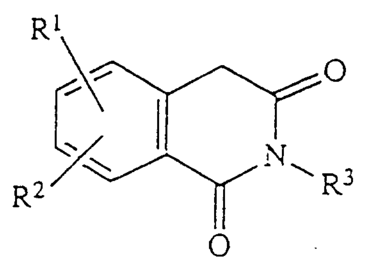

(VI)

dans laquelle $R^1$, $R^2$ et $R^3$ sont tels que définis ci-dessus, avec une base dans un solvant classique et l'addition subséquente d'un agent réactif carbométhoxylant pour produire le composé de formule (VII) :

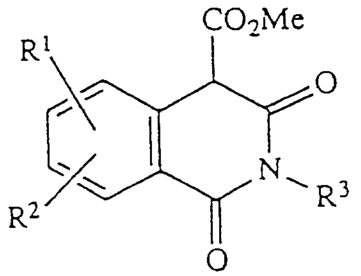

(VII)

dans laquelle $R^1$, $R^2$ et $R^3$ sont tels que définis ci-dessus;
b) la réaction dudit composé de formule (VII) avec une base inorganique dans un solvant classique et l'addition subséquente de bromacétate de t-butyle pour produire le composé de formule (VIII) :

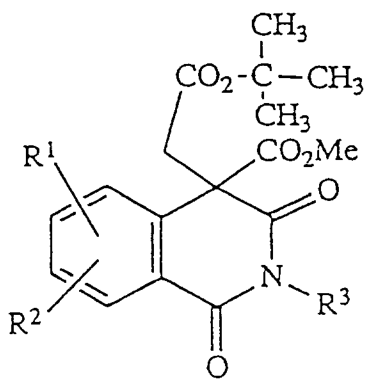

(VIII)

dans laquelle $R^1$, $R^2$ et $R^3$ sont tels que définis ci-dessus;
c) l'hydrolyse dudit composé de formule (VIII) avec un acide organique dans un solvant classique pour produire le composé de formule (IX) :

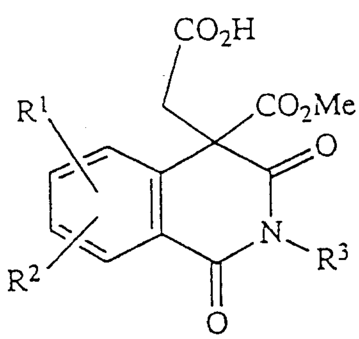

(IX)

dans laquelle $R^1$, $R^2$ et $R^3$ sont tels que définis ci-dessus;
d) la réaction dudit composé de formule (IX) avec un agent de couplage dans un solvant classique et l'addition subséquente d'une solution d'ammonium dans du tétrahydrofuranne pour produire le composé de formule (X) :

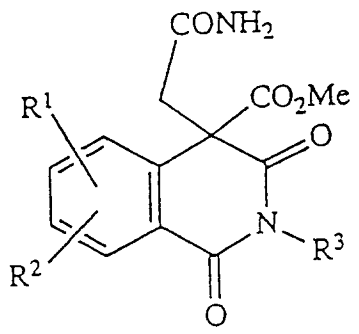

(X)

dans laquelle $R^1$, $R^2$ et $R^3$ sont tels que définis ci-dessus;
e) la réaction dudit composé de formule (X) avec une base dans un solvant classique pour produire le composé de formule (XI).

**7.** Procédé suivant la revendication 6, pour la production de composés de formule (XI) dans laquelle $R^1$ et $R^2$ sont tels que définis dans la revendication 6 et $R^3$ est un radical alcoyle contenant 1 à 6 atomes de carbone, dans laquelle le composé de formule (X), où $R^1$ et $R^2$ sont tels que définis ci-dessus, $R^3$ est un radical alcoyle tel que défini ci-dessus, est mis à réagir avec une base telle que le bis-(triméthylsilyl)amidure de lithium, dans un solvant classique pour produire le composé de formule (XI), où $R^1$ et $R^2$ sont tels que définis ci-dessus, $R^3$ est un radical alcoyle tel que défini ci-dessus.

**8.** Procédé de préparation d'un composé de formule (VII) :

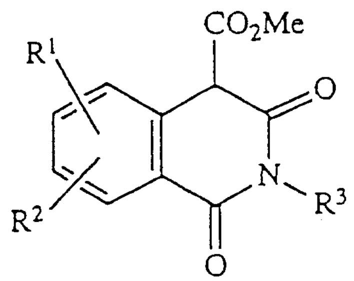

(VII)

dans laquelle $R^1$ et $R^2$ sont des atomes d'hydrogène ou d'halogène; $R^3$ est un radical alcoyle contenant 1 à 6 atomes de carbone, un radical (alcoyle inférieur)aryle ou (alcoyle inférieur)aryle dihalogéno-substitué où le radical aryle contient 6 à 10 atomes de carbone et le radical alcoyle inférieur contient 1 à 6 atomes de carbone qui comprend la réaction du composé de formule (XII) :

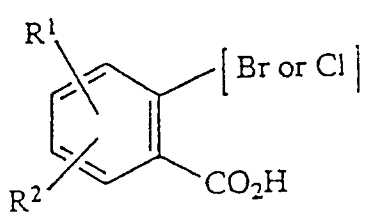

(XII)

dans laquelle $R^1$ et $R^2$ sont tels que définis ci-dessus avec le malonate de diméthyle et du NaH en présence d'une quantité catalytique de CuBr pour produire le composé de formule (XIII) :

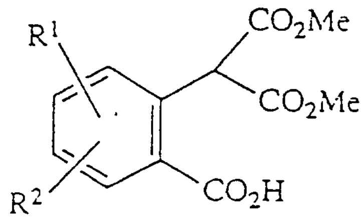

(XIII)

dans laquelle $R^1$ et $R^2$ sont tels que définis ci-dessus et la réaction dudit composé de formule (XIII) avec du chlorure de thionyle et l'addition subséquente d'un solvant inerte, de triéthylamine et d'une amine de formule $R^3NH_2$ pour produire le composé de formule (VII).

**9.** Procédé de préparation d'une composition pharmaceutique qui comprend la mise en association d'un composé de formule (I) ou d'un analogue ou d'un sel pharmaceutiquement acceptable de celui-ci tel que défini dans la revendication 1 avec un excipient pharmaceutiquement acceptable.

**10.** Procédé de préparation d'un composé de formule (XXIV) :

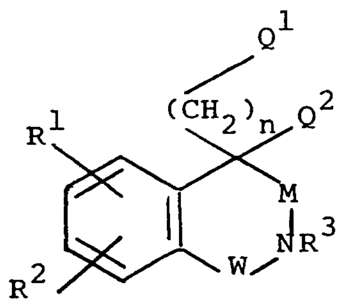

(XXIV)

dans laquelle un des $Q^1$ et $Q^2$ est $-CZNH_2$ ou -CN et l'autre des $Q^1$ et $Q^2$ est $-COOR^5$ ; n est un nombre de 1 à 3 et M, W, Z, $R^1$, $R^2$, $R^3$ et $-COOR^5$ sont tels que définis dans la revendication 1, qui comprend :

a) la réaction d'un acide de formule (XXV) :

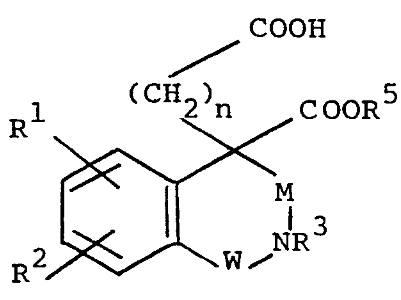

(XXV)

dans laquelle $R^1$, $R^2$, $R^3$, M, W et $-COOR^5$ sont tels que définis ci-dessus avec un agent de couplage et de l'ammoniac pour donner un composé correspondant de formule (I) dans laquelle $Q^1$ est $-CONH_2$ ; ou

b) la réaction d'un composé de formule (L) :

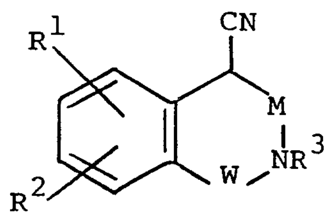

(L)

dans laquelle $R^1$, $R^2$, M, W et $R^3$ sont tels que définis dans la revendication 1 avec un composé de formule :

$Br(CH_2)_nCOOR^5$

dans laquelle n est un nombre de 1 à 3 et $-COOR^5$ est un radical ester pour donner un composé de formule (XXIV) où $Q^1$ est $-COOR^5$ et $Q^2$ est -CN;
c) la réaction d'un composé de formule (G) :

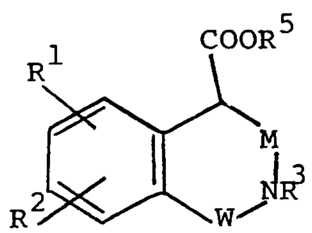

(G)

dans laquelle $R^1$, $R^2$, $R^3$, M, W et $-COOR^5$ sont tels que définis dans la revendication 1 avec un composé de formule :

$Br(CH_2)_nCN$

où n est tel que défini ci-dessus pour donner un composé de formule (XXIV) où $Q^1$ est -CN et $Q^2$ est $-COOR^5$; ou
d) la conversion d'un nitrile de formule (XXIV) où $Q^2$ est -CN en un amide ou un thioamide de formule (XXIV) où $Q^2$ est $-CONH_2$ ou $-CSNH_2$ de manière connue; ou
e) la conversion d'un nitrile de formule (XXIV) où $Q^1$ est -CN en un thioamide de formule (XXIV) où $Q^1$ est $-CSNH_2$ en utilisant $HSP(=S)(OEt)_2/HCl$.

**11.** Composé de formule (XXIV) :

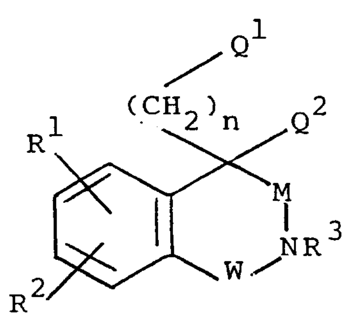

(XXIV)

dans laquelle un des $Q^1$ et $Q^2$ est $-CZNH_2$ ou -CN; l'autre des $Q^1$ et $Q^2$ est $-COOR^5$; Z, $R^1$, $R^2$, $R^3$, M et w sont tels que définis dans la revendication 1, $-COOR^5$ est un radical ester et n est un nombre de 1 à 3.

**12.** Composé suivant la revendication 11, où $Q^1$ est $-CONH_2$, $R^1$ et $R^2$ sont des atomes d'hydrogène ou d'halogène; n est 1; M et W sont tous deux un radical carbonyle et $R^3$ est un radical alcoyle inférieur contenant 1 à 6 atomes de carbone, un radical benzyle ou un radical benzyle dihalogéno-substitué.

**13.** Composé suivant la revendication 11, qui est :

l'ester méthylique de l'acide 4-(2-amino-2-oxoéthyl)-2-[(3,4-dichlorophényl)méthyl]-1,2,3,4-tétrahydro-1,3-dioxo-4-isoquinoléinecarboxylique;

l'ester méthylique de l'acide 4-(2-amino-2-oxoéthyl)-2-[(4-bromo-2-fluorophényl)méthyl]-1,2,3,4-tétrahydro-1,3-dioxo-4-isoquinoléinecarboxylique;

l'ester méthylique de l'acide 4-(2-amino-2-oxoéthyl)-2-[(4-bromo-2-fluorophényl)méthyl]-7-chloro-1,2,3,4-tétrahydro-1,3-dioxo-4-isoquinoléinecarboxylique;

l'ester méthylique de l'acide 4-(2-amino-2-oxoéthyl)-2-[(4-bromo-2-fluorophényl)méthyl]-6-chloro-1,2,3,4-tétrahydro-1,3-dioxo-4-isoquinoléinecarboxylique;

l'ester méthylique de l'acide 4-(2-amino-2-oxoéthyl)-2-[(4-bromo-2-fluorophényl)méthyl]-6-fluoro-1,2,3,4-tétrahydro-1,3-dioxo-4-isoquinoléinecarboxylique;

l'ester méthylique de l'acide 4-(2-amino-2-oxoéthyl)-1,2,3,4-tétrahydro-2-méthyl-1,3-dioxo-4-isoquinoléinecarboxylique;

l'ester méthylique de l'acide 4-(2-amino-2-oxoéthyl)-7-chloro-1,2,3,4-tétrahydro-2-méthyl-1,3-dioxo-4-isoquinoléinecarboxylique;

l'ester méthylique de l'acide 4-(2-amino-2-oxoéthyl)-6-chloro-1,2,3,4-tétrahydro-2-méthyl-1,3-dioxo-4-isoquinoléinecarboxylique;

l'ester méthylique de l'acide 4-(2-amino-2-oxoéthyl)-2-éthyl-1,2,3,4-tétrahydro-1,3-dioxo-4-isoquinoléinecarboxylique;

l'ester méthylique de l'acide 4-(2-amino-2-oxoéthyl)-1,2,3,4-tétrahydro-2-propyl-1,3-dioxo-4-isoquinoléinecarboxylique;

l'ester méthylique de l'acide 4-(2-amino-2-oxoéthyl)-2-butyl-1,2,3,4-tétrahydro-1,3-dioxo-4-isoquinoléinecarboxylique;

l'ester méthylique de l'acide 4-(2-amino-2-oxoéthyl)-6-bromo-1,2,3,4-tétrahydro-2-méthyl-1,3-dioxo-4-isoquinoléinecarboxylique;

l'ester menthylique de l'acide 4-(2-amino-2-oxoéthyl)-2-[(4-bromo-2-fluorophényl)méthyl]-1,2,3,4-tétrahydro-1,3-dioxo-4-isoquinoléinecarboxylique;

l'ester menthylique de l'acide 4-(2-amino-2-oxoéthyl)-2-[(4-bromo-2-fluorophényl)méthyl]-6-fluoro-1,2,3,4-tétrahydro-1,3-dioxo-4-isoquinoléinecarboxylique.